# EUROPÄISCHE PATENTANMELDUNG

(11) **EP 3 395 171 A1**
(43) Veröffentlichungstag der Anmeldung: **31.10.2018**
(21) Anmeldenummer: 18168893.8
(22) Anmeldetag: 12.12.2012
(51) Int. Cl.: A01N 37/52, C07C 317/42, C07C 323/36, C07C 323/44, C07C 323/63

(54) **N-ARYLAMIDINE-SUBSTITUIERTE TRIFLUOROETHYLSULFID-DERIVATE ALS AKARIZIDE UND INSEKTIZIDE**

(30) Priorität: 21.12.2011 EP 11194855
(62) Teilanmeldung aus: 12806011.8
(71) Anmelder: Bayer CropScience Aktiengesellschaft, 40789 Monheim (DE)
(72) Erfinder: KÖHLER, Adeline, 40764 Langenfeld (DE); ALIG, Bernd, 53639 Königswinter (DE); BECKER, Angela, 06650 Opio (FR); VOERSTE, Arnd, 50674 Köln (DE); GÖRGENS, Ulrich, 40882 Ratingen (DE); FISCHER, Reiner, 40789 Monheim (DE); MORADI, Wahed Ahmed, 40789 Monheim (DE); CEREZO-GALVEZ, Silvia, 40764 Langenfeld (DE); HAHN, Julia Johanna, 40589 Düsseldorf (DE); ILG, Kerstin, 50670 Köln (DE); SCHWARZ, Hans-Georg, 46282 Dorsten (DE); GOMIBUCHI, Takuya, 305-0032 Ibaraki (JP); ITO, Masahito, Ibaraki, 300-1232 (JP); YAMAZAKI, Daiei, Yamaguchi, 747-0001 (JP); SHIBUYA, Katsuhiko, Tochigi, 329-0433 (JP); SHIMOJO, Eiichi, Osaka, 586-0018 (JP)
(74) Vertreter: BIP Patents

(57) **Zusammenfassung**

Die vorliegende Erfindung betrifft neue N-Arylamidine-substituierte Trifluoroethylsulfid-Derivate der Formel (I), 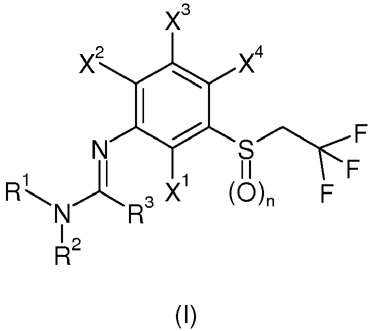 - in welcher X¹, X², X³, X⁴, R¹, R², R³, n die in der Beschreibung angegebenen Bedeutungen haben- deren Anwendung als Akarizide und Insektizide zur Bekämpfung tierischer Schädlinge und Verfahren sowie Zwischenprodukte zu ihrer Herstellung

## Beschreibung

Die vorliegende Erfindung betrifft neue N-Arylamidine-substituierte Trifluoroethylsulfid-Derivate, deren Anwendung als Akarizide und Insektizide zur Bekämpfung tierischer Schädlinge und Verfahren und Zwischenprodukte zu ihrer Herstellung.

Verschiedene substituierte N-Arylamidine und deren insektizide und akarizide Wirkung sind in der Literatur bereits beschrieben in WO 2007/131680.

Die gemäß den oben genannten Schriften bereits bekannten Wirkstoffe weisen bei ihrer Anwendung Nachteile auf, sei es, dass sie keine oder aber eine nur unzureichende insektizide und/oder akarizide Wirkung gegen tierische Schädlinge, insbesondere bei niedrigeren Aufwandmengen besitzen.

Aufgabe der vorliegenden Erfindung ist es daher, entsprechende N-Arylamidine-substituierte Trifluoroethylsulfid-Derivate bereitzustellen, welche als Insektizide und/oder Akarizide mit einer zufrieden stellenden insektiziden und/oder akariziden Wirkung gegen tierische Schädlinge, insbesondere bei niederigeren Aufwandmengen, mit einer hohen Selektivität und verbesserten Verträglichkeit in Nutzpflanzenkulturen eingesetzt werden können.

Es wurden nun neue Verbindungen der Formel (I) 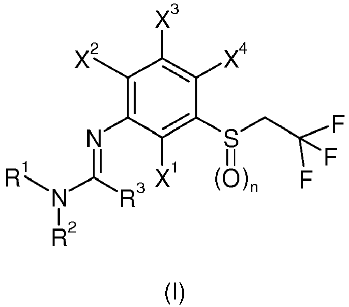 gefunden,
in welcher
n für die Zahl 0, 1 oder 2 steht,
X¹, X², X³, X⁴ unabhängig von einander für Wasserstoff, Halogen, Hydroxy, Amino, OCN, SCN, SF₅, Trialkylsilyl,Alkyl, Halogenalkyl, Cyanoalkyl, Hydroxyalkyl, Alkoxycarbonylalkyl, Alkoxyalkyl, Alkenyl, Halogenalkenyl, Cyanoalkenyl, Alkinyl, Halogenalkinyl, Cyanoalkinyl, Alkoxy, Halogenalkoxy, Cyanoalkoxy, Hydroxycarbonylalkoxy, Alkoxycarbonylalkoxy, Alkoxyalkoxy, Alkylhydroxyimino, Alkoxyimino, Alkylalkoxyimino, Halogenalkylalkoxyimino, Alkylthio, Halogenalkylthio, Alkoxyalkylthio, Alkylthioalkyl, Alkylsulfinyl, Halogenalkylsulfinyl, Alkoxyalkylsulfinyl, Alkylsulfinylalkyl, Alkylsulfonyl, Halogenalkylsulfonyl, Alkoxyalkylsulfonyl, Alkylsulfonylalkyl, Alkylsulfonyloxy,Alkylcarbonyl, Halogenalkylcarbonyl, Carboxyl, Alkylcarbonyloxy, Alkoxycarbonyl, Halogenalkoxycarbonyl, Alkoxycarbonylalkyl, Aminocarbonyl, Alkylaminocarbonyl, Dialkylaminocarbonyl, Alkenylaminocarbonyl, Dialkenylaminocarbonyl, Cycloalkylaminocarbonyl, Alkylsulfonylamino, Aminosulfonyl, Alkylaminosulfonyl, Dialkylaminosulfonyl, Alkylsulfoximino, Aminothiocarbonyl, Alkylaminothiocarbonyl, Dialkylaminothiocarbonyl,
oder für gegebenenfalls substituiertes Phenylalkyl, Phenoxy, Phenylalkyloxy, Phenoxyalkyl, Phenylthio, Phenylthioalkyl, Phenylsulfinyl, Phenylsulfonyl, Hetarylalkyl, Hetaryloxy, Hetarylalkyloxy Hetarylthio, Hetarylsulfinyl, Hetarylsulfonyl,
oder für gegebenenfalls substituiertes gesättigtes oder ungesättigtes Cycloalkylalkyl, Cycloalkyloxy, Cycloalkylalkoxy, Cycloalkylthio, Cycloalkylalkylthio, Cycloalkylsulfinyl, Cycloalkylalkylsulfinyl, Cycloalkylsulfonyl, Cycloalkylalkylsulfonyl,
oder für NR⁴R⁵, wobei R⁴ und R⁵ unabhängig voneinander für Wasserstoff, Cyano, Alkyl, Halogenalkyl, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, Acyl, Alkoxycarbonyl stehen oder R⁴ und R⁵ gemeinsam mit dem N-Atom, an dem sie gebunden sind, einen gegebenenfalls substituierten und gegebenenfalls durch Heteroatome aus der Reihe O, S oder N unterbrochenen gesättigten oder ungesättigten fünf- bis achtgliedrigen Ring bilden können, stehen,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcarbonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes, gegebenenfalls durch Heteroatome aus der Reihe O, S oder N unterbrochenes Cycloalkyl, Cycloalkoxy, Cycloalkylalkyl oder Cycloalkylalkoxy steht,
oder, X² und X³, oder X³ und X⁴, bilden einen 5 oder 6-gliedrigen Ring mit den C-Atome an die sie gebunden sind aus, der gegebenenfalls subsitutiert ist und gegebenenfalls durch Heteroatome aus der Reihe O, S, N oder CO unterbrochen ist.
R³ für Wasserstoff, (C₂-C₈)Alkyl, Cyano, Halogenalkyl, Alkoxyalkyl, Cyanoalkyl, Alkylthioalkyl, Alkenyl, Halogenalkenyl, Alkinyl, Halogenalkinyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy steht,
R¹ und R² unabhängig voneinander für Wasserstoff, Cyano, Alkyl, Halogenalkyl, Alkoxy, Cyanoalkyl, Hydroxyalkyl, Alkoxyalkyl, Alkylthioalkyl, Alkenyl, Halogenalkenyl, Cyanoalkenyl, Alkinyl, Halogenalkinyl, Cyanoalkinyl, für gegebenenfalls substituierte Alkylcarbonyl, Alkoxycarbonyl, Arylcarbonyl, Hetarylcarbonyl, Aryloxycarbonyl, Hetaryloxycarbonyl, Alkylsulfinyl, Halogenalkylsulfinyl, Arylsulfinyl, Arylalkylsulfinyl, Hetarylsulfinyl, Hetarylalkylsulfinyl, Alkylsulfonyl, Halogenalkylsulfonyl, Arylsulfonyl, Arylalkylsulfonyl, Hetarylsulfonyl, Hetarylalkylsulfonyl stehen,
oder für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy stehen,
oder für -(CH₂)ₘ-R⁶, -O-(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, Alkyl, Halogenalkyl, Alkenyl, Halogenalkenyl, Alkinyl, Alkoxy, Halogenalkoxy, Alkylthio, Alkylsulfinyl, Alkylsulfonyl, Alkylsulfonyloxy, Halogenalkylthio, Halogenalkylsulfinyl, Halogenalkylsulfonyl, Alkylamino, Dialkylamino, Alkylcabonylamino, Alkoxycarbonyl, Alkylcarbonyl, Alkylcarbonyloxy, Aminocarbonyl, Alkylaminocarbonyl, Dialkylaminocarbonyl, Aminothiocarbonyl, Alkylaminothiocarbonyl, Dialkylaminothiocarbonyl, Cycloalkylamino, Alkylsulfonylamino, Aminosulfonyl, Alkylaminosulfonyl oder Dialkylaminosulfonyl, oder mit gegebenenfalls substituiertes gegebenenfalls durch Heteroatome aus der Reihe O, S oder N unterbrochenes Cycloalkyl, Cycloalkoxy, Cycloalkylalkyl oder Cycloalkylalkoxy stehen, wobei m für die Zahl 1, 2, 3 oder 4 steht, oder
R¹ und R² auch zusammen mit dem N-Atom, an das sie gebunden sind, einen gesättigten oder ungesättigten und gegebenenfalls substituierten 4- bis 8-gliedrigen Ring bilden können, der gegebenenfalls ein oder mehrere weitere Heteroatome aus der Reihe Schwefel, Sauerstoff (wobei Sauerstoffatome nicht unmittelbar benachbart sein dürfen) und Stickstoff und/oder gegebenenfalls mindestens eine Carbonylgruppe enthalten kann, oder
R¹ und R³ auch zusammen mit den Atomen, an die sie gebunden sind, einen gesättigten oder ungesättigten und gegebenenfalls substituierten 4- bis 8-gliedrigen Ring bilden können, der gegebenenfalls ein oder mehrere weitere Heteroatome aus der Reihe Schwefel, Sauerstoff (wobei Sauerstoffatome nicht unmittelbar benachbart sein dürfen) und Stickstoff und/oder gegebenenfalls mindestens eine Carbonylgruppe enthalten kann,

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere sowie Rotamere, Tautomere und Salze der Verbindungen der Formel (I).

Insbesondere umfassen die Verbindungen der Formel (I) E/Z Diastereomere wie zum Beispiel

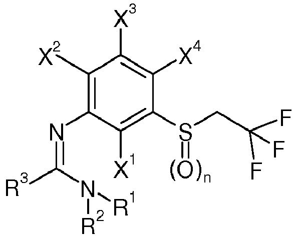

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden definiert,
wobei
n bevorzugt für die Zahl 0, 1 oder 2 steht,
X¹, X², X³, X⁴ unabhängig von einander bevorzugt für Wasserstoff, Halogen, Hydroxy, Amino, OCN, SCN, SF₅, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₄)-alkoxy, (C₁-C₆)Alkoxycarbonyl-(C₁-C₄)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₇)Alkoxycarbonyl-(C₁-C₆)alkoxy, (C₁-C₆)Alkoxy-(C₁-C₆)alkoxy, (C₁-C₇)Alkylhydroxyimino, (C₁-C₇)Alkoxyimino, (C₁-C₆)Alkyl-(C₁-C₇)alkoxyimino, (C₁-C₆)Halogenalkyl-(C₁-C₇)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy,(C₁-C₇)Alkylcarbonyl, (C₁-C₇)Halogenalkylcarbonyl, Carboxyl, (C₁-C₇)Alkylcarbonyloxy, (C₁-C₇)Alkoxycarbonyl, (C₁-C₇)Halogenalkoxycarbonyl, (C₁-C₇)Alkoxycarbonyl-(C₁-C₆)alkyl, Aminocarbonyl, (C₁-C₇)Alkylaminocarbonyl, Di-(C₁-C₇)alkylaminocarbonyl, (C₁-C₇)Alkenylaminocarbonyl, Di-(C₁-C₇)-alkenylaminocarbonyl, (C₃-C₈)Cycloalkylaminocarbonyl, (C₁-C₆)Alkylsulfonylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl,
oder für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl substituiertes gegebenenfalls durch ein oder zwei Heteroatome aus der Reihe O, S oder N unterbrochenes, gegebenenfalls gesättigtes oder ungesättigtes (C₃-C₈)Cycloalkyl, (C₃-C₈)Cycloalkoxy, (C₃-C₈)Cycloalkyl(C₁-C₆)alkyl oder (C₃-C₈)Cycloalkyl-(C₁-C₆)alkoxy substituiertes Phenyl-(C₁-C₆)alkyl, Phenoxy, Phenyl-(C₁-C₄)alkyloxy, Phenoxy-(C₁-C₄)alkyl, Phenylthio, Phenylthio-(C₁-C₄)alkyl, Phenylsulfinyl, Phenylsulfonyl, Hetaryl-(C₁-C₆)alkyl, Hetaryloxy, Hetaryl-(C₁-C₄)alkyloxy, Hetarylthio, Hetarylsulfinyl, Hetarylsulfonyl, gegebenenfalls gesättigtes oder ungesättigtes (C₃-C₈)Cycloalkyl-(C₁-C₆)alkyl, (C₃-C₈)Cycloalkyloxy, (C₃-C₈)Cycloalkyl-(C₁-C₆)alkoxy, (C₃-C₈)Cycloalkylthio, (C₃-C₈)Cycloalkyl-(C₁-C₆)alkylthio, (C₃-C₈)Cycloalkylsulfinyl, (C₃-C₈)Cycloalkyl-(C₁-C₆)alkylsulfinyl, (C₃-C₈)Cycloalkylsulfonyl, (C₃-C₈)Cycloalkyl-(C₁-C₆)alkylsulfonyl,
oder für NR⁴R⁵, wobei R⁴ und R⁵ unabhängig voneinander für Wasserstoff, Cyano, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkyl-(C₁-C₆)thioalkyl, (C₂-C₈)Alkenyl, (C₂-C₈)Halogenalkenyl, (C₂-C₈)Cyanoalkenyl, (C₂-C₈)Alkinyl, (C₂-C₈)Halogenalkinyl, (C₂-C₈)Cyanoalkinyl, Acyl, (C₁-C₇)Alkoxycarbonyl stehen oder R⁴ und R⁵ gemeinsam mit dem N-Atom, an dem sie gebunden sind, einen gegebenenfalls gleich oder verschieden durch Halogen, Cyano, Hydroxy, Amino, (C₁-C₆)Alkyl, (C₁-C₆)Halogenalkyl, (C₁-C₆)Alkoxy, (C₁-C₆)Halogenalkoxy, oder durch gegebenenfalls gleich oder verschieden durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl oder (C₃-C₈)Cycloalkyl(C₁-C₆)alkyl, substituierten und gegebenenfalls durch Heteroatome aus der Reihe O, S oder N unterbrochenen gesättigten oder ungesättigten fünf- bis siebengliedrigen Ring bilden können, stehen,
oder für einen (C₃-C₈)Cycloalkyl, (C₃-C₈)Cycloalkoxy, (C₃-C₈)Cycloalkylthio, (C₃-C₈)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, der gegebenenfalls einfach oder mehrfach, gleich oder verschieden substituiert ist mit Halogen, Cyano, Nitro, (C₁-C₆)Alkyl, (C₁-C₆)Halogenalkyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, oder durch gegebenenfalls durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl(-C₁-C₆)alkyl oder (C₃-C₈)Cycloalkyl-(C₁-C₆)alkoxy steht,
oder, X² und X³, oder X³ und X⁴, können die folgenden 5 oder 6-gliedrigen Ring ausbilden, die gegebenenfalls gleich oder verschieden durch Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, subsitutiert sind, 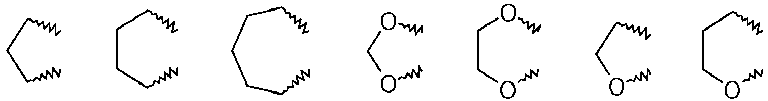
oder X² und X³, oder X³ und X⁴, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, (C₁-C₆)Alkoxy(C₁-C₆)alkyl, (C₁-C₆)Alkylthio, (C₁-C₆)Alkylsulfonyl, Amino, (C₁-C₆)Alkylamino, Di-(C₁-C₆)-alkylamino oder (C₃-C₈)Cycloalkylamino, 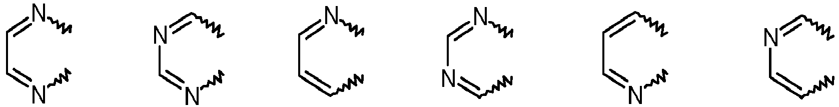 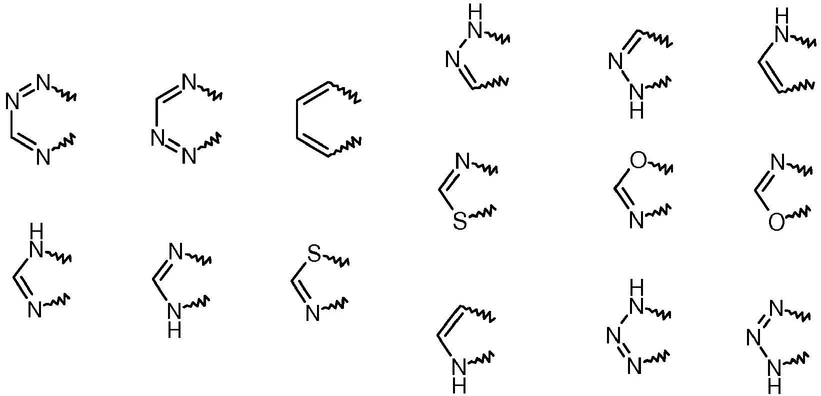 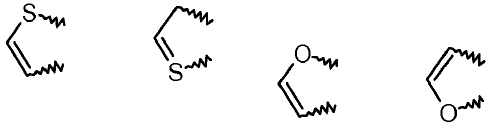
R³ bevorzugt für Wasserstoff, (C₂-C₆)Alkyl, Cyano, (C₁-C₆)Halogenalkyl, (C₁-C₆)Alkoxy-(C₁-C₆)alkyl, (C₁-C₆)Cyanoalkyl, (C₁-C₆)Alkylthio-(C₁-C₆)alkyl, (C₂-C₆)Alkenyl, (C₂-C₆)Halogenalkenyl, (C₂-C₆)Alkinyl, (C₂-C₆)Halogenalkinyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₆)Cycloalkylamino, (C₁-C₆)Alkylsulfonylamino, Aminosulfonyl, (C₁-C₆)Alkylaminosulfonyl oder Di-(C₁-C₆)alkylaminosulfonyl, oder substituiert mit gegebenenfalls gleich oder verschieden durch Halogen, Cyano, Nitro, Hydroxy, Amino, Carboxy, (C₁-C₆)Alkyl, (C₁-C₆)Halogenalkyl, (C₁-C₆)Alkoxy, (C₁-C₆)Halogenalkoxy substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy steht,
R¹ und R² unabhängig voneinander bevorzugt für Wasserstoff, Cyano, (C₁-C₆)Alkyl, (C₁-C₆)Halogenalkyl, (C₁-C₆)Alkoxy, (C₁-C₆)Cyanoalkyl, (C₁-C₆)Hydroxyalkyl, (C₁-C₆)Alkoxy-(C₁-C₆)alkyl, (C₁-C₆)Alkylthio-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl,
für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylamino, Di-(C₁-C₆)alkyl-amino substituierte (C₁-C₆)Alkylcarbonyl, (C₁-C₇)Alkoxycarbonyl, Arylcarbonyl, Hetarylcarbonyl, Aryloxycarbonyl, Hetaryloxycarbonyl, (C₁-C₆)Alkylsulfinyl, (C₁-C₆)Halogenalkylsulfinyl, Arylsulfinyl, Aryl(C₁-C₆)alkylsulfinyl, Hetarylsulfinyl, Hetaryl(C₁-C₆)alkylsulfinyl, (C₁-C₆)Alkylsulfonyl, (C₁-C₆)Halogenalkylsulfonyl, Arylsulfonyl, Aryl(C₁-C₆)alkylsulfonyl, Hetarylsulfonyl, Hetaryl(C₁-C₆)alkylsulfonyl stehen,
oder für einen 3- bis 6-gliedrigen gesättigten, teilgesättigten oder aromatischen Ring, der gegebenenfalls ein bis drei Heteroatome aus der Reihe O, S oder N enthalten kann, gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)-alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)-alkylaminosulfonyl, oder mit gegebenenfalls substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy stehen,
oder für -(CH₂)ₘ-R⁶, -O-(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkylamino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl, oder gleich oder verschieden mit gegebenenfalls durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy stehen, wobei m für die Zahl 1, 2 oder 3 steht, oder
R¹ und R² zusammen mit den Atomen, an das sie gebunden sind, einen gesättigten oder ungesättigten und gegebenenfalls einfach oder mehrfach durch (C₁-C₄)Alkyl, (C₁-C₄)Alkoxy, Halogen, (C₁-C₄)Halogenalkyl substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe stehen,
R¹ und R³ zusammen mit den Atomen, an die sie gebunden sind, einen gesättigten oder ungesättigten und gegebenenfalls einfach oder mehrfach gleich oder verschieden durch (C₁-C₄)Alkyl, (C₁-C₄)Alkoxy, Halogen, (C₁-C₄)Halogenalkyl, Cyano, (C₃-C₆)Cycloalkyl, (C₃-C₆)Halogencycloalkyl, (C₃-C₆)Cyanocycloalkyl, (C₁-C₂)Alkyl(C₃-C₆)Cycloalkyl (C₂-C₄)Alkandiyl, (C₂-C₄)Alkendiyl, Butandienyl (wobei Alkandiyl, Alkendiyl und Butandienyl gegebenenfalls durch (C₁-C₄)Alkyl, (C₁-C₄)Alkoxy, Halogen, (C₁-C₄)Halogenalkyl substituiert sein können und/oder gegebenenfalls durch mindestens ein Sauerstoff- oder/und Stickstoffatom unterbrochen sein können) substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe enthalten kann,

Die erfindungsgemäßen Verbindungen sind durch die Formel (I) allgemein definiert. Besonders bevorzugte Substituenten bzw. Bereiche der in der oben und nachstehend erwähnten Formeln aufgeführten Reste werden definiert,
wobei
n besonders bevorzugt für die Zahl 0 oder 1 steht,
X¹, X², X³, X⁴ unabhängig von einander besonders bevorzugt für Wasserstoff, Fluor, Chlor, Brom, Jod, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, Aminothiocarbonyl,
oder für gegebenenfalls einfach oder zweifach gleich oder verschieden durch Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, oder durch gegebenenfalls durch ein Heteroatom aus der Reihe O, S oder N unterbrochenes, gegebenenfalls gesättigtes oder ungesättigtes (C₃-C₆)Cycloalkyl substituiertes Phenyl-(C₁-C₄)alkyl, Phenoxy, Hetaryl-(C₁-C₄)alkyl, Hetaryloxy, gegebenenfalls gesättigtes oder ungesättigtes (C₃-C₆)Cycloalkyl-(C₁-C₄)alkyl, (C₃-C₆)Cycloalkyloxy, (C₃-C₆)Cycloalkyl-(C₁-C₄)alkoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl, der gegebenenfalls einfach oder zweifach, gleich oder verschieden substituiert ist mit Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, (C₃-C₆)Cycloalkyl steht,
oder, X² und X³, oder X³ und X⁴, können die folgenden 5 oder 6-gliedrigen Ring ausbilden, die gegebenenfalls einfach bis vierfach durch Fluor oder (C₁-C₄)Alkyl subsitutiert sind, 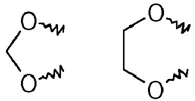
oder X² und X³, oder X³ und X⁴, können die folgenden anellierten Ringe ausbilden, die gegebenenfalls einfach oder zwei, gleich oder verschieden substituiert sind, wobei die Substituenten unabhängig voneinander ausgewählt sein können aus Wasserstoff, Fluor, Chlor, Brom, Cyano, (C₁-C₄)Alkyl, (C₃-C₆)Cycloalkyl, (C₁-C₄)Halogenalkyl, (C₁-C₄)Alkoxy, (C₁-C₄)Halogenalkoxy, 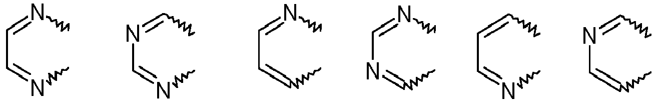 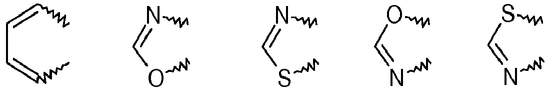
R³ besonders bevorzugt für Wasserstoff, (C₂-C₄)Alkyl, Cyano, (C₁-C₄)Halogenalkyl, (C₁-C₄)Alkoxy-(C₁-C₄)alkyl, (C₁-C₄)Cyanoalkyl,
oder für einen 3- bis 6-gliedrigen gesättigten, teilgesättigten oder aromatischen Ring, der gegebenenfalls ein bis zwei Heteroatome aus der Reihe O, S oder N enthalten kann und der gegebenenfalls einfach oder dreifach, gleich oder verschieden substituiert ist mit Fluor, Chlor, Brom, Jod, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, oder substituiert mit (C₃-C₆)Cycloalkyl steht,
R¹ und R² unabhängig voneinander besonders bevorzugt für Wasserstoff, Cyano, (C₁-C₄)Alkyl, (C₁-C₄)Halogenalkyl, (C₁-C₄)Alkoxy, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Alkinyl,
für gegebenenfalls einfach bis dreifach unabhängig voneinander durch Fluor, Chlor, Brom, Cyano, Nitro, (C₁-C₄)Alkyl, (C₁-C₄)Halogenalkyl, (C₂-C₄)Alkenyl, (C₂-C₄)Halogenalkenyl, (C₂-C₄)Alkinyl, (C₁-C₄)Alkoxy, (C₁-C₄)Halogenalkoxy, (C₁-C₄)Alkylthio, (C₁-C₄)Alkylsulfinyl, (C₁-C₄)Alkylsulfonyl, (C₁-C₄)Alkylamino, Di-(C₁-C₄)alkyl-amino substituiertes (C₁-C₄)Alkylcarbonyl, (C₁-C₅)Alkoxycarbonyl, Arylcarbonyl, Thiophenylcarbonyl, Pyridylcarbonyl, Pyrimidylcarbonyl, Thiazolylcarbonyl, Pyrazolylcarbonyl, (C₁-C₄)Alkylsulfinyl, (C₁-C₄)Halogenalkylsulfinyl, Arylsulfinyl, Aryl(C₁-C₄)alkylsulfinyl, Hetarylsulfinyl, Hetaryl(C₁-C₄)alkylsulfinyl, (C₁-C₄)Alkylsulfonyl, (C₁-C₄)Halogenalkylsulfonyl, Arylsulfonyl, Aryl(C₁-C₄)alkylsulfonyl, Hetarylsulfonyl, Hetaryl(C₁-C₄)alkylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl stehen,
oder für -(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl stehen, wobei m für die Zahl 1 oder 2 steht, oder
R¹ und R² zusammen mit dem N-Atom, an das sie gebunden sind, einen gesättigten oder ungesättigten und gegebenenfalls einfach oder vierfach durch Fluor, Chlor, (C₁-C₄)Alkyl, (C₁-C₄)Alkoxy, (C₁-C₄)Halogenalkyl substituierten 3- bis 6-gliedrigen Ring bilden können, der gegebenenfalls durch ein weiteres Heteroatom aus der Reihe Schwefel, Sauerstoff und Stickstoff enthalten kann und/oder mindestens eine Carbonylgruppe stehen,
R¹ und R³ zusammen mit den Atomen, an die sie gebunden sind, einen gesättigten oder ungesättigten und gegebenenfalls einfach oder mehrfach durch Methyl, Ethyl, Methoxy, Ethoxy, Fluor, Chlor, Trifluoromethyl, Cyclopropyl, Cyano, Chlorcyclopropyl, Fluorcyclopropyl, Cyanocyclopropyl, Methylcyclopropyl, (C₂-C₄)Alkandiyl, (C₂-C₄)Alkendiyl, Butandienyl (wobei Butandienyl gegebenenfalls einfach oder zweifach durch Methyl, Fluor, Chlor, Brom, Methoxy oder Trifluoromethyl substituiert sein können und/oder gegebenenfalls durch mindestens ein Sauerstoff- oder/und Stickstoffatom unterbrochen sein können) substituierten 5- bis 6-gliedrigen Ring bilden können, der gegebenenfalls ein weiteres Schwefel- oder Sauerstoff- oder Stickstoffatom und/oder eine Carbonylgruppe enthalten kann,

Die erfindungsgemäßen Verbindungen sind durch die Formel (I) allgemein definiert. Ganz besonders bevorzugte Substituenten bzw. Bereiche der in der oben und nachstehend erwähnten Formeln aufgeführten Reste werden definiert,
wobei
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Wasserstoff, Fluor, Chlor, Brom, Methyl, Ethyl, Trifluoromethyl, Difluoromethyl, Difluoromethoxy, Trifluoromethoxy, OCH₂CF₃,
insbesonders bevorzugt stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ ganz besonders bevorzugt für Wasserstoff, Ethyl, Propyl, Cyano, Trifluoromethyl, Difluoromethyl, Dichlormethyl, Chlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl,
oder für Aryl steht, insbesondere bevorzugt für Phenyl steht,
R¹ ganz besonders bevorzugt für Wasserstoff, Methyl, Ethyl, Propyl, Butyl, *sec*-Butyl, *iso*-Propyl, *tert-*Butyl, (2,2,2)Trifluoroethyl, (2,2)Difluoromethyl, Methoxy, Ethoxy, Methoxymethyl, Methoxyethyl,
oder für Aryl steht, welches gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere wird Phenyl explizit genannt: welches gegebenenfalls mit den unter ganz besonders bevorzugt genannten Subtituenten substituiert sein kann,
oder für -(CH₂)ₘ-R⁶, wobei R⁶ für Aryl steht, welches gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere wird Phenyl für R⁶ explizit genannt, welches gegebenenfalls mit den unter ganz besonders bevorzugt genannten Subtituenten substituiert sein kann,
R² ganz besonders bevorzugt für Wasserstoff, Methyl oder Ethyl steht, oder
R¹ und R² zusammen mit dem N-Atom, an das sie gebunden sind, einen gesättigten oder ungesättigten 5- bis 6-gliedrigen Ring bilden können, der gegebenenfalls ein Sauerstoffatom enthalten kann, insbesondere werden folgende Ringe explizit genannt, 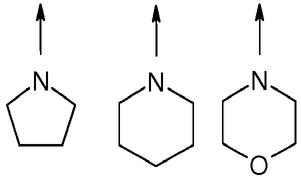 in denen das N-Atom, an das R¹ und R² gebunden sind, den Cyclus darstellen und der Pfeil zum Rest des Moleküls zeigt,
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, Difluoromethyl, Cyano, Cyclopropyl, Chlorcyclopropyl, Fluorcyclopropyl, Cyanocyclopropyl, Methylcyclopropyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls eine Carbonylgruppe enthalten kann, insbesondere stehen R¹ und R³ zusammen mit den Atomen, an die sie gebunden sind für folgende Gruppe, 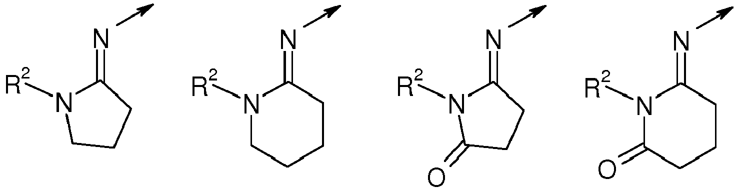 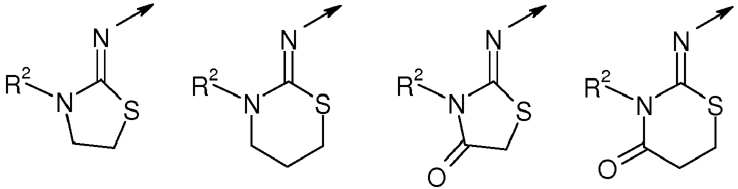 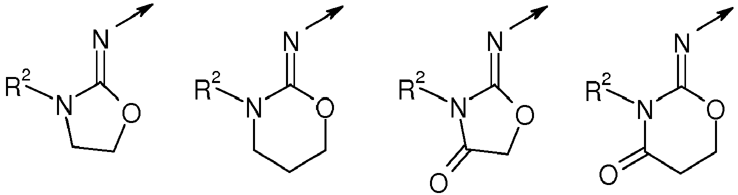 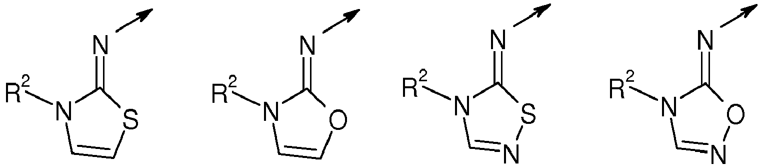
die jeweils gegebenenfalls einfach oder zweifach durch Methyl, Ethyl, Methoxy, Ethoxy, Fluor, Chlor, Trifluoromethyl, Difluoromethyl, Cyclopropyl, Chlorcyclopropyl, Fluorcyclopropyl, Cyanocyclopropyl, Methylcyclopropyl substituiert sein können,
   wobei der Pfeil zum Rest des Moleküls zeigt.

Die erfindungsgemäßen Verbindungen sind durch die Formel (I) allgemein definiert. Ebenfalls ganz besonders bevorzugte Substituenten bzw. Bereiche der in der oben und nachstehend erwähnten Formeln aufgeführten Reste werden definiert,
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Wasserstoff, Fluor, Chlor, Brom, Methyl, Ethyl, Trifluoromethyl, Difluoromethyl, Vinyl, Ethinyl, Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders bevorzugt stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl, Methoxy/Methyl, Methyl/Methoxy, Methyl/H, Ethyl/H, Chlor/H, Brom/H, Fluor/H, Methoxy/H, H/Chlor, H/Fluor, H/Brom, H/Methyl, H/Methoxy, H/Ethyl
R³ ganz besonders bevorzugt für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ ganz besonders bevorzugt für
Cyano, Cyanomethyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für Oxetanyl, Thietanyl, Trimethylensulfonyl, Trimethylensulfinyl, Oxanyl oder Thianyl, steht, die gegebenenfalls einfach oder mehrfach, gleich oder verschieden sein können mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
oder für Cyclopropyl, Cyclobutyl, Cyclopentyl, Cyclohexyl, steht, die einfach oder mehrfach, gleich oder verschieden sind mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl,
R² ganz besonders bevorzugt für Wasserstoff, Methyl oder Ethyl steht, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der ein oder zwei weitere Heteroatome aus der Reihe Schwefel oder Stickstoff enthält,
oder zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 4-gliedrigen Ring bilden können, der gegebenenfalls ein weiteres Heteroatom aus der Reihe Sauerstoff, Schwefel oder Stickstoff enthält
insbesondere werden folgende Ringe explizit genannt, 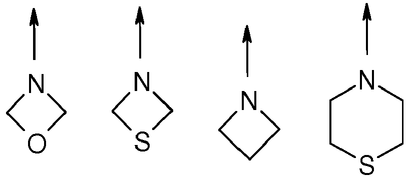 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

Die erfindungsgemäßen Verbindungen sind durch die Formel (I) allgemein definiert. Ebenfalls ganz besonders bevorzugte Substituenten bzw. Bereiche der in der oben und nachstehend erwähnten Formeln aufgeführten Reste werden definiert,
wobei
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Wasserstoff, Fluor, Chlor, Brom, Methyl, Ethyl, Trifluoromethyl, Difluoromethyl, Vinyl, Ethinyl, Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders bevorzugt stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl, Methoxy/Methyl, Methyl/Methoxy, Methyl/H, Ethyl/H, Chlor/H, Brom/H, Fluor/H, Methoxy/H, H/Chlor, H/Fluor, H/Brom, H/Methyl, H/Methoxy, H/Ethyl
R³ ganz besonders bevorzugt für Methoxymethyl, Methoxyethyl, Cyanomethyl, Allyl, Butenyl, Oxolanyl, Pentandienyl, Butadienyl,
oder für Cyclopropyl, Cyclobutyl, Cyclopentyl, Cyclohexyl, Pyridyl, Pyrimidyl, Pyrazinyl, Pyridazinyl, Pyrrolyl, Furanyl, Thiazolyl, Pyrazolyl oder Thienyl steht, die gegebenenfalls einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert sein können,
oder für Aryl, insbesondere für Phenyl steht, welches einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert ist,
R¹ und R² ganz besonders bevorzugt unabhängig voneinander für Wasserstoff, Cyano, Methyl, Ethyl, Propyl, Butyl, *sec*-Butyl, *iso*-Propyl, *tert*-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 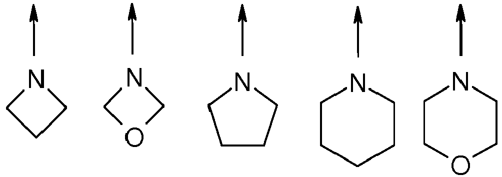
in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

Die erfindungsgemäßen Verbindungen sind durch die Formel (I) allgemein definiert. Ebenfalls ganz besonders bevorzugte Substituenten bzw. Bereiche der in der oben und nachstehend erwähnten Formeln aufgeführten Reste werden definiert,
n ganz besonders bevorzugt für die Zahl 0 oder 1 steht,
X¹ und X³ ganz besonders bevorzugt für Wasserstoff stehen,
X² und X⁴ ganz besonders bevorzugt für folgende Kombinationen X²/X⁴ stehen: Vinyl/H, H/Vinyl, Ethinyl/H, H/Ethinyl, Methoxy/H, H/Methoxy, Ethoxy/H, H/Ethoxy, Aminothiocarbonyl/H, H/Aminothiocarbonyl, Vinyl/Methyl, Methyl/Vinyl, Ethinyl/Methyl, Methyl/Ethinyl, Methoxy/Methyl, Methyl/Methoxy, Ethoxy/Methyl, Methyl/Ethoxy, Aminothiocarbonyl/Methyl, Methyl/Aminothiocarbonyl, Vinyl/F, F/Vinyl, Ethinyl/F, F/Ethinyl, Methoxy/F, F/Methoxy, Ethoxy/F, F/Ethoxy, Aminothiocarbonyl/F, F/Aminothiocarbonyl, Vinyl/Cl, Cl/Vinyl, Ethinyl/Cl, Cl/Ethinyl, Methoxy/Cl, Cl/Methoxy, Ethoxy/Cl, Cl/Ethoxy, Aminothiocarbonyl/Cl, Cl/Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
R³ ganz besonders bevorzugt für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ und R² ganz besonders bevorzugt unabhängig voneinander für Wasserstoff, Cyano, Methyl, Ethyl, Propyl, Butyl, *sec*-Butyl, *iso*-Propyl, *tert*-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 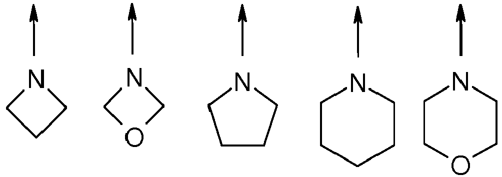
in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können,
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, Difluoromethyl, Cyano, Cyclopropyl, Chlorcyclopropyl, Fluorcyclopropyl, Cyanocyclopropyl, Methylcyclopropyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls ein weiteres Schwefel- oder Sauerstoff- oder Stickstoffatom und/oder eine Carbonylgruppe enthalten kann, insbesondere stehen R¹ und R³ zusammen mit den Atomen, an die sie gebunden sind für folgende Gruppe, 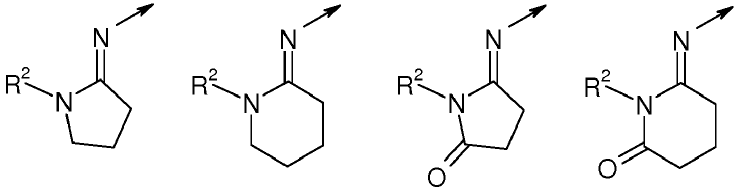 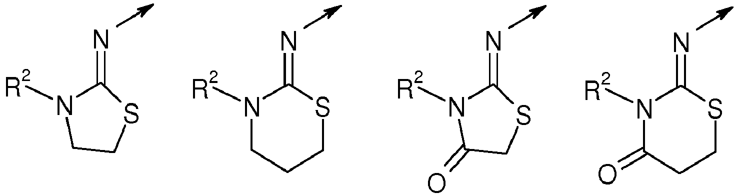 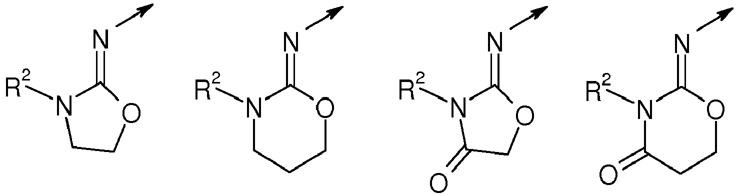 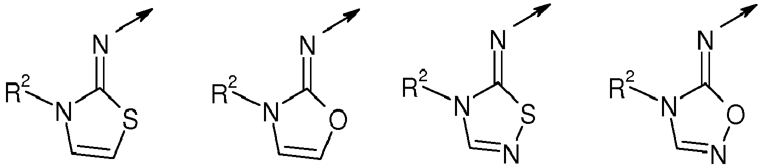
die jeweils gegebenenfalls einfach oder zweifach durch Methyl, Ethyl, Methoxy, Ethoxy, Fluor, Chlor, Trifluoromethyl, Difluoromethyl, Cyclopropyl, Chlorcyclopropyl, Fluorcyclopropyl, Cyanocyclopropyl, Methylcyclopropyl substituiert sein können,
   wobei der Pfeil zum Rest des Moleküls zeigt.

Die erfindungsgemäßen Verbindungen sind durch die Formel (I) allgemein definiert. Ebenfalls ganz besonders bevorzugte Substituenten bzw. Bereiche der in der oben und nachstehend erwähnten Formeln aufgeführten Reste werden definiert,
n ganz besonders bevorzugt für die Zahl 0 oder 1 steht,
X¹ und X³ ganz besonders bevorzugt für Wasserstoff stehen,
X² und X⁴ ganz besonders bevorzugt unabhängig voneinander für Wasserstoff, Fluor, Chlor, Brom, Methyl, Ethyl, Trifluoromethyl, Difluoromethyl, Vinyl, Ethinyl, Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders bevorzugt stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl, Methoxy/Methyl, Methyl/Methoxy, Methyl/H, Ethyl/H, Chlor/H, Brom/H, Fluor/H, Methoxy/H, H/Chlor, H/Fluor, H/Brom, H/Methyl, H/Methoxy, H/Ethyl
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, Difluoromethyl, Cyano, Cyclopropyl, Chlorcyclopropyl, Fluorcyclopropyl, Cyanocyclopropyl, Methylcyclopropyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls ein weiteres Schwefel- oder Sauerstoff- oder Stickstoffatom und/oder eine Carbonylgruppe enthalten kann, insbesondere stehen R¹ und R³ zusammen mit den Atomen, an die sie gebunden sind für folgende Gruppe, 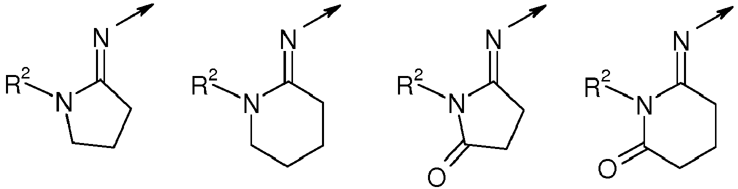 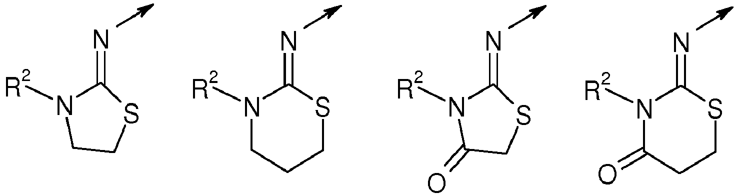 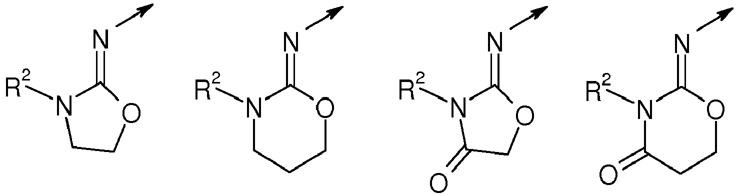 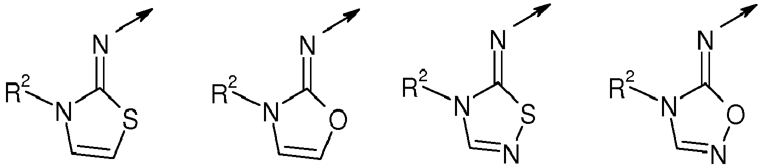
die jeweils gegebenenfalls einfach oder zweifach durch Methyl, Ethyl, Methoxy, Ethoxy, Fluor, Chlor, Trifluoromethyl, Difluoromethyl, Cyclopropyl, Chlorcyclopropyl, Fluorcyclopropyl, Cyanocyclopropyl, Methylcyclopropyl substituiert sein können,
   wobei der Pfeil zum Rest des Moleküls zeigt.
R² ganz besonders bevorzugt für Cyano, (2,2,2)Trifluoroethyl, (2,2)Difluoromethyl, 2-Chloro-(2,2)difluoroethyl, (2,2)Dichloro-2-fluoroethyl, (2,2,2)Trichloroethyl, Pentafluoroethyl, 2-Chlorotetrafluoroethyl, Allyl, Butenyl, Propinyl, Methylsulfinyl, Trifluoromethylsulfinyl, Methylsulfonyl, Trifluoromethylsulfonyl stehen,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl stehen, wobei m für die Zahl 1, insbesondere werden folgende R⁶ explizit genannt: Cyclopropyl, Cyclobutyl, Cyclopentyl, Cyclohexyl, Phenyl und Pyridyl, die gegebenenfalls mit den unter ganz besonderen bevorzugten genannten Subtituenten substituiert sein können,

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

In den als bevorzugt gennanten Restedefinitionen steht Halogen für Fluor, Chlor, Brom und Jod, besonders bevorzugt für Fluor, Chlor und Brom, und ganz besonders bevorzugt für Fluor und Chlor.

### Herstellverfahren

Die Verbindungen der allgemeinen Formel (I) können mit den in der Anmeldung WO 2007/131680 beschriebenen Methoden hergestellt werden. Abweichend von diesen beschriebenen Methoden können die Verbindungen der Formel (I) auch nach Verfahren A, Verfahren B, Verfahren C, Verfahren E, Verfahren F, Verfahren G, Verfahren H, Verfahren I, Verfahren J und Verfahren K hergestellt werden. Verfahren D stellt eine Alternativ zur Herstellung der Vorstufen.

Einige allgemeine Verfahren zur Herstellung von Amidinen sind beschrieben in S. Patai and Z. Rappoport, The Chemistry of Amidines and Imidates, Vol. 27, John Wiley & Sons, New York, NY, USA, 1991.

Ferner sind in der Literatur Palladium(II)- katalysierte Synthesen von Arylamidinen mit Hilfe von Trifluorboraten beschrieben, siehe z.B. J.Sävmarker, Dissertation Universität Uppsala, 2012 oder J.Sävmarker, Org. Lett. 14 (2012), 2394-2397.

### Verfahren A

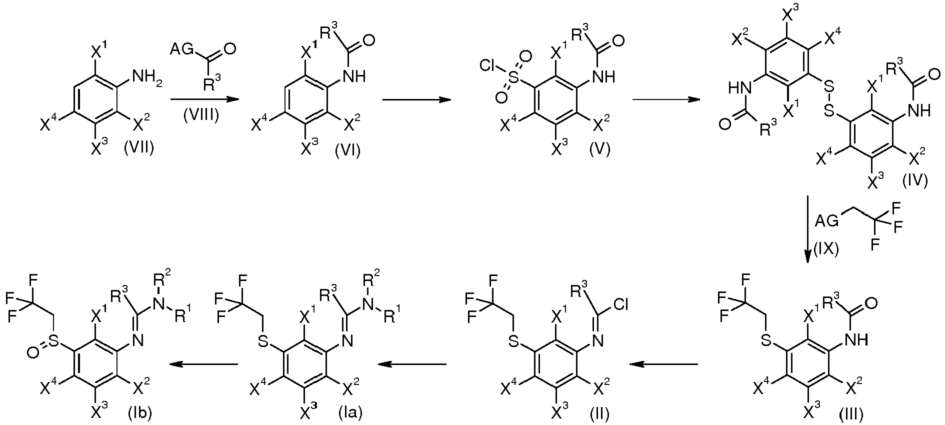

Wobei X¹, X², X³, X⁴, R¹, R² und R³ die oben angegebenen Bedeutungen haben, AG für eine Abgangsgruppe und PG für eine Schutzgruppe steht.

Aniline der Formel (VII) sind entweder kommerziell erhältlich oder können durch bekannte Methoden hergestellt werden. In Gegenwart von Säuren, Säureanhydriden oder Säurechloriden der Formel (VIII) werden die Aniline (VII) in die entsprechenden Anilide (VI) überführt. Die Chlorsulfonierung der Anilide (VI) mit Chlorsulfonsäure liefert die entsprechenden Sulfonylchloride (V). Die Reduktion der Sulfonylchloride (V) in die Disulfide (IV) ist mit literaturbekannten Methoden wie zum Beispiel Eisen in Salzsäure oder Iodid möglich. Die Umsetzung der Disulfide (IV) mit Trifluorethylelektrophilen der Formel (XVI), wobei AG für eine Abgangsgruppe wie zum Beispiel Chlor, Brom, Tosylat, Mesylat oder Triflat steht, liefern die Sulfide (III).

Die Anilide (III) werden beispielsweise mit Diphenylchlorphosphat oder Phosphorpentachlorid zu Imidoylchloriden der Formel (II) chloriert und mit Aminen zu Amidinen (Ia) umgesetzt.

Die Thioether (Ia) werden in die entsprechenden Sulfoxide (Ib) durch Umsetzung mit Oxidationsmitteln wie zum Beispiel meta-Chlorperbenzoesäure überführt.

Die Oxidation von Verbindungen der Formel (Ia) zu Verbindungen der Formel (Ib) kann durch ein Oxidationsmittel in einem geeignetem Lösungs- und Verdünnungsmittel erfolgen. Als Oxidationsmittel eignen sich zum Beispiel verdünnte Salpetersäure, Wasserstoffperoxid und Peroxycarbonsäuren, wie *meta-*Chlorperbenzoesäure. Als Lösungsmittel eignen sich inerte organische Lösungsmittel, typischerweise Acetonitril und halogenierte Lösungsmittel wie Dichlormethan, Chloroform oder Dichlorethan.

Zur Erzeugung enantiomeren angereicherten Sulfoxide eignen sich eine Vielfalt von Methoden, wie von A.R. Maguire in ARKIVOC, 2011(i), 1-110 oder von WO2011/006646 beschrieben: metall-katalysierte asymmetrische Oxidationen von Thioethern, zum Beispiel mit Titatium und Vanadium als meistbenutzten Katalysatorquellen, in Form von Ti(OⁱPr₄) und VO(acac)₂, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nicht-metall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, in dem sie zum Beispiel präparativ auf einer chiralen HPLC getrennt werden.

Die Verbindungen der Formel (II), (III), (IV) und (V) im Verfahren A lassen sich insbesondere nach den in den Herstellbeispielen genannten Bedingungen herstellen.

Alternativ können die Thioether der Formel (III) nach Verfahren B hergestellt werden.

### Verfahren B

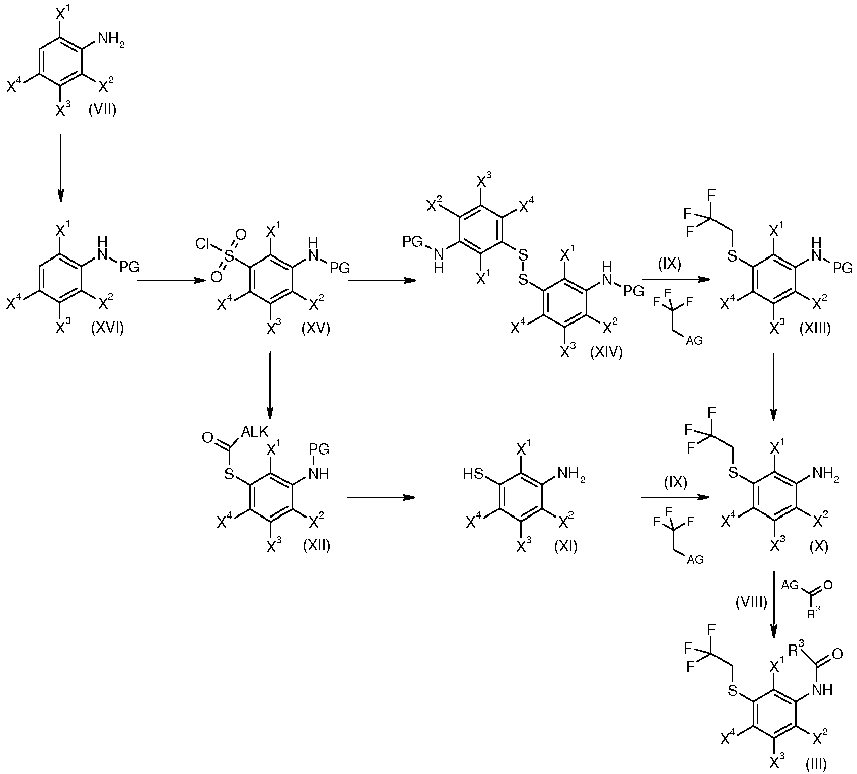

wobei X¹, X², X³, X⁴ und R³ die oben angegebenen Bedeutungen haben, AG für eine Abgangsgruppe und PG für eine Schutzgruppe steht.

Die Aniline (VII) können mit einer geeigneten literaturbekannten Schutzgruppe wie zum Beispiel einer Acetylgruppe zu Verbindungen der Formel (XVI) geschützt werden. Die Chlorsulfonierung der Verbindungen der Formel (XVI) mit Chlorsulfonsäure liefert die entsprechenden Sulfonylchloride (XV). Die Reduktion der Sulfonylchloride (XV) in die Disulfide (XIV) ist mit literaturbekannten Methoden wie zum Beispiel Eisen in Salzsäure oder Iodid möglich. Die Umsetzung der Disulfide (XIV) mit Trifluorethylelektrophilen der Formel (IX), wobei AG für eine Abgangsgruppe wie zum Beispiel Chlor, Brom, Tosylat, Mesylat oder Triflat steht, liefern die Sulfide (XIII). Die Schutzgruppe wird durch geeignete literaturbekannte Methoden abgespalten, so dass man Aniline der Formel (X) erhält. Diese werden in Gegenwart von Säuren, Säureanhydriden oder Säurechloriden der Formel (VIII) in die entsprechenden Anilide (III) überführt.

Statt der Reduktion zum Disulfid (XIV), kann mit einem geeigneten Reduktionsmittel wie zum Beispiel Iod/Phosphor auch zum Alkylthioat (XI) reduziert und anschließend durch eine geeignete Methode wie zum Beispiel mit Kaliumhydoxidlösung zu Verbindungen der Formel (X) entschützt werden. Die Umsetzung der Thiole (XI) mit Trifluorethylelektrophilen der Formel (IX), wobei AG für eine Abgangsgruppe wie zum Beispiel Chlor, Brom, Tosylat, Mesylat oder Triflat steht, liefern die Sulfide (X). Diese werden in Gegenwart von Säuren, Säureanhydriden oder Säurechloriden der Formel (VIII) in die entsprechenden Anilide (III) überführt.

Die Verbindungen der Formel (III), (X), (XI), (XII), (XV) und (XVI) im Verfahren B lassen sich insbesondere nach den in den Herstellbeispielen genannten Bedingungen herstellen.

### Verfahren C

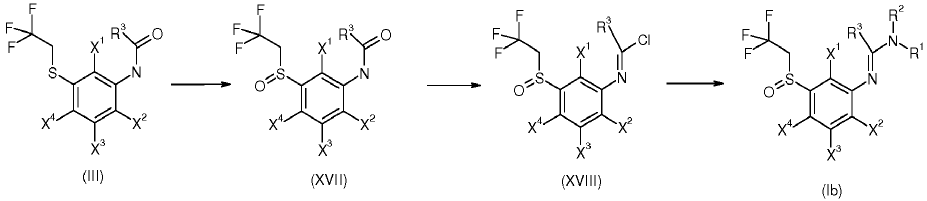

wobei X¹, X², X³, X⁴, R¹, R² und R³ die oben angegebenen Bedeutungen haben.

Weiterhin wurde gefunden, dass man chirale Verbindungen (Ib) nach Verfahren C erhält, wenn man Verbindungen der Formel (III) chiral oxidiert zu Sulfoxide der Formel (XVII).

Die Anilide (XVII) werden beispielsweise mit Diphenylchlorphosphat oder Phosphorpentachlorid zu Imidoylchloriden der Formel (XVIII) chloriert und mit Aminen zu Amidinen (Ib) umgesetzt.

Die Verbindungen der Formel (XVII) im Verfahren C sind teilweise neu und lassen sich insbesondere nach den in den Herstellbeispielen genannten Bedingungen herstellen.

### Verfahren D

Alternativ können die Thioether der Formel (X) nach Verfahren D hergestellt werden. 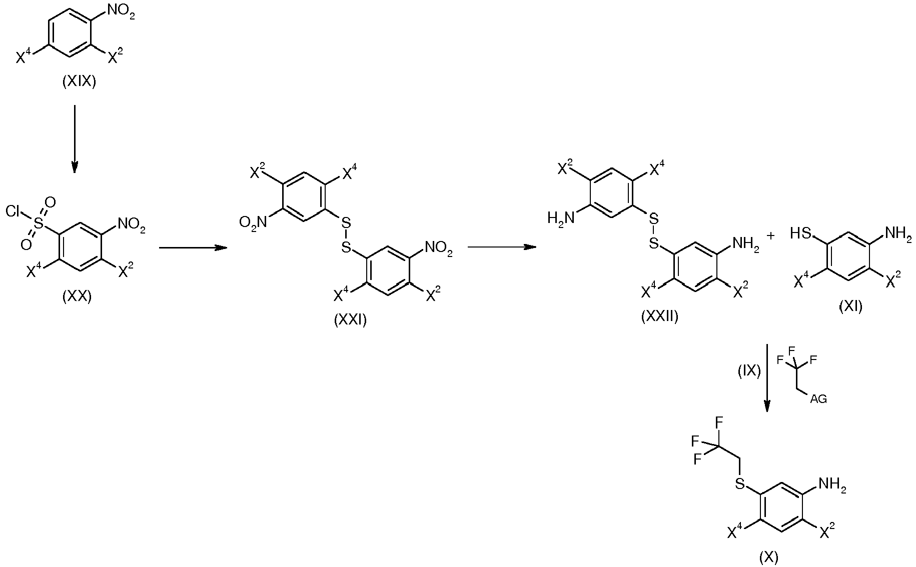 X², X⁴ können unabhängig voneinander besonders bevorzugt für Wasserstoff, Fluor, Chlor, Brom stehen.

Chlorsulfonierung der Nitroaromaten der Formel (XIX) mit Chlorsulfonsäure liefert die entsprechenden Sulfonylchloride (XX). Die Reduktion der Sulfonylchloride (XX) in die Bis(nitroaryl)disulfide (XXI) ist mit literaturbekannten Methoden wie zum Beispiel Iodid möglich. Die Reduktion der Disulfide (XXI) in die Disulfanediyldianiline (XXII), die teilweise als Gemisch mit den entsprechenden Aminoarylthiolen (XI) entstehen, ist mit Reduktionsmittel wie zum Beispiel Wasserstoff mit Hilfe heterogener Katalysatoren wie zum Beispiel Raney-Nickel, Platin auf Aktivkohle oder Palladium auf Aktivkohle möglich. Die Umsetzung der Disulfide (XXII) bzw. Thiophenole (XI) mit Trifluorethylelektrophilen der Formel (IX), wobei AG für eine Abgangsgruppe wie zum Beispiel Chlor, Brom, Iod, Tosylat, Mesylat oder Triflat steht, liefert die 3-[(2,2,2-Trifluorethyl)sulfanyl]aniline der Formel (X).

Die Verbindungen der Formel (X), (XI), (XII), (XX) und (XXI) im Verfahren D lassen sich insbesondere nach den in den Herstellbeispielen genannten Bedingungen herstellen.

### Verfahren E

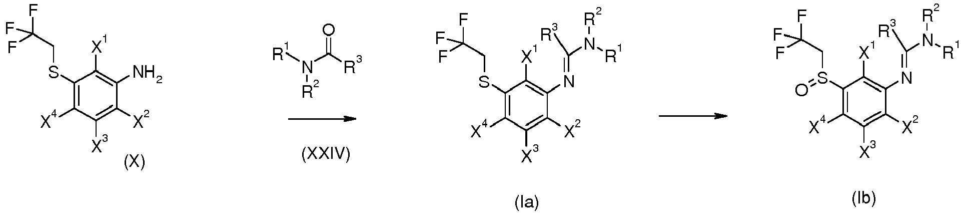

wobei X¹, X², X³, X⁴, R¹, R² und R³ die oben angegebenen Bedeutungen haben.

Aniline der Formel (X) können zu den Trifluorethylsulfid-Derivaten der Formel (Ia) nach literaturbekannten Methoden hergestellt werden, zum Beispiel durch die Umsetzung mit Amiden der Formel (XXIV) und Phosphorylchlorid, gegebenenfalls in Anwesenheit eines inerten, organischen Lösungsmittels.

Durch Oxidation der Thioether (Ia) nach literaturbekannten Methoden können die Sulfoxide (Ib) erhalten werden.

Amide der Formel (XXIV) in der R³ für gegebenenfalls substituiertes Cyclopropyl steht und R¹ für Wasserstoff steht und R² für gegebenenfalls substituiertes Haloalkyl steht, führen bei der Umsetzung mit Anilinen der Formel (X) in Gegenwart von Phosphorylchlorid zu einem Gemisch der erfindungsgemäßen *N*-Arylamidine der allgemeinen Formel (Ia-a) und (Ia-b). 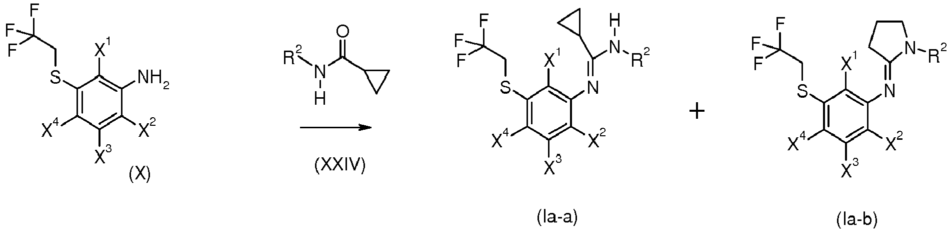 wobei X¹, X², X³, X⁴ und R² die oben angegebenen Bedeutungen haben.

Durch Oxidation der Thioether (Ia-a) erhält man nach literaturbekannten Methoden Sulfoxide der Formel (Ib), wobei R3 für gegebenenfalls substituiertes Cyclopropyl steht und R¹ für Wasserstoff steht und R² für gegebenenfalls substituiertes Haloalkyl steht.

Durch Oxidation der Thioether (Ia-b) erhält man nach literaturbekannten Methoden Sulfoxide der Formel (Ib), wobei R¹ und R³ zusammen einen 5-Ring bilden und R² für gegebenenfalls substituiertes Haloalkyl steht.

Amide der Formel (XXIV) sind kommerziell erhältlich oder können nach hinlänglich bekannten Verfahren, z.B. durch die Umsetzung von Säurechloriden mit Aminen, gegebenenfalls in Gegenwart von Basen und gegebenenfalls in Gegenwart eines Lösungsmittels erhalten werden, beispielsweise analog Houben-Weyl VIII, 655.

Die Verbindungen der Formel (X) und (XXIV) im Verfahren E lassen sich insbesondere nach den in den Herstellbeispielen genannten Bedingungen herstellen.

### Verfahren F

Die 3-substituierten 2-Arylimino-1,3-thiazolidine (n=0, A=S), 2-Arylimino-1,3-thiazinane (n=1, A=S), 2-Arylimino-1,3-oxazolidine (n=0, A=O) und 2-Arylimino-1,3-oxazinane (n=1, A=O) der allgemeinen Formel (I) können beispielsweise nach Verfahren F hergestellt werden. 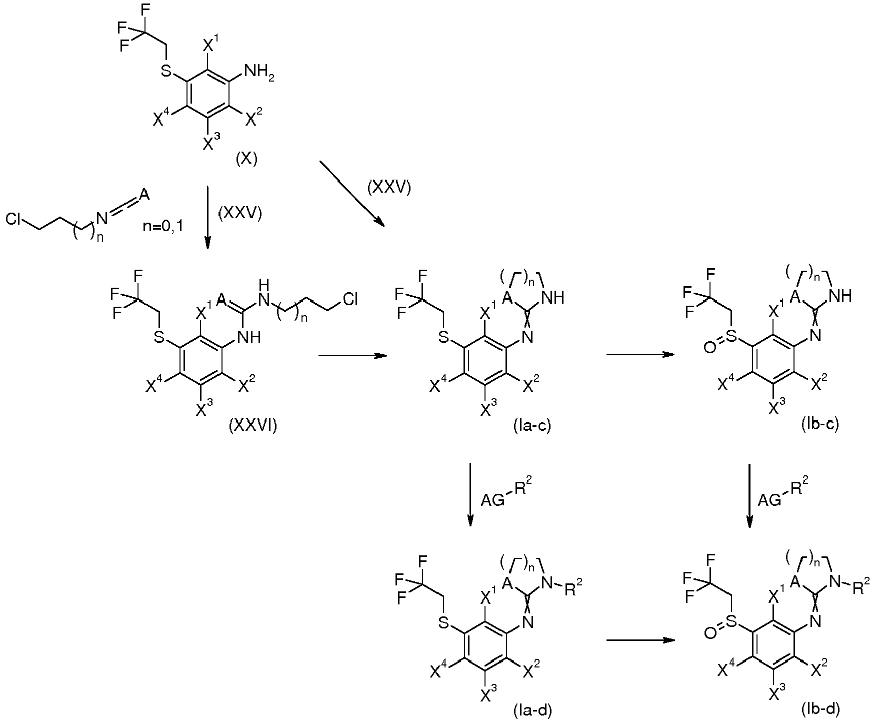 wobei X¹, X², X³, X⁴ und R² die oben angegebenen Bedeutungen haben.

Aniline der Formel (X) können durch die Umsetzung mit Isocyanaten (XXV, A=O) oder Isothiocyanaten (XXV, A=S) direkt zu den cyclischen Amidinen der Formel (Ia-c) umgesetzt werden. Im Falle der Zwischenisolierung der (Thio)harnstoffe der Formel (XXVI) können diese in Gegenwart von Basen in die cyclischen Amidine der Formel (Ia-c) umgewandelt werden.

(Thio)harnstoffe und deren Sulfoxide werden im Patent JP2011/042611 als Akarizide beansprucht. Die (Thio)harnstoffe der Formel (XXVI) sind neu und ebenfalls Gegenstand der Erfindung.

Durch Oxidation der Thioether der Formel (Ia-c) nach literaturbekannten Methoden können die Sulfoxide der Formel (Ib-c) erhalten werden.

Die Thioether (Ia-c) sowie die Sulfoxide (Ib-c) können dann nach literaturbekannten Methoden zu den Verbindungen der allgemeinen Formel (Ia-d) bzw. (Ib-d), wobei R² die oben angegebenen Bedeutungen haben kann, umgesetzt werden. Geeignete Mittel zur Alkylierung sind zum Beispiel Alkylhalogenide (-chloride, -bromide und -iodide), -triflate, -mesylate und Dialkylsulfate.

Durch Oxidation der Thioether der Formel (Ia) nach literaturbekannten Methoden können die Sulfoxide der Formel (Ib) erhalten werden.

Die Verbindungen der Formel (X) und (XXVI) im Verfahren F lassen sich insbesondere nach den in den Herstellbeispielen genannten Bedingungen herstellen.

### Verfahren G

Die 3-substituierten 2-Arylimino-1,3-oxazole der allgemeinen Formel (I) können beispielsweise nach Verfahren G hergestellt werden. 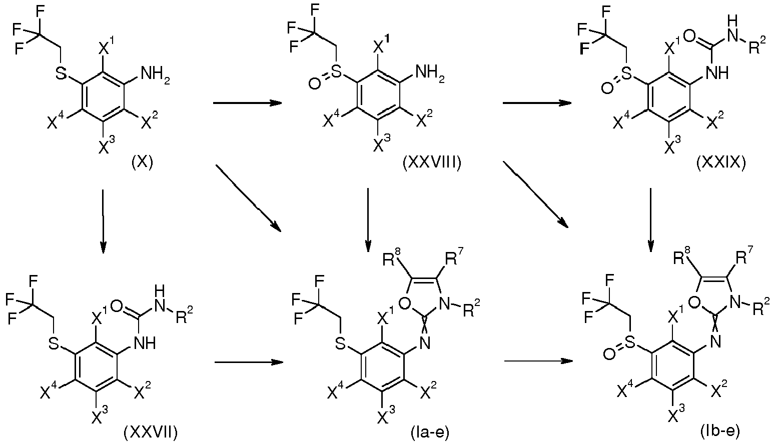 wobei X¹, X², X³, X⁴ und R² die oben angegebenen Bedeutungen haben.

R⁷ und R⁸ sind die Substituenten an dem Ring, der von R¹ und R³ zusammen mit den Atomen, an die sie gebunden sind, gebildet wird.

Aniline der Formel (XXVIII) können durch Oxidation der Aniline der Formel (X) hergestellt werden, zum Beispiel mit *meta*-Chlorbenzoesäure als Oxidationsmittel in einem inerten, organischen Lösungsmittel. Die Aniline der Formel (XXVIII) sind neu und ebenfalls Gegenstand der Erfindung.

Aniline der Formel (X) bzw. (XXVIII) können zu den Harnstoffen der Formel (XXVII) bzw. (XXIX) nach literaturbekannten Methoden umgesetzt werden, zum Beispiel nach JP2011/042611, in dem sie mit Isocyanaten gegebenenfalls in Anwesenheit einer Base und gegebenfalls in Anwesenheit eines organischen Lösungsmittels versetzt werden. Alternativ können diese Aniline nach allgemein bekannten Methoden in deren Isocyanaten umgewandelt werden und diese dann mit Aminen zu den Harnstoffen umgesetzt werden.

Aus den Harnstoffen (XXVII) bzw. (XXIX) kann die Synthese der 3-substituierten 2-Aryliminooxazole (Ia-e) bzw. (Ib-e) erfolgen, zum Beispiel nach der Methode von M. Han in Bull. Korean Chem. Soc. 2012, 33(4), 1371-1374, in dem zuerst mit Einsatz von Tetrachlorkohlenstoff und Triphenylphosphin ein *N'-*substituiertes *N*-Arylimidoformamid hergestellt wird und dieses anschließend mit einer adäquaten Hydroxycarbonylverbindung unter Ku(I)-Katalyse in einem inerten, hochsiedenden Lösungsmittel selektiv zum 2-Aryliminooxazol umgesetzt wird.

Durch Oxidation des Thioethers (Ia) nach literaturbekannten Methoden können die Sulfoxide (Ib) erhalten werden.

Auch ist die Synthese dieser 2-Aryliminooxazole (I) direkt aus den Anilinen der Formel (X) oder (XXVIII) möglich, zum Beispiel durch Umsetzung mit 3-Alkyl-1,3-oxazolinthionen der Formel (J) oder deren Oxazoliumsalzen, gegebenenfalls in Anwesenheit einer Base und gegebenenfalls in einem inerten Lösungsmittel, im Allgemeinen bei hohen Temperaturen, wie von Gompper in Chem. Ber. 1959, 92, 1928-1932 beschrieben.

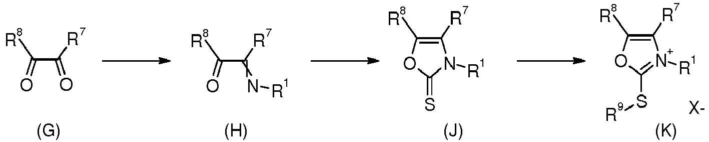

3-Substituierte 1,3-Oxazolinthione der Formel (J) können aus kommerziellen Quellen bezogen werden oder nach literaturbekannten Methoden beschrieben hergestellt werden, beispielsweise aus Dicarbonylverbindungen der Formel (G) über die Syntheseintermediate (H), in Analogie zu den Vorschriften von K. N. Mehrotra in Bull. Chem. Soc. Jap. 1985, 58 (8), 2399-2402 für R⁷=R⁸=Aryl, mit Einsatz von Schwefelkohlenstoff als Schwefel-Quelle.

Die Oxazoliiumsalze der Formel (K) können durch Alkylierung der entsprechenden Oxazolinthione der Formel (J) hergestellt werden. Geeignete Mittel zur Alkylierung sind zum Beispiel Alkylhalogenide (-chloride, -bromide und -iodide), -triflate, -mesylate und Dialkylsulfate. Die Natur des Anions X auf der allgemeinen Formel (K) wird von diesen Mittel bestimmt und kann beispielsweise Chlorid, Bromid, Iodid, Triflat, Mesylat oder Sulfat sein.

Die Verbindungen der Formel (X) und (XXVIII) im Verfahren G lassen sich insbesondere nach den in den Herstellbeispielen genannten Bedingungen herstellen.

### Verfahren H

Die 3-substituierten 2-Aryliminothiadiazole der allgemeinen Formel (I) können beispielsweise nach Verfahren H hergestellt werden. 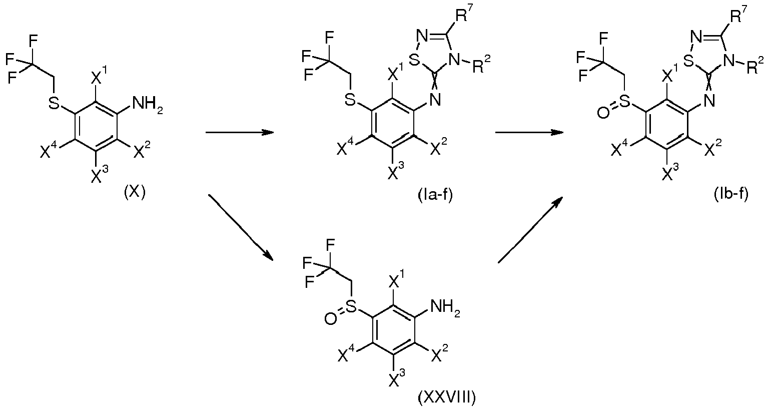 wobei X¹, X², X³, X⁴ und R² die oben angegebenen Bedeutungen haben.

R⁷ ist der Substituent an dem Ring, der von R¹ und R³ zusammen mit den Atomen, an die sie gebunden sind, gebildet wird.

Aniline der allgemeinen Formel (X) oder der allgemeinen Formel (XXVIII) können beispielsweise mit Thiadiazolinthionen der Formel (XXX) oder mit den entsprechenden Thiadiazoliumsalzen der Formel (XXXI), zu den 2-Aryliminothiadiazolen der Formel (Ia-f) bzw. (Ib-f) umgesetzt werden. Dies gelingt gegebenenfalls in Anwesenheit einer Base gegebenenfalls in einem inerten Lösungsmittel oder in einem Mittel, das gleichzeitig als Base und Lösungsmittel dienen kann, wie zum Beispiel Pyridin.

Die Synthese der Thiadiazolinthione der Formel (XXX) und deren Thiadiazoliumsalzen (XXXI) kann beispielweise nach folgendem Verfahren durchgeführt werden:

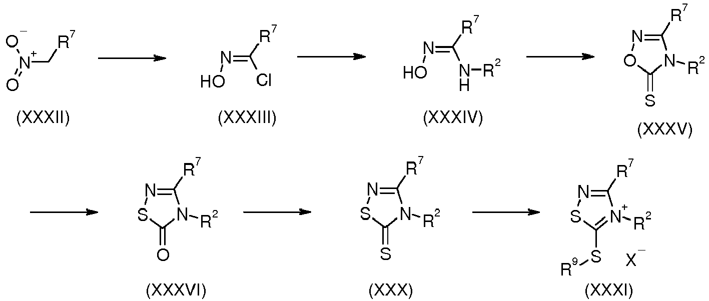

Die Synthese von *N*-substitutierten Amidoximen der Formel (XXXIV) ist gut in der Literatur beschrieben. Diese lassen sich zum Beispiel nach J. Org. Chem. 1980, 45 (21), 4198 aus den entsprechenden Imidoylchloriden der Formel (XXXIII) durch Umsetzung mit den gewünschten Aminen. Dies erfolgt gegebenenfalls in einem inerten Lösungsmittel bei Temperaturen zwischen 0 °C und 100 °C mit Reaktionszeiten von 1 h bis 36 h. Für die Herstellung der Imidoylchloride können literaturbekannten Verfahren angewandt werden, wie beispielswiese die Reaktion von den adäquaten Nitroverbindungen der Formel (XXXII) mit Alkoxylaten gegebenenfalls in dem entsprechenden Alkohol als Lösungsmittel und anschließend Versetzung mit einem Chlorierungsmittel, beispielsweise Titaniumtetrachlorid.

Zur Synthese der Oxadiazolinthionen der Formel (XXXV) sind in der Literatur eine Reihe Methoden bekannt, wie zum Beispiel die Umsetzung von 1,2,4-Oxadiazolin-5-onen mit Phosphorpentasulfid, von D. Sümengen in J. Chem. Soc. Perkin Trans 1 1983, 4, 687-691, beschrieben. Die benötigten 1,2,4-Oxadiazolin-5-one sind kommerziell erhältlich oder können aus den *N*-substituierten Amidoximen (XXXIV) durch Umsetzung mit einem geeignetem Halocarbonylderivaten erhalten werden, wie von H. Argibas in Phosp., Sulf., Silicon and rel. elem. 1998, 134/135, 381-319, beschrieben. Alternativ können die Oxadiazolinthionen direkt aus *N*-substitutierten Amidoximen der Formel (XXXIV) hergestellt werden, beispielsweise durch Umsetzung mit Thiophosgen, wie ebenfalls von D. Sümengen publiziert.

Für die Synthese der Thiadiazolinone der Formel (XXXVI) aus Oxadiazolinthionen der Formel (XXXV) lässt sich die Methode von D. Sümengen in J. Chem. Soc. Perkin Trans 1 1983, 4, 687-691 anwenden: es handelt sich um eine Umlagerung von 3,4-disubstituierten 1,2,4-Oxadiazolin-5-thionen unter Kupfer-Katalyse in einem hochsiedenden, inerten Lösungsmittel bei höher Temperatur.

Die Herstellung von Thiadiazolinthionen der Formel (XXX) kann beispielsweise aus Thiadiazolinonen der Formel (XXXVI) erfolgen, durch Versetzung mit geeigneten Schwefelungsreagenzien wie zum Beispiel mit Phosphorpentasulfid oder Lawessons Reagenz in gegebenenfalls einem inerten Lösungsmittel bei Temperaturen bis zu 140 °C.

Die Thiadiazoliumsalze der Formel (XXXI) können nach literaturbekannten Methoden durch Alkylierung der entsprechenden Thiadiazolinthione der Formel (XXX) hergestellt werden. Geeignete Mittel zur Alkylierung sind zum Beispiel Alkylhalogenide (-chloride, -bromide und -iodide), -triflate, -mesylate und Dialkylsulfate.

Die Verbindungen der Formel (XXVIII) im Verfahren H lassen sich insbesondere nach den in den Herstellbeispielen genannten Bedingungen herstellen.

### Verfahren I

Die 3-substituierten 2-Arylimino-1-3-oxazolidin-4-one (A=O, n=0), 2-Arylimino-1-3-oxazinan-4-one (A=O, n=1), 2-Arylimino-1,3-thiazolidin-4-one (A=S, n=0) und 2-Arylimino-1,3-thiazinan-4-one (A=S, n=1) der allgemeinen Formel (I) können beispielsweise nach Verfahren I hergestellt werden. 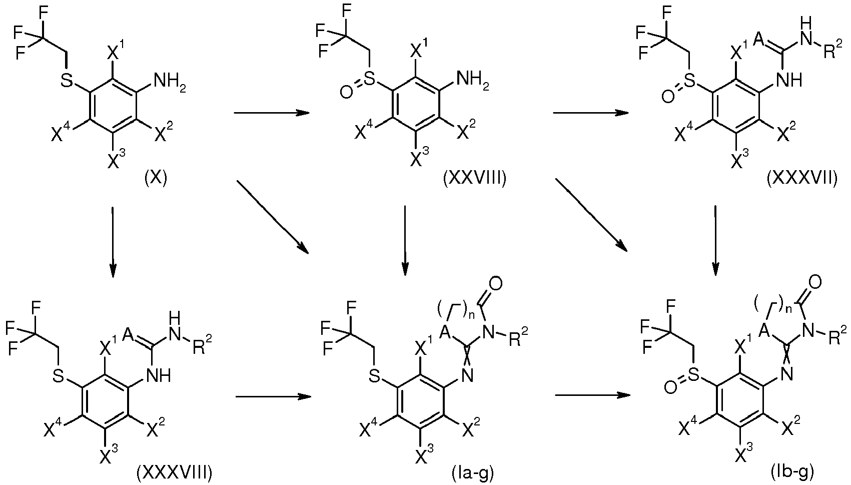 wobei X¹, X², X³, X⁴ und R² die oben angegebenen Bedeutungen haben und n kann für 0 oder 1 stehen.

Aniline der Formel (X) und (XXVIII) können zu den Hanstoffen (A=O) und Thioharnstoffen (S) der Formel (XXXVIII) bzw. (XXXVII) nach literaturbekannten Methoden umgesetzt werden, zum Beispiel nach JP2011/042611, in dem sie mit Isocyanaten (für A=O) und Isothiocyanaten (für A=S) gegebenenfalls in Anwesenheit einer Base und gegebenfalls in Anwesenheit eines organischen Lösungsmittels versetzt werden oder in dem sie nach allgemein bekannten Methoden in deren Isocyanaten (A=O) und Isothiocyanaten (A=S) umgewandelt und diese mit Aminen zu den Thioharnstoffen umgesetzt werden.

Aus den Harnstoffen (A=O) und Thioharnstoffen (A=S) der allgemeinen Formel (XXXVIII) bzw. (XXXVII) kann die Synthese der 2-Arylimino-1,3-oxazolidin-4-one (A=O) und -thiazolidin-4-one (A=S) der allgemeinen Formel (Ia-g) bzw. (Ib-g) erfolgen, zum Beispiel durch Cycloacylierung mit einem adäquaten Halocarbonylderivaten in einem inerten Lösungsmittel, meistens bei Temperaturen höher als 100 °C. Geeignete Halocarbonylderivaten sind zum Beispiel für n=0 Chloressigsäure und deren Säurechlorid, Bromessigsäure und deren Säurechlorid oder -bromid; für n=1 werden in der Literatur 3-substituierte 2-Propenoylchloride genannt, wie von C. F. Howell in J. Org. Chem. 1962, 27, 1686 und 1691 für analogue Umsetzungen zu Oxazolidinonen und V.N.Britsun in Russ. J. Org. Chem. 2006, 41 (11), 1719-1729 für Thiazolidinonen beschrieben.

Die Verbindungen der Formel (XXVIII), (XXXVIII) und (XXXVII) im Verfahren I lassen sich insbesondere nach den in den Herstellbeispielen genannten Bedingungen herstellen.

### Verfahren J

Die 3-substituierten 2-Arylimino-1,3-thiazole der allgemeinen Formel (I) können beispielsweise nach Verfahren J hergestellt werden. 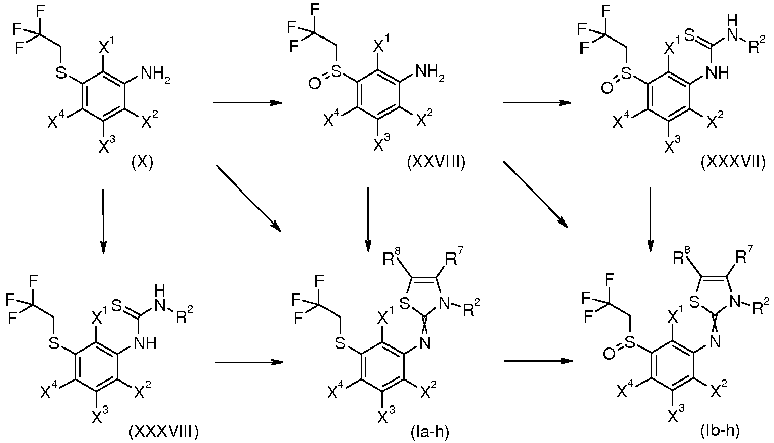 wobei X¹, X², X³, X⁴ und R² die oben angegebenen Bedeutungen haben.

R⁷ und R⁸ sind die Substituenten an dem Ring, der von R¹ und R³ zusammen mit den Atomen, an die sie gebunden sind, gebildet wird.

Aus den Thioharnstoffen (XXXVIII) bzw. (XXXVII) kann die Synthese der 3-substituierten 2-Aryliminothiazole (Ia-h) bzw. (Ib-h) erfolgen, zum Beispiel durch Reaktion mit einem adäquaten Halocarbonylderivaten in einem inerten Lösungsmittel. Dies kann zum Beispiel durch Cycloacylierung mit α -Haloketoverbindungen oder deren Derivaten, beispielsweise mit 3-Brom-2-butanon für R⁷=R⁸=Methyl, geschehen, wie vom A. Yahyazadeh in J. Pharm. Res. 1998, 9, 536-537(S), 2126-2139(M), oder mit 2-Chloro-1,1-diethoxyethan für R⁷=R⁸=H wie im Patent US4079144 beschrieben.

Durch Oxidation des Thioethers (Ia) nach literaturbekannten Methoden können die Sulfoxide (Ib) erhalten werden. Hierfür sind bekannte Methoden der enantioselektiven Anreicherung der Sulfoxide anwendbar.

Auch ist die Synthese dieser 2-Arylimino-1,3-thiazole (I) direkt aus den Anilinen der Formel (X) oder (XXVIII) möglich, zum Beispiel durch Umsetzung mit 3-Alkyl-1,3-thiazolinthione der Formel (A) oder deren Thiazoliniumsalzen (B), gegebenenfalls in Anwesenheit einer Base und gegebenenfalls in einem inerten Lösungsmittel.

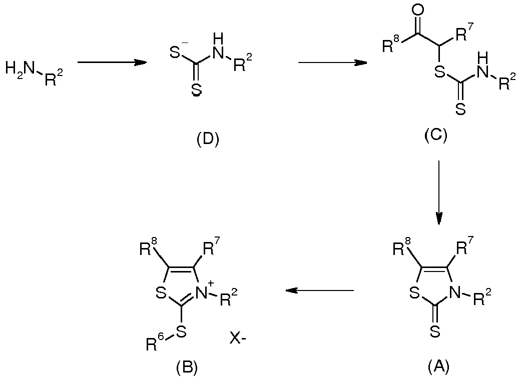

3-Substituierte 1,3-thiazolinthione der Formel (A) können aus kommerziellen Quellen bezogen werden oder nach literaturbekannten Methoden wie zum Beispiel von K. Janikowska in Phosp. Sulf. Sil. and rel.elem. 2011, 186(1), 12-20 beschrieben hergestellt werden. Wie auch im J. Amer. Chem. Soc. 1987, 109 (2), 492-507, lassen sich aus Aminen durch Umsetzung mit Schwefelkohlenstoff Dithiocarbamate der Formel (D) erzielen, die dann mit einem adäquaten Halocarbonylsynthon zu alkylierten Verbindungen der Formel (C) führen. Geeignete Halocarbonylverbindungen sind zum Beispiel Chlorethanal oder 2-Chloro-1,1-diethoxyethan für R⁷=R⁸=H, Chloropropanon für R⁷=H und R⁸=Methyl, 3-Brom-2-butanon für R⁷=R⁸=Methyl, 2-Brom-3-pentanon für R⁷=Methyl und R⁸=Ethyl, etc. Die Verbindungen der Formel (C) können entweder spontan zu den gewünschten Thiazolinthionen (A) cyclisieren oder erst nach Dehydratisierung, meistens unter sauren Bedingungen und Hitzeeinwirkung.

Die Thiazoliniumsalze der Formel (B) können durch Alkylierung der entsprechenden Thiadiazolinthione der Formel (B) hergestellt werden. Geeignete Mittel zur Alkylierung sind zum Beispiel Alkylhalogenide (-chloride, -bromide und -iodide), -triflate, -mesylate und Dialkylsulfate.

Die Verbindungen der Formel (XXVIII), (XXXVII) und (XXXVIII) im Verfahren J lassen sich insbesondere nach den in den Herstellbeispielen genannten Bedingungen herstellen.

### Verfahren K

Die 3-substituierten 2-Aryliminooxadiazole der allgemeinen Formel (I) können beispielsweise nach Verfahren K hergestellt werden. 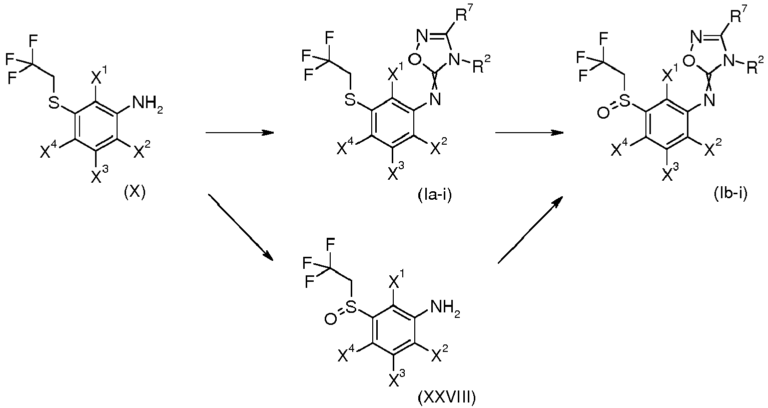 wobei X¹, X², X³, X⁴ und R² die oben angegebenen Bedeutungen haben.

R⁷ ist der Substituent an dem Ring, der von R¹ und R³ zusammen mit den Atomen, an die sie gebunden sind, gebildet wird.

Aniline der allgemeinen Formel (X) oder der allgemeinen Formel (XXVIII) können beispielsweise mit Oxadiazolinthionen der Formel (XXXV) oder mit den entsprechenden Oxadiazoliumsalzen der Formel (XXXIX) zu den 2-Aryliminooxadiazolen der Formel (Ia-i) bzw. (Ib-i) umgesetzt werden. Dies gelingt gegebenenfalls in Anwesenheit einer Base gegebenenfalls in einem inerten Lösungsmittel oder in einem Mittel, das beide Rollen annehmen kann, wie zum Beispiel Pyridin.

Die Synthese der Oxadiazolinthione der Formel (XXXV) und deren Oxadiazoliumsalze (XXXIX) kann beispielweise nach folgendem Verfahren durchgeführt werden:

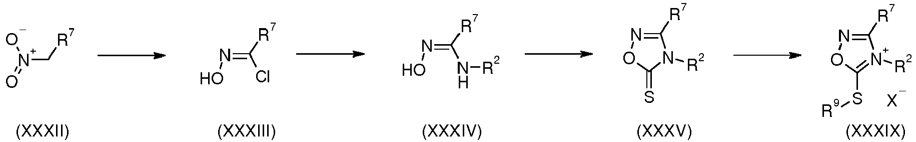

Die Oxadiazoliumsalze der Formel (XXXIX) können nach literaturbekannten Methoden durch Alkylierung der entsprechenden Oxadiazolinthione der Formel (XXXV) hergestellt werden. Geeignete Mittel zur Alkylierung sind zum Beispiel Alkylhalogenide (-chloride, -bromide und -iodide), -triflate, -mesylate und Dialkylsulfate.

Die Verbindungen der Formel (XXVIII) im Verfahren K lassen sich insbesondere nach den in den Herstellbeispielen genannten Bedingungen herstellen.

Alle diese Verfahren führen zu erfindungsgemäßen Verbindungen der allgemeinen Formel (I).

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphorenthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiologika, Düngemittel, Lockstoffen, Phytotonics, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Kombinationen das Pflanzenwachstum verbessern, die Toleranz gegenüber abiotischen Faktoren wie z. B. hohen oder niedrigen Temperaturen, gegen Trockenheit oder gegen erhöhten Wasser- bzw. Bodensalzgehalt. Auch lässt sich das Blüh- und Fruchtverhalten verbessern, die Keimfähigkeit und Bewurzelung optimieren die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl in Vor-, Tank- oder Fertigmischungen als auch in Saatgutanwendungen verwendet werden.

Besonders günstige Mischungspartner sind z.B. die folgenden:

### Insektizide/Akarizide/Nematizide

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemetonmethyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
   Cyclodien-organochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin.
(5) Nikotinerge Acetylcholin-Rezeptor (nAChR) allosterische Aktivatoren, wie beispielsweise
   Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder
   Fenoxycarb; oder Pyriproxyfen.
(8) Wirkstoffe mit unbekannten oder nicht spezifischen Wirkmechanismen, wie beispielsweise
   Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
   Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder Organozinnverbindungen, z.B. Azocyclotin, Cyhexatin und Fenbutatin-oxid; oder
   Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartap-hydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
   Tetron- und Tetramsäurederivate, z.B. Spirodiclofen, Spiromesifen und Spirotetramat.
(24) Komplex-IV-Elektronentransportinhibitoren, wie beispielsweise
   Phosphine, z.B. Aluminiumphosphid, Calciumphosphid, Phosphin und Zinkphosphid; oder
   Cyanid.
(25) Komplex-II-Elektronentransportinhibitoren, wie beispielsweise Cyenopyrafen.
(28) Ryanodinrezeptor-Effektoren, wie beispielsweise
   Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:

3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-l-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (lE)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925) und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233).

### Fungizide

(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazol-p (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).
(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0) (bekannt aus WO2010025451), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2) (bekannt aus WO 2004/058723), (3.9) Famoxadon (131807-57-3) (bekannt aus WO 2004/058723), (3.10) Fenamidon (161326-34-7) (bekannt aus WO 2004/058723), (3.11) Fenoxystrobin (918162-02-4), (3.12) Fluoxastrobin (361377-29-9) (bekannt aus WO 2004/058723), (3.13) Kresoxim-Methyl (143390-89-0) (bekannt aus WO 2004/058723), (3.14) Metominostrobin (133408-50-1) (bekannt aus WO 2004/058723), (3.15) Orysastrobin (189892-69-1) (bekannt aus WO 2004/058723), (3.16) Picoxystrobin (117428-22-5) (bekannt aus WO 2004/058723), (3.17) Pyraclostrobin (175013-18-0) (bekannt aus WO 2004/058723), (3.18) Pyrametostrobin (915410-70-7) (bekannt aus WO 2004/058723), (3.19) Pyraoxystrobin (862588-11-2) (bekannt aus WO 2004/058723), (3.20) Pyribencarb (799247-52-2) (bekannt aus WO 2004/058723), (3.21) Triclopyricarb (902760-40-1), (3.22) Trifloxystrobin (141517-21-7) (bekannt aus WO 2004/058723), (3.23) (2E)-2-(2-{[6-(3-Chlor-2-methylphenoxy)-5-fluorpyrimidin-4-yl]oxy}phenyl)-2-(methoxyimino)-N-methylethanamid (bekannt aus WO 2004/058723), (3.24) (2E)-2-(Methoxyimino)-N-methyl-2-(2-{[({(1E)-1-[3-(trifluormethyl)phenyl]ethyliden}amino)oxy]methyl}phenyl)ethanamid (bekannt aus WO 2004/058723), (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).
(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).
(5) Verbindungen mit Multisite-Aktivität, wie beispielsweise (5.1) Bordeauxmischung (8011-63-0), (5.2) Captafol (2425-06-1), (5.3) Captan (133-06-2) (bekannt aus WO 02/12172), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).
(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).
(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7) (bekannt aus WO2005070917).
(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).
(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).
(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).
(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6) (bekannt aus WO2005042474).
(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).
(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).
(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).
(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9) (bekannt aus EP-A 1 559 320), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2) (bekannt aus WO2003035617), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0) (bekannt aus WO2005070917), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4) (bekannt aus WO2009094442), (15.75) 5-Fluor-2-[(4-methylbenzyl)oxy]pyrimidin-4-amin (1174376-25-0) (bekannt aus WO2009094442), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl} -2-phenylacetamid (221201-92-9), (15.86) N'- {4-[(3-Tert-butyl-4-cyano-1 ,2-thiazol-5-yl)oxy] -2-chlor-5-methylphenyl} -N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid (bekannt aus EP-A 1 559 320), (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N-[4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken.

### Herstellbeispiel 1:2,2,2-Trifluor-N'-{2-fluor-4-methyl-5-[(2,2,2-trifluorethyl)sulfinyl]phenyl}-N,N-dimethylethanimidamid (Ib-01)

### Stufe 1: 2,2,2-Trifluor-N-(2-fluor-4-methylphenyl)acetamid (VI-1)

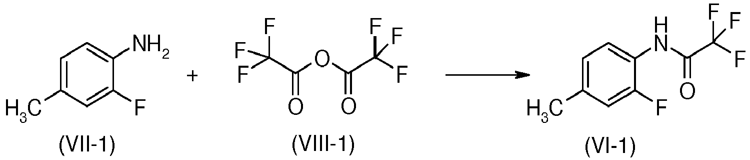

27,5 g 2-Fluor-4-Methylanilin werden bei 0 °C in 300 mL Dichlormethan vorgelegt, 26,7 g Triethylamin werden zugegeben und anschließend werden 50,8 g Trifluoressigsäureanhydrid zugetropft. Es wird 2 h bei 0 °C nachgerührt und anschließend einrotiert. Der Rückstand wird in Wasser aufgenommen und zweimal mit Ethylacetat extrahiert. Die vereinigten organischen Phasen werden über Natriumsulfat getrocknet, filtriert, und das Lösemittel wird im Vakuum entfernt. Es werden 49,0 g (100% d.Th.) des Trifluoracetamids (VI-1) erhalten.
logP(HCOOH): 2,40

### Stufe 2: 4-Fluor-2-methyl-5-[(trifluoracetyl)amino]benzolsulfonylchlorid (V-1)

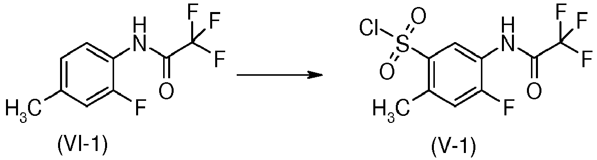

258 g Chlorsulfonsäure werden vorgelegt und bei Raumtemperatur werden 49 g 2,2,2-Trifluor-N-(2-fluor-4-methylphenyl)acetamid (VI-1) in Portionen zugesetzt. Bei Raumtemperatur wird noch 16 h nachgerührt. Die Mischung wird unter Rühren auf Eis gegeben und zweimal mit Ethylacetat extrahiert. Die vereinigten organischen Phasen werden über Natriumsulfat getrocknet, filtriert, und das Lösemittel wird im Vakuum entfernt. Es werden 70,8 g des Chlorsulfonyls (V-1) erhalten. Das Rohprodukt wird sofort weiter umgesetzt.

### Stufe 3: N,N'-[Disulfanediylbis(6-fluor-4-methylbenzol-3,1-diyl)]bis(2,2,2-trifluoracetamid) (IV-1)

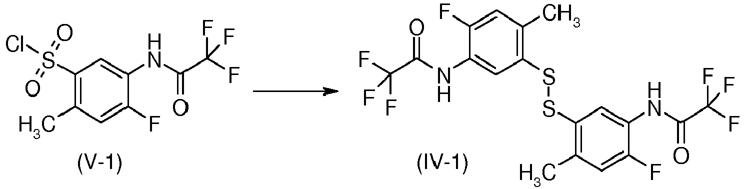

298,8 g Natriumiodid werden in 1000 mL Trifluoressigsäure gelöst und 70,8 g 4-Fluor-2-methyl-5-[(trifluoracetyl)amino]benzolsulfonylchlorid (V-1) werden bei Raumtemperatur zugegeben. Es wird 16 h bei Raumtemperatur nachgerührt und anschließend das Lösemittel im Vakuum entfernt. Der Rückstand wird mit Wasser verrührt und abgesaugt. Es werden 62,3 g (86% d.Th.) des Disufids (IV-1) als Feststoff erhalten.
logP(HCOOH): 4,41

### Stufe 4: 2,2,2-Trifluor-N-{2-fluor-4-methyl-5-[(2,2,2-trifluorethyl)sulfanyl]phenyl}acetamid (III-1)

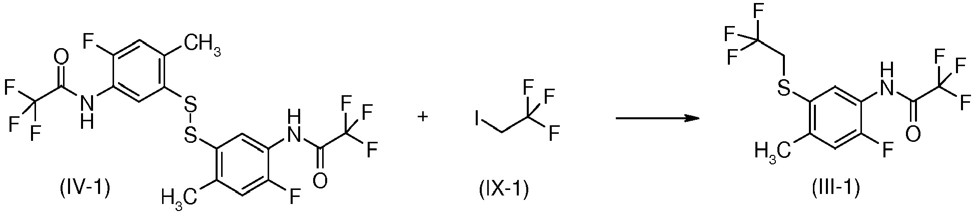

3,4 g N,N'-[Disulfanediylbis(6-fluor-4-methylbenzol-3,1-diyl)]bis(2,2,2-trifluoracetamid) (IV-1) werden in 150 ml N,N-Dimethylformamid gelöst und mit 1,86 g Kaliumcarbonat, 3,11 g 1,1,1-Trifluoriodethan (XVI-1), 2,39 g Rongalit und einigen Tropfen Wasser versetzt. Die Reaktionsmischung wird 16 h bei Raumtemperatur gerührt. Die Hauptmenge des N,N-Dimethylformamids wird im Vakuum abdestilliert. Der Rückstand wird in Wasser aufgenommen und zweimal mit Ethylacetat extrahiert. Die vereinigten organischen Phasen werden über Natriumsulfat getrocknet, filtriert, und anschließend wird das Lösemittel im Vakuum entfernt. Es werden 4,48 g (90% d.Th.) des Thioethers (III-1) erhalten.
logP(HCOOH): 3,31

### Stufe 5: 2,2,2-Trifluor-N-{2-fluor-4-methyl-5-[(2,2,2-trifluorethyl)sulfanyl]phenyl} ethanimidoylchlorid (II-1)

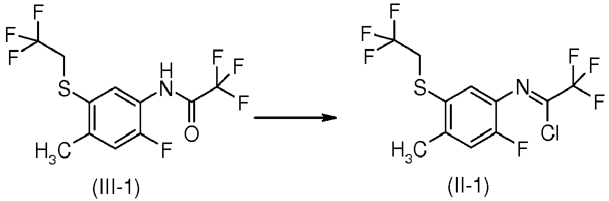

1 g 2,2,2-Trifluor-N-{2-fluor-4-methyl-5-[(2,2,2-trifluorethyl)sulfanyl]phenyl}acetamid (III-1), 1,51 g Triethylamin und 4,01 g Diphenylchlorphosphat werden in 20 mL Acetonitril 16 h refluxiert. Nach Abkühlen wird Ethylacetat zugegeben, der ausgefallene Feststoff abfiltriert und verworfen. Das Filtrat wird auf Kieselgel gezogen und mit Cyclohexan/Ethylacetat (98/2) chromatographiert. Nach Entfernen des Lösemittels im Vakuum erhält man 1 g des Imidoylchlorids (II-1).

### Stufe 6: 2,2,2-Trifluor-N'-{2-fluor-4-methyl-5-[(2,2,2-trifluorethyl)sulfanyl]phenyl}-N,N-dimethylethanimidamid (Ia-01)

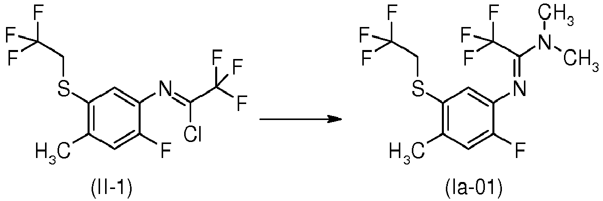

0,77 g Dimethylamin (2 M in THF) werden in 40 ml Acetonitril vorgelegt, und bei Raumtemperatur wird 1 g 2,2,2-Trifluor-N-{2-fluor-4-methyl-5-[(2,2,2-trifluorethyl)sulfanyl]phenyl} ethanimidoylchlorid (II-1) gelöst in 10 mL Acetonitril zugetropft. Die Reaktionsmischung wird 16 h bei Raumtemperatur gerührt und anschließend wird das Lösemittel im Vakuum entfernt. Der Rückstand wird in Wasser aufgenommen und zweimal mit Ethylacetat extrahiert. Die vereinigten organischen Phasen werden über Natriumsulfat getrocknet, filtriert und anschließend wird das Lösemittel im Vakuum entfernt. Es werden 0,36 g (35% d.Th.) des Amidins (Ia-01) erhalten.
logP(HCOOH): 4,48

### Stufe 7: 2,2,2-Trifluor-N'-{2-fluor-4-methyl-5-[(2,2,2-trifluorethyl)sulfinyl]phenyl}-N,N-dimethylethanimidamid (Ib-01)

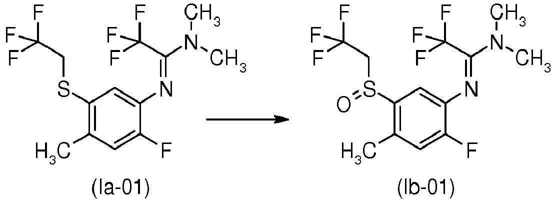

0,36 g 2,2,2-Trifluor-N'-{2-fluor-4-methyl-5-[(2,2,2-trifluorethyl)sulfanyl]phenyl}-N,N-dimethylethanimidamid (Ia-01) werden in 30 mL Dichlormethan gelöst und bei Raumtemperatur werden 0,21 g meta-Chlorperbenzoesäure zugegeben. Die Reaktionsmischung wird 16 h bei Raumtemperatur nachgerührt und anschließend mit Natriumcarbonatlösung alkalisch gestellt. Überschüssige meta-Chlorperbenzoesäure wird mit Natriumthiosulfat reduziert. Nach Phasentrennung wird das Lösemittel im Vakuum entfernt. Der Rückstand wird mit Cyclohexan/Aceton (9/1) chromatographiert. 0,31 g (79% d.Th) des Amidins (Ib-01) werden so erhalten.
logP(HCOOH): 3,15

### Herstellbeispiel 2: 5-Amino-4-fluor-2-methylbenzolthiol (XI-1)

### Stufe 1: S-(5-Acetamido-4-fluor-2-methylphenyl)ethanthioat (XII-1)

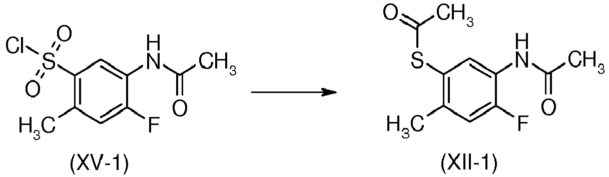

99,3 g 5-Acetamido-4-fluor-2-methylbenzolsulfonylchlorid (XV-1) werden in 700 mL Eisessig suspendiert, mit 0,9 g Iod und 38,7 g rotem Phosphor versetzt und 5 h bei Rückfluß nachgerührt. Nach Abkühlen wird der Feststoff abfiltriert und das Filtrat einrotiert. Der Rückstand wird mit Wasser verrührt und abgesaugt. 57,6 g (67% d.Th.) des Thioats (XII-1) werden als Feststoff erhalten.
logP(HCOOH): 1,78

### Stufe 2: 5-Amino-4-fluor-2-methylbenzolthiol (XI-1)

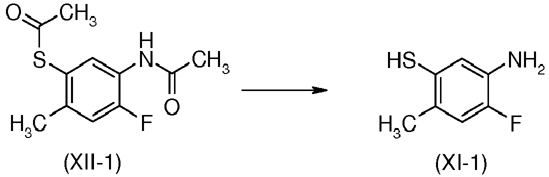

57,4 g S-(5-Acetamido-4-fluor-2-methylphenyl)ethanthioat (XII-1) werden in 750 mL Wasser und 96,6 g Kaluimhydroxid gelöst. Die Reaktionsmischung wird 16 h bei Rückfluß gekocht. Nach Abkühlen wird die Lösung mit Salzsäure auf pH 2-3 gestellt und der ausgefallene Feststoff abgesaugt. 35,8 g (94% d.Th.) des Thiols (XI-1) werden als Feststoff erhalten.
logP(HCOOH): 3,70

### Chirale Oxidation von (III) zu (XVII)

### Beispiel 1: Synthese von 2,2,2-Trifluor-N-{4-fluor-2-methyl-5-[(2,2,2-trifluorethyl)sulfinyl]-phenyl} acetamid

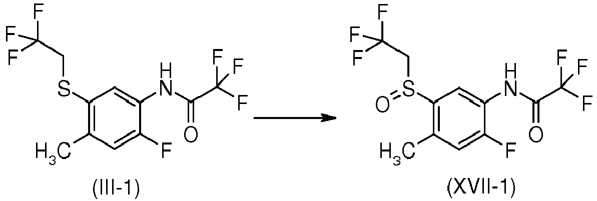

In einem Dreihalskolben wurden 500 mg (1,49 mmol) 2,2,2-Trifluor-N-{4-fluor-2-methyl-5-[(2,2,2-trifluorethyl)sulfanyl]phenyl}acetamid in 5 g Chloroform gelöst und auf 15 °C abgekühlt. Zu dieser Mischung wurde eine Lösung aus 15,82 mg (0,06 mmol) Vanadiumacetylacetonat und 29,84 mg (0,089 mmol) (S)(2,4-Di-*tert*.-butyl-6-{(E)-[(1-hydroxy-3,3-dimethylbutan-2-yl)imino]methyl}phenol 1 g Chloroform zugesetzt. Nach 5 Minuten wurde eine Lösung aus 225,5 mg (1,79 mmol) 30%iger H₂O₂ und 300 mg Puffer-Lösung pH 7 (KH₂PO₄/Na₂HPO₄) über 20 Minuten zudosiert. Der Verlauf der Umsetzung wurde mittels HPLC verfolgt. Nach 2 h Reaktionszeit wurden 100 mg Thiosulfatlösung zugesetzt und Chloroform weitgehend im Vakuum verdampft. Zu dem Rückstand wurden 5 g Cyclohexan zugegeben und der ausgefallene Feststoff wurde abfiltriert.

Man erhielt 1-{2,4-Dimethyl-5-[(2,2,2-trifluorethyl)-sulfinyl]phenyl}-3-(trifluormethyl)-1H-1,2,4-triazol (91,24% HPLC-Reinheit) als beigen Feststoff. Der Enantiomerenüberschuss wurde mittels HPLC an chiraler Phase (Daicel Chiracel OJ-RH 150) mit einem Verhältnis von 25,90 : 74,10 bestimmt.

### Chirale Oxidation von (Ia) zu (Ib)

### Beispiel 1: Synthese von N'-{2,4-Dichlor-5-[(2,2,2-trifluorethyl)sulfinyl]phenyl}-2,2,2-trifluorethanimidamid

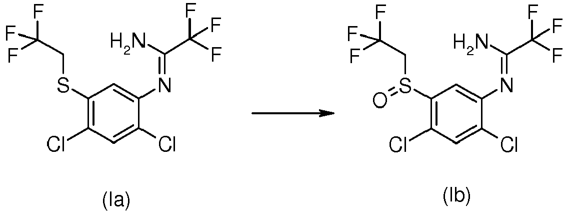

In einem Dreihalskolben wurden 1 g (2,69 mmol) N'-{2,4-Dichlor-5-[(2,2,2-trifluorethyl)sulfanyl]phenyl}-2,2,2-trifluorethanimidamid in 10 mL Chloroform gelöst und auf 15 °C abgekühlt. Zu dieser Mischung wurde eine Lösung aus 29 mg (0,10 mmol) Vanadiumacetylacetonat und 54 mg (0,16 mmol) (S)(2,4-Di-*tert*.-butyl-6-{(E)-[(1-hydroxy-3,3-dimethylbutan-2-yl)imino]methyl}phenol in 2 mL Chloroform zugesetzt. Eine Lösung aus 367 mg (3,23 mmol) 30%iger H₂O₂ und 750 mg Puffer-Lösung pH 7 (KH₂PO₄/Na₂HPO₄) wurde über 4 Stunden zudosiert. Der Verlauf der Umsetzung wurde mittels DC verfolgt. Nach 2 h Reaktionszeit wurden 128 mg Thiosulfatlösung (1M) zugesetzt und über Nacht gerührt. Die Phasen wurden getrennt und Chloroform weitgehend (bis auf ca. 3 mL) im Vakuum verdampft. Zu dem Rückstand wurde Cyclohexan zugegeben und der ausgefallene Feststoff wurde abfiltriert.

Man erhielt 980 mg N'-{2,4-Dichlor-5-[(2,2,2-trifluorethyl)sulfinyl]phenyl}-2,2,2-trifluorethanimidamid (>99% HPLC-Reinheit) als Feststoff. Der Enantiomerenüberschuss wurde mittels HPLC an chiraler Phase (Chiracel OD-RH 150) mit einem Verhältnis von 89,63 : 10,37 bestimmt.

### Herstellbeispiel 3: N'-{4-Chlor-3-[(2,2,2-trifluorethyl)sulfinyl]phenyl}-2,2,2-trifluorethanimidamid (Ib-108)

### Stufe 1: 1,1'-Disulfanediylbis(2-chlor-5-nitrobenzol)

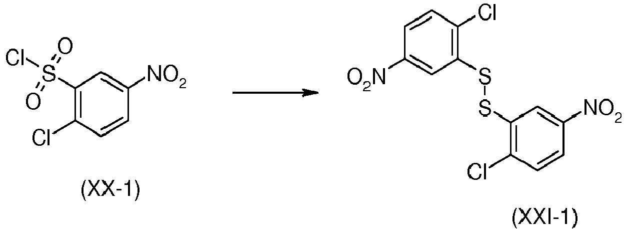

52,0 g (203,1 mmol) 2-Chlor-5-nitrobenzolsulfonylchlorid werden unter starkem Rühren mit 236,1 g (1,02 mol) Chlorsulfonsäure versetzt und über Nacht bei Raumtemperatur gerührt. Nach Zugabe von 40%iger wässriger Natriumhydrogensulfit-Lösung wird der gebildete Feststoff abgesaugt, mit Wasser gewaschen und über Nacht auf einer Tonplatte getrocknet. Es werden 36,1 g (100% Reinheit, 94% d.Th.) der Titelverbindung als graubrauner Feststoff erhalten.
logP(HCOOH): 5,03; logP(neutral): 5,01; 1H-NMR (D6-DMSO, 400MHz) δ ppm 8,40(d,2H), 8,18-8,16(m,2H), 7,91(d,2H) ; GC-MS: EI-Masse (m/z): 376 (2Cl) [M]+

### Stufe 2: 3,3'-Disulfanediylbis(4-chloranilin)

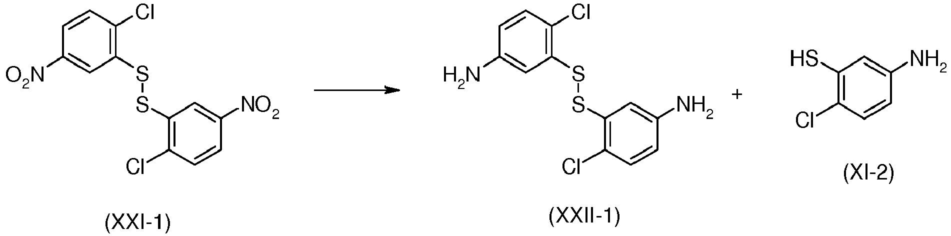

8,00 g (21,2 mmol) 1,1'-Disulfanediylbis(2-chlor-5-nitrobenzol) werden in 150 mL THF gelöst, mit 1,6 g Raney-Nickel versetzt und 72 h bei 50 °C unter Wasserstoffatmosphäre (20 bar) gerührt. Die Reaktionsmischung wird mit THF über Kieselgur filtriert, das Filtrat wird unter vermindertem Druck vom Lösungsmittel berfreit. Es werden 6,64 g (90% Reinheit, 89% der Theorie) eines Gemisches aus 1,1'-Disulfanediylbis(2-chlor-5-nitrobenzol) und 5-Amino-2-chlorbenzolthiol erhalten, das ohne weitere Aufreinigung alkyliert wird.

### 1,1'-Disulfanediylbis(2-chlor-5-nitrobenzol):

logP(HCOOH): 3,31; logP(neutral): 3,35; 1H-NMR (D6-DMSO, 400MHz) δ ppm 7,10(d,2H), 6,73(d,2H), 6,47-6,44(m,2H), 5,51(breit,4H); GC-MS: EI-Masse (m/z): 316 (2Cl) [M]+

### 5-Amino-2-chlorbenzolthiol:

logP(HCOOH): 1,64; logP(neutral): nicht messbar; 1H-NMR (D6-DMSO, 400MHz) δ ppm 7,01(d,1H), 6,54(d,1H), 6,35-6,32(m,1H), 5,28(breit,3H); GC-MS: EI-Masse (m/z): 159 (1Cl) [M]+

### Stufe 3: 4-Chlor-3-[(2,2,2-trifluorethyl)sulfanyl]anilin

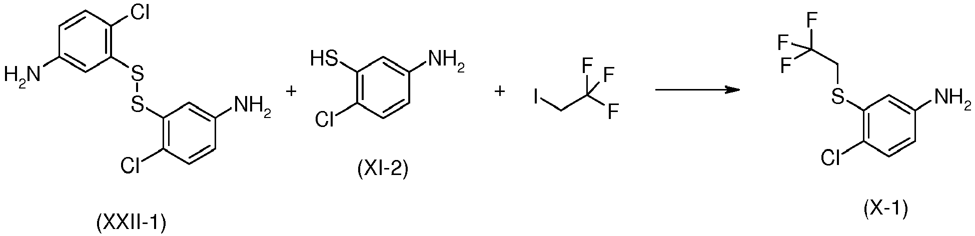

6,40 g eines Gemisches aus Disulfanediylbis(2-chlor-5-nitrobenzol) und 5-Amino-2-chlorbenzolthiol (ca. 20 mmol) werden in 100 mL N,N-Dimethylformamid vorgelegt und mit 7,02 g (40,3 mmol) Natriumdithionit, 5,58 g (40,3 mmol) Kaliumcarbonat und 5,49 g (40,3 mmol) Rongalit versetzt und auf 0 °C abgekühlt. 9,32 g 1,1,1-Trifluor-2-iodethan werden bei 0 °C zugetropft. Die Reaktionsmischung wird über Nacht bei Raumtemperatur gerührt. Ein Großteil des Lösungsmittels wird unter vermindertem Druck entfernt, der Rückstand wird mit Wasser versetzt und mit Essigester extrahiert. Die vereinigten organischen Phasen werden sukzessive mit Wasser, gesättigter wässriger Natriumchlorid-Lösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 4,70 g (98% Reinheit, 47% der Theorie) der Titelverbindung als gelbe Flüssigkeit.
logP(HCOOH): 2,64; logP(neutral): 2,69; 1H-NMR (D6-DMSO, 400MHz) δ ppm 7,09(d,1H), 6,78(d,1H), 6,49-6,46(m,1H), 5,37(breit,2H), 3,90(q,2H) ; GC-MS: EI-Masse (m/z): 241 (1Cl) [M]+

### Stufe 4: N-{4-Chlor-3-[(2,2,2-trifluorethyl)sulfanyl]phenyl}-2,2,2-trifluoracetamid

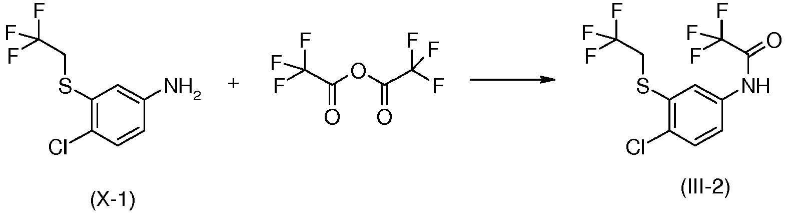

1,00 g (4,14 mmol) 4-Chlor-3-[(2,2,2-trifluorethyl)sulfanyl]anilin werden in 14 mL Dichlormethan vorgelegt und bei 0 °C mit 0,50 g (4,97 mmol) Triethylamin versetzt. 0,96 g (4,55 mmol) Trifluoressigsäureanhydrid werden bei 0 °C zugetropft. Die Reaktionsmischung wird über Nacht bei Raumtemperatur gerührt, dann mit Wasser und gesättigter wässriger Natriumchlorid-Lösung gewaschen, über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 1,00 g (99% Reinheit, 71% der Theorie) der Titelverbindung als farblosen Feststoff.
logP(HCOOH): 3,46; logP(neutral): 3,41; 1H-NMR (D6-DMSO, 400MHz) δ ppm 11,43(s,1H), 7,91(d,1H), 7,63-7,69(m,1H), 7,56-7,58(m,1H), 4,05(q,2H); 1H-NMR (CDCl₃, 400MHz) δ ppm 7,85(breit,1H), 7,82(d,1H), 7,52-7,45(m,2H), 3,53(q,2H); GC-MS: EI-Masse (m/z): 337 (1Cl) [M]+

### Stufe 5: N-{4-Chlor-3-[(2,2,2-trifluorethyl)sulfanyl]phenyl}-2,2,2-trifluorethanimidoylchlorid

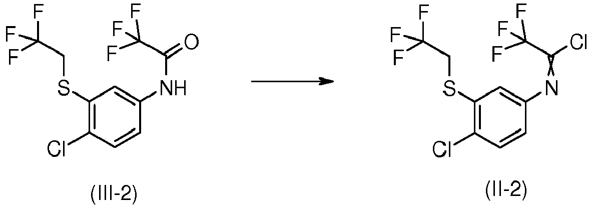

880 mg (2,61 mmol) N-{4-Chlor-3-[(2,2,2-trifluorethyl)sulfanyl]phenyl}-2,2,2-trifluoracetamid werden in 17 mL Acetonitril vorgelegt, bei Raumtemperatur mit 1,32 g (13,03 mmol) Triethylamin und 3,50 g (13,03 mmol) Diphenylchlorphosphat versetzt und über Nacht zum Rückfluss erhitzt. Nach Abkühlen wird Essigester zugegeben, filtriert und konzentriert. Der Rückstand wird unmittelbar einer säulenchromatographischen Reinigung an Kieselgel mittels MPLC mit Cyclohexan/Essigester als Laufmittel unterzogen. Man erhält 640 mg (93% Reinheit, 64% der Theorie) der Titelverbindung als gelbes Öl,
logP(HCOOH): 4,77; logP(neutral): 4,73; 1H-NMR (CDCl₃, 400MHz) δ ppm 7,51(d,1H), 7,24(d,1H), 7,02-7,00(m,1H), 3,51(q,2H); GC-MS: EI-Masse (m/z): 355 (2Cl) [M]+

### Stufe 6: N'-{4-Chlor-3-[(2,2,2-trifluorethyl)sulfanyl]phenyl}-2,2,2-trifluorethanimidamid (Ia-204)

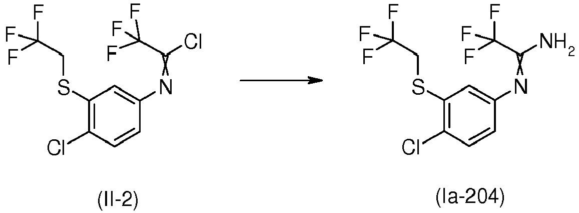

210 mg (0,59 mmol) N-{4-Chlor-3-[(2,2,2-trifluorethyl)sulfanyl]phenyl}-2,2,2-trifluorethanimidoylchlorid werden in 5 mL Acetonitril vorgelegt und mit einer Lösung aus 241 mg (3,54 mmol) 25%iger wässriger Ammoniaklösung in 5 mL Acetonitril versetzt. Die Reaktionsmischung wird über Nacht bei Raumtemperatur gerührt, dann konzentriert. Der Rückstand wird mit Wasser und Essigester extrahiert. Die vereinigten organischen Phasen werden über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 120 mg (100% Reinheit, 60% der Theorie) der Titelverbindung als gelbes Öl.
logP(HCOOH): 3,10; logP(neutral): 3,10; 1H-NMR (D6-DMSO, 400MHz) δ ppm 7,42(d,1H), 7,26(breit,2H), 7,05(d,1H), 6,75-6,72(m,1H), 4,13(q,2H)

### Stufe 7: N'-{4-Chlor-3-[(2,2,2-trifluorethyl)sulfinyl]phenyl}-2,2,2-trifluorethanimidamid (Ib-108)

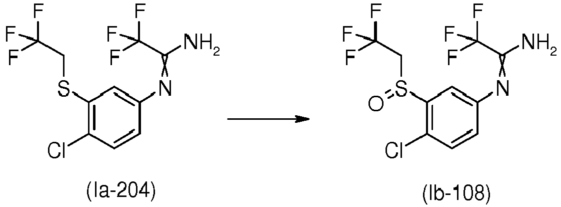

90 mg (0,27 mmol) N'-{4-Chlor-3-[(2,2,2-trifluorethyl)sulfanyl]phenyl}-2,2,2-trifluorethanimidamid werden in 6 mL Dichlormethan gelöst und bei 0 °C mit 68 mg (0,29 mmol) meta-Chlorperbenzoesäure versetzt. Nach zweistündigem Rühren bei 0 °C werden erneut 23 mg (0,13 mmol) zugegeben. Die Reaktionsmischung wird über Nacht bei Raumtemperatur gerührt und dann mit Dichlormethan verdünnt und sukzessive mit 40%iger Natriumhydrogensulfit-Lösung und gesättigter wässriger Natrumhydrogencarbonat gewaschen. Die organische Phase wird über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 90 mg (95% Reinheit, 91% der Theorie) der Titelverbindung als beigen Feststoff.
logP(HCOOH): 2,25; logP(neutral): 2,21; 1H-NMR (D6-DMSO, 400MHz) δ ppm 7,57(d,1H), 7,42(breit,2H), 7,30(d,1H), 7,10-7,07(m,1H), 4,22-4,04(m,2H)

### Herstellbeispiel 4: N'-{2-Fluor-4-methyl-5-[(2,2,2-trifluorethyl)sulfanyl]phenyl}-N-methyl-4-(trifluormethyl)benzolcarboximidamid (Ia-250)

### Stufe 1: N-{2-Fluor-4-methyl-5-[(2,2,2-trifluorethyl)sulfanyl]phenyl}-4-(trifluormethyl)benzamid

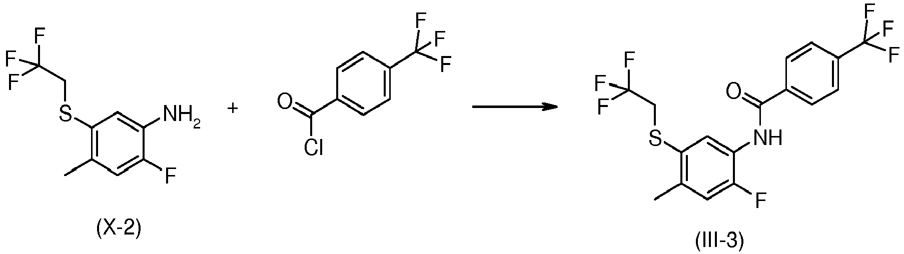

1,00 g (4,18 mmol) 2-Fluor-4-methyl-5-[(2,2,2-trifluorethyl)sulfanyl]anilin werden in 25 mL Dichlormethan vorgelegt, mit 0,47 g (4,60 mmol) Triethylamin versetzt und auf 0 °C abgekühlt. 0,96 g (4,60 mmol) 4-(Trifluormethyl)benzoylchlorid in 25 mL Dichlormethan werden bei 0 °C zugetropft. Die Reaktionsmischung wird über Nacht bei Raumtemperatur gerührt. 0,17 g (0,84 mmol) 4-(Trifluormethyl)benzoylchlorid werden zugesetzt und weitere 3 h bei Raumtemperatur gerührt. Die Reaktionsmischung wird mit Dichlormethan verdünnt, sukzessive mit halbgesättigter wässriger Ammoniumchloridlösung und gesättigter wässriger Natriumchlorid-Lösung gewaschen, über Magnesiumsulfat getrocknet, filtriert und unter vermindertem Druck vom Lösungsmittel befreit. Der Rückstand wird in Petrolether verrührt, abgesaugt und getrocknet. Man erhält 1,69 g (95% Reinheit, 93% der Theorie) der Titelverbindung als farblosen Feststoff.
logP(HCOOH): 4,20; logP(neutral): 4,17; 1H-NMR (D6-DMSO, 400MHz) δ ppm 10,40(s,1H), 8,17(d,2H), 7,93(d,2H), 7,82(d,1H), 7,31(d,1H), 3,89(q,2H), 2,42(s,3H); 1H-NMR (CD₃CN, 400MHz) δ ppm 8,70(breit,1H), 8,12(d,1H), 8,08(d,2H), 7,84(d,2H), 7,17(d,1H), 3,57(q,2H), 2,46(s,3H); GC-MS: EI-Masse (m/z): 411 [M]+

### Stufe 2: N-{2-Fluor-4-methyl-5-[(2,2,2-trifluorethyl)sulfanyl]phenyl}-4-(trifluormethyl)benzol-carboximidoylchlorid

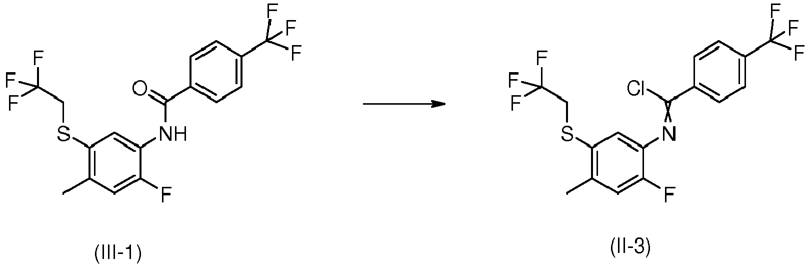

1,00 g (2,43 mmol) N-{2-Fluor-4-methyl-5-[(2,2,2-trifluorethyl)sulfanyl]phenyl}-4-(trifluormethyl)-benzamid werden in 20 mL Dichlormethan vorgelegt, mit 0,51 g (2,43 mmol) Phosphorpentachlorid versetzt und über Nacht bei Raumtemperatur gerührt. 0,51 g (2,43 mmol) Phosphorpentachorid werden erneut zugesetzt und über Nacht bei Raumtemperatur gerührt. Weitere 0,51 g (2,43 mmol) Phosphorpentachorid werden zugegeben, die Reaktionsmischung wird 3 h bei Raumtemperatur gerührt, dann über Kieselgel filtriert und konzentriert. Man erhält 0,91 g (74% Reinheit, 65% der Theorie) der Titelverbindung als gelbes Öl, welches direkt weiter umgesetzt wird.
1H-NMR (CD₃CN, 400MHz) δ ppm 8,32(d,2H), 7,86(d,2H), 7,32(d,1H), 7,21(d,1H), 3,58(q,2H), 2,47(s,3H); GC-MS: EI-Masse (m/z): 429 (1Cl) [M]+

### Stufe 3: N'-{2-Fluor-4-methyl-5-[(2,2,2-trifluorethyl)sulfanyl]phenyl}-N-methyl-4-(trilluormethyl)benzol-carboximidamid

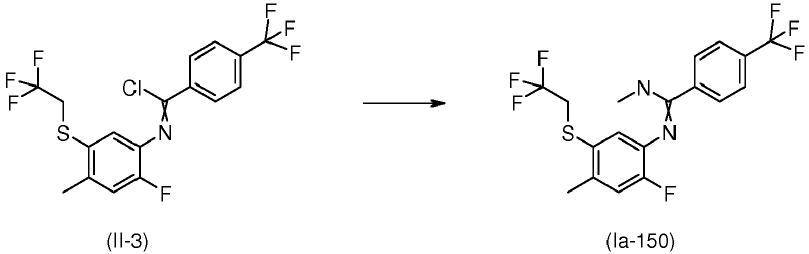

150 mg (4,65 mmol) Methylamin (2M in THF) werden in 25 mL Acetonitril vorgelegt, bei 0 °C mit 400 mg (0,93 mmol) N-{2-Fluor-4-methyl-5-[(2,2,2-trifluorethyl)sulfanyl]phenyl}-4-(trifluormethyl)benzol-carboximidoylchlorid in 25 mL Acetonitril versetzt und über Nacht bei Raumtemperatur gerührt, dann konzentriert. Der Rückstand wird säulenchromatographisch an Kieselgel mittels MPLC mit Cyclohexan/Essigester als Laufmittel gereinigt. Man erhält 220 mg (99% Reinheit, 56% der Theorie) der Titelverbindung als gelben Feststoff.
logP(HCOOH): 1,96; logP(neutral): 4,08; 1H-NMR (D6-DMSO, 400MHz) δ ppm 7,64(d,2H), 7,46(d,1H), 7,39(d,2H), 6,88(d,1H), 7,76(d,1H), 3,53(q,2H), 2,89(d,3H), 2,20(s,3H); GC-MS: EI-Masse (m/z): 424 [M]+

### Herstellbeispiel 5: 5-Chlor-N'-{2-fluor-4-methyl-5-[(2,2,2-trifluorethyl)sulfanyl]phenyl}-N,N-dimethylthiophen-2-carboximidamid (Ia-149)

### Stufe 1: 5-Chlor-N'-{2-fluor-4-methyl-5-[(2,2,2-trifluorethyl)sulfanyl]phenyl}-N,N-dimethylthiophen-2-carboximidamid

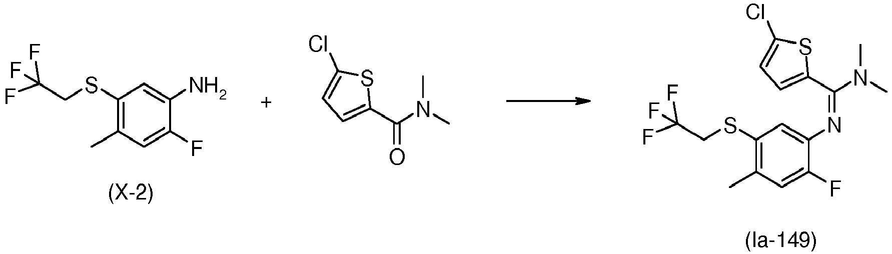

200 mg (0,84 mmol) 2-Fluor-4-methyl-5-[(2,2,2-trifluorethyl)sulfanyl]anilin und 206 mg (1,09 mmol) 5-Chlor-N,N-dimethylthiophen-2-carboxamid werden mit 743 mg (4,85 mmol) Phosphorylchlorid versetzt und über Nacht zum Rückfluss erhitzt. Nach Abkühlen wird die Reaktionsmischung in Wasser gegossen, mit Kaliumcarbonat basisch gestellt und mit Dichlormethan extrahiert. Die vereinigten organischen Phasen werden über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 165 mg (92% Reinheit, 44% der Theorie) der Titelverbindung als rotbraunes Öl.
logP(HCOOH): 2,11; logP(neutral): 4,89; 1H-NMR (D6-DMSO, 400MHz) δ ppm 7,00(d,1H), 7,93(d,1H), 6,87-6,84(m,2H), 3,68(q,2H), 2,97(breit,6H), 2,23(s,3H)

### Herstellbeispiel 6: N'-{2-Fluor-4-methyl-5-[(2,2,2-trifluorethyl)sulfanyl]phenyl}-N,N,1-trimethyl-1H-pyrrol-2-carboximidamid (Ia-162)

### Stufe 1: N'-12-Fluor-4-methyl-5-[(2,2,2-trifluorethyl)sulfanyl]phenyl}-N,N,1-trimethyl-1H-pyrrol-2-carboximidamid

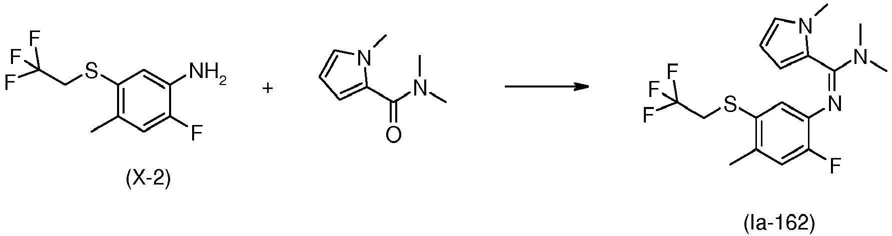

200 mg (0,84 mmol) 2-Fluor-4-methyl-5-[(2,2,2-trifluorethyl)sulfanyl]anilin und 165 mg (1,09 mmol) N,N,1-Trimethyl-1H-pyrrol-2-carboxamid werden mit 743 mg (4,85 mmol) Phosphorylchlorid versetzt und über Nacht zum Rückfluss erhitzt. Nach Abkühlen wird die Reaktionsmischung in Wasser gegossen, mit Kaliumcarbonat basisch gestellt und mit Dichlormethan extrahiert. Die vereinigten organischen Phasen werden über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 177 mg (99% Reinheit, 56% der Theorie) der Titelverbindung als gelbes Öl.
logP(HCOOH): 1,67; logP(neutral): 4,11; 1H-NMR (D6-DMSO, 400MHz) δ ppm 6,88(d,1H), 6,70-6,68(m,1H), 6,64(d,1H), 5,91-5,89(m,2H), 3,59(q,2H), 3,39(s,3H), 3,05(breit,3H), 2,77(breit,3H), 2,21(s,3H)

### Herstellbeispiel 7: N'-{2-Fluor-4-methyl-5-[(2,2,2-trifluorethyl)sulfanyl]phenyl}-N,N-dimethylfuran-2-carboximidamid (Ia-166)

### Stufe 1: N'-{2-Fluor-4-methyl-5-[(2,2,2-trifluorethyl)sulfanyl]phenyl}-N,N-dimethylfuran-2-carboximidamid

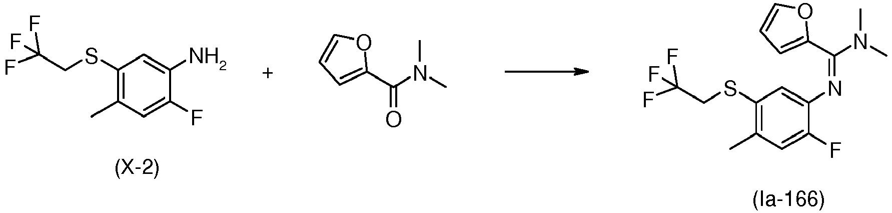

200 mg (0,84 mmol) 2-Fluor-4-methyl-5-[(2,2,2-trifluorethyl)sulfanyl]anilin und 139 mg (1,09 mmol) N,N-Dimethyl-2-furanamid werden mit 743 mg (4,85 mmol) Phosphorylchlorid versetzt und über Nacht zum Rückfluss erhitzt. Nach Abkühlen wird die Reaktionsmischung in Wasser gegossen, mit Kaliumcarbonat basisch gestellt und mit Dichlormethan extrahiert. Die vereinigten organischen Phasen werden über Magnesiumsulfat getrocknet, filtriert und unter vermindertem Druck vom Lösungsmittel befreit. Nach säulenchromatographischer Reinigung an Kieselgel mittels MPLC mit Cyclohexan/Essigester als Laufmittel erhält man 64 mg (98% Reinheit, 21% der Theorie) der Titelverbindung als gelbes Öl.
logP(HCOOH): 1,57; logP(neutral): 3,94; 1H-NMR (D6-DMSO, 400MHz) δ ppm 7,69-7,67(m,1H), 6,91(d,1H), 6,76(d,1H), 6,43-6,40(m,1H), 6,25(d,1H), 3,69(q,2H), 2,94(s,6H), 2,24(s,3H)

### Herstellbeispiel 8: 2-Methoxy-4-methyl-5-[(2,2,2-trifluorethyl)sulfanyl]anilin

### Stufe 1: 5-Acetamido-4-methoxy-2-methylbenzolsulfonylchlorid

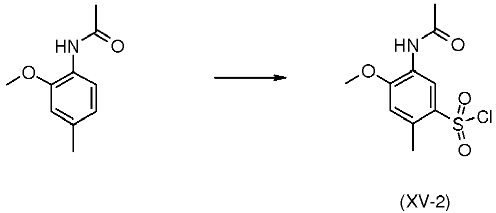

19,5 g (108,8 mmol) N-(2-Methoxy-4-methylphenyl)acetamid [CAS-RN 89345-81-3] werden portionsweise zu 150 g (1287 mmol) Chlorsulfonsäure gegeben und 4 h bei 80°C nachgerührt. Nach dem Abkühlen wird auf Eiswasser gegeben und der erhaltene Feststoff abgesaugt, es verbleiben 25,4 g Produkt (84,1 % der Theorie, Reinheit 100 % nach 1H-NMR).
1H-NMR(D6-DMSO) δ ppm: 9,04(s,1H), 8,14(s,1H), 6,80(s,1H), 3,80(s,3H), 2,48(s,3H), 2,04(s,3H)

### Stufe 2: N,N'-[Disulfanediylbis(6-methoxy-4-methylbenzol-3,1-diyl)]diacetamid

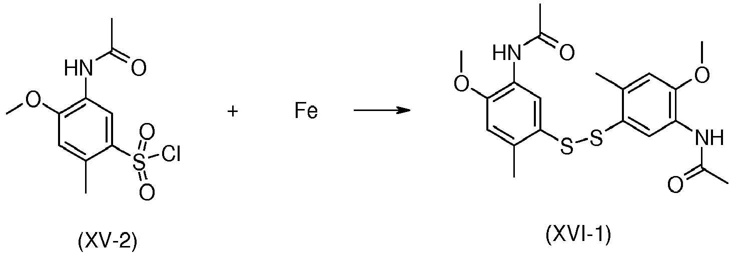

25,3 g (91,1 mmol) 5-Acetamido-4-methoxy-2-methylbenzolsulfonylchlorid werden mit 14,6 g (261,4 mmol) Eisenpulver in 400 ml Ethanol und 36,7 g konzentrierter Salzsäure 12 h unter Rückfluss erhitzt.Nach dem Entfernen des Lösungsmittels unter vermindertem Druck wird der Rückstand mit Wasser verrührt und abgesaugt und man erhält 9,4 g Rohprodukt (49,1 % der Theorie, Reinheit 86,4 % nach LC/MS) als hellbraunen Feststoff.
logP(HCOOH): 2,73

### Stufe 3: N-{2-Methoxy-4-methyl-5-[(2,2,2-trifluorethyl)sulfanyl]phenyl}acetamid

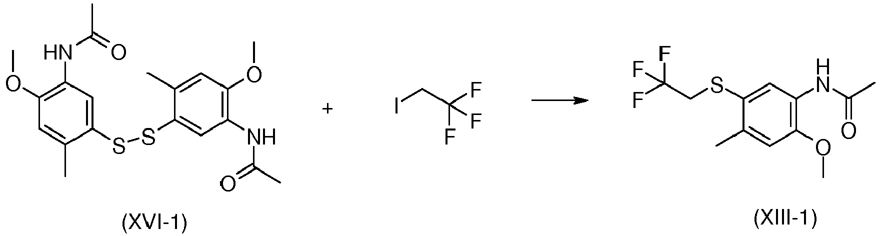

9,4 g (22,35 mmol) N,N'-[Disulfanediylbis(6-methoxy-4-methylbenzol-3,1-diyl)]diacetamid werden in 60 ml Dimethylformamid vorgelegt und mit 6,5 g Natrium-Dithionit, 15,9 g Kaliumcarbonat und 5,45 g Natriumbiphosphat und 40 ml Wasser versetzt und anschliessend 3 h bei 60°C gerührt. Nach dem Abkühlen werden 12,5 g (59,54 mmol) 1,1,1-Trifluor-2-iodethan dazugegeben und weitere 12 h bei 75°C gerührt. Nach Entfernen des Lösungsmittels unter vermindertem Druck wird der verbleibende Rückstand mit konzentrierter Salzsäure angesäuert und der entstandene Niederschlag abgesaugt. Es verbleiben 5,3 g Produkt (80,8 % der Theorie, Reinheit > 95 % nach 1H-NMR).
1H-NMR(D6-DMSO) δ ppm : 9,15(s,1H), 8,14(s,1H), 6,98(s,1H), 3,83(s,3H), 3,69-3,61(q,2H), 2,40(s,3H), 2,06(s,3H)

### Stufe 4: 2-Methoxy-4-methyl-5-[(2,2,2-trifluorethyl)sulfanyl]anilin

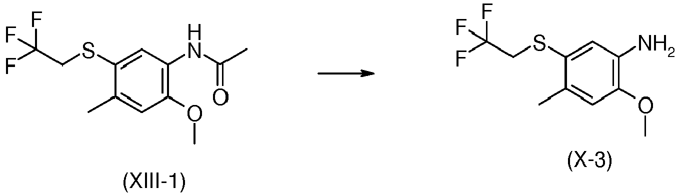

5,3 g (22,35 mmol) N-{2-Methoxy-4-methyl-5-[(2,2,2-trifluorethyl)sulfanyl]phenyl}acetamid werden in 125 ml 5 molarer Salzsäure 18 h unter Rückfluß gerührt. Das Reaktionsgemisch wird mit Natronlauge basisch gestellt und mit Dichlormethan extrahiert. Die organische Phase wird abgetrennt, über Natriumsulfat getrocknet und unter Vakuum vom Lösungsmittel befreit. Es verbleiben 3,6 g Produkt als dunkeloranges Öl (79,3 % der Theorie, Reinheit 96,8 % nach LC/MS).
1H-NMR(D6-DMSO) δ ppm: 6,83(s,1H), 6,72(s,1H), 4,64(breit,2H), 3,75(s,3H), 3,64-3,56(q,2H), 2,30(s,3H)

### Herstellbeispiel 9: 3-[(2,2,2-Trifluorethyl)sulfanyl]anilin

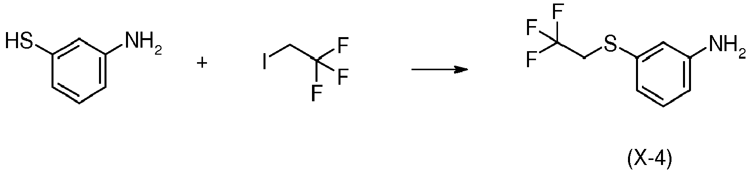

6,5 g (51,92 mmol) 3-Aminobenzolthiol werden in 200 ml Acetonitril vorgelegt und mit 13,7 g Kaliumcarbonat, 1,6 g festem Natriumhydroxid, 1 ml Dimethylsulfoxid und 13 g (61,93 mmol) 1,1,1-Trifluor-2-iodethan versetzt und 18 h bei 45°C gerührt. Das Reaktionsgemisch wird mit 250 ml Wasser verdünnt und mit Dichlormethan extrahiert. Die organische Phase wird abgetrennt, über Natriumsulfat getrocknet und unter Vakuum vom Lösungsmittel befreit. Es verbleiben 9,5 g Produkt als braunes Öl (88,3 % der Theorie, Reinheit 97,9 % nach LC/MS).
1H-NMR(D6-DMSO) δ ppm: 7,00-6,97(m,1H), 6,61(m,1H), 6,59(m,1H), 6,47-6,45(m,1H), 5,19(breit,2H), 3,88-3,80(q,2H)
logP(HCOOH): 2,01

### Herstellbeispiel 10: N-(2,2-Difluorethyl)cyclopropancarboxamid (XXIV-1)

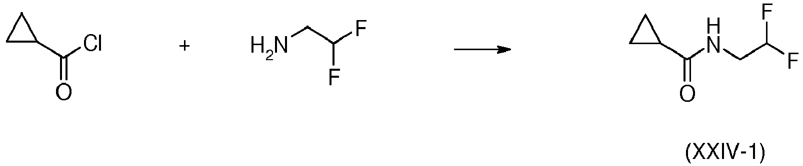

14,4 g (137,75 mmol) Cyclopropancarbonsäurechlorid werden in 300 ml wasserfreiem Tetrahydrofuran gelöst.Nach der Zugabe von 1 g Triethylamin wird eine Lösung von 16,8 g (207,24 mmol) 2,2-Difluorethanamin in 50 ml wasserfreiem Tetrahydrofuran zugetropft. Nach beendeter Zugabe wird noch 18 h bei Raumtemperatur und anschliessend noch 1 h bei 40°C nachgerührt. Man verdünnt mit 200 ml Wasser und extrahiert mit Dichlormethan. Die vereinigten organischen Phasen werden abgetrennt, über Natriumsulfat getrocknet und im Vakuum vom Lösungsmittel befreit. Als Rückstand verbleiben 18,9 g Produkt (92 % der Theorie, Reinheit 100 % nach 1H-NMR) als weisser Feststoff.
1H-NMR(D6-DMSO) δ ppm: 8,46(t,1H), 5,99(tt,1H), 3,54-3,43(m,2H), 1,65-1,59(m,1H), 0,70-0,60(m,4H)

### Herstellbeispiel 11: 1-Fluorcyclopropancarboxamid (XXIV-2)

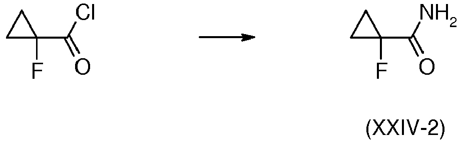

Zu 15 g Ammoniumhydroxid (28-30 Gew. % NH₃-Lösung in Wasser) gibt man portionsweise 600 mg (4,9 mmol) 1-Fluorcyclopropancarbonylchlorid [CAS-RN 149961-53-5] und rührt noch 18 h bei Raumtemperatur. Der anfallende Niederschlag wird abgesaugt und man erhält 470 mg Produkt (93,1 % der Theorie, Reinheit 100 % nach 1H-NMR) als weissen Feststoff.
1H-NMR(D6-DMSO) δ ppm : 7,80(breit,1H), 7,58(breit,1H), 1,29-1,12(m,4H)

### Herstellbeispiel 12: (4-Chlorphenyl)(4,4-difluorpiperidin-1-yl)methanon (XXIV-3)

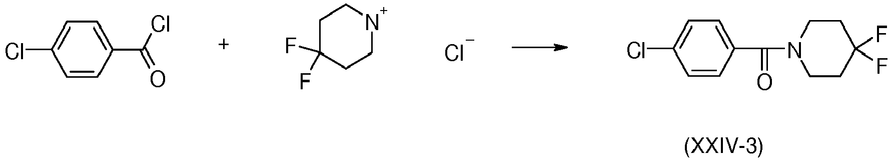

470 mg (2,98 mmol) 4,4-Difluorpiperidiniumchlorid werden in 35 ml wasserfreiem Toluol vorgelegt. Nach der Zugabe von 1,4 g Triethylamin werden portionsweise 690 mg (3,94 mmol) 4-Chlorbenzoylchlorid dazugegeben und es wird noch 18 h bei 100°C gerührt. Das Reaktionsgemisch wird mit 250 ml Wasser verdünnt, die organische Phase wird abgetrennt, über Natriumsulfat getrocknet und unter Vakuum vom Lösungsmittel befreit. Es verbleiben 480 m g Produkt als oranges Öl (62 % der Theorie, Reinheit 88 % nach LC/MS).
1H-NMR(D6-DMSO) δ ppm : 7,54-7,49(m,4H), 3,70(m,2H), 3,39(m,2H), 2,03(m,4H)

### Herstellbeispiel 13: N-{2-[3-Chlor-5-(trifluormethyl)pyridin-2-yl]ethyl}cyclopropancarboxamid (XXIV-4)

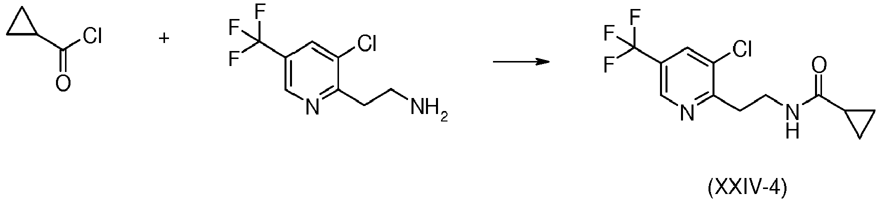

550 mg (2,11 mmol) 2-[3-Chlor-5-(trifluormethyl)pyridin-2-yl]ethanamin werden in 20 ml wasserfreiem Dichlormethan vorgelegt. Nach der Zugabe von 1 g Triethylamin werden 270 mg (2,58 mmol) Cyclopropancarbonsäurechlorid gelöst in 5 ml wasserfreiem Dichlormethan zugetropft, anschliessend wird noch 12 h bei 50°C gerührt. Das Reaktionsgemisch wird mit 50 ml Wasser verdünnt, die organische Phase wird abgetrennt, über Magnesiumsulfat getrocknet und im Vakuum vom Lösungsmittel befreit. Es verbleiben 450 m g Produkt als oranger Feststoff (73 % der Theorie, Reinheit 87,1 % nach LC/MS).
1H-NMR(D6-DMSO) δ ppm: 8,89(d,1H), 8,41(d,1H), 8,17(t,1H), 3,53-3,48(m,2H), 3,11(t,2H), 1,51-1,46(m,1H), 0,63-0,59(m,4H)

### Herstellbeispiel 14: 1-Fluor-N'-{2-fluor-4-methyl-5-[(2,2,2-trifluorethyl)sulfinyl]phenyl}cyclopropancarboximidamid (Ib-19)

### Stufe 1: 1-Fluor-N'-{2-fluor-4-methyl-5-[(2,2,2-trifluorethyl)sulfanyl]phenyl}cyclopropancarboximidamid (Ia-27)

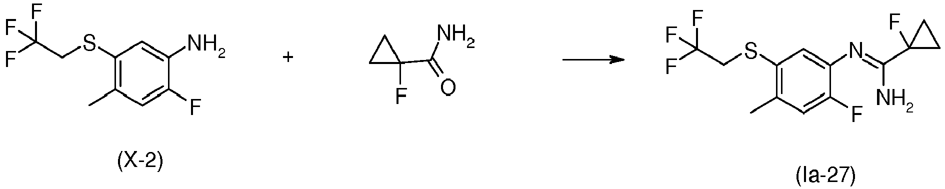

Zu 400 mg (1,67 mmol) 2-Fluor-4-methyl-5-[(2,2,2-trifluorethyl)sulfanyl]anilin und 470 mg (4,56 mmol) 1-Fluorcyclopropancarboxamid gibt man 1,4 g (9,13 mmol) Phosphorylchlorid und rührt 18 h bei 95°C. Der nach dem Einrotieren verbleibende Rückstand wird mit Wasser verrührt, mit festem Kaliumcarbonat basisch gestellt und mit Dichlormethan extrahiert. Die vereinigten organischen Phasen werden abgetrennt, über Natriumsulfat getrocknet und im Vakuum vom Lösungsmittel befreit. Als Rückstand verbleiben 380 mg Produkt (70,1 % der Theorie, Reinheit 88,8 % nach LC/MS).
logP(HCOOH): 1,2

### Stufe 2: 1-Fluor-N'-{2-fluor-4-methyl-5-[(2,2,2-trifluorethyl)sulfinyl]phenyl}cyclopropancarboximidamid (Ib-19)

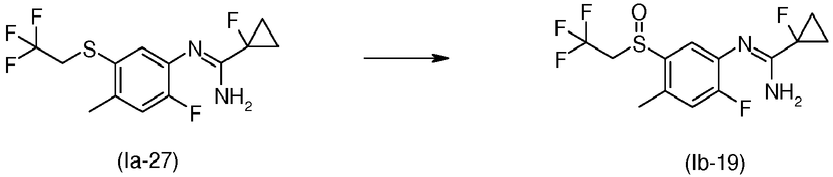

310 mg (0,96 mmol) 1-Fluor-N'-{2-fluor-4-methyl-5-[(2,2,2-trifluorethyl)sulfanyl]phenyl}cyclopropancarboximidamid (Ia-27) werden bei 0-4 °C in 30 ml Trichlormethan vorgelegt. Nach der Zugabe von 335 mg Puffer-Lösung pH 7 (KH2PO4/Na2HPO4) und 65 mg Benzyltriethylammoniumchlorid werden bei 0-4 °C portionsweise 230 mg (70%ig, 1,03 mmol) m-Chlorperbenzoesäure dazugegeben und das Reaktionsgemisch wird 24 h bei Raumtemperatur gerührt. Nach Versetzung mit einer 33%igen wäßrigen Natrium-Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand säulenchromatographisch mittels MPLC über RP(C-18) mit Wasser/Acetonitril aufgereinigt. Man erhält 42 mg Produkt (12,9 % der Theorie, Reinheit 98,1 % nach LC/MS).

### Herstellbeispiel 15: 2,2,2-Trifluor-N'-{2-methoxy-4-methyl-5-[(2,2,2-trifluorethyl)sulfanyl]phenyl}ethanimidamid (Ia-236)

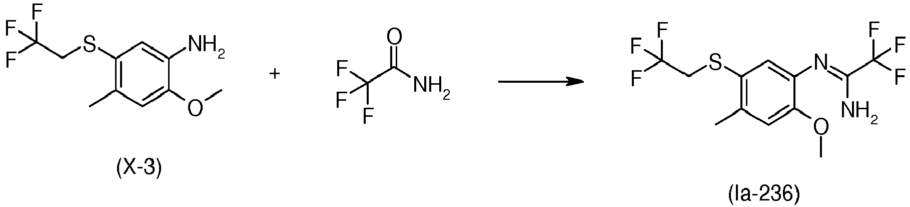

Zu 300 mg (1,19 mmol) 2-Methoxy-4-methyl-5-[(2,2,2-trifluorethyl)sulfanyl]anilin und 400 mg (3,54 mmol) Trifluoracetamid gibt man 2,5 g (16,3 mmol) Phosphorylchlorid und rührt 18 h bei 95°C. Der nach dem Einrotieren verbleibende Rückstand wird mit Wasser verrührt, mit festem Kaliumcarbonat basisch gestellt und mit Dichlormethan extrahiert. Die vereinigten organischen Phasen werden abgetrennt, über Natriumsulfat getrocknet und im Vakuum vom Lösungsmittel befreit. Als Rückstand verbleiben 61,3 mg Produkt (14,1 % der Theorie, Reinheit > 95 % nach 1H-NMR).

### Herstellbeispiel 16: N-[(4-Chlorphenyl)(morpholin-4-yl)methylen]-2-methoxy-4-methyl-5-[(2,2,2-trifluorethyl)sulfanyl]anilin (Ia-268)

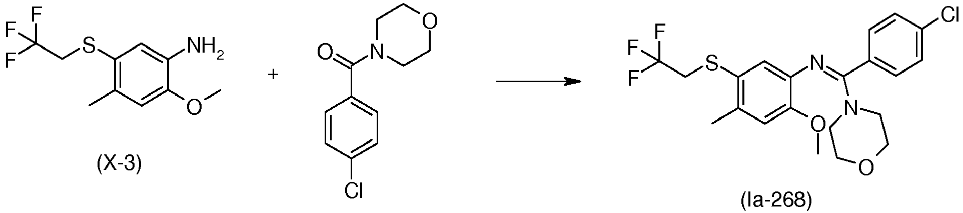

Zu 300 mg (1,19 mmol) 2-Methoxy-4-methyl-5-[(2,2,2-trifluorethyl)sulfanyl]anilin und 360 mg (1,60 mmol) (4-Chlorphenyl)(morpholin-4-yl)methanon gibt man 2,5 g (16,3 mmol) Phosphorylchlorid und rührt 18 h bei 95°C. Der nach dem Einrotieren verbleibende Rückstand wird mit Wasser verrührt, mit festem Kaliumcarbonat basisch gestellt und mit Dichlormethan extrahiert. Die vereinigten organischen Phasen werden abgetrennt, über Natriumsulfat getrocknet und im Vakuum vom Lösungsmittel befreit. Als Rückstand verbleiben 247 mg Produkt (40,9 % der Theorie, Reinheit 90,9 % nach LC/MS).

### Herstellbeispiel 17: N'-{2-Fluor-4-methyl-5-[(2,2,2-trifluorethyl)sulfinyl]phenyl}-N-methylpyridin-3-carboximidamid (Ib-28)

### Stufe 1: N'-{2-Fluor-4-methyl-5-[(2,2,2-trifluorethyl)sulfanyl]phenyl}-N-methylpyridin-3-carboximidamid (Ia-61)

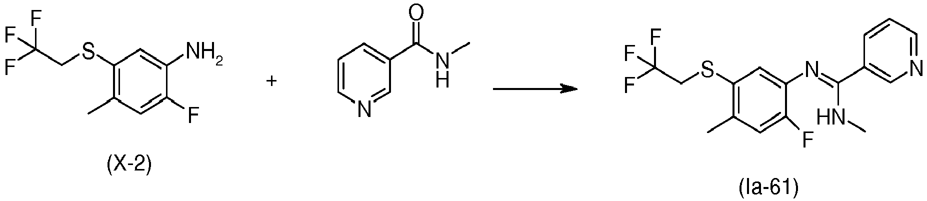

Zu 400 mg (1,67 mmol) 2-Fluor-4-methyl-5-[(2,2,2-trifluorethyl)sulfanyl]anilin und 450 mg (3,31 mmol) N-Methylnicotinamid gibt man 1,5 g (9,78 mmol) Phosphorylchlorid und rührt 18 h bei 95°C. Der nach dem Einrotieren verbleibende Rückstand wird mit Wasser verrührt, mit festem Kaliumcarbonat basisch gestellt und mit Dichlormethan extrahiert. Die vereinigten organischen Phasen werden abgetrennt, über Natriumsulfat getrocknet und im Vakuum vom Lösungsmittel befreit. Als Rückstand verbleiben 550 mg Produkt (92,1 % der Theorie, Reinheit 92,9 % nach LC/MS).

### Stufe 2: N'-{2-Fluor-4-methyl-5-[(2,2,2-trifluorethyl)sulfinyl]phenyl}-N-methylpyridin-3-carboximidamid (Ib-28)

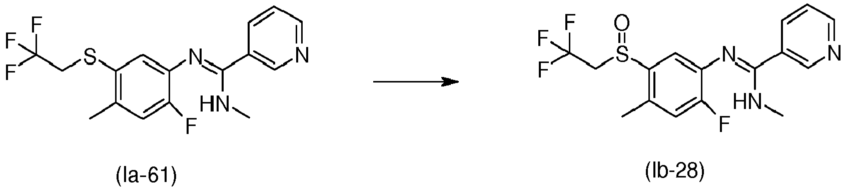

460 mg (1,29 mmol) N'-{2-Fluor-4-methyl-5-[(2,2,2-trifluorethyl)sulfanyl]phenyl}-N-methylpyridin-3-carboximidamid (Ia-61) werden bei 0-4 °C in 40 ml Trichlormethan vorgelegt. Nach der Zugabe von 460 mg Puffer-Lösung pH 7 (KH₂PO₄/Na₂HPO₄) und 90 mg Benzyltriethylammoniumchlorid werden bei 0-4 °C portionsweise 320 mg (70%ig, 0,261 mmol) m-Chlorperbenzoesäure dazugegeben und das Reaktionsgemisch wird 24 h bei RT gerührt. Nach der Zugabe einer 33%igen wäßrigen Natrium-Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand säulenchromatographisch mittels MPLC über RP(C-18) mit Wasser/Acetonitril aufgereinigt. Man erhält 145 mg Produkt (30,2 % der Theorie, Reinheit 98,7 % nach HPLC).

### Herstellbeispiel 18: N'-{2-Fluor-4-methyl-5-[(2,2,2-trifluorethyl)sulfinyl]phenyl}pyrazin-2-carboximidamid (Ib-62)

### Stufe 1: N'-{2-Fluor-4-methyl-5-[(2,2,2-trilluorethyl)sulfanyl]phenyl}pyrazin-2-carboximidamid (Ia-117)

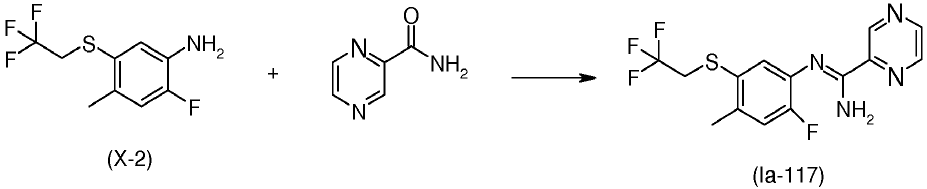

Zu 300 mg (1,25 mmol) 2-Fluor-4-methyl-5-[(2,2,2-trifluorethyl)sulfanyl]anilin und 190 mg (1,54 mmol) Pyrazincarboxamid gibt man 2 g (13,04 mmol) Phosphorylchlorid und rührt 18 h bei 95°C. Der nach dem Einrotieren verbleibende Rückstand wird mit Wasser verrührt, mit festem Kaliumcarbonat basisch gestellt und mit Dichlormethan extrahiert. Die vereinigten organischen Phasen werden abgetrennt, über Natriumsulfat getrocknet und im Vakuum vom Lösungsmittel befreit. Als Rückstand verbleiben 365 mg Produkt (84,5 % der Theorie, Reinheit 91,2 % nach LC/MS).
logP(HCOOH): 1,24

### Stufe 2: N'-{2-Fluor-4-methyl-5-[(2,2,2-trifluorethyl)sulfinyl]phenyl}pyrazin-2-carboximidamid (Ib-62)

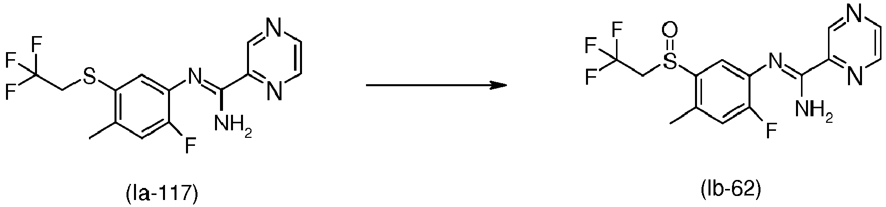

305 mg (0,89 mmol) N'-{2-Fluor-4-methyl-5-[(2,2,2-trifluorethyl)sulfanyl]phenyl}pyrazin-2-carboximidamid (Ia-117) werden bei 0-4 °C in 30 ml Trichlormethan vorgelegt. Nach der Zugabe von 320 mg Puffer-Lösung pH 7 (KH₂PO₄/Na₂HPO₄) und 60 mg Benzyltriethylammoniumchlorid werden bei 0-4 °C portionsweise 225 mg (70%ig, 1,04 mmol) m-Chlorperbenzoesäure dazugegeben und das Reaktionsgemisch wird 24 h bei RT gerührt. Nach Versetzung mit einer 33%igen wäßrigen Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand auf RP(C-18) Material aufgezogen. Nach säulenchromatographischer Aufreinigung mittels MPLC über RP(C-18) mit Wasser/Acetonitril erhält man 55 mg (17,2 % der Theorie, Reinheit 100 % nach LC/MS) Produkt als hellbeigen Feststoff.
logP(HCOOH): 0,84 logP(neutral): 1,77

### Herstellbeispiel 19: 2-Fluor-4-methyl-N-[1-(2,2,2-trifluorethyl)pyrrolidin-2-yliden]-5-[(2,2,2-trifluorethyl)sulfinyl]anilin (Ib-115)

### Stufe 1: N'- {2-Fluor-4-methyl-5- [(2,2,2-trifluorethyl)sulfanyl]phenyl} -N-(2,2,2 trifluorethyl)cyclopropancarboximidamid (Ia-145) und 2-Fluor-4-methyl-N-[1-(2,2,2-trifluorethyl)pyrrolidin-2-yliden]-5-[(2,2,2-trifluorethyl)sulfanyl]anilin (Ia-189)

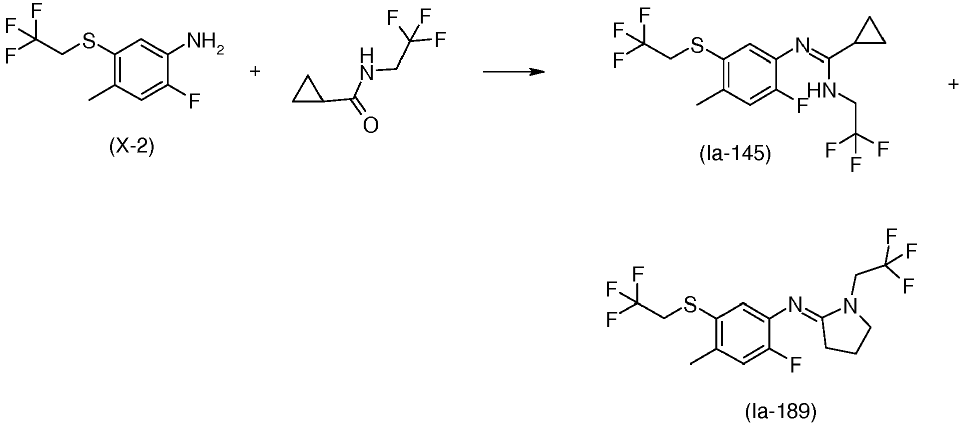

Zu 300 mg (1,25 mmol) 2-Fluor-4-methyl-5-[(2,2,2-trifluorethyl)sulfanyl]anilin und 430 mg (2,57 mmol) N-(2,2,2-Trifluorethyl)cyclopropancarboxamid gibt man 1,5 g (9,78 mmol) Phosphorylchlorid und rührt 18 h bei 95°C. Der nach dem Einrotieren verbleibende Rückstand wird mit Wasser verrührt, mit festem Kaliumcarbonat basisch gestellt und mit Dichlormethan extrahiert. Die vereinigten organischen Phasen werden abgetrennt, über Natriumsulfat getrocknet und im Vakuum vom Lösungsmittel befreit. Als Rückstand verbleiben 630 mg Rohprodukt als ca. 3:1 Gemisch der oben beschriebenen Isomere.

Nach säulenchromatographischer Aufreinigung mittels MPLC über RP(C-18) mit Wasser/Acetonitril erhält man 239,8 mg (49,2 % der Theorie, Reinheit 80 % nach 1H-NMR) N'-{2-Fluor-4-methyl-5-[(2,2,2-trifluorethyl)sulfanyl]phenyl}-N-(2,2,2 trifluorethyl)cyclopropancarboximidamid (Ia-145)

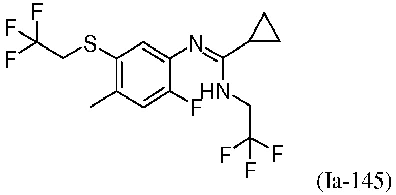

13C-NMR(D6-DMSO) δ ppm: 161.0, 153.5, 137.0, 133.9, 128.1, 127.2, 117.5,41.0, 35.6, 19.6, 11.6, 6.2
logP(HCOOH): 1,99 logP(neutral): 4,12
und 72,4 mg (14,9 % der Theorie, Reinheit 100 % nach HPLC) 2-Fluor-4-methyl-N-[1-(2,2,2-trifluorethyl)pyrrolidin-2-yliden]-5-[(2,2,2-trifluorethyl)sulfanyl]anilin (Ia-189).

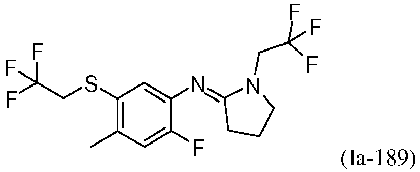

1H-NMR(D6-DMSO) δ ppm: 7,09-7,01(m,2H), 4,28-4,21(q,2H), 3,89-3,81(q,2H), 3,51-3,47(t,2H), 2,36-2,30(t,2H), 2,32(s,3H), 1,98-1,90(m,2H).
logP(HCOOH): 2,32 logP(neutral): 4,37

### Stufe 2: 2-Fluor-4-methyl-N-[1-(2,2,2-trifluorethyl)pyrrolidin-2-yliden]-5-[(2,2,2-trifluorethyl)sulfinyl]anilin (Ib-115)

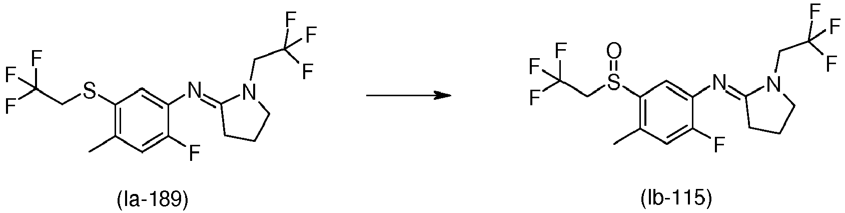

900 mg (2,32 mmol) 2-Fluor-4-methyl-N-[1-(2,2,2-trifluorethyl)pyrrolidin-2-yliden]-5-[(2,2,2-trifluorethyl)sulfanyl]anilin (Ia-189) werden bei 0-4 °C in 40 ml Trichlormethan vorgelegt. Nach der Zugabe von 850 mg Puffer-Lösung pH 7 (KH₂PO₄/Na₂HPO₄) und 160 mg Benzyltriethylammoniumchlorid werden bei 0-4 °C portionsweise 600 mg (77%ig, 2,68 mmol) m-Chlorperbenzoesäure dazugegeben und das Reaktionsgemisch wird 24 h bei RT gerührt. Nach der Zugabe einer 33%igen wäßrigen Natrium-Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand säulenchromatographisch mittels MPLC über RP(C-18) mit Wasser/Acetonitril aufgereinigt. Man erhält 70 mg Produkt (7,5 % der Theorie, Reinheit 95,2 % nach LC/MS).
13C-NMR(D6-DMSO) δ ppm: 164.2, 155.6, 138.3, 136.0, 129.8, 125.1, 124.2, 119.9, 117.9, 56.8, 49.5, 44.4, 27.0, 19.5, 16.4

### Herstellbeispiel 20: 2,4-Dimethyl-N-[1,3-oxazolidin-2-yliden]-5-[(2,2,2-trifluorethyl)sulfanyl]anilin

### Stufe 1: 1-(2-Chlorethyl)-3-{2,4-dimethyl-5-[(2,2,2-trifluorethyl)sulfanyl]phenyl}harnstoff

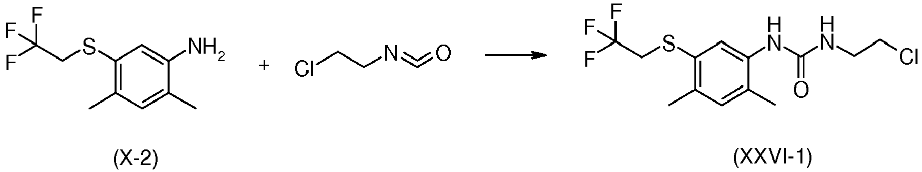

Zu einer Lösung von 700 mg (6,63 mmol) 2-Chlorethylisocyanat in 50 ml tert.-Butylmethylether und einer katalytischen Menge 1,8-Diazabicyclo[5.4.0] undec-7-en (DBU) werden portionsweise 1,45 g (6,16 mmol) 2,4-Dimethyl-5-[(2,2,2-trifluorethyl)sulfanyl]anilin dazugegeben und anschliessend noch 18 h bei Raumtemperatur gerührt. Das Gemisch wird fast vollständig im Vakuum vom Lösungsmittel befreit, der anfallende weisse Feststoff wird abgesaugt. Es verbleiben 2,00 g Produkt (88,5 % der Theorie, Reinheit 94,4 % nach LC/MS).
1H-NMR(D6-DMSO) δ ppm: 8,01(s,1H), 7,83(s,1H), 7,04(s,1H), 6,83(t,1H), 3,77-3,69(q,2H), 3,68-3,65(m,2H), 3,45-3,40(m,2H), 2,30(s,3H), 2,14(s,3H).

### Stufe 2: 2,4-Dimethyl-N-[1,3-oxazolidin-2-yliden]-5-[(2,2,2-trifluorethyl)sulfanyl]anilin

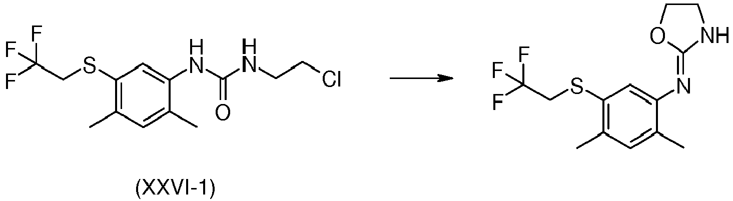

900 mg (2,64 mmol) 1-(2-Chlorethyl)-3-{2,4-dimethyl-5-[(2,2,2-trifluorethyl)sulfanyl]phenyl}harnstoff werden in einem Gemisch aus 15 ml Wasser und 20 ml Propionitril mit 4 g Kaliumcarbonat 18 h unter Rückfluß erhitzt. Das Gemisch wird im Vakuum vom Lösungsmittel befreit und der verbleibende Feststoffbrei wird mit verdünnter Salzsäure angesäuert. Man lässt 18 h stehen und saugt anschliessend den beigen Niederschlag ab. Man erhält 560 mg Rohprodukt. Nach säulenchromatographischer Aufreinigung mittels einer Biotage Isolera One und Essigester / Cyclohexan 2:1 v/v als Laufmittel werden 130 mg Produkt erhalten (16,2 % der Theorie, Reinheit nach LC/MS 81,5 %).
logP(HCOOH): 1,36

### Herstellbeispiel 21: 2-Fluor-4-methyl-N-[1,3-thiazinan-2-yliden]-5-[(2,2,2-trifluorethyl)sulfanyl]anilin (Ia-279)

### Stufe 1: 2-Fluor-4-methyl-N-[(1,3-thiazinan-2-yliden]-5-[(2,2,2-trifluorethyl)sulfanyl]aniliniumchlorid (Ia-278)

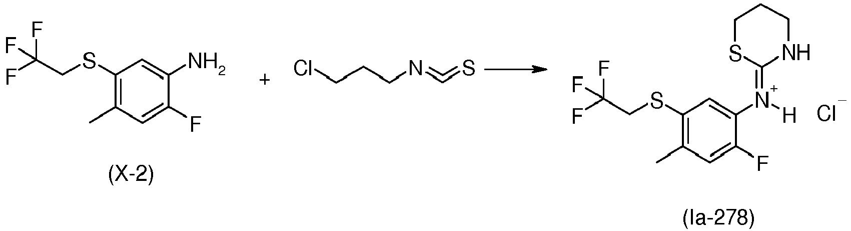

Zu einer Lösung von 1 g (7,37 mmol) 3-Chlorpropylisocyanat in 30 ml tert.-Butylmethylether und einer katalytischen Menge 1,8-Diazabicyclo[5.4.0] undec-7-en (DBU) werden portionsweise 1,61 g (6,72 mmol) 2-Fluor-4-methyl-5-[(2,2,2-trifluorethyl)sulfanyl]anilin dazugegeben und anschliessend noch 18 h bei Raumtemperatur gerührt. Das Gemisch wird im Vakuum vom Lösungsmittel befreit. Als Rückstand verbleibt ein hellbeiges Öl das langsam durchkristallisiert. Es verbleiben 2,3 g Produkt (91,3 % der Theorie, Reinheit nach LC/MS 95 %).
13C-NMR(D6-DMSO) δ ppm: 166.0 (breit), 156.1, 142.1, 131.7, 129.4, 126.1, 120.6, 118.6, 41.3, 34.9, 26.6, 20.6
logP(HCOOH): 1,34

### Stufe 2: 2-Fluor-4-methyl-N-[1,3-thiazinan-2-yliden]-5-[(2,2,2-trifluorethyl)sulfanyl]anilin (Ia-279)

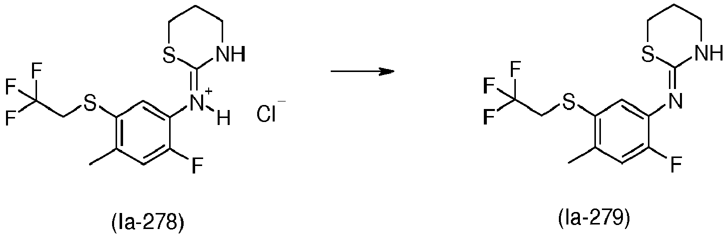

2,2 g (5,87 mmol) 2-Fluor-4-methyl-N-[1,3-thiazinan-2-yliden]-5-[(2,2,2-trifluorethyl)sulfanyl]aniliniumchlorid (Ia-278) werden in 50 ml Wasser gelöst und mit Ammoniumhydroxid (28-30 Gew. % NH₃-Lösung in Wasser) basisch gestellt. Man extrahiert mehrmals mit Dichlormethan. Die vereinigten organischen Phasen werden abgetrennt, über Natriumsulfat getrocknet und im Vakuum vom Lösungsmittel befreit. Als Rückstand verbleiben 1,56 g Produkt (68,3 % der Theorie, Reinheit 100 % nach 1H-NMR) als bernsteinfarbene, glänzende Kristalle.

### Herstellbeispiel 22: 2-Fluor-4-methyl-N-[1,3-thiazolidin-2-yliden]-5-[(2,2,2-trifluorethyl)sulfinyl]anilin

### Stufe 1: 1-(2-Chlorethyl)-3-{2-fluor-4-methyl-5-[(2,2,2-trifluorethyl)sulfanyl]phenyl}thioharnstoff

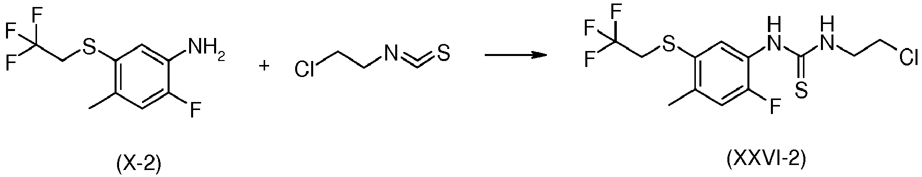

Zu einer Lösung von 1 g (8,22 mmol) 2-Chlorethylisothiocyanat in 50 ml tert.-Butylmethylether und einer katalytischen Menge 1,8-Diazabicyclo[5.4.0] undec-7-en (DBU) werden portionsweise 1,9 g (7,94 mmol) 2-Fluor-4-methyl-5-[(2,2,2-trifluorethyl)sulfanyl]anilin dazugegeben und anschliessend noch 18 h bei Raumtemperatur gerührt. Das Gemisch wird fast vollständig im Vakuum vom Lösungsmittel befreit, der anfallende weisse Feststoff wird abgesaugt. Es verbleiben 2,88 g Produkt (97,1 % der Theorie, Reinheit 100 % nach 1H-NMR).
1H-NMR(D6-DMSO) δ ppm: 7,68-7,67(m,1H), 7,43-7,40(m,1H), 4,03-3,93(m,4H), 3,61(t,2H), 2,41(s,3H).

### Stufe 2: 2-Fluor-4-methyl-N-[1,3-thiazolidin-2-yliden]-5-[(2,2,2-trifluorethyl)sulfanyl]anilin (Ia-238)

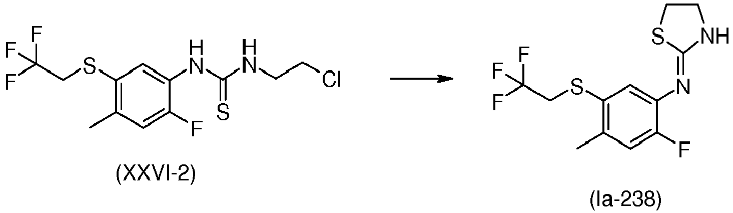

2,75 g (7,62 mmol) 1-(2-Chlorethyl)-3-{2-fluor-4-methyl-5-[(2,2,2-trifluorethyl)sulfanyl]phenyl}harnstoff werden in einem Gemisch aus 3 ml Wasser und 100 ml Propionitril mit 2 g Kaliumcarbonat, 3,5 g Caesiumcarbonat, 0,1 g Natriumhydroxid und 0,1 g Kaliumiodid 48 h unter Rückfluß erhitzt. Das Gemisch wird im Vakuum vom Lösungsmittel befreit und der verbleibende Feststoffbrei wird mit verdünnter Salzsäure neutral gestellt. Man extrahiert mehrmals mit Dichlormethan. Die vereinigten organischen Phasen werden abgetrennt, über Magnesiumsulfat getrocknet und im Vakuum vom Lösungsmittel befreit. Man erhält 2,38 g braunes Öl als Rohprodukt. Nach säulenchromatographischer Aufreinigung mittels einer Biotage Isolera One unter Verwendung einer 50 g Snap Cartridge Kartusche und Essigester / Cyclohexan 1:1 v/v als Laufmittel werden 590 mg Produkt erhalten (23,9 % der Theorie, Reinheit 92,9 % nach LC/MS).
13C-NMR(D6-DMSO) δ ppm: 163.3, 153.7, 134.8, 126.9(breit), 126.9, 126.0, 117.0, 45.0(breit), 35.5, 29.8, 19.4

### Stufe 3: 2-Fluor-4-methyl-N-[1,3-thiazolidin-2-yliden]-5-[(2,2,2-trifluorethyl)sulfinyl]anilin

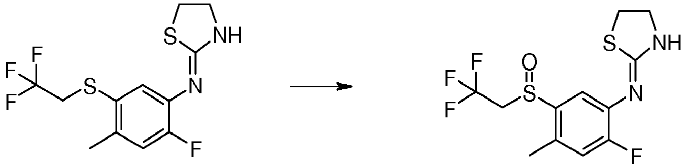

99 mg (0,305 mmol) 2-Fluor-4-methyl-N-[1,3-thiazolidin-2-yliden]-5-[(2,2,2-trifluorethyl)sulfanyl]anilin (Ia-238) werden bei 0-4 °C in 10 ml Trichlormethan vorgelegt. Nach der Zugabe von 120 mg Puffer-Lösung pH 7 (KH₂PO₄/Na₂HPO₄) und 20 mg Benzyltriethylammoniumchlorid werden bei 0-4 °C portionsweise 80 mg (70%ig, 0,357 mmol) m-Chlorperbenzoesäure dazugegeben und das Reaktionsgemisch wird 24 h bei RT gerührt. Nach der Zugabe einer 33%igen wäßrigen Natrium-Bisulfit-Lösung wird das Gemisch zweimal mit Dichlormethan extrahiert. Die vereinigten organischen Phasen werden mit Wasser gewaschen, über Natriumsulfat getrocknet und filtriert. Nach Entfernen des Lösungsmittels unter vermindertem Druck wird der Rückstand säulenchromatographisch mittels MPLC über RP(C-18) mit Wasser/Acetonitril aufgereinigt. Man erhält 15 mg Produkt (14,4 % der Theorie, Reinheit 100 % nach LC/MS).
13C-NMR(D6-DMSO) δ ppm: 165.3, 157.5, 137.2, 131.5, 125.6, 120.6, 119.5, 58.5, 46.4, 31.3
logP(HCOOH): 0,80

### Herstellbeispiel 23: 2-Fluor-4-methyl-N-[(2E)-1-phenylpyrrolidin-2-yliden]-5-[(2,2,2-trifluorethyl)sulfinyl]anilin (Ib-55)

### Stufe 1: 2-Fluor-4-methyl-N-[(2E)-1-phenylpyrrolidin-2-yliden]-5-[(2,2,2-trifluorethyl)sulfanyl]anilin

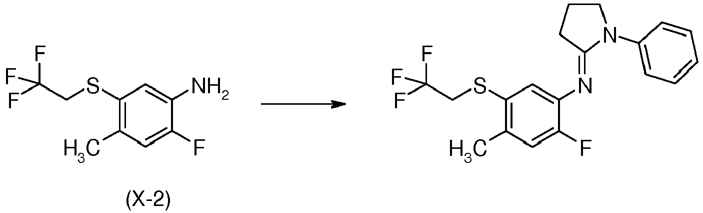

150 mg (0,63 mmol) 2-Fluor-4-methyl-5-[(2,2,2-trifluorethyl)sulfanyl]anilin und 202 mg (1,25 mmol) N-Phenyl-2-pyrrolidinon werden vorgelegt. 0,29 ml (3,14 mmmol) Phosphorylchlorid werden langsam hinzugetropft und das Reaktionsgemisch wird 2 h bei 100°C gerührt. Nach Abkühlen wird es auf Eiswasser gegossen, der pH-Wert wird auf 8-9 mit Hilfe von Natronlauge eingestellt und das Gemisch wird mit Essigester dreimal extrahiert. Die vereinten organischen Phasen werden mit gesättigter Natriumchloridlösung gewaschen, über Natriumsulfat getrocknet, filtriert und eingeeent. Der Rückstand wird in Acetonitril aufgenommen, auf RP(C-18) aufgezogen, mittels MPLC über RP(C-18) mit Wasser/Acetonitril aufgereinigt. Es werden zwei Fraktionen isoliert: 89 mg (98% Reinheit, 37% d.Th.) und 65 mg (98% Reinheit, 27% d.Th.) der Titelverbindung.
logP(HCOOH): 1,83; logP(neutral): 4,92; 1H-NMR (D6-DMSO, 400MHz) δ ppm 7,85(d,2H), 7,36(dd,2H), 7,12-7,09(m,3H), 3,92-3,84(m,4H), 2H unter dem DMSO-Peak, 2,33(s,3H), 2,05-1,97(m,2H)

### Stufe 2: 2-Fluor-4-methyl-N-[(2E)-1-phenylpyrrolidin-2-yliden]-5-[(2,2,2-trifluorethyl)sulfinyl]anilin (Ib-55)

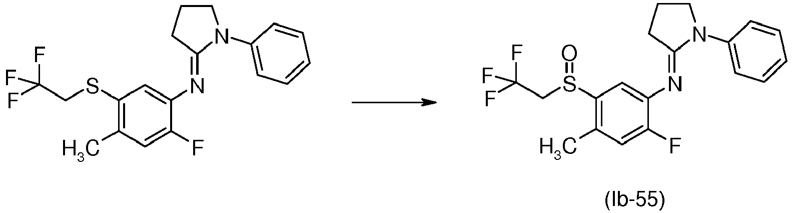

89 g (0,23 mmol) 2-Fluor-4-methyl-*N*-[(2E)-1-phenylpyrrolidin-2-yliden]-5-[(2,2,2-trifluorethyl)sulfanyl]anilin werden bei 0-4 °C in 3 mL Dichlormethan vorgelegt, 69 mg (0,28 mmol) *meta*-Chlorperbenzoesäure (70%ig) werden dazu gegeben und das Reaktionsgemisch wird 2 h bei Raumtemperatur nachgerührt. Anschließend mit einer 33%igen Natriumthiosulfatlösung (Peroxid-Test durchgeführt) und einer gesättigten Natriumhydrogencarbonatlösung versetzt und zweimal mit Dichlormethan ausgeschüttelt. Die vereinigten organischen Phasen werden mit einer gesättigten Natriumcarbonatlösung gewaschen, über Natriumsulfat getrocknet, filtriert, und das Lösemittel wird im Vakuum entfernt. Der Rückstand enthält 98 mg (94% Reinheit, 99% d.Th.) der Titelverbindung als braunes Öl.
logP(HCOOH): 1,27; logP(neutral): 3,47; 1H-NMR (D6-DMSO, 400MHz) δ ppm 7,86(d,2H), 7,37(m,3H), 7,22(d,1H), 7,09(dd,1H), 4,14-4,02(m,2H), 3,73(dd,2H), 2,67-2,57(m,2H), 2,33(s,3H), 2,07-1,99(m,2H)

### Herstellbeispiel 24: 2-({2-Fluor-4-methyl-5-[(2,2,2-trifluorethyl)sulfinyl]phenyl}imino)-3-(2,2,2-trifluorethyl)-1,3-thiazolidin-4-on (Ib-71)

### Stufe 1.a: 1-{2-Fluor-4-methyl-5-[(2,2,2-trifluorethyl)sulfanyl]phenyl}-3-(2,2,2-trifluorethyl)thioharnstoff bekannt aus JP 2011-42611 (Beispiel 250)

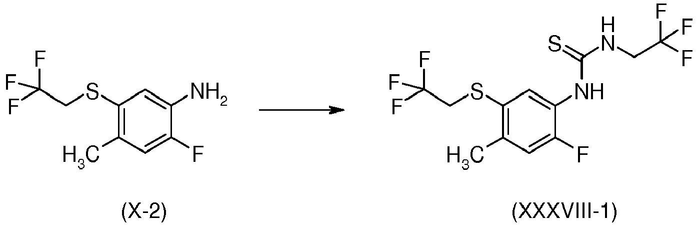

1,00 g (4,18 mmol) 2-Fluor-4-methyl-5-[(2,2,2-trifluorethyl)sulfanyl]anilin wird in 5 ml Dichlormethan vorgelegt und dazu werden 0,006 ml (0,042 mmol) Triethylamin gegeben. Nach der Zugabe von 0,59 g (4,18 mmol) 1,1,1-Trifluor-2-isothiocyanatoethan wird das Reaktionsgemisch über Nacht bei Raumtemperatur gerührt. Unter Vakuum wird das Lösungsmittel entfert, der Rückstand wird mit etwas Toluol verrührt und der unlösliche Teil wird abgesaugt und getrocknet. Es werden 0,31 g (100% Reinheit, 20% d.Th.) der Titelverbindung als weißer Feststoff erhalten. Das Filtrat wird unter Vakuum von Lösungsmittel befreit. Der Rückstand aus 1,30 g enthält die Titelverbindung mit einer Reinheit von 77%.
logP(HCOOH): 3,32; logP(neutral): 3,24; 1H-NMR (D6-DMSO, 400MHz) δ ppm 9,62(bs,1H), 8,34(bs,1H), 7,76(d,1H), 7,26(d,1H), 4,46-4,40(m,2H), 3,87(q,2H), 2,38(s,3H)

### Stufe 2.a: 2-({2-Fluor-4-methyl-5-[(2,2,2-trifluorethyl)sulfanyl]phenyl}imino)-3-(2,2,2-trifluorethyl)-1,3-thiazolidin-4-on (Ia-175)

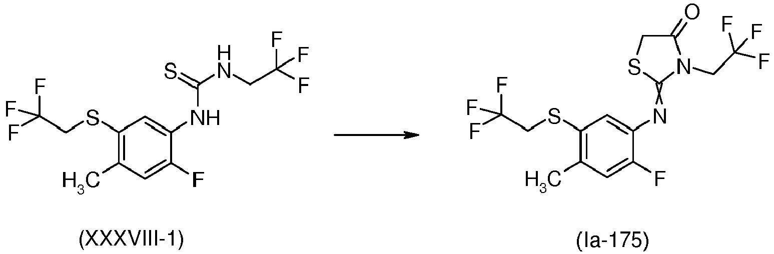

In 2 ml Toluol werden 75 mg (97% Reinheit, 0,19 mmol) 1-{2-Fluor-4-methyl-5-[(2,2,2-trifluorethyl)sulfanyl]phenyl}-3-(2,2,2-trifluorethyl)thioharnstoff und 27 mg (0,19 mmol) Bromessigsäure vorgelegt und 6 h bei Rückfluss gerührt. Nach Abkühlen wird das Reaktionsgemisch mit einer gesättigten Natriumchloridlösung versetzt, die organische Phase wird getrennt, über Natriumsulfat getrocknet und unter Vakuum vom Lösungsmittel befreit. Der Rückstand wird auf RP(C-18) Material aufgezogen und mittels MPLC über RP(C-18) mit Wasser/Acetonitril aufgereinigt. Es werden 18 mg (100% Reinheit, 23% d. Th.) der Titelverbindung als weißer Feststoff isoliert.
logP(HCOOH): 4,09; logP(neutral): 3,99; 1H-NMR (D6-DMSO, 400MHz) δ ppm 7,27(d,1H), 7,21(d,1H), 4,58(q,2H), 4,24(s,2H), 3,87(q,2H), 2,39(s,3H)

### Stufe 3.a: 2-({2-Fluor-4-methyl-5-[(2,2,2-trifluorethyl)sulfinyl]phenyl}imino)-3-(2,2,2-trifluorethyl)-1,3-thiazolidin-4-on (Ib-71)

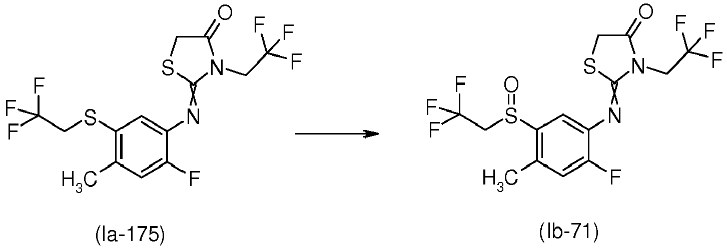

136 g (0,32 mmol) 2-({2-Fluor-4-methyl-5-[(2,2,2-trifluorethyl)sulfanyl]phenyl}imino)-3-(2,2,2-trifluorethyl)-1,3-thiazolidin-4-on werden bei 0-4 °C in 3 mL Dichlormethan vorgelegt, 84 mg (0,32 mmol) *meta*-Chlorperbenzoesäure (70%ig) werden dazu gegeben und das Reaktionsgemisch wird 2 h bei Raumtemperatur nachgerührt. Anschließend mit einer 33%igen Natriumthiosulfatlösung (Peroxid-Test durchgeführt) und einer gesättigten Natriumhydrogencarbonatlösung versetzt und zweimal mit Dichlormethan ausgeschüttelt. Die vereinigten organischen Phasen werden mit einer gesättigten Natriumcarbonatlösung gewaschen, über Natriumsulfat getrocknet, filtriert, und das Lösemittel wird im Vakuum entfernt. Der Rückstand enthält 136 mg (100% Reinheit, 96% d.Th.) der Titelverbindung als heller Öl, das mit der Zeit zum weißen Feststoff kristallisiert.
logP(HCOOH): 2,93; logP(neutral): 2,87; 1H-NMR (D6-DMSO, 400MHz) δ ppm 7,51(d,1H), 7,38(d,1H), 4,62-4,57(m,2H), 4,26-4,14(m,3H), 4,04-3,94(m,1H), 2,36(s,3H)

Alternativ kann die Synthese wie folgt durchgeführt werden:

### Stufe 1.b: 2-Fluor-4-methyl-5-[(2,2,2-trifluorethyl)sulfinyl]anilin

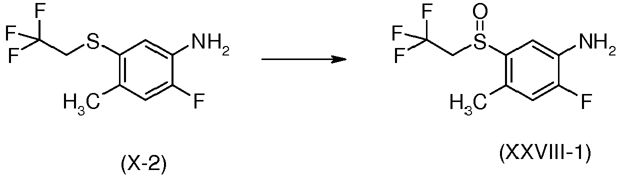

5,00 g (0,21 mmol) 2-Fluor-4-methyl-5-[(2,2,2-trifluorethyl)sulfanyl]anilin werden bei 0-4 °C in 100 ml Dichlormethan vorgelegt, 6,18 g (0,25 mmol) meta-Chlorperbenzoesäure werden dazu gegeben und das Reaktionsgemisch wird 2 h bei Raumtemperatur nachgerührt. Anschließend mit einer 33%igen Natriumthiosulfatlösung versetzt (Peroxid-Test durchgeführt) und zweimal mit Dichlormethan ausgeschüttelt. Die vereinigten organischen Phasen werden mit einer gesättigten Natriumcarbonatlösung gewaschen, über Natriumsulfat getrocknet, filtriert, und das Lösemittel wird im Vakuum entfernt. Der Rückstand enthält 5,10 g (90% Reinheit, 86% d.Th.) der Titelverbindung als brauner Öl.
logP(HCOOH): 1,77; logP(neutral): 1,72; 1H-NMR (D6-DMSO, 400MHz) δ ppm 7,26(d,1H), 7,02(d,1H), 5,45(bs,2H), 4,08-3,95(m,1H), 3,88-3,75(m,1H), 2,19(s,3H)

### Stufe 2.b: 1-{2-Fluor-4-methyl-5-[(2,2,2-trifluorethyl)sulfinyl]phenyl}-3-(2,2,2-trifluorethyl)thioharnstoff

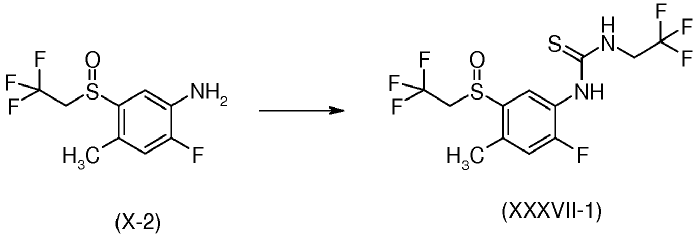

1,00 g (3,53 mmol) 2-Fluor-4-methyl-5-[(2,2,2-trifluorethyl)sulfanyl]anilin (90%ig) wird in 5 ml Dichlormethan vorgelegt und dazu werden 0,005 ml (0,035 mmol) Triehtylamin gegeben. Nach der Zugabe von 0,50 g (3,53 mmol) 1,1,1-Trifluor-2-isothiocyanatoethan wird das Reaktionsgemisch über Nacht bei Raumtemperatur gerührt. Der unlösliche Teil wird abgesaugt und getrocknet. Es werden 0,60 g (100% Reinheit, 43% d.Th.) der Titelverbindung als weißer Feststoff erhalten. Das Filtrat wird unter Vakuum von Lösungsmittel befreit. Der Rückstand aus 0,81 g enthält die Titelverbindung mit einer Reinheit von 54%.
logP(HCOOH): 2,34; logP(neutral): 2,30; 1H-NMR (D6-DMSO, 400MHz) δ ppm 9,75(bs,1H), 8,50(bs,1H), 8,12(bd,1H), 7,36(d,1H), 4,52-4,40(m,1H), 4,21-4,15(m,1H), 4,05-3,95(m,1H), 2,36(s,3H)

### Stufe 3.c: 2-({2-Fluor-4-methyl-5-[(2,2,2-trifluorethyl)sulfinyl]phenyl}imino)-3-(2,2,2-trifluorethyl)-1,3-thiazolidin-4-on (Ib-71)

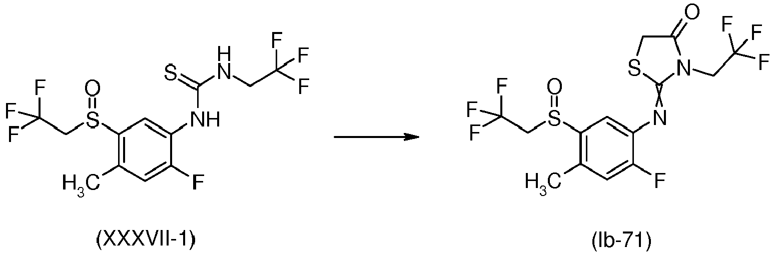

In 2 ml Toluol werden 200 mg (0,51 mmol) 1-{2-Fluor-4-methyl-5-[(2,2,2-trifluorethyl)sulfinyl]phenyl}-3-(2,2,2-trifluorethyl)thioharnstoff und 70 mg (0,51 mmol) Bromessigsäure vorgelegt und 6 h bei Rückfluss verrührt. Das Reaktionsgemisch wird mit einer gesättigten Natriumchloridlösung versetzt, die organische Phase wird getrennt, über Natriumsulfat getrocknet und unter Vakuum vom Lösungsmittel befreit. Der Rückstand wird auf RP(C-18) Material aufgezogen und mittels MPLC über RP(C-18) mit Wasser/Acetonitril aufgereinigt. Es werden 63 mg (97% Reinheit, 28% d. Th.) der Titelverbindung als weißer Feststoff isoliert.

### Herstellbeispiel 25: Synthese von 3,4-Dimethyl-5-(methylsulfanyl)-1,2,4-thiadiazol-4-iummethylsulfat

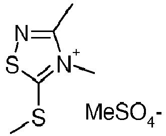

### Stufe 1: 3,4-Dimethyl-1,2,4-thiadiazol-5(4H)-on

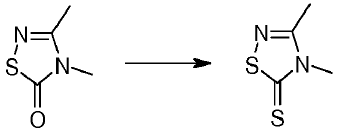

Zu einer Lösung aus 1,80 g (13,80 mmol) 3,4-Dimethyl-1,2,4-thiadiazol-5(4H)-on (roh, hergestellt nach J. Chem. Soc. Perkin Trans. 1 1983, 4, 687-691) in Xylol werden bei 0 °C 6,13 g (27,6 mmol) Phosphorpentasulfid gegeben und anschließend 4 h bei 100 °C erhitzt. Nach wässriger Aufarbeitung und chromatographischer Aufreinigung erhält man 0,60 g (30% d.Th.) der Titelverbindung.

### Stufe 2: 3,4-Dimethyl-5-(methylsulfanyl)-1,2,4-thiadiazol-4-iummethylsulfat

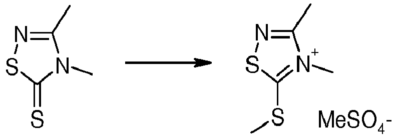

0,62 g (4,52 mmol) Dimethylsulfat werden unter Rühren zu einer Lösung aus 0,60 g (4,10 mmol) 3,4-Dimethyl-1,2,4-thiadiazol-5(4H)-thion in Acetonitril gegeben und 5 h bei Rückfluss gerührt. Nach Abkühlen wird das Lösungsmittel unter Vakuum entfernt und der erhaltene Rückstand (1,50 g) wird roh weiter umgesetzt.

### Herstellbeispiel 26: N-(3,4-Dimethyl-1,2,4-thiadiazol-5(4H)-yliden)-2,4-dimethyl-5-[(2,2,2-trifluorethyl)sulfinyl]anilin (Ib-173)

### Stufe 1: N-(3,4-Dimethyl-1,2,4-thiadiazol-5(4H)-yliden)-2,4-dimethyl-5-[(2,2,2-trifluorethyl)sulfanyl]anilin

Gemäß den oben beschriebenen Herstellverfahren lassen sich die Verbindungen der Formel (I) erhalten, beispielsweise die folgenden Verbindungen der Formel (I):

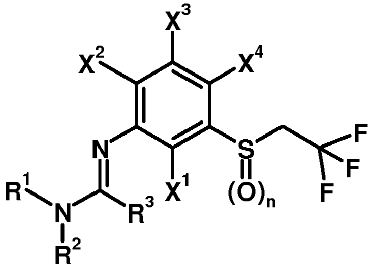

| **Bsp** | **n** | **R¹** | **R²** | **R³** | **X¹** | **X²** | **X³** | **X⁴** | **Log P** |
|---|---|---|---|---|---|---|---|---|---|
| Ia-01 | 0 | CH₃ | CH₃ | CF₃ | H | F | H | CH₃ | 4,48^{[a]};4,5^{[b]} |
| Ia-02 | 0 | Propyl | H | CF₃ | H | F | H | CH₃ | 4,45^{[a]};4,43^{[b]} |
| Ia-03 | 0 | H | H | CHF₂ | H | F | H | CH₃ | 2,2^{[c]} |
| Ia-04 | 0 | Pyridin-2-ylmethyl | H | CF₃ | H | F | H | CH₃ | 3,68^{[a]} |
| Ia-05 | 0 | Propan-2-yl | H | CF₃ | H | F | H | CH₃ | 4,51^{[a]};4,52^{[b]} |
| Ia-06 | 0 | H | H | PD-F-Heptyl | H | F | H | CH₃ | |
| Ia-07 | 0 | H | H | CF₃ | H | CH₃ | H | CH₃ | 3,36^{[a]};3,4^{[b]} |
| Ia-08 | 0 | Pyridin-2-ylmethyl | Pyridin-2-ylmethyl | CF₃ | H | F | H | CH₃ | |
| Ia-09 | 0 | CH₃ | H | CF₃ | H | CH₃ | H | F | 3,72^{[a]};3,69^{[b]} |
| Ia-10 | 0 | CH₃ | CH₃ | DD-F-Hexyl | H | F | H | CH₃ | |
| Ia-11 | 0 | Methylsulfonyl | H | CF₃ | H | F | H | CH₃ | 3,05^{[a]} |
| Ia-12 | 0 | CH₃ | H | CF₃ | H | CH₃ | H | CH₃ | 3,95^{[a]};4,01^{[b]} |
| Ia-13 | 0 | CH₃ | H | CF₃ | H | F | H | CH₃ | 3,7^{[a]};3,71^{[b]} |
| Ia-14 | 0 | H | H | CF₃ | H | Cl | H | Cl | |
| Ia-15 | 0 | CH₃ | H | CF₃ | H | Cl | H | Cl | 4,2^{[a]};4,2^{[b]} |
| Ia-16 | 0 | (Trifluormethyl)sulfonyl | H | CF₃ | H | F | H | CH₃ | |
| Ia-17 | 0 | H | H | CF₃ | H | CH₃ | H | F | 3,16^{[a];}3,14^{[b]} |
| Ia-18 | 0 | Ethyl | H | CF₃ | H | F | H | CH₃ | 4,12^{[a]};4,12^{[b]} |
| Ia-19 | 0 | CH₃ | H | CF₃ | H | H | H | CF₃ | |
| Ia-20 | 0 | CH₃ | H | CF₃ | H | Cl | H | CH₃ | |
| Ia-21 | 0 | H | H | CF₃ | H | F | H | CH₃ | 3,13^{[a]};3,17^{[a]};3,12^{[b]} |
| Ia-22 | 0 | H | H | DD-F-Hexyl | H | F | H | CH₃ | |
| Ia-23 | 0 | CH₃ | CH₃ | CF₃ | H | CH₃ | H | CH₃ | 4,87^{[a]};4,87^{[b]} |
| Ia-24 | 0 | CH₃ | CH₃ | CF₃ | H | CH₃ | H | F | 4,53^{[a]};4,47^{[b]} |
| Ia-25 | 0 | H | H | CF₃ | H | H | H | CF₃ | |
| Ia-26 | 0 | Cpr | H | CF₃ | H | F | H | CH₃ | 4,03^{[a]};4,02^{[b]} |
| Ib-01 | 1 | CH₃ | CH₃ | CF₃ | H | F | H | CH₃ | |
| Ib-02 | 1 | Ethyl | H | CF₃ | H | F | H | CH₃ | |
| Ib-03 | 1 | Propan-2-yl | | H | C F₃ | H | F | H | CH₃ |
| Ib-04 | 1 | CH₃ | H | CF₃ | H | CH₃ | H | CH₃ | 3,15^{[a]};3,09^{[b]} |
| Ib-05 | 1 | CH₃ | H | CF₃ | H | CH₃ | H | F | 2,86^{[a]};2,79^{[b]} |
| Ib-06 | 1 | Propyl | H | CF₃ | H | F | H | CH₃ | 3,24^{[a]};3,17^{[b]} |
| Ib-07 | 1 | CH₃ | H | CF₃ | H | F | H | CH₃ | 2,64^{[a]};2,61^{[b]} |
| Ib-08 | 1 | Cpr | H | CF₃ | H | F | H | CH₃ | 2,68^{[a]};2,67^{[b]} |
| Ib-09 | 1 | CH₃ | CH₃ | CF₃ | H | CH₃ | H | CH₃ | 3,23^{[a]};3,19^{[b]} |
| Ib-10 | 1 | CH₃ | H | CF₃ | H | H | H | CF₃ | 2,52^{[a]};2,49^{[b]} |
| Ib-11 | 1 | H | H | CF₃ | H | F | H | CH₃ | 2,77^{[a]};2,71^{[b]} |
| Ib-12 | 1 | H | H | CF₃ | H | CH₃ | H | CH₃ | 3,36^{[a]};3,3^{[b]} |
| Ib-13 | 1 | H | H | CF₃ | H | CH₃ | H | F | |
| Ib-14 | 1 | H | H | CF₃ | H | Cl | H | Cl | 2,05^{[a]};2,02^{[b]} |
| Ib-15 | 1 | CH₃ | CH₃ | CF₃ | H | CH₃ | H | F | 2,17^{[a]};2,14^{[b]} |
| Ib-16 | 1 | H | H | CF₃ | H | H | H | CF₃ | 2,2^{[a]};2,19^{[b]} |

### Abkürzungen:

PD-F-Heptyl = Pentadecafluorheptyl;DD-F-Hexyl = 1,1,2,2,3,3,4,4,5,5,6,6-Dodecafluorhexyl;CHF₂ = Difluormethyl;Cpr = Cyclopropyl;Cl = Chlor;F = Fluor.

Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden:
^{[a]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. Ist auch logP(HCOOH) genannt.
^{[b]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. Ist auch logP(neutral) genannt.
^{[c]} Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

Die NMR-Daten ausgewählter Beispiele werden entweder in klassischer Form (δ-Werte, Anzahl der H-Atome, Multiplettaufspaltung) oder als NMR-Peak-Listen aufgeführt.
NMR-Peak-Listenverfahren

Wenn die 1H-NMR-Daten ausgewählter Beispiele in Form von 1H-NMR-Peaklisten notiert werden, wird zu jedem Signalpeak erst der δ-Wert in ppm und dann die Signalintensität durch ein Leerzeichen getrennt aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ ₁ Intensität₁;δ₂ IntenSität₂; ........ ;δᵢ Intensitätᵢ;......; δₙ Intensitätₙ

Das Lösungsmittel, in welchem das NMR-Spektrum aufgenommen wurde, wird in eckigen Klammern hinter der Nummer des Beispieles und vor der NMR-Peakliste bzw. der klassischen NMR-Interpretationsliste aufgeführt.

| **Beispiels-nummer** | **1H-NMR Daten** |
|---|---|
| Ia-01 | 1H-NMR(D6-DMSO): 7,12(d,1H), 7,07(d,1H), 3,84-3,92(m,2H), 2,98(breit,6H), 2,33(s,3H) |
| Ia-02 | 1H-NMR (CDCl3): 7,03-7,09(m,1H), 6,91-6,93(m,1H), 5,29(breit,1H), 3,28-3,38 (m,4H), 2,42(s,3H), 1,69 (breit, 2H), 1,01(breit,2H), 0,83-0,88(m,1H) |
| Ia-03 | 1H-NMR(D6-DMSO): 7,11-7,14(m,1H), 7,06-7,04(m,1H), 6,92-6,98(breit,2H), 6,30(tt,1H), 3,83-3,91(q,2H), 2,34(s,3H) |
| Ia-05 | [CDCl₃] 7.26 45.31;7.09 0.35;7.03 0.72;6.93 0.95;6.91 0.85;5.09 0.50;4.13 0.50;3.30 1.93;3.28 1.82;2.42 16.00;1.57 44.22;1.43 0.36;1.33 0.54;1.29 4.62;1.28 4.60;1.26 1.33;1.25 1.06;1.25 1.27;1.23 0.74;1.08 0.47;0.01 0.72;0.00 24.21;-0.01 0.77 |
| Ia-07 | 1H-NMR(D6-DMSO): 7,10(s,1H), 6,98(breit, 2H), 6,85(s,1H), 3,87(q,2H), 2,30(s,3H), 1,98(s,3H) |
| Ia-09 | [DMSO-D₆] 7.09 3.30;7.07 3.26;6.87 0.35;6.85 0.33;4.06 0.56;4.04 1.74;4.02 1.77;4.00 0.62;3.92 0.37;3.86 1.00;3.35 143.04;3.34 312.18;2.79 0.68;2.78 0.62;2.74 0.37;2.73 0.35;2.68 0.56;2.67 0.68;2.67 0.49;2.54 0.38;2.53 0.96;2.51 54.21;2.50 72.95;2.34 0.46;2.33 0.64;2.33 0.79;2.33 0.64;2.32 0.46;2.07 1.95;2.04 0.35;2.01 16.00;1.99 8.08;1.36 0.69;1.24 0.46;1.19 2.26;1.17 4.49;1.16 2.17;0.88 0.50;0.86 0.36;0.00 9.11 |
| Ia-12 | [DMSO-D₆] 7.01 4.28;4.03 0.82;4.02 0.83;3.79 0.35;3.37 0.42;3.34 331.71:3.33 0.37;3.32 2.34;2.79 0.50;2.62 0.35;2.61 0.48;2.61 0.35;2.52 0.66;2.52 0.84;2.52 0.90;2.51 23.83;2.51 51.40;2.50 70.93;2.50 51.33;2.50 23.51;2.44 0.53;2.39 0.35;2.39 0.53;2.38 0.38;2.36 0.58;2.33 0.49;2.31 0.74;2.28 16.00;2.19 0.55;1.99 3.80;1.97 7.19;1.19 0.93;1.17 1.84;1.16 0.90;0.01 0.74;0.00 23.54;-0.01 0.71 |
| Ia-13 | [CDCl₃] 7.26 51.97;7.09 0.34;7.04 0.43;6.93 0.64;6.92 0.56;3.73 0.51;3.72 0.51;3.49 0.37;3.30 1.06;3.29 1.10;3.29 0.94;3.28 0.80;3.01 1.54;2.42 16.00;2.36 0.92;1.57 10.32;1.26 0.76;1.25 1.28;1.23 0.63;0.01 0.86;0.00 28.20;-0.01 0.93 |
| Ia-14 | 1H-NMR(D6-DMSO): 7,67(s,1H), 7,15(s,1H), 4,16(q,2H) |
| Ia-15 | 1H-NMR(D6-DMSO): 8,30(breit,1H), 7,61(s,1H), 7,20(s,1H), 4,14(q,2H), 2,62(breit,3H) |
| Ia-17 | 1H-NMR(D6-DMSO): 7,15(d,1H), 7,10(breit, 2H), 6,92(d,1H), 3,90(q,2H), 2,01(s,3H) |
| Ia-18 | [CDCl₃] 7.26 18.05;7.26 0.39;7.25 0.35;7.24 0.34;7.04 0.64;6.94 0.46;6.93 1.00;6.92 0.76;6.91 0.76;6.84 0.35;6.82 0.33;5.26 0.47;3.45 1.08;3.41 0.41;3.39 0.32;3.30 1.56;3.28 1.39;2.47 0.74;2.42 16.00;1.58 15.92;1.29 1.95;1.26 0.51;1.25 0.43;1.25 0.48;1.09 0.37;0.00 9.63;-0.01 0.33 |
| Ia-19 | 1H-NMR (CDCl3): 7,58 (d,1H), 7,13 (s,1H), 6,85 (d,1H), 5,31 (breit,1H), 3,45 (q,2H), 2,93 (breit,3H) |
| Ia-21 | 1H-NMR(D6-DMSO): 7,17(d,1H), 7,09(d,1H), 3,91(q,2H), 2,34(s,3H) |
| Ia-23 | 1H-NMR(D6-DMSO): 7,02(s,1H), 6,79(s,1H), 3,83(q,2H), 2,87(breit,6H), 2,28(s,3H), 1,96(s,3H) |
| Ia-24 | 1H-NMR(D6-DMSO): 7,09(d,1H), 6,86(breit,1H), 3,89(q,2H), 2,90(breit,3H), 2,00(s,3H) |
| Ia-25 | 1H-NMR (CDCl3): 7,72 (d,1H), 7,24 (m,1H), 6,96-6,99 (m,1H), 4,98 (breit,2H), 3,48(q,2H) |
| Ia-26 | [CDCl₃] 7.26 29.38;6.94 1.54;6.92 1.55;3.73 0.38;3.72 0.38;3.49 0.34;3.33 0.70;3.32 0.83;2.47 0.43;2.42 16.00;1.57 6.79;1.26 0.58;1.25 1.07;1.23 0.54;0.48 0.53;0.43 0.39;0.01 0.45;0.00 15.80;-0.01 0.53 |

| **Beispiels-nummer** | **1H-NMR Daten** |
|---|---|
| Ib-01 | 1H-NMR(D6-DMSO): 7,29(d,1H), 7,23(d,1H), 4,02-4,14(m,2H), 3,01(breit,6H), 2,31(s,3H) |
| Ib-02 | 1H-NMR(D6-DMSO): 8,30(breit,1H), 7,29(d,1H), 7,22(d,1H), 4,08-4,14(m,2H), 3,99(breit,2H), 2,31(s,3H), 1,11(breit,3H) |
| Ib-03 | 1H-NMR(D6-DMSO): 8,02(breit,1H), 7,28(d,1H), 7,21(d,1H), 4,08-4,14(m,2H), 3,96-4,00(m,1H), 2,31(s,3H), 1,12-1,17(breit,6H) |
| Ib-04 | 1H-NMR(D6-DMSO): 8,01(breit,1H), 7,00-7,20(m,2H), 3,70-4,10(m,2H), 2,80(breit,3H), 2,27(s,3H), 2,07(s,3H) |
| Ib-05 | [DMSO-D₆] 7.76 0.68;7.75 0.68;7.48 0.42;7.46 0.41;7.27 5.26;7.26 5.26;7.09 0.34;7.02 0.49;4.32 0.34;4.30 0.38;4.27 0.47;4.26 0.80;4.24 1.46;4.22 2.04;4.20 1.69;4.18 0.75;3.35 821.56;3.34 2.15;3.33 2.32;3.17 0.58;3.16 0.56;2.81 0.80;2.73 0.36;2.62 0.57;2.62 1.01;2.62 1.31;2.61 0.98;2.61 0.52;2.54 1.16;2.52 3.60;2.52 4.50;2.52 5.20;2.51 63.77;2.51 130.55;2.50 175.10;2.50 122.46;2.50 53.43;2.48 0.53;2.39 0.67;2.39 1.11;2.39 1.41;2.38 1.11;2.38 0.73;2.37 6.18;2.33 0.38;2.31 0.37;2.27 4.25;2.11 16.00;2.09 5.30;2.08 1.22;2.02 0.44;1.91 0.99;1.23 0.51;1.14 1.46;0.01 0.51;0.00 12.92 |
| Ib-06 | 1H-NMR(D6-DMSO): 7,28(d,1H), 7,22(d,1H), 4,10-4,14(m,1H), 3,90-4,09(m,1H), 3,10-3,30(breit,2H), 2,31(s,3H), 1,51-1,56(breit,2H), 0,84-0,91(breit,3H) |
| Ib-07 | 1H-NMR(D6-DMSO): 7,30(d,1H), 7,22(d,1H), 3,99-4,15(m,2H), 2,66-2,73(breit,3H), 2,31(s,3H) |
| Ib-08 | [DMSO-D₆] 8.43 0.75;7.39 0.32;7.22 1.80;7.20 1.79;4.37 0.45;4.13 0.35;4.11 0.82;4.09 0.84;4.07 0.47;4.05 0.32;4.03 0.53;4.02 0.49;3.45 0.50;3.44 0.51;3.44 0.56;3.43 0.58;3.43 0.36;3.42 0.38;3.40 0.48;3.40 0.57;3.39 1.02;3.38 1.14;3.35 1478.79;3.33 18.72;2.62 0.34;2.62 0.79;2.62 1.07;2.61 0.78;2.61 0.36;2.54 0.67;2.52 1.59;2.52 2.06;2.52 2.16;2.51 57.91;2.51 127.88;2.50 173.56;2.50 123.42;2.50 53.41;2.39 0.33;2.39 0.75;2.39 1.04;2.38 0.74;2.38 0.33;2.31 16.00;2.08 2.40;1.99 1.93;1.23 0.45;1.19 0.50;1.17 1.09;1.16 0.49;1.07 0.79;1.05 1.55;1.04 0.73;0.42 0.41;0.41 0.44;0.01 0.39;0.00 13.49;-0.01 0.41 |
| Ib-09 | 1H-NMR(D6-DMSO): 7,12(s,1H), 7,03(s,1H), 4,03-4,07(m,2H), 2,91(breit,6H), 2,27(s,3H), 2,07(s,3H) |
| Ib-10 | 1H-NMR (CDCl3): 7,63-7,71 (m,2H), 7,07 (d,1H), 5,63 (breit,1H), 3,25-3,58 (m,2H), 2,94 (d,3H) |
| Ib-11 | 1H-NMR(D6-DMSO): 7,37(d,1H), 7,28(d,1H), 4,03-4,10(m,2H), 2,34(s,3H) |
| Ib-12 | 1H-NMR(D6-DMSO): 7,19(s,1H), 7,17(s,1H), 3,96-4,04(m,2H), 2,30(s,3H), 2,07(s,3H) |
| Ib-13 | 1H-NMR(D6-DMSO): 7,31(d,1H), 7,10(d,1H), 4,23-4,29(m,1H), 4,13-4,18(m,1H), 2,10(s,3H) |
| Ib-14 | 1H-NMR(D6-DMSO): 7,87(s,1H), 7,54(breit,2H), 7,33(s,1H), 4,10-4,18(m,2H) |
| Ib-15 | 1H-NMR(D6-DMSO): 7,27(d,1H), 6,98(d,1H), 4,23(m,2H), 2,94(breit,6H), 2,09(s,3H) |

| **Beispiels nummer** | **Struktur** | **Analytische Daten** |
|---|---|---|
| Ia-27 | 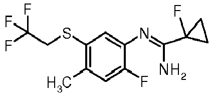 | 1H-NMR(D6-DMSO): 7,11-7,08(m,1H), 7,04-7,02(m,1H), 6,68(breit,2H), 3,89-3,81(q,2H), 2,33(s,3H), 1,37-1,30(m,4H) |
| Ia-28 | 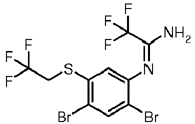 | 1H-NMR(D6-DMSO): 7,87(s,1H), 7,60-7,27(breit,2H), 7,10(s,1H), 4,15(q,2H) |
| Ia-29 | 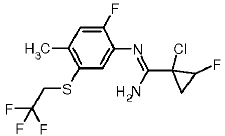 | 1H-NMR(D6-DMSO): 7,10-7,07(m,1H), 7,00-6,98(m,1H), 6,60(breit,2H), 5,03(m,1H), 3,88-3,80(q,2H), 2,32(s,3H), 2,05-1,98(m,1H), 1,72-1,63(m,1H) |
| Ia-30 | 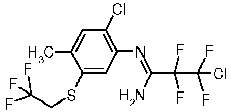 | 1H-NMR(D6-DMSO): 7,37(s,1H), 7,26(breit,2H), 6,99(s,1H), 4,00-3,92(q,2H), 2,32(s,3H) |
| Ia-31 | 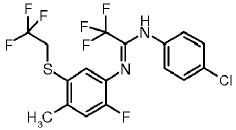 | logP(HCOOH)=4,73, logP(neutral)=4,67 |
| Ia-32 | 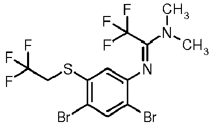 | 1-H-NMR(D6-DMSO): 7,82(s,1H), 7,12(s,1H), 4,14(q,2H), 3,00(s,6H) |
| Ia-33 | 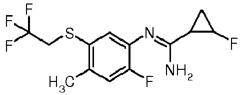 | 1H-NMR(D6-DMSO): 7,06-7,03(m,1H), 6,94-6,92(m,1H), 6,37(breit,2H), 4,93-4,76(m,1H), 3,86-3,78(q,2H), 2,31(s,3H), 2,09-2,05(m,1H), 1,42-1,24(m,2H) |
| Ia-34 | 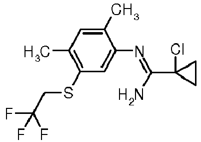 | 1H-NMR(D6-DMSO): 7,03(s,1H), 6,76(s,1H), 5,99(breit,2H), 3,86-3,78(q,2H), 2,29(s,3H), 1,91(s,3H), 1,59-1,55(m,2H), 1,29-1,26(m,2H) |
| Ia-35 | 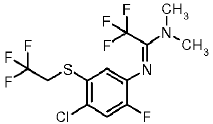 | 1H-NMR(D6-DMSO): 7,48(d,1H), 7,20(d,1H), 4,05(q,2H), 3,01(s,6H) |
| Ia-36 | 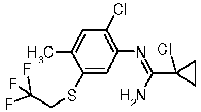 | 1H-NMR(D6-DMSO): 7,29(m,1H), 6,94(m,1H), 6,32(breit,2H), 3,97-3,89(q,2H), 2,29(s,3H), 1,60(m,2H), 1,28(m,2H) |
| Ia-37 | 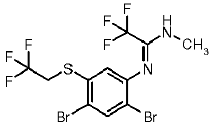 | 1H-NMR(D6-DMSO): 8,22(breit,1H), 7,82(s,1H), 7,16(s,1H), 4,13(q,2H), 2,65(bs,3H) |
| Ia-38 | 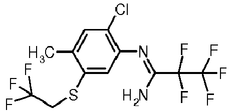 | 1H-NMR(D6-DMSO): 7,37(s,1H), 7,32(breit,2H), 7,00(s,1H), 4,01-3,93(q,2H), 2,31(s,3H) |
| Ia-39 | 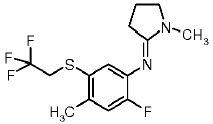 | 1H-NMR(D6-DMSO): 7,01(m,2H), 3,84(q,2H), 3,38-3,32(m,2H), 2,88(s,3H), 2,30(s,3H), 2,25(t,2H), 1,88(q,2H) |
| Ia-40 | 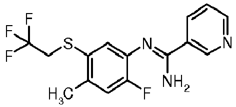 | 1H-NMR(D6-DMSO): 9,12-9,11(m,1H), 8,68-8,67(m,1H), 8,32-8,28(m,1H), 7,50-7,47(m,1H), 7,17-7,12(m,2H), 6,79(breit,2H), 3,93-3,85(q,2H), 2,36(s,3H) |
| Ia-41 | 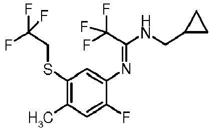 | logP(HCOOH)= 4,62 |
| Ia-42 | 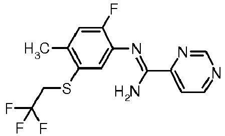 | 1H-NMR(D6-DMSO): 9,32(m,1H), 9,00-8,98(m,1H), 8,27-8,26(m,1H), 7,19-7,16(m,2H), 7,05-6,98(breit,2H), 3,93-3,85(q,2H), 2,37(s,3H) |
| Ia-43 | 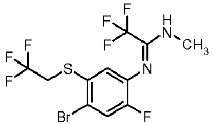 | 1H-NMR(D6-DMSO): 8,32(bs,1H), 7,58(d,1H), 7,22-7,20(m,1H), 4,05(q,2H), 2,80-2,60(breit,3H) - Peaks vom Hauptisomer |
| Ia-44 | 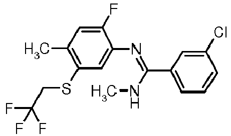 | 1H-NMR(D6-DMSO): 7,42-7,35(m,2H), 7,28-7,24(m,2H), 7,08-7,06(m,1H), 6,88-6,83(m,2H), 3,65-3,57(q,2H), 2,86-2,85(d,3H), 2,21(s,3H) |
| Ia-45 | 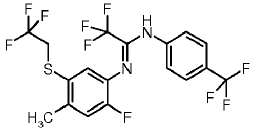 | LogP(HCOOH)=4,82, logP(neutral)=4,71 |
| Ia-46 | 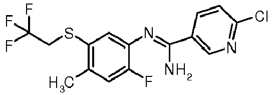 | 1H-NMR(D6-DMSO): 8,94(m,1H), 8,37-8,34(m,1H), 7,63-7,61(m,1H), 7,17-7,12(m,2H), 6,86(breit,2H), 3,92-3,84(q,2H), 2,36(s,3H) |
| Ia-47 | 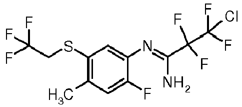 | 1H-NMR(D6-DMSO): 7,35(breit,2H), 7,17-7,15(m,1H), 7,06-7,04(m,1H), 3,92-3,85(q,2H), 2,35(s,3H) |
| Ia-48 | 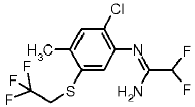 | 1H-NMR(D6-DMSO): 7,34(m,1H), 6,99(s,1H), 6,84(breit,2H), 6,31(t,1H), 3,99-3,91(q,2H), 2,30(s,3H) |
| Ia-49 | 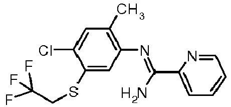 | 1H-NMR(D6-DMSO): 8,63-8,64(m,1H), 8,34-8,32(m,1H), 7,98-7,94(m,1H), 7,59-7,55(m,1H), 7,36(s,1H), 7,02(s,1H), 6,60(breit,2H), 4,05-3,98(q,2H), 2,05(s,3H) |
| Ia-50 | 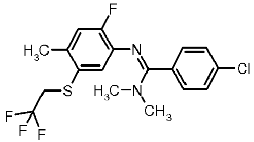 | 1H-NMR(D6-DMSO): 7,51-6,74(m,6H), 3,64-3,56(q,2H), 2,98(s,3H), 2,89(s,3H), 2,18(s,3H) |
| Ia-51 | 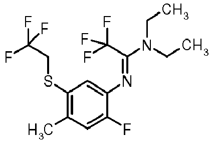 | 1H-NMR(D6-DMSO): 7,10(d,1H), 7,04(d,1H), 3,86(q,2H), 3,40-3,36(m,4H), 2,32(s,3H), 1,15-1,11(m,6H) |
| Ia-52 | 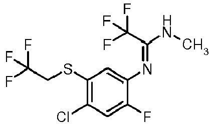 | 1H-NMR(D6-DMSO): 8,32(bs,1H), 7,49-7,44(m,1H), 7,32-7,21(m,1H), 4,04(q,2H), 2,70(bs,3H) |
| Ia-53 | 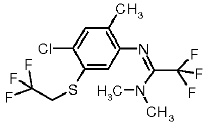 | 1H-NMR(D6-DMSO): 7,29(s,1H), 6,90(s,1H), 4,04-3,98(q,2H), 2,92(breit,6H), 1,98(s,3H) |
| Ia-54 | 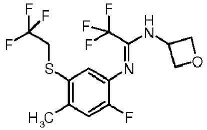 | 1H-NMR(D6-DMSO): 8,86(breit,1H), 7,13-7,05(breit,2H), 4,77(breit,2H), 4,60(breit,2H), 4,40(breit,1H), 3,85(breit,2H), 2,33(s,3H) |
| Ia-55 | 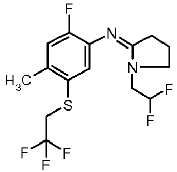 | 1H-NMR(D6-DMSO): 7,08-7,04(m,2H), 6,23(tt,1H), 3,87-3,77(m,4H), 3,48-3,46(t,2H), 2,31(s,3H), 2,32-2,29(t,2H);1,94-1,90(m,2H) |
| Ia-56 | 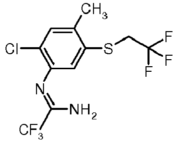 | 1H-NMR(D6-DMSO): 7,37(s,1H), 7,27(breit,2H), 7,03(s,1H), 4,03-3,95(q,2H), 2,31(s,3H) |
| Ia-57 | 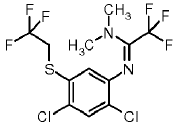 | 1H-NMR(D6-DMSO): 7,62(s,1H), 7,17(s,1H), 4,17-4,12(m,2H), 3,00(s,6H) |
| Ia-58 | 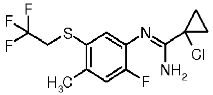 | 1H-NMR(D6-DMSO): 7,10-7,07(m,1H), 7,00-6,98(m,1H), 6,42(breit,2H), 3,88-3,80(q,2H), 2,32(s,3H), 1,57(m, 2H), 1,29(m,2H) |
| Ia-59 | 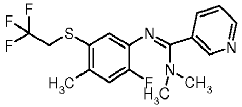 | 1H-NMR(D6-DMSO): 8,46-8,44(m,1H), 8,32-8,31(m,1H), 7,60-7,58(m,1H), 7,33-7,30(m,1H), 6,83-6,80(m,2H), 3,69-3,61(q,2H), 3,01(s,3H), 2,93(s,3H), 2,17(s,3H) |
| Ia-60 | 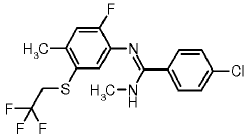 | 1H-NMR(D6-DMSO): 7,37-7,32(m,3H), 7,19-7,17(m,2H), 6,88-6,85(m,1H), 6,80-6,78(m,1H), 3,64-3,56(q,2H), 2,86-2,85(d,3H), 2,21(s,3H) |
| Ia-61 | 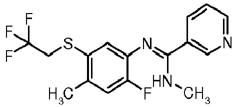 | 1H-NMR(D6-DMSO): 8,47-8,46(m,1H), 8,36(m,1H), 7,57-7,55(m,1H), 7,51-7,48(m,1H), 7,30-7,27(m,1H), 6,89-6,83(m,2H), 3,70-3,62(q,2H), 2,89-2,88(d,3H), 2,20(s,3H) |
| Ia-62 | 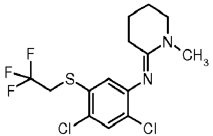 | 1H-NMR(D6-DMSO): 7,54(s,1H), 7,02(breit,1H), 4,11(q,2H), 3,28(t,2H), 2,96(s,3H), 2,08(t,2H), 1,77-1,71(m,2H), 1,62-1,56(m,2H) |
| Ia-63 | 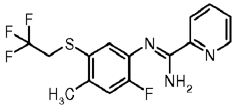 | 1H-NMR(D6-DMSO): 8,65-8,64(m,1H), 8,31-8,29(m, 1H), 7,98-7,94(m,1H), 7,59-7,56(m,1H), 7,17-7,14(m,2H), 6,80(breit,2H), 3,93-3,85(q,2H), 2,36(s,3H) |
| Ia-64 | 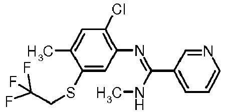 | 1H-NMR(D6-DMSO): 8,46-8,45(m,1H), 8,37(s,1H), 7,57-7,55(m,1H), 7,49-7,48(breit,1H), 7,30-7,27(m,1H), 7,13(s,1H), 6,78(s,1H), 3,73-3,65(q,2H), 2,90-2,88(d,3H), 2,17(s,3H) |
| Ia-65 | 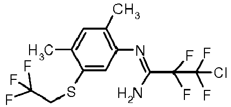 | 1H-NMR(D6-DMSO): 7,10(s,1H), 6,98(breit,2H), 6,83(s,1H), 3,89-3,81(q,2H), 2,31(s,3H), 1,98(s,3H) |
| Ia-66 | 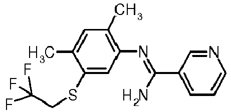 | 1H-NMR(D6-DMSO): 9,14(m,1H), 8,67-8,66(m,1H), 8,33-8,30(m,1H), 7,50-7,46(m,1H), 7,10(s,1H), 6,91(s,1H), 6,41(breit,2H), 3,89-3,81(q,2H), 2,33(s,3H), 2,04(s,3H) |
| Ia-67 | 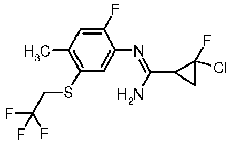 | 1H-NMR(D6-DMSO): 7,09-7,06(m,1H), 6,94-6,92(m,1H), 6,53(breit,2H), 3,86-3,78(m,2H), 2,67(m,1H), 2,33(s,3H), 1,96-1,90(m,1H), 1,76-1,70(m,1H) |
| Ia-68 | 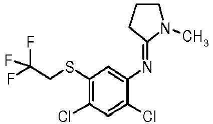 | 1H-NMR(D6-DMSO): 7,53(s,1H), 7,10(s,1H), 4,11(q,2H), 3,39(t,2H), 2,90(s,3H), 2,26(t,2H), 1,90(q,2H) |
| Ia-69 | 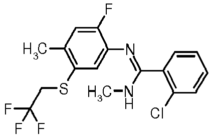 | 1H-NMR(D6-DMSO): 8,33(breit,1H), 7,50-7,24(m,4H), 6,88-6,85(m,1H), 6,76-6,73(m,1H), 3,49-3,45(q,2H), 2,89-2,88(d,3H), 2,18(s,3H) |
| Ia-70 | 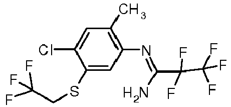 | 1H-NMR(D6-DMSO): 7,36(s,1H), 7,22(breit,2H), 6,92(s,1H), 4,07-3,99(q,2H), 1,98(s,3H) |
| Ia-71 | 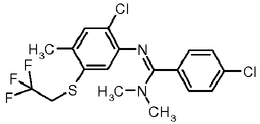 | 1H-NMR(D6-DMSO): 7,35-7,32(m,2H), 7,19-7,16(m,2H), 7,09(s,1H), 6,70(s,1H), 3,68-3,60(q,2H), 2,90(breit,6H), 2,15(s,3H) |
| Ia-72 | 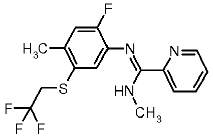 | 1H-NMR(D6-DMSO): 8,55-8,54(m,1H), 8,04-7,96(m,2H), 7,72(m,1H), 7,60-7,57(m,1H), 7,37(m,1H), 6,93-6,90(m,1H), 3,63(q,2H), 2,83-2,81(d,3H), 2,24(s,3H) |
| Ia-73 | 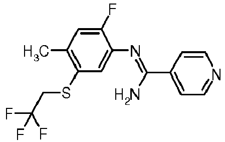 | 1H-NMR(D6-DMSO): 8,69-8,68(m,2H), 7,90-7,89(m,2H), 7,17-7,11(m,2H), 6,85(breit,2H), 3,93-3,85(q,2H), 2,36(s,3H) |
| Ia-74 | 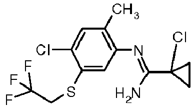 | 1H-NMR(D6-DMSO): 7,27(s,1H), 6,84(s,1H), 6,21(breit,2H), 4,04-3,94(q,2H), 1,93(s,3H), 1,30-1,15(m,4H) |
| Ia-75 | 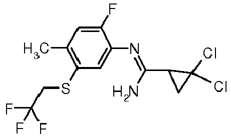 | 1H-NMR(D6-DMSO): 7,09-7,06(m,1H), 6,94-6,92(m,1H), 6,65(breit,2H), 3,85-3,77(q,2H), 2,68-2,62(m,1H), 2,33(s,3H), 2,17-2,14(m,1H), 1,91-1,87(m,1H) |
| Ia-76 | 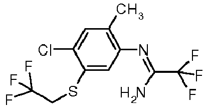 | 1H-NMR(D6-DMSO): 7,36(m,1H), 7,15(breit,2H), 6,95(s,1H), 4,08-4,00(q,2H), 1,99(s,3H) |
| Ia-77 | 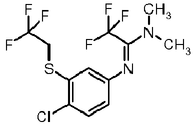 | 1H-NMR(D6-DMSO): 7,33(d,1H), 6,99(d,1H),6,64-6,61(m,1H), 4,15-4,07 (m,2H),2,92(s,6H) |
| Ia-78 | 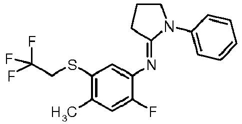 | 1H-NMR(D6-DMSO): 7,85(d,2H), 7,36(dd,2H), 7,12-7,09(m,3H), 3,92-3,84(m,4H),2H unter dem DMSO-Peak, 2,33(s,3H), 2,05-1,97(m,2H) |
| Ia-79 | 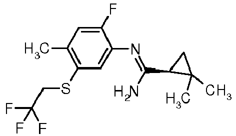 | 1H-NMR(D6-DMSO): 7,05-7,01(m,2H), 6,10(breit,2H), 3,88-3,80(q,2H), 2,32(s,3H), 2,31(s,3H), 2,30(s,3H), 1,74(m,1H), 1,17(m,2H) |
| Ia-80 | 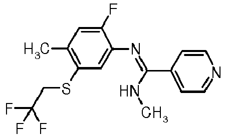 | 1H-NMR(D6-DMSO): 8,49-8,48(m,2H), 7,51-7,49(m,1H), 7,15-7,14(m,2H), 6,87-6,85(m,2H), 3,69-3,61(q,2H), 2,88-2,87(d,3H), 2,20(s,3H) |
| Ia-81 | 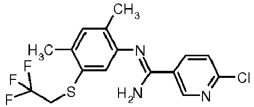 | 1H-NMR(D6-DMSO): 8,95-8,96(m,1H), 8,36-8,39(m,1H), 7,61-7,63(m,1H), 7,10(s,1H), 6,91(s,1H), 6,49(breit,2H), 3,81-3,89(q,2H), 2,32(s,3H), 2,03(s,3H) |
| Ia-82 | 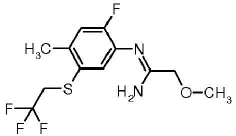 | 1H-NMR(D6-DMSO): 7,09-7,06(m,1H), 7,01-6,99(m,1H), 6,19(breit,2H), 3,95(s,2H), 3,88-3,80(q,2H), 3,33(s,3H), 2,32(s,3H) |
| Ia-83 | 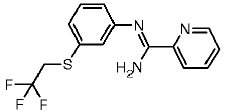 | 1H-NMR(D6-DMSO): 8,64-8,63(m,1H), 8,30-8,28(m,1H), 7,98-7,93(m,1H), 7,58-7,55(m,1H), 7,33-7,29(m,1H), 7,13-7,11(m,1H), 7,02(s,1H), 6,84-6,82(m,1H), 6,64(breit,2H), 4,04-3,96(q,2H) |
| Ia-84 | 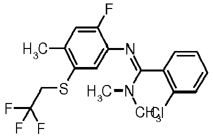 | 1H-NMR(D6-DMSO): 7,40-7,17(m,4H), 6,87-6,84(m,1H), 6,77-6,75(m,1H), 3,59-3,46(m,2H), 3,14(breit,3H), 2,71(breit,3H), 2,17(s,3H) |
| Ia-85 | 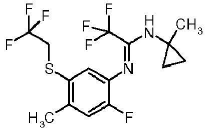 | 1H-NMR(D6-DMSO): 8,33(breit,1H), 7,11-7,06(m,2H), 3,83(q,2H), 2,33(s,3H), 1,34(breit,3H), 0,76(breit, 2H), 0,57(breit,2H) |
| Ia-86 | 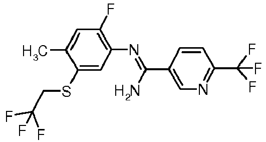 | 1H-NMR(D6-DMSO): 9,27-9,25(m,1H), 8,58-8,56(m,1H), 8,03-8,01(m,1H), 7,18-7,14(m,2H), 7,01(breit,2H), 3,93-3,85(q,2H), 2,37(s,3H) |
| Ia-87 | 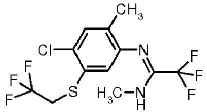 | 1H-NMR(D6-DMSO): 8,00(breit,1H), 7,28(s,1H), 6,95(breit,1H), 4,09-4,01(q,2H), 2,68-2,67(breit,3H), 2,00(s,3H) |
| Ia-88 | 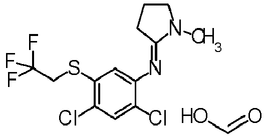 | 1H-NMR(D6-DMSO): 7,53(s,1H), 7,102(s,1H), 4,12(q,2H), 3,39(dd,2H), 2,90(s,3H), 2,26(dd,2H), 1,90(m,2H) |
| Ia-89 | 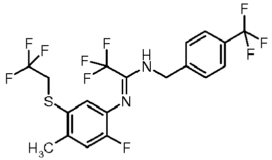 | logP(HCOOH)=5,15, logP(neutral)=5,15 |
| Ia-90 | 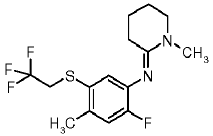 | 1H-NMR(D6-DMSO): 7,03(d,1H), 6,93(d,1H), 3,84(q,2H), 3,25(t,2H), 2,94(s,3H), 2,30(s,3H), 2,10(t,2H), 1,76-1,70(m,2H), 1,60-1,54(m,2H) |
| Ia-91 | 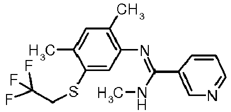 | 1H-NMR(D6-DMSO): 8,44-8,43(m,1H), 8,32(m,1H), 7,51-7,48(m,1H), 7,28-7,21(m,2H), 6,89(s,1H), 6,41(breit,1H), 3,47-3,39(q,2H), 2,88(breit,3H), 2,17(s,3H), 2,07(s,3H) |
| Ia-92 | 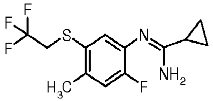 | 1H-NMR(D6-DMSO): 7,04-7,01(m,1H), 6,93-6,91(m,1H), 6,12(breit,2H), 3,86-3,78(q,2H), 2,31(s,3H), 1,52(m,1H), 0,88-0,84(m,2H), 0,72-0,71(m,2H) |
| Ia-93 | 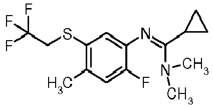 | 1H-NMR(D6-DMSO): 7,10-7,07(m,2H), 3,87-3,79(q,2H), 3,11(breit,6H), 2,34(s,3H), 1,69-1,68(m,1H), 0,68-0,66(m,2H), 0,33(m,2H) |
| Ia-94 | 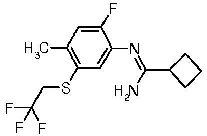 | 1H-NMR(D6-DMSO): 7,10-7,07(m,1H), 6,98-6,96(m,1H), 6,15(breit,2H), 3,88-3,81(q,2H), 3,12-3,08(m,1H), 2,33(s,3H), 2,37-1,71(m,6H) |
| Ia-95 | 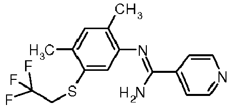 | 1H-NMR(D6-DMSO): 8,69-8,67(m,2H), 7,92-7,91(m,2H), 7,11(s,1H), 6,90(s,1H), 6,47(breit,2H), 3,89-3,81(q,2H), 2,32(s,3H), 2,02(s,3H) |
| Ia-96 | 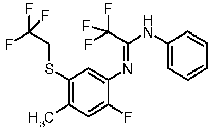 | logP(HCOOH)=4,37, logP(neutral)=4,37 |
| Ia-97 | 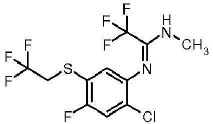 | 1H-NMR(D6-DMSO): 8,20(bs,1H), 7,48(d,1H), 7,32-7,17(m,1H), 3,98(q,2H), 2,90-2,50(breit,3H) |
| Ia-98 | 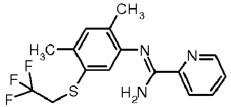 | 1H-NMR(D6-DMSO): 8,64-8,63(m,1H), 8,35-8,33(m,1H), 7,98-7,93(m,1H), 7,58-7,54(m,1H), 7,11(s,1H), 6,94(s,1H), 6,41(breit,2H), 3,89-3,82(q,2H), 2,33(s,3H), 2,03(s,3H) |
| Ia-99 | 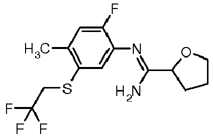 | 1H-NMR(D6-DMSO): 7,09-7,02(m,2H), 6,22(breit,2H), 4,40-4,36(t,1H), 3,97-3,92(m,1H), 3,88-3,80(q,2H), 3,76-3,72(m,1H), 2,33(s,3H), 2,23-2,16(m,1H), 2,06-1,98(m,1H),1,89-1,77(m,2H) |
| Ia-100 | 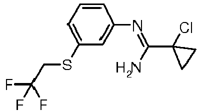 | 1H-NMR(D6-DMSO): 7,25-7,21(m,1H), 7,07-7,05(m,1H), 6,83(m,1H), 6,65-6,63(m,1H), 6,18(breit,2H), 4,00-3,92(q,2H), 1,55(m,2H), 1,26(m,2H) |
| Ia-101 | 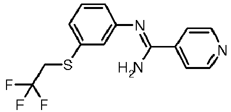 | 1H-NMR(D6-DMSO): 8,69-8,67(m,2H), 7,91-7,89(m,2H), 7,32-7,29(m,1H), 7,12-7,10(m,1H), 6,98(s,1H), 6,79-6,77(m,1H), 6,65(breit,2H), 4,04-3,96(q,2H) |
| Ia-102 | 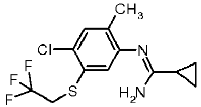 | 1H-NMR(D6-DMSO): 7,24(s,1H), 6,79(s,1H), 5,95(breit,2H), 4,00-3,92(q,2H), 1,93(s,3H), 1,51(breit,1H), 0,89-0,86(m,2H), 0,73-0,70(m,2H) |
| Ia-103 | 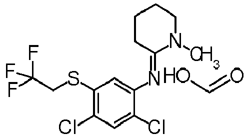 | 1H-NMR(D6-DMSO): 7,54(bs,1H), 7,02(bs,1H), 4,11(q,2H), 3,33(dd,2H), 2,96(s,3H), 2,08(dd,2H), 1,74(m,2H), 1,58(m,2H) |
| Ia-104 | 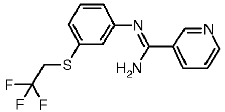 | 1H-NMR(D6-DMSO): 9,12(s,1H), 8,67-8,66(m,1H), 8,31-8,29(m,1H), 7,50-7,46(m,1H), 7,32-7,28(m,1H), 7,12-7,10(m,1H), 6,99(s,1H), 6,80-6,78(m,1H), 6,66-6,59(breit,2H), 4,04-3,96(q, 2H) |
| Ia-105 | 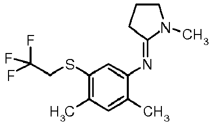 | 1H-NMR(D6-DMSO): 7,33(bs,1H), 7,20(bs,1H), 3,92(q,2H), 3,66(bs,2H), 3,34(m,2H), 3,13(bs,3H), 2,33(bs,3H), 2,16(bs,3H), 2,02-1,95(m,2H) |
| Ia-106 | 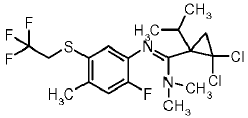 | 1H-NMR(D6-DMSO): 7,05(m,1H), 6,88(m,1H), 3,75(q,2H), 2,98(s,3H), 2,87(s,3H), 2,33(s,3H), 1,83(m,2H), 0,95-0,93(m,7H) |
| Ia-107 | 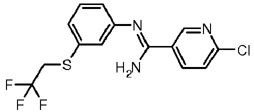 | 1H-NMR(D6-DMSO): 8,94-8,93(m,1H), 8,41-8,34(m,2H), 7,82-7,80(m,1H), 7,63-7,61(m,1H), 7,32-7,28(m,1H), 6,99(s,1H), 6,68-6,65(breit,2H), 4,00(q,2H) |
| Ia-108 | 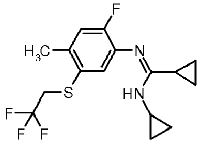 | 1H-NMR(D6-DMSO): 7,15-7,09(m,2H), 3,89-3,81(q,2H), 2,61(m,1H), 2,33(s,3H), 1,45(m,1H), 0,84-0,80(m,3H), 0,69-0,61(m,4H), 0,50(m,2H) |
| Ia-109 | 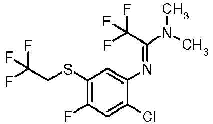 | 1H-NMR(D6-DMSO): 7,48(d,1H), 7,15(d,1H), 4,00(q,2H), 2,98(s,6H) |
| Ia-110 | 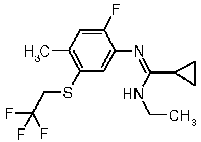 | 1H-NMR(D6-DMSO): 7,18(d,1H), 7,04-6,97(m,2H), 3,83(q,2H), 3,15(m,2H), 2,31(s,3H), 1,35(m,1H), 0,99(t,3H), 0,79-0,77(m,2H), 0,60-0,57(m,2H) |
| Ia-111 | 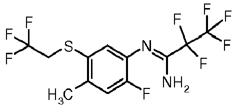 | 1H-NMR(D6-DMSO): 7,40(breit,2H), 7,18-7,15(m,1H), 7,07-7,05(m,1H), 3,93-3,85(q,2H), 2,35(s,3H) |
| Ia-112 | 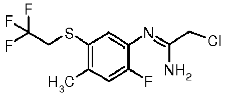 | 1H-NMR(D6-DMSO): 7,14-7,12(m,1H), 7,06-7,04(m,1H), 6,75(breit,2H), 4,18(s,2H), 3,90-3,82(q,2H), 2,34(s,3H) |
| Ia-113 | 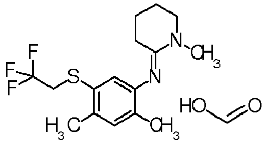 | 1H-NMR(D6-DMSO): 8,15(s,1H), 7,33(bs,1H), 7,21(d,1H), 3,94(q,2H), 3,49(breit,2H), 3,19(breit,3H), 2,34(s,3H), 2,24-2,06(m,5H), 1,78(breit,2H), 1,61(breit,2H) |
| Ia-114 | 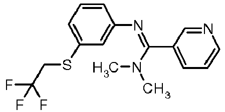 | 1H-NMR(D6-DMSO): 8,44-8,42(m,1H), 8,31-8,30(m,1H), 7,60-7,58(m,1H), 7,32-7,29(m,1H), 6,96-6,92(m,1H), 6,79-6,77(m,1H), 6,60-6,59(m,1H), 6,41-6,38(m,1H), 3,79-3,71(q,2H), 3,00(breit,3H); 2,92(breit,3H) |
| Ia-115 | 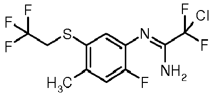 | 1H-NMR(D6-DMSO): 7,36-7,28(breit,2H), 7,17-7,15(m,1H), 7,09-7,06(s,1H), 3,94-3,86(q,2H), 2,34(s,3H) |
| Ia-116 | 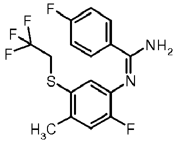 | 1H-NMR(D6-DMSO): 8,04-8,01(m,2H), 7,29-7,25(m,2H), 7,14-7,09(m,2H), 6,61(breit,2H), 3,87(q,2H), 2,36(s,3H) |
| Ia-117 | 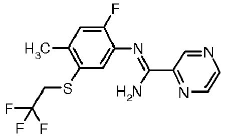 | 1H-NMR(D6-DMSO): 9,45-9,44(m,1H), 8,83-8,82(m,1H), 8,72-8,71(m,1H), 7,20-7,16(m,2H), 6,98-6,92(breit,2H), 3,93-3,85(q, 2H), 2,37(s,3H) |
| Ia-118 | 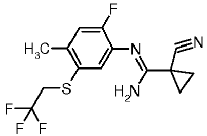 | 1H-NMR(D6-DMSO): 7,11-7,08(m,1H), 7,01-6,99(m,1H), 6,26(breit,2H), 3,90-3,82(q,2H), 2,32(s,3H), 1,66-1,56(m,4H) |
| Ia-119 | 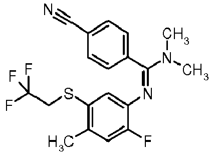 | 1H-NMR(D6-DMSO): 7,77-7,75(m,2H), 7,36-7,34(m,2H), 6,83(d,1H), 6,74(d,1H), 3,57(q,2H), 3,10(breit,3H), 2,73(breit,3H), 2,17(s,3H) |
| Ia-120 | 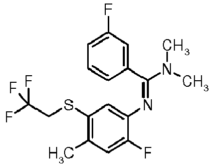 | 1H-NMR(D6-DMSO): 7,35-7,29(m,1H), 7,12-7,07(m,1H), 7,02-7,01(m,1H), 7,00-6,93(m,1H), 6,84-6,78(m,2H), 3,60(q,2H), 3,07(breit,3H), 2,73(breit,3H), 2,17(s,3H) |
| Ia-121 | 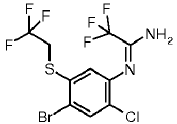 | 1H-NMR(D6-DMSO): 7,76(s,1H), 7,48-7,33(breit,2H), 7,13(s,1H), 4,20-4,12(m,2H) |
| Ia-122 | 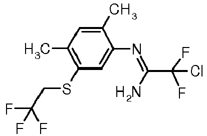 | 1H-NMR(D6-DMSO): 7,09(s,1H), 6,90(breit,2H), 6,84(s,1H), 3,90-3,82(q,2H), 2,31(s,3H), 1,99(s,3H) |
| Ia-123 | 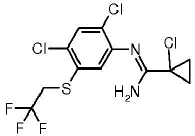 | 1H-NMR(D6-DMSO): 7,56(s,1H), 7,04(s,1H), 6,53(breit,2H), 4,13-4,05(q,2H), 1,62-1,59(m,2H), 1,31-1,28(m,2H) |
| Ia-124 | 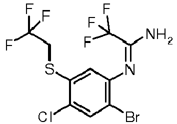 | 1H-NMR(D6-DMSO): 7,78(s,1H), 7,57-7,33(breit,1H), 7,12(s,1H), 4,19-4,11(2H) |
| Ia-125 | 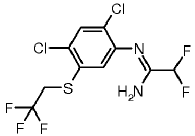 | 1H-NMR(D6-DMSO): 7,63(s,1H), 7,09(s,1H), 7,02-6,97(breit,2H), 6,34(t,1H), 4,16-4,09(q,2H) |
| Ia-126 | 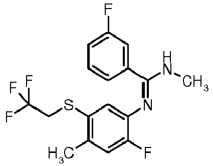 | 1H-NMR(D6-DMSO): 7,40-7,39(m,1H), 7,33-7,27(m,1H), 7,16-7,11(m,1H), 7,02-6,97(m,2H), 6,87-6,82(t,2H), 3,64-3,56(q,2H), 2,87-2,86(m,3H), 2,21(s,3H) |
| Ia-127 | 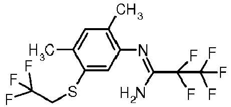 | 1H-NMR(D6-DMSO): 7,10(s,1H), 7,05(breit,2H), 6,84(s,1H), 3,90-3,82(q,2H), 2,31(s,3H), 1,96(s,3H) |
| Ia-128 | 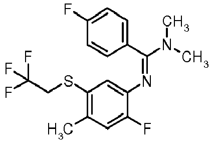 | 1H-NMR(D6-DMSO): 7,19-7,16(m,2H), 7,13-7,08(m,2H), 6,82(d,1H), 6,72(d,1H), 3,58(q,2H), 3,01(breit,3H), 2,72(breit,3H), 2,17(s,3H) |
| Ia-129 | 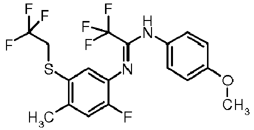 | logP(HCOOH)=4,2, logP(neutral)=4,2 |
| Ia-130 | 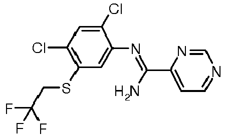 | 1H-NMR(D6-DMSO): 9,33(m,1H), 9,01-9,00(m,1H), 8,28-8,27(m,1H), 7,66(s,1H), 7,21(s,1H), 7,12-7,00(breit,2H), 4,16-4,08(q,2H) |
| Ia-131 | 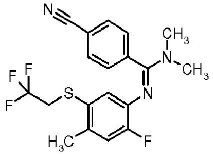 | 1H-NMR(D6-DMSO): 7,77-7,75(m,2H), 7,36-7,34(m,2H), 6,83(d,1H), 6,74(d,1H), 3,58(q,2H), 3,10(breit,3H), 2,70(breit,3H), 2,17(s,3H) |
| Ia-132 | 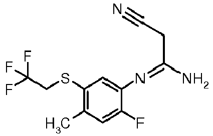 | 1H-NMR(D6-DMSO): 7,11-7,10(m,1H), 7,07-7,01(m,1H), 6,49(breit,2H), 3,88-3,83(q,2H), 3,65(s,2H), 2,33(s,3H) |
| Ia-133 | 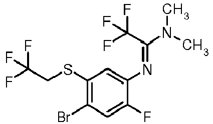 | 1H-NMR(D6-DMSO, 400MHz): 7,58(d,1H), 7,18(d,1H), 4,06(q,2H), 3,01(s,6H) |
| Ia-134 | 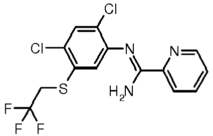 | 1H-NMR(D6-DMSO): 8,66-8,65(m,1H), 8,32-8,30(m,1H), 8,00-7,95(m,1H), 7,64(s,1H), 7,60-7,57(m,1H), 7,20(s,1H), 6,95(breit,2H), 4,16-4,08(m,2H) |
| Ia-135 | 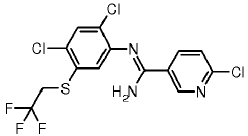 | 1H-NMR(D6-DMSO): 8,95(breit,1H), 8,38-8,32(m,1H), 7,64(m,2H), 7,19(s,1H), 6,94(breit,2H), 4,15-4,08(q,2H) |
| Ia-136 | 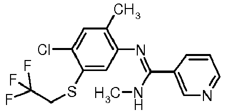 | 1H-NMR(D6-DMSO): 8,47-8,45(m,1H), 8,35(s,1H), 7,56-7,54(m,1H), 7,39-7,38(m,1H), 7,31-7,27(m,1H), 7,15(s,1H), 6,54(breit,1H), 3,71-3,64(q,2H), 2,89-2,88(d,3H), 2,08(s,3H), |
| Ia-138 | 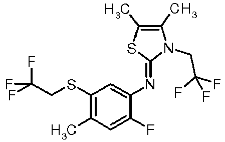 | 1H-NMR(D6-DMSO): 7,20-7,16(m,2H), 4,79(q,2H), 3,83(q,2H), 2,35(s,3H), 2,10(s,3H), 2,03(s,3H) |
| Ia-139 | 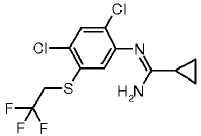 | 1H-NMR(D6-DMSO): 7,52(s,1H), 6,98(s,1H), 6,24-6,15(breit,2H), 4,11-4,03(q,2H), 1,51(m,1H), 0,92-0,88(m,2H), 0,76-0,71(m,2H) |
| Ia-140 | 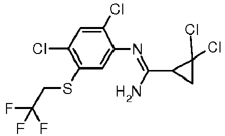 | 1H-NMR(D6-DMSO): 7,58(s,1H), 6,95(s,1H), 6,70(breit,2H), 4,07-4,00(m,2H), 2,17-2,14(m,1H), 1,95-1,93(m,1H) |
| Ia-141 | 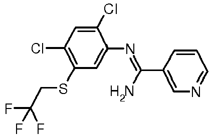 | 1H-NMR(D6-DMSO): 9,13(m,1H), 8,69(m,1H), 8,32(m,1H), 7,64(s,1H), 7,49(m,1H), 7,18(s,1H), 6,89(breit,2H), 4,16-4,08(breit,2H) |
| Ia-143 | 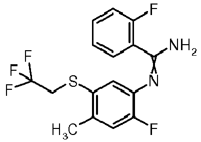 | logP(HCOOH)=1,37, logP(neutral)=3,25 |
| Ia-144 | 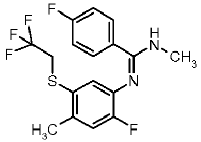 | 1H-NMR(D6-DMSO): 7,34-7,33(m,1H), 7,23-7,20(m,2H), 7,12-7,07(m,2H), 6,85(d,1H), 6,77(d,1H), 3,58(q,2H), 2,86-2,85(m,3H), 2,21(s,3H) |
| Ia-145 | 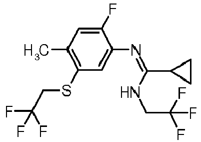 | 1H-NMR(D6-DMSO): 7,09-7,07(m,1H), 6,99-6,98(m,1H), 6,72(breit,1H), 3,85-3,79(m,4H), 2,33(s,3H), 1,41(breit,1H), 0,85-0,83(m,2H), 0,68-0,65(m,2H) |
| Ia-146 | 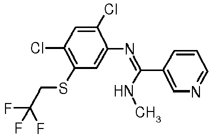 | 1H-NMR(D6-DMSO): 8,50-8,49(m,1H), 8,41(s,1H), 7,71(breit,1H), 7,50-7,58(m,1H), 7,52-7,48(m,1H), 7,34-7,30(m,1H), 6,95(breit,1H), 3,96-3,88(q,2H), 2,92-2,90(d,3H) |
| Ia-147 | 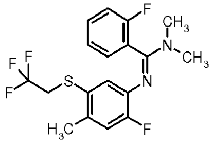 | 1H-NMR(D6-DMSO): 7,36-7,30(m,1H), 7,17-7,08(m,3H), 6,83(d,1H), 6,73(d,1H), 3,55(q,2H), 3,13(breit,3H), 2,76(breit,3H), 2,17(s,3H) |
| Ia-148 | 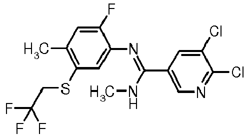 | 1H-NMR(D6-DMSO): 8,46-8,45(m,1H), 8,03(s,1H), 7,64-7,63(q,1H), 6,90-6,94(m,2H), 3,72-3,64(q,2H), 2,89(d,3H), 2,22(s,3H) |
| Ia-149 | 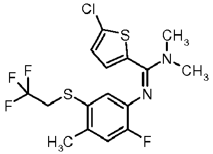 | 1H-NMR(D6-DMSO): 7,00(m,1H), 6,94-6,91(d,1H), 6,86-6,84(m,2H), 3,68(q,2H), 2,97(s,6H), 2,23(s,3H) |
| Ia-150 | 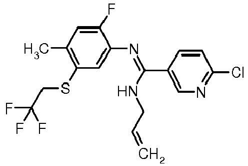 | 1H-NMR(D6-DMSO): 8,24-8,23(m,1H), 7,76(breit,1H), 7,66-7,63(m,1H), 7,45-7,43(m,1H), 6,90-6,87(m,2H), 6,03-5,97(m,1H), 5,32-5,28(m,1H), 5,16-5,13(m,1H), 4,02(breit,2H), 3,72-3,64(q,2H), 2,22(s,3H) |
| Ia-151 | 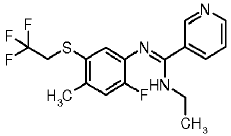 | 1H-NMR(D6-DMSO): 8,90(m,1H), 8,67(m,2H), 7,89-7,88(m,1H), 7,67-7,66(m,1H), 7,46-7,45(m,1H), 7,22-7,19(m,1H), 3,89-3,73(m,4H), 2,30(s,3H), 1,38-1,35(t,3H) |
| Ia-152 | 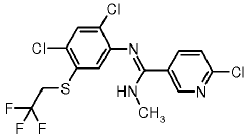 | 1H-NMR(D6-DMSO): 8,25-8,20(m,2H), 7,73(breit,1H), 7,66-7,61(m,1H), 7,48-7,46(m,1H), 6,94(breit,1H), 3,97-3,89(q,2H), 2,91-2,90(d,3H) |
| Ia-153 | 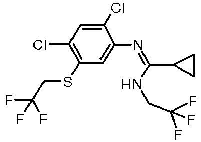 | 1H-NMR(D6-DMSO): 8,58(t,1H), 7,60(m,1H), 7,10(m,1H), 4,10-3,87(m,4H), 1,38(m,1H), 0,88(m,2H), 0,71(m,2H) |
| Ia-155 | 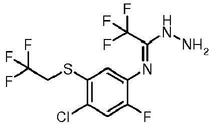 | 1H-NMR(D6-DMSO): 7,96(bs,1H), 7,48(d,1H), 7,33(bs,2H), 6,51(d,1H), 3,90-3,70(m,2H) |
| Ia-156 | 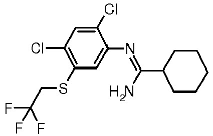 | 1H-NMR(D6-DMSO): 7,54(s,1H), 6,98(s,1H), 6,10(breit,2H), 4,10-4,02(q,2H), 2,14(m,1H), 1,84-1,15(m,10H) |
| Ia-158 | 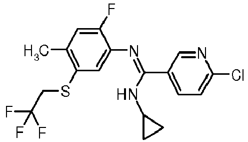 | 1H-NMR(D6-DMSO): 8,21-8,18(m,1H), 7,65-7,58(m,2H), 7,43-7,41(m,1H), 6,90-6,87(m,2H), 3,73-3,66(q,2H), 2,22(s,3H), 2,92(m,1H), 0,74-0,67(m,2H), 0,60-0,56(m,2H) |
| Ia-159 | 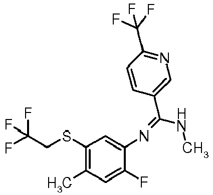 | 1H-NMR(D6-DMSO): 8,84(m,1H), 8,21-8,19(m,1H), 8,02-8,00(m,1H), 7,61-7,59(m,1H), 7,29-7,26(m,2H), 3,76-3,69(q,2H), 2,84(breit,3H), 2,30(s,3H) |
| Ia-160 | 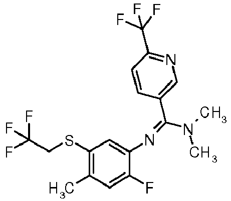 | 1H-NMR(D6-DMSO): 8,57(m,1H), 7,96-7,94(m,1H), 7,86-7,84(m,1H), 6,91-6,88(m,2H), 3,65-3,57(q,2H), 3,15(breit,3H), 2,80(breit,3H), 2,18(s,3H) |
| Ia-161 | 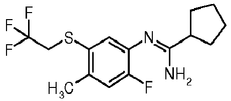 | 1H-NMR(D6-DMSO): 7,08-7,05(m,1H), 6,98(m,1H), 6,20(breit,2H), 3,87-3,79(q,2H), 2,33(s,3H), 2,30(m,1H), 1,84-1,52(m,8H) |
| Ia-162 | 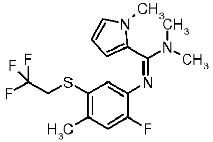 | 1H-NMR(D6-DMSO): 6,88(d,1H), 6,70-6,69(m,1H), 6,63(d,1H), 5,91-5,89(m,2H), 3,58(q,2H), 3,39(s,3H), 3,05(breit,3H), 2,77(breit,3H), 2,21(s,3H) |
| Ia-163 | 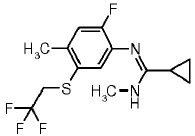 | 1H-NMR(D6-DMSO): 7,05-6,99(m,2H), 6,24(breit,1H), 3,86-3,78(q,2H), 2,65-2,64(d,3H), 2,31(s,3H) 1,35(m,1H), 0,79-0,75(m,2H), 0,62-0,57(m,2H) |
| Ia-165 | 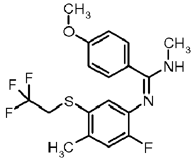 | logP(HCOOH)=1,68, logP(neutral)=3,49 |
| Ia-166 | 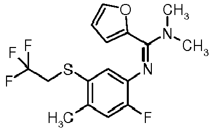 | 1H-NMR(D6-DMSO): 7,68(m,1H), 6,91(d,1H), 6,75(d,1H), 6,42-6,41(m,1H), 6,25-6,24(m,1H), 3,69(q,2H), 2,94(s,6H), 2,24(s,3H) |
| Ia-167 | 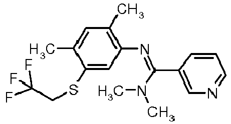 | 1H-NMR(D6-DMSO): 8,42-8,40(m,1H), 8,29(m,1H), 7,59-7,56(m,1H), 7,30-7,27(m,1H), 6,83(s,1H) 2,04(s,3H), 6,42(s,1H), 2,13(s,3H), 3,51-3,43(q,2H), 2,92(breit,6H) |
| Ia-168 | 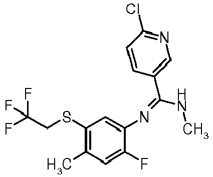 | 1H-NMR(D6-DMSO): 8,35-8,33(m,1H), 7,76(m,1H), 7,54-7,52(m,1H), 7,25(m,1H), 7,07(m,2H), 3,74(q,2H), 3,07(d,3H), 2,27(s,3H) |
| Ia-169 | 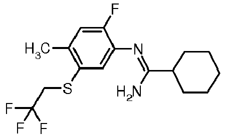 | 1H-NMR(D6-DMSO): 7,05-7,02(m,1H), 6,93-6,91(m,1H), 5,90(breit,2H), 3,85-3,77(q,2H), 2,3(s,3H), 2,16-2,10(m,1H), 1,83-1,15(m,10H) |
| Ia-171 | 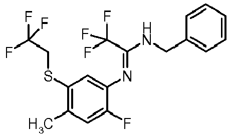 | 1H-NMR(D6-DMSO): 8,73(breit,1H), 7,32-7,25(breit,5H), 7,08-7,05(m,1H), 6,96-6,94(m,1H), 4,48(breit,2H) 3,78(q,2H), 2,32(s,3H) |
| Ia-172 | 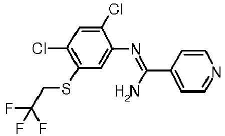 | 1H-NMR(D6-DMSO): 8,70(m,2H), 7,90-7,87(m,2H), 7,64(m,1H), 7,18(s,1H), 6,94(breit,2H), 4,15-4,08(q,2H) |
| Ia-173 | 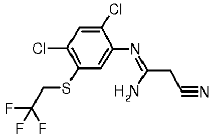 | 1H-NMR(D6-DMSO): 7,60(s,1H), 7,06(s,1H), 6,63-6,54(breit,2H), 4,14-4,07(q,2H), 3,67(s,2H) |
| Ia-175 | 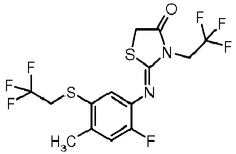 | 1H-NMR(D6-DMSO): 7,27(d,1H), 7,21(d,1H), 4,58(q,2H), 4,24(s,2H), 3,87(q,2H), 2,39(s,3H) |
| Ia-176 | 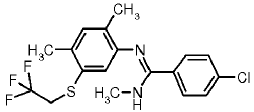 | 1H-NMR(D6-DMSO): 7,31-7,29(m,2H), 7,14-7,08(m,3H), 6,89(s,1H), 6,36(s,1H), 3,41-3,33(q,2H), 2,85(breit,3H), 2,18(s,3H), 2,07(s,3H) |
| Ia-177 | 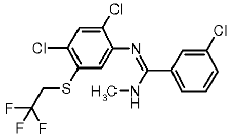 | 1H-NMR(D6-DMSO): 7,59-7,58(m,1H), 7,43(s,1H), 7,40-7,27(m,3H), 7,08-7,06(m,1H), 6,88(s,1H), 3,90-3,82(q,2H), 2,88-2,87(d,3H) |
| Ia-178 | 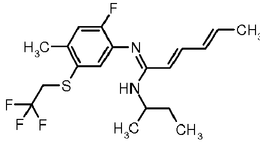 | 1H-NMR(D6-DMSO): 7,73(m,1H), 7,03-6,93(m,3H), 6,19-5,87(m,3H), 3,80-3,71(m,3H), 2,32(s,3H), 1,80-1,78(d,3H), 1,48(m,2H), 1,03-1,02(d,3H), 0,81(t,3H) |
| Ia-180 | 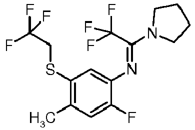 | 1H-NMR(D6-DMSO): 7,07-7,04(m,2H), 3,80(q,2H), 3,18(breit,4H), 2,33(s,3H), 1,84(breit,4H) |
| Ia-181 | 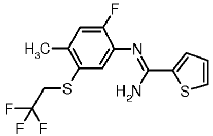 | 1H-NMR(D6-DMSO): 7,76(m,1H), 7,65-7,64(m,1H), 7,15-7,08(m,3H), 6,68(breit,2H), 3,92-3,84(q,2H), 2,35(s,3H) |
| Ia-182 | 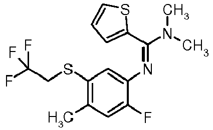 | 1H-NMR(D6-DMSO): 7,59-7,56(m,1H), 6,98-6,95(m,2H), 6,87(d,1H), 6,77(d,1H), 3,63(q,2H), 2,96(s,6H), 2,20(s,3H) |
| Ia-183 | 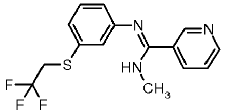 | 1H-NMR(D6-DMSO): 8,46-8,45(m,1H), 8,36(s,1H), 7,57-7,55(m,1H), 7,33-7,27(m,2H), 7,01-6,97(m,1H), 6,84-6,82(m,1H), 6,64(s,1H), 6,44-6,42(m,1H), 3,79-3,71(q,2H), 2,87-2,86(d,3H) |
| Ia-184 | 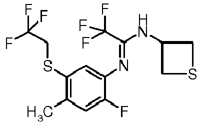 | 1H-NMR(D6-DMSO): 8,48(breit,1H), 7,11-7,01(m,2H), 4,90(breit,1H), 3,79(q,2H), 3,50(breit,2H), 3,18(breit,2H), 2,34(s,3H) |
| Ia-185 | 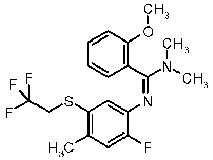 | 1H-NMR(D6-DMSO): 7,25-7,21(m,1H), 6,96-6,90(m,2H), 6,84-6,81(m,2H), 6,60(d,1H), 3,73(s,3H), 3,50-3,37(m,2H), 3,10(breit,3H), 2,69(breit,3H), 2,17(s,3H) |
| Ia-186 | 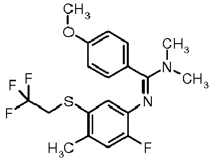 | 1H-NMR(D6-DMSO): 7,06-7,02(m,2H), 6,84-6,80(m,3H), 6,70(d,1H), 3,68(s,3H), 3,54(q,2H), 2,91(breit,6H), 2,17(s,3H) |
| Ia-187 | 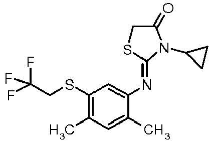 | 1H-NMR(D6-DMSO): 7,14(s,1H), 7,00(s,1H), 3,92(s,2H), 3,82(q,2H), 2,71(septet,1H), 2,33(s,3H), 2,06(s,3H), 1,00-0,90(m,4H) |
| Ia-188 | 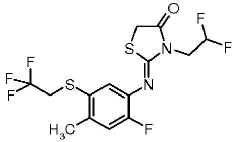 | 1H-NMR(D6-DMSO): 7,28-7,25(m,2H), 6,32(tt,1H), 4,21-4,12(m,4H), 3,87(q,2H), 2,38(s,3H) |
| Ia-189 | 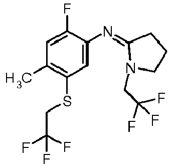 | 1H-NMR(D6-DMSO) 7,09-7,01(m,2H), 4,24(q,2H), 3,85(q,2H), 3,49(t,2H), 2,33(t,3H), 2,32(s,3H), 1,98-1,90(m,2H) |
| Ia-190 | 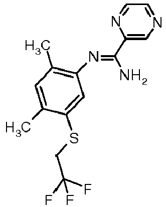 | 1H-NMR(D6-DMSO): 9,49(m,1H), 8,81(m,1H), 8,71(m,1H), 7,13(s,1H), 6,98(s,1H), 6,53(breit,2H), 3,86(q,2H), 2,33(s,3H), 2,06(s,3H) |
| Ia-191 | 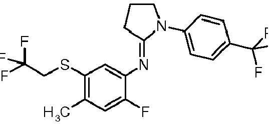 | 1H-NMR(D6-DMSO): 8.15(d,2H), 7.72(d,2H), 7.14(d,2H), 3.95-3.85(m,2x2H), 2.54(m,2H), 2.34(s,3H), 2.04(m,2H) |
| Ia-192 | 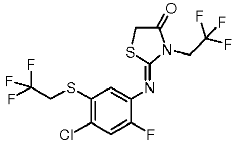 | 1H-NMR(D6-DMSO): 7,67(d,1H), 7,36(d,1H), 4,59(q,2H), 4,27(s,2H), 4,06(q,2H) |
| Ia-193 | 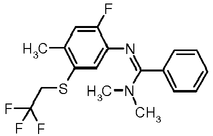 | 1H-NMR(D6-DMSO): 7,30-7,25(m,3H), 7,13-7,11(m,2H), 6,81-6,78(m,1H), 6,73-6,71(m,1H), 3,54(q,2H), 3,08(breit,3H), 2,75(breit,3H), 2,16(s,3H) |
| Ia-194 | 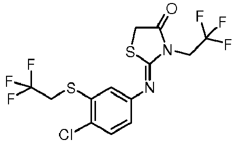 | 1H-NMR(D6-DMSO): 7,51(d,1H), 7,19(d,1H), 6,85(dd,1H), 4,57(q,2H), 4,21(s,2H), 4,12(q,2H) |
| Ia-195 | 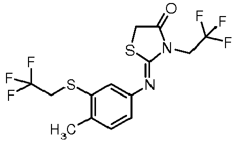 | 1H-NMR(D6-DMSO): 7,27(d,1H), 7,09(d,1H), 6,79(dd,1H), 4,57(q,2H), 4,18(s,2H), 3,94(q,2H), 2,35(s,3H) |
| Ia-196 | 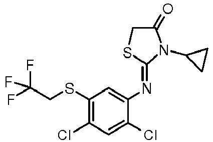 | 1H-NMR(D6-DMSO): 7,76(s,1H), 7,35(s,1H), 4,13(q,2H), 4,02(s,2H), 2,71(septet,1H), 1,02-0,94(m,4H) |
| Ia-197 | 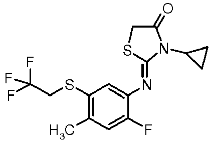 | 1H-NMR(D6-DMSO): 7,25-7,20(m,2H), 3,98(s,2H), 3,87(q,2H), 2,74-2,60(m,1H), 2,40(s,3H), 1,00-0,87(m,4H) |
| Ia-198 | 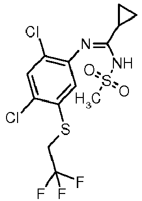 | 1H-NMR(D6-DMSO): 9,30(breit,1H), 7,85(s,1H), 7,75(s,1H), 4,21(q,2H), 2,87(s,3H), 1,20(m,2H), 1,05(m,2H) |
| Ia-199 | 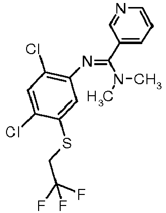 | 1H-NMR(D6-DMSO): 8,47-8,46(m,1H), 8,37-8,36(m,1H), 7,66-7,63(m,1H), 7,37(s,1H), 7,35-7,31(m,1H), 6,92(s,1H), 3,92(q,2H), 3,08(breit,3H), 2,85(breit,3H) |
| Ia-200 | 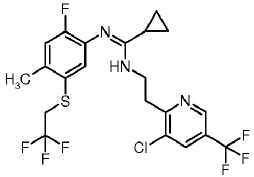 | 1H-NMR(D6-DMSO): 8,87(s,1H), 8,38(m,1H), 7,04-7,02(m,1H), 6,94(s,1H), 6,33(breit,1H), 3,79(q,2H), 3,63-3,58(m,2H), 3,23(t,2H), 2,31(s,3H), 1,34(m,1H), 0,76-0,72(m,2H), 0,60-0,55(m,2H) |
| Ia-201 | 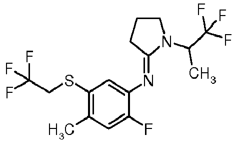 | 1H-NMR(D6-DMSO): 7,10-7,05(m,2H), 5,14-5,06(m,1H), 3,89-3,81(q,2H), 3,51-3,49(m,2H), 2,32(s,3H), 2,30-2,28(m,2H), 1,98-1,89(m,2H), 1,38-1,36(breit,3H) |
| Ia-202 | 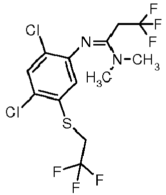 | 1H-NMR(D6-DMSO): 7,59(s,1H), 7,05(s,1H), 4,10(q,2H), 3,70(s,2H), 3,08(breit,6H) |
| Ia-203 | 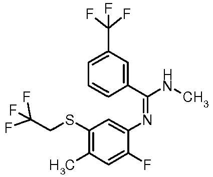 | 1H-NMR(D6-DMSO): 7,66(d,1H) 7,55-7,41(m,4H), 6,87-6,83(m,2H), 3,56(q,2H), 2,89(d,3H), 2,19(s,3H) |
| Ia-204 | 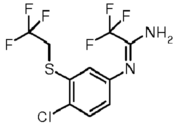 | 1H-NMR(D6-DMSO): 7,42(d,1H), 7,26(breit,2H), 7,05(d,1H), 6,75-6,72(m,1H), 4,13(q,2H) |
| Ia-205 | 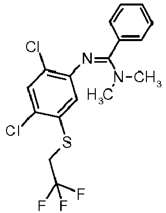 | 1H-NMR(D6-DMSO): 7,35(s,1H), 7,31-7,27(m,3H), 7,18-7,16(m,2H), 6,81(s,1H), 3,82(q,2H), 3,10(breit,3H), 2,82(d,3H) |
| Ia-206 | 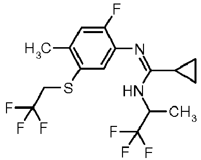 | 1H-NMR(D6-DMSO): 7,09-7,06(m,1H), 7,01-6,99(m,1H), 6,47-6,44(m,1H), 3,85(q,2H), 2,65-2,59(m,2H), 2,33(s,3H), 1,41(m,1H), 1,27(d,3H), 0,96-0,61(m,4H) |
| Ia-207 | 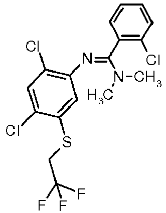 | 1H-NMR(D6-DMSO): 7,45-6,76(m,6H), 3,86(q,2H), 3,17(breit,3H), 2,73(breit,3H) |
| Ia-208 | 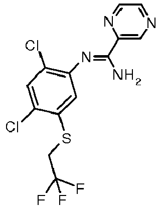 | 1H-NMR(D6-DMSO): 9,45(s,1H), 8,84(m,1H), 8,74-8,73(m,1H), 7,66(s,1H), 7,23(s,1H), 7,03(breit,2H), 4,12(q,2H) |
| Ia-209 | 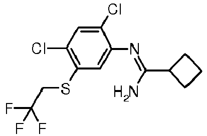 | 1H-NMR(D6-DMSO): 7,61(breit,1H), 7,11(breit,1H), 6,20(breit,2H), 4,10(q,2H), 3,14(m,1H), 2,32-1,78(m,6H) |
| Ia-210 | 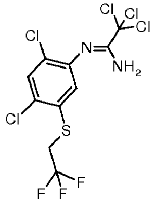 | 1H-NMR(D6-DMSO): 7,65(s,1H), 7,15(breit,2H), 7,10(s,1H), 4,15(q,2H) |
| Ia-211 | 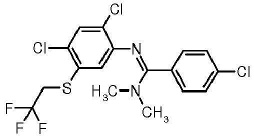 | 1H-NMR(D6-DMSO): 7,39-7,35(m,3H), 7,21-7,19(m,2H), 6,85(s,1H), 3,86(q,2H), 3,07(breit,3H), 2,79(breit,3H) |
| Ia-212 | 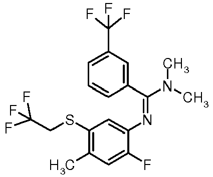 | 1H-NMR(D6-DMSO): 7,63(d,1H), 7,54-7,46(m,2H), 7,42(d,1H), 6,83-6,78(m,2H), 3,56(q,2H), 3,11(breit,3H), 2,76(breit,3H), 2,16(s,3H) |
| Ia-213 | 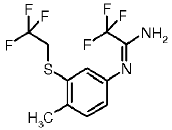 | 1H-NMR(D6-DMSO): 7,20(d,1H), 7,10(breit,2H), 6,96(d,1H), 6,69-6,67(m,1H), 3,97(q,2H), 2,31(s,3H) |
| Ia-214 | 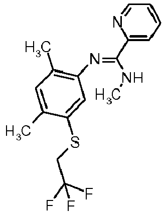 | 1H-NMR(D6-DMSO): 8,56-8,55(m,1H), 7,69-7,68(m,2H), 7,38-7,22(m,3H), 6,93(s,1H), 3,48(breit,2H), 2,77(breit,3H), 2,22(s,3H), 2,06(s,3H) |
| Ia-215 | 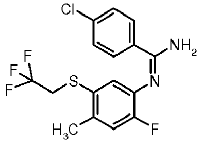 | 1H-NMR(D6-DMSO): 7,99(d,2H), 7,51(d,2H), 7,14-7,09(m,2H), 6,65(breit,2H), 3,87(q,2H), 2,35(s,3H) |
| Ia-216 | 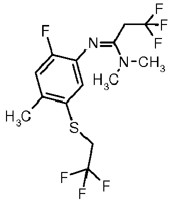 | 1H-NMR(D6-DMSO): 7,10-7,07(m,1H), 6,94-6,92(m,1H), 3,83(q,2H), 3,52(q,2H), 3,04(breit,6H), 2,32(s,3H) |
| Ia-217 | 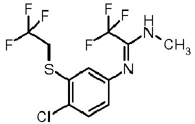 | 1H-NMR(D6-DMSO): 8,05(breit,1H), 7,32(d,1H), 7,03(breit,1H), 6,65(breit,1H), 4,09(q,2H), 2,70(breit,3H) |
| Ia-218 | 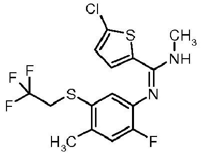 | 1H-NMR(D6-DMSO): 7,57(d,1H), 7,49(breit,1H), 7,17(d,1H), 7,05-6,99(m,2H), 3, |
| Ia-219 | 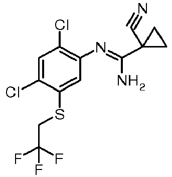 | 1H-NMR(D6-DMSO): 7,58(s,1H), 7,07(s,1H), 6,40(breit,2H), 3,93(q,2H), 1,75-1,61(m,4H) |
| Ia-220 | 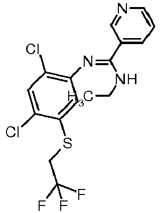 | 1H-NMR(D6-DMSO): 8,49-8,47(m,1H), 8,39(m,1H), 7,65(t,1H), 7,59-7,57(m,1H), 7,42(s,1H), 7,37-7,29(m,1H), 6,90(s,1H), 3,90(q,2H), 3,43-3,38(m,2H), 1,23(t,3H) |
| Ia-221 | 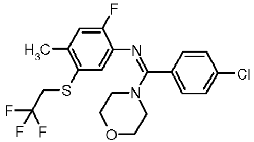 | 1H-NMR(D6-DMSO): 7,36-7,34(m,2H), 7,20-7,18(m,2H), 6,87-6,84(m,1H), 6,82-6,80(m,1H), 3,66-3,58(m,8H), 3,30(breit,2H), 2,19(s,3H) |
| Ia-222 | 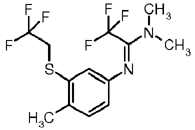 | 1H-NMR(D6-DMSO): 7,10(d,1H), 6,89(breit,1H), 6,55(d,1H), 3,93(q,2H), 2,87(breit,6H), 2,29(s,3H) |
| Ia-223 | 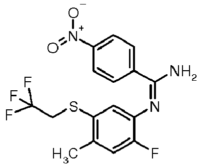 | 1H-NMR(D6-DMSO): 8,30(d,2H), 8,21(d,2H), 7,17-7,13(m,2H), 6,89(breit,2H), 3,89(q,2H), 2,37(s,3H) |
| Ia-224 | 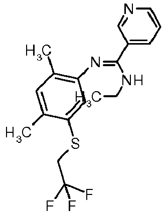 | 1H-NMR(D6-DMSO): 8,43-8,42(m,1H), 8,31(m,1H), 7,52-7,50(m,1H), 7,27-7,20(m,2H), 6,89(s,1H), 6,39(s,1H), 3,45-3,33(m,4H), 2,17(s,3H), 2,07(s,3H), 1,22(t,3H) |
| Ia-225 | 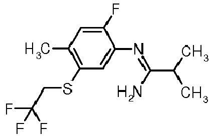 | 1H-NMR(D6-DMSO): 7,07-7,04(m,1H), 6,95-6,94(m,1H), 5,95(breit,2H), 3,82(q,2H), 2,32(s,3H), 1,14(d,6H) |
| Ia-226 | 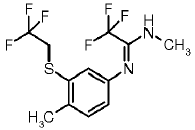 | 1H-NMR(D6-DMSO): 7,90(breit,1H), 7,09(d,1H), 6,89(breit,1H), 6,58(breit,1H), 4,03(q,2H) 2,76(breit,3H), 2,29(s,3H) |
| Ia-227 | 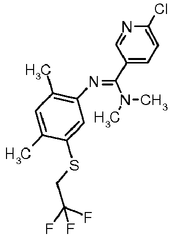 | 1H-NMR(D6-DMSO): 8,15(m,1H), 7,68-7,65(m,1H), 7,42-7,40(m,1H), 6,86(s,1H), 6,45(s,1H), 3,52(q,2H), 2,92(breit,6H), 2,16(s,3H), 2,04(s,3H) |
| Ia-228 | 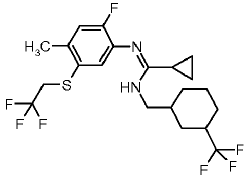 | 1H-NMR(D6-DMSO): 7,05-6,95(m,2H), 6,26(breit,1H), 3,80(q,2H), 3,09-3,01(m,2H), 2,32(s,3H), 2,22-0,60(m,15H) |
| Ia-229 | 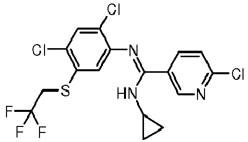 | 1H-NMR(D6-DMSO): 8,32-8,27(m,1H), 7,82(breit,1H), 7,65(m,1H), 7,48-7,46(m,2H), 7,02(breit,1H), 3,97(q,2H), 2,83(m,1H), 0,69-0,61(m,4H) |
| Ia-230 | 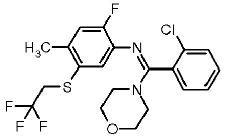 | 1H-NMR(D6-DMSO): 7,40-7,23(m,4H), 6,89-6,86(m,1H), 6,81-6,79(m,1H), 3,65-3,49(m,8H), 3,02-3,00(m,2H), 2,18(s,3H) |
| Ia-231 | 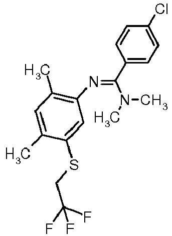 | 1H-NMR(D6-DMSO): 7,32-7,30(m,2H), 7,14-7,12(m,2H), 6,83(s,1H), 6,39(s,1H), 3,44(q,2H), 2,89(breit,6H), 2,15(s,3H), 2,04(s,3H) |
| Ia-232 | 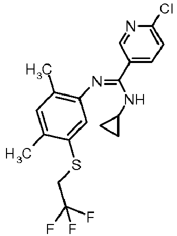 | 1H-NMR(D6-DMSO): 8,14(m,1H), 7,55-7,53(m,1H), 7,38-7,37(m,2H), 6,91(s,1H), 6,47(s,1H), 3,50(q,2H), 2,87(m,1H), 2,19(s,3H), 2,08(s,3H), 0,71(m,2H), 0,58(m,2H) |
| Ia-233 | 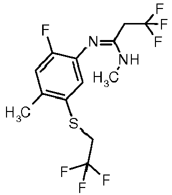 | 1H-NMR(D6-DMSO): 7,26(breit,1H), 7,09-7,06(m,1H), 6,97-6,95(m,1H), 3,84(q,2H), 3,14(q,2H), 2,77(d,3H), 2,32(s,3H) |
| Ia-234 | 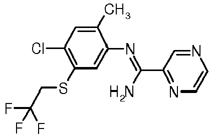 | 1H-NMR(D6-DMSO): 9,50-9,49(m,1H), 8,83-8,82(m,1H), 8,72(s,1H), 7,37(s,1H), 7,07(s,1H), 6,75(breit,2H), 4,03(q,2H), 2,08(s,3H) |
| Ia-235 | 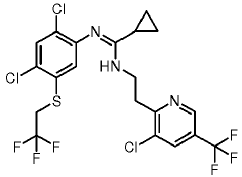 | 1H-NMR(D6-DMSO): 8,88(s,1H), 8,39(m,1H), 7,54(s,1H), 7,05(s,1H), 6,53(breit,1H), 4,07(q,2H), 3,64-3,59(m,2H), 3,27(t,2H), 1,35-1,30(m,1H), 0,79-0,76(m,2H), 0,60-0,59(m,2H) |
| Ia-236 | 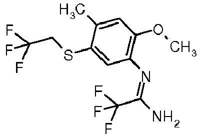 | 1H-NMR(D6-DMSO): 6,96-6,92(m,4H), 3,75(q,2H), 3,73(s,3H), 2,39(s,3H) |
| Ia-237 | 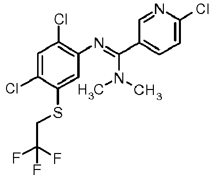 | 1H-NMR(D6-DMSO): 8,23-8,22(m,1H), 7,73-7,70(m,1H), 7,49-7,47(m,1H), 7,42(s,1H), 6,95(s,1H), 3,94(q,2H), 3,10(breit,3H), 2,83(breit,3H) |
| Ia-238 | 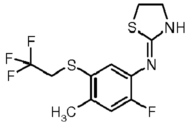 | 1H-NMR(D6-DMSO): 7,78(breit,1H), 7,12-7,07(m,2H), 3,83-3,75(m,2H), 3,55(breit,2H), 3,29(t,2H), 2,33(s,3H) |
| Ia-239 | 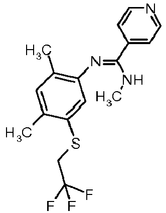 | 1H-NMR(D6-DMSO): 8,46-8,45(m,2H), 7,19(breit,1H), 7,11(m,2H), 6,90(s,1H), 6,42(breit,1H), 3,44(q,2H), 2,87(breit,3H), 2,17(s,3H), 2,07(s,3H) |
| Ia-240 | 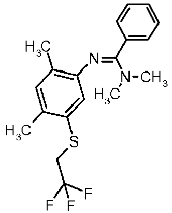 | 1H-NMR(D6-DMSO): 7,28-7,22(m,3H), 7,10-7,08(m,2H), 6,81(s,1H), 6,37(s,1H), 3,36(q,2H), 2,90(breit,6H), 2,13(s,3H), 2,05(s,3H) |
| Ia-241 | 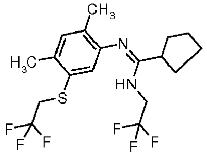 | 1H-NMR(D6-DMSO): 7,01(s,1H), 6,89(t,1H), 6,63(s,1H), 4,10-4,02(m,2H), 3,77(q,2H), 2,50(m,1H), 2,29(s,3H), 1,92(s,3H), 1,67-1,63(m,6H), 1,36-1,35(m,2H) |
| Ia-242 | 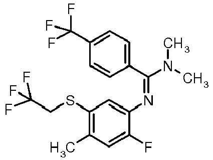 | 1H-NMR(D6-DMSO): 7,65(d,2H), 7,38(d,2H), 6,84(d,1H), 6,73(d,1H), 3,53(q,2H), 3,11(breit,3H), 2,73(breit,3H), 2,17(s,3H) |
| Ia-243 | 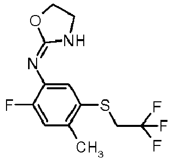 | 1H-NMR(D6-DMSO): 7,65(s,1H), 7,20(s,1H), 7,03-7,01(m,1H), 4,36(t,2H), 3,80(q,2H), 3,50(breit,2H), 2,31(s,3H) |
| Ia-244 | 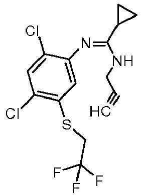 | 1H-NMR(D6-DMSO): 8,04(s,1H), 7,95(s,1H), 6,63(s,1H), 4,32(q,2H), 1,88(s,2H), 1,41-1,34(m,1H), 0,83-0,67(m,4H) |
| Ia-245 | 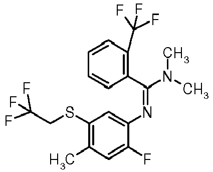 | 1H-NMR(D6-DMSO): 7,68-7,60(m,2H), 7,54-7,49(m,1H), 7,40(d,1H), 6,85(d,1H), 6,66(d,1H), 3,55-3,43(m,2H), 3,12(breit,3H), 2,63(breit,3H), 2,17(s,3H) |
| Ia-247 | 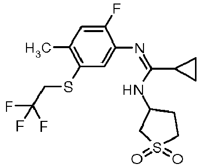 | 1H-NMR(D6-DMSO): 7,09-7,04(m,2H), 6,40-6,39(m,1H), 4,60-4,55(m,1H), 3,84(q,2H), 3,49-2,04(m,6H), 1,37-1,36(m,1H), 0,86-0,83(m,2H), 0,65-0,61(m,2H) |
| Ia-248 | 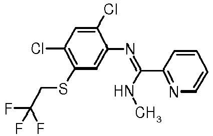 | 1H-NMR(D6-DMSO): 8,56-8,55(m,1H), 7,79-7,75(m,2H), 7,47(s,1H), 7,42-7,39(m,2H), 6,86(s,1H), 3,89(q,2H), 2,81(d,3H) |
| Ia-249 | 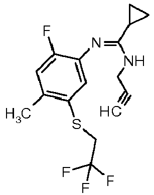 | 1H-NMR(D6-DMSO): 7,74-7,72(m,1H), 7,50-7,48(m,1H), 6,61(s,1H), 4,08(q,2H), 2,44(s,3H), 1,91(s,3H), 1,46-1,40(m,1H), 0,82-0,68(m,4H) |
| Ia-250 | 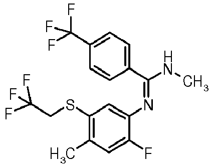 | 1H-NMR(D6-DMSO): 7,64(d,2H), 7,46(d,1H), 7,39(d,2H), 6,88(d,1H), 7,76(d,1H), 3,53(q,2H), 2,89(d,3H), 2,20(s,3H), |
| Ia-251 | 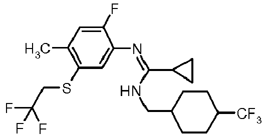 | logP(HCOOH)=2,23 |
| Ia-252 | 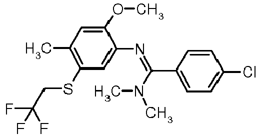 | 1H-NMR(D6-DMSO): 7,29-7,27(m,2H), 7,10-7,08(m,2H), 6,59(m,2H), 3,60(s,3H), 3,46(q,2H), 2,87(breit,6H), 2,22(s,3H) |
| Ia-253 | 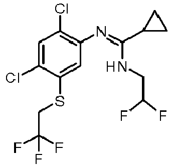 | 1H-NMR(D6-DMSO): 7,59(s,1H), 7,14(s,1H), 6,85(breit,1H), 6,20(tt,1H), 4,11(q,2H), 3,57-3,47(m,2H), 1,34(m,1H), 0,86-0,82(m,2H), 0,69-0,64(m,2H) |
| Ia-254 | 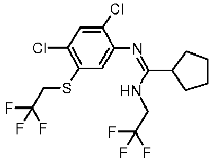 | 1H-NMR(D6-DMSO): 7,58(s,1H), 7,27(breit,1H), 6,96(s,1H), 4,14-4,02(m,4H), 2,46-2,44(m,1H), 1,67-1,63(m,6H), 1,39-1,38(m,2H) |
| Ia-255 | 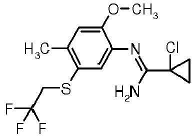 | 1H-NMR(D6-DMSO): 6,87(s,1H), 6,81(s,1H), 5,98(breit,2H), 3,72(q,2H), 3,69(s,3H), 2,37(s,3H), 1,57-1,54(m,2H), 1,27-1,24(m,2H) |
| Ia-256 | 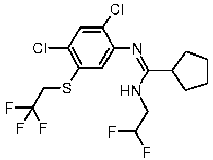 | 1H-NMR(D6-DMSO): 7,58(s,1H), 7,23(breit,1H), 7,04(s,1H), 6,23(tt,1H), 4,12(q,2H), 3,62-3,52(m,2H), 2,45-2,41(m,1H), 1,68-1,60(m,6H), 1,39-1,37(m,2H) |
| Ia-257 | 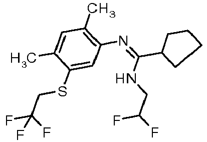 | 1H-NMR(D6-DMSO): 7,00(s,1H), 6,79(breit,1H), 6,68(s,1H), 6,16(tt,1H), 3,78(q,2H), 3,57(m,2H), 2,49(m,1H), 2,28(s,3H), 1,93(s,3H), 1,68-1,61(m,6H), 1,35-1,34(m,2H) |
| Ia-258 | 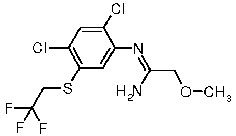 | 1H-NMR(D6-DMSO): 7,56(s,1H), 7,05(s,1H), 6,32(breit,2H), 4,09(q,2H), 3,98-3,96(m,2H), 3,33(s,3H) |
| Ia-259 | 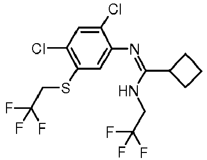 | 1H-NMR(D6-DMSO): 7,55(s,1H), 7,34(breit,1H), 6,99(s,1H), 4,14-4,02(m,4H), 3,26-3,17(m,1H), 2,12-1,56(m,6H) |
| Ia-260 | 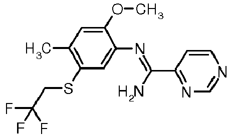 | 1H-NMR(D6-DMSO): 9,30-9,29(m,1H), 8,97-8,96(m,1H), 8,26-8,25(m,1H), 7,01(s,1H), 6,96(s,1H), 6,60(breit,2H), 3,75(q,2H), 3,73(s,3H), 2,42(s,3H) |
| Ia-261 | 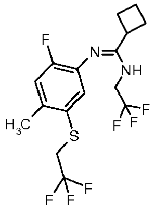 | 1H-NMR(D6-DMSO): 7,22(t,1H), 7,06-7,03(m,1H), 6,90-6,88(m,1H), 4,10-4,01(m,2H), 3,82(q,2H), 3,20-3,16(m,1H), 2,32(s,3H), 2,11-2,06(m,2H), 1,79-1,59(m,4H) |
| Ia-262 | 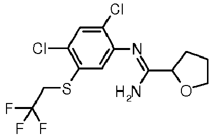 | 1H-NMR(D6-DMSO): 7,56(s,1H), 7,06(s,1H), 6,26(breit,2H), 4,39(m,1H), 4,09(q,2H), 3,98-3,92(m,1H), 3,77-3,75(m,1H), 2,17-2,08(m,2H), 1,92-1,86(m,2H) |
| Ia-263 | 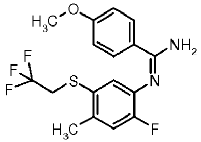 | 1H-NMR(D6-DMSO): 7,94(d,2H), 7,13-7,07(m,2H), 6,97(d,2H), 6,44(breit,2H), 3,86(q,2H), 3,81(s,3H), 2,35(s,3H) |
| Ia-264 | 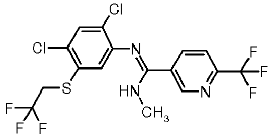 | 1H-NMR(D6-DMSO): 8,59(s,1H), 7,88-7,82(m,3H), 7,48(s,1H), 6,91(s,1H), 3,87(q,2H), 2,93(d,3H) |
| Ia-265 | 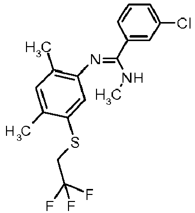 | 1H-NMR(D6-DMSO): 7,34-7,01(m,5H), 6,89(s,1H), 6,41(s,1H), 3,39(q,2H), 2,86(breit,3H), 2,18(s,3H), 2,07(s,3H) |
| Ia-266 | 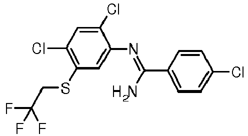 | 1H-NMR(D6-DMSO): 8,03-7,99(m,2H), 7,62(s,1H), 7,53-7,51(m,2H), 7,15(s,1H), 6,75(breit,2H), 4,13(q,2H) |
| Ia-267 | 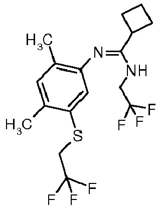 | 1H-NMR(D6-DMSO): 7,02(t,1H), 6,98(s,1H), 6,63(s,1H), 4,11-4,02(m,2H), 3,78(q,2H), 3,14(m,1H), 2,28(s,3H), 2,12-2,04(m,2H), 1,91(s,3H), 1,75-1,59(m,4H) |
| Ia-268 | 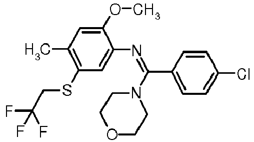 | 1H-NMR(D6-DMSO): 7,30-7,28(m,2H), 7,14-7,11(m,2H), 6,64-6,62(m,2H), 3,63(m,6H), 3,59(s,3H), 3,48(q,2H), 3,34-3,29(breit,2H), 2,23(s,3H) |
| Ia-269 | 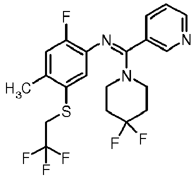 | 1H-NMR(D6-DMSO): 8,48-8,46(m,1H), 8,40-8,39(m,1H), 7,67-7,65(m,1H), 7,33-7,30(m,1H), 6,90-6,83(m,2H), 3,66(q,2H), 3,59-3,49(m,4H), 2,18(s,3H), 2,15-2,06(m,4H) |
| Ia-270 | 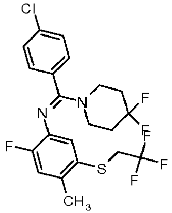 | 1H-NMR(D6-DMSO): 7,37-7,35(m,2H), 7,25-7,23(m,2H), 6,87-6,82(m,2H), 3,61(q,2H), 3,47(m,4H), 2,19(s,3H), 2,03(m,4H) |
| Ia-271 | 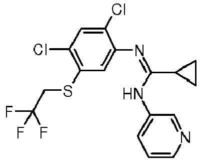 | 1H-NMR(D6-DMSO): 8,14(m,1H), 8,02(m,1H), 7,86-7,83(m,2H), 7,23-7,22(m,1H), 7,15-7,14(m,1H), 4,26-4,18(m,2H), 1,52(m,1H), 1,28(m,2H), 0,85-0,83(m,2H) |
| Ia-272 | 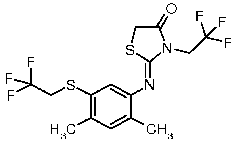 | 1H-NMR(D6-DMSO): 7,17(s,1H), 7,00(s,1H), 4,59(q,2H), 4,18(s,2H), 3,81(q,2H), 2,33(s,3H), 2,04(s,3H) |
| Ia-273 | 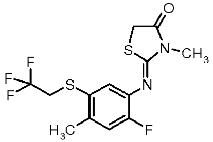 | 1H-NMR(D6-DMSO): 7,26-7,23(m,2H), 4,09(s,2H), 3,87(q,2H), 3,17(s,3H), 2,37(s,3H) |
| Ia-274 | 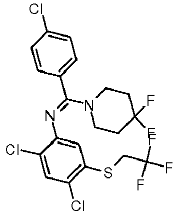 | 1H-NMR(D6-DMSO): 7,44(m,1H), 7,39-7,37(m,2H), 7,28-7,26(m,2H), 6,92(s,1H), 3,90-3,83(q,2H), 3,60(m,4H), 2,06(m,4H) |
| Ia-275 | 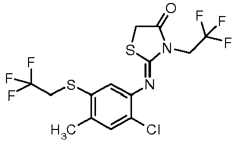 | 1H-NMR(D6-DMSO): 7,47(s,1H), 7,20(s,1H), 4,60(q,2H), 4,25(s,2H), 3,94(q,2H), 2,35(s,3H) |
| Ia-276 | 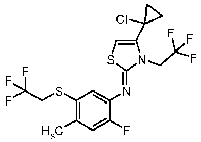 | 1H-NMR(D6-DMSO): 7,24-7,19(m,2H), 6,72(s,1H), 4,93(q,2H), 3,86(q,2H), 2,36(s,3H), 1,52-1,44(m,4H) |
| Ia-277 | 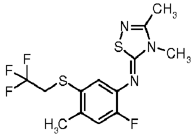 | 1H-NMR(D6-DMSO): 7,24-7,19(m,2H), 3,86(q,2H), 3,44(s,3H), 2,36(s,6H) |
| Ia-278 | 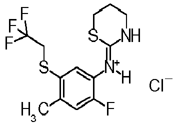 | 1H-NMR(D6-DMSO): 7,65-7,64(m,1H), 7,42-7,40(m,1H), 4,02-3,96(q,2H), 3,45(t,2H), 3,41(breit,1H), 3,27(t,2H), 2,41(s,3H), 2,09-2,05(m,2H) |
| Ia-279 | 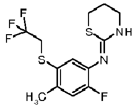 | 1H-NMR(D6-DMSO): 7,55(breit,1H), 7,05(s,1H), 7,02(s,1H), 3,83-3,75(q,2H), 3,26-3,23(m,2H), 2,97-2,94(m,2H), 2,31(s,3H), 1,96-1,90(m,2H). |
| Ib-17 | 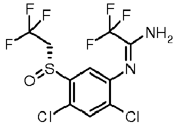 | 1H-NMR(D6-DMSO): 7,87(s,1H), 7,54(breit,2H), 7,33(s,1H), 4,18-4,10(m,2H) |
| Ib-18 | 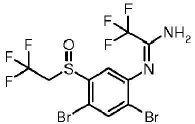 | 1H-NMR(D6-DMSO): 8,10(s,1H), 7,80-7,30(breit,1H), 7,29(s,1H), 4,16-4,05(m,2H) |
| Ib-19 | 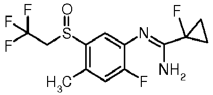 | 1H-NMR(D6-DMSO): 7,29-7,27(m,1H), 7,18-7,15(m,1H), 6,74(breit,2H), 4,06-3,98(m,2H), 2,30(s,3H), 1,39-1,29(m,4H) |
| Ib-20 | 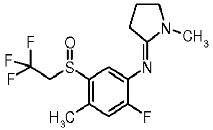 | 1H-NMR(D6-DMSO): 7,27(d,1H), 7,14(d,1H), 4,09-3,98(m,2H), 3,34-3,33(m,2H), 2,90(s,3H), 2,38-2,22(m,5H), 1,93-1,88(m,2H) |
| Ib-21 | 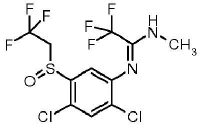 | logP(HCOOH)=3,32, logP(neutral=3,32) |
| Ib-22 | 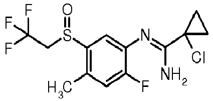 | 1H-NMR(D6-DMSO): 7,27-7,24(m,1H), 7,19-7,16(m,1H), 6,57(breit,2H), 4,05-3,97(m,2H), 2,31(s,3H), 1,57(m,2H), 1,31-1,28(m,2H) |
| Ib-23 | 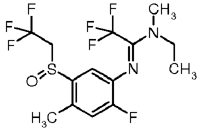 | 1H-NMR(D6-DMSO): 7,28(d,1H), 7,21(d,1H), 4,14-3,98(m,2H), 3,48-3,43(m,2H), 2,97(s,3H), 2,31(s,3H), 1,15(t,3H) |
| Ib-24 | 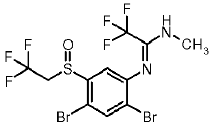 | 1H-NMR(D6-DMSO): 8,41(breit,1H), 8,04(s,1H), 7,27(s,1H), 4,25-4,02(m,2H), 2,66(breit,3H) |
| Ib-25 | 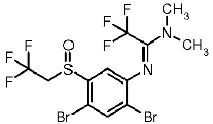 | 1H-NMR(D6-DMSO): 8,03(s,1H), 7,22(s,1H), 4,23-4,05(m,2H), 3,03(breit,3H) und 3H unter dem DMSO-Peak |
| Ib-26 | 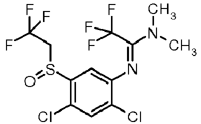 | 1H-NMR(D6-DMSO): 7,83(s,1H), 7,25(s,1H), 4,16-4,12(m,1H), 4,27-4,21(m,1H), 3,04(s,6H) |
| Ib-27 | 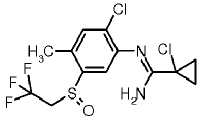 | 1H-NMR(D6-DMSO): 7,41(m,1H), 7,23(m,1H), 6,53(breit,2H), 4,06-3,97(m,2H), 2,29(s,3H), 1,62-1,59(m,2H), 1,32-1,29(m,2H) |
| Ib-28 | 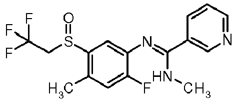 | 1H-NMR(D6-DMSO): 8,50-8,49(m,1H), 8,38(breit,1H), 7,66-7,60(m,2H), 7,34-7,31(m,1H), 7,16-7,14(m,1H), 6,96-6,93(m,1H), 3,97-3,88(m,1H), 3,76-3,70(m,1H), 2,91-2,90(d,3H), 2,20(s,3H) |
| Ib-29 | 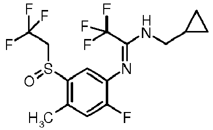 | 1H-NMR(D6-DMSO): 8,40(s,breit,1H), 7,28(d,1H), 7,21(d,1H), 4,15-4,06(m,1H), 4,00-3,94(m,1H), 3,08(s,breit,2H), 2,31(s,3H), 1,09(s,breit,1H), 0,46-0,44(m,2H), 0,22(s,breit,2H) |
| Ib-30 | 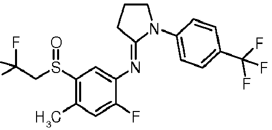 | 1H-NMR(D6-DMSO): 8,12(d,2H), 7,73(d,2H), 7,41(d,1H), 7,26(d,1H), 4,15-4,05(m,2H), 3,96(t,2H), 2,69-2,50(m,4H), 2,34(s,3H), 2,09-2,02(m,2H) |
| Ib-31 | 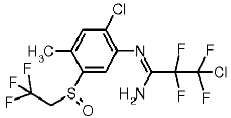 | 1H-NMR(D6-DMSO): 7,50(s,1H), 7,40(breit,2H), 7,28(s,1H), 4,10-4,02(q,2H), 2,32(s,3H) |
| Ib-32 | 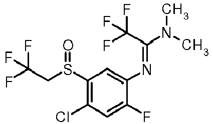 | 1H-NMR(D6-DMSO): 7,68(d,1H), 7,32(d,1H), 4,28-4,10(m,2H), 3,05(s,6H) |
| Ib-33 | 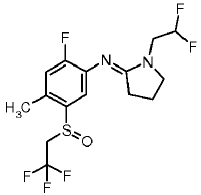 | 1H-NMR(D6-DMSO): 7,33-7,31(m,1H), 7,19-7,16(m,1H), 6,26(tt,1H), 4,12-3,98(m,2H), 3,87-3,78(dt,2H), 3,52-3,48(m,2H), 2,35(m,2H), 2,30(s,3H), 1,98-1,90(m,2H) |
| Ib-34 | 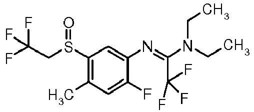 | 1H-NMR(D6-DMSO): 7,27(d,1H), 7,21(d,1H), 4,15-3,98(m,2H), 3,45-3,40(m,4H), 2,30(s,3H), 1,18-1,14(m,6H) |
| Ib-35 | 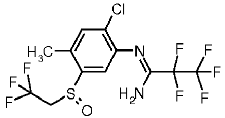 | 1H-NMR(D6-DMSO): 7,51(s,1H), 7,45(breit,2H), 7,29(s,1H), 4,11-4,01(m,2H), 2,32(s,3H) |
| Ib-36 | 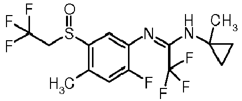 | 1H-NMR(D6-DMSO): 8,48(s,1H), 7,30(d,1H), 7,21(d,1H), 4,15-3,95(m,2H), 2,31(s,3H), 1,33-1,24(m,3H), 0,77(s,2H), 0,57(s,2H) |
| Ib-37 | 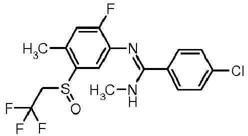 | 1H-NMR(D6-DMSO): 7,52(breit,1H), 7,37-7,35(m,2H), 7,22-7,20(m,2H), 7,06-7,04(m,1H), 6,97-6,95(m,1H), 3,92-3,83(m,1H), 3,64-3,58(m,1H), 2,89-2,88(d,3H), 2,20(s,3H) |
| Ib-38 | 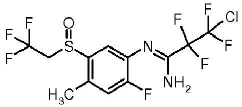 | 1H-NMR(D6-DMSO): 7,50(breit,2H), 7,34-7,32(m,1H), 7,28-7,25(m,1H), 4,21-4,02(m,2H), 2,34(s,3H) |
| Ib-39 | 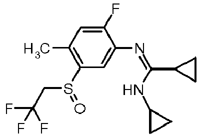 | 1H-NMR(D6-DMSO): 7,26-7,25(m,1H), 7,13-7,11(m,1H), 6,45(breit,1H), 4,05-3,96(m,2H), 2,29(s,3H), 2,21-2,18(m,1H), 1,87-1,84(m,1H), 0,81-0,80(m,2H), 0,66-0,54(m,4H), 0,44(m,2H) |
| Ib-40 | 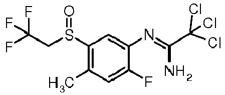 | 1H-NMR(D6-DMSO): 7,34-7,32(m,1H), 7,29-7,26(m,1H), 7,23(breit,2H), 4,13-4,01(m,2H), 2,34(s,3H) |
| Ib-41 | 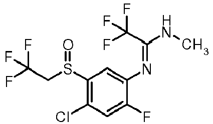 | 1H-NMR(D6-DMSO): 8,48(bs,1H,), 7,68(d,1H), 7,32(d,1H), 4,28-4,05(m,2H), 2,72(bs,3H) |
| Ib-42 | 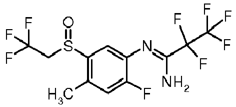 | 1H-NMR(D6-DMSO): 7,55(breit,2H), 7,35-7,33(m,1H), 7,29-7,26(m,1H), 4,12-4,01(m,2H), 2,34(s,3H) |
| Ib-43 | 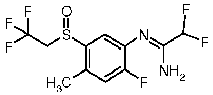 | 1H-NMR(D6-DMSO): 8,22-8,20(m,1H), 7,44-7,41(m,1H), 6,49(tt,1H), 4,21-3,93(m,2H), 2,36(s,3H) |
| Ib-44 | 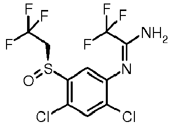 | 1H-NMR(D6-DMSO): 7,87(s,1H), 7,54(breit,2H), 7,33(s,1H), 4,18-4,10(m,2H) |
| Ib-45 | 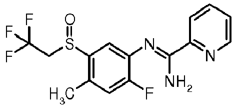 | 1H-NMR(D6-DMSO): 8,66-8,65(m,1H), 8,33-8,31(m,1H), 8,00-7,95(m,1H), 7,60-7,57(m,1H), 7,46-7,44(m, H), 7,27-7,24(m,1H), 6,95(breit,2H), 4,11-4,03(q,2H), 2,35(s,3H) |
| Ib-46 | 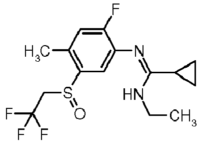 | 1H-NMR(D6-DMSO): 7,28-7,26(m,1H), 7,15-7,12(m,1H), 6,30(breit,1H), 4,02(m,2H), 3,19(m,2H), 2,29(s,3H), 1,90(m,1H), 1,08(t,3H), 0,85-0,81(m,2H), 0,64-0,62(m,2H) |
| Ib-47 | 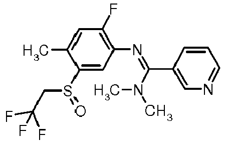 | 1H-NMR(D6-DMSO): 8,48-8,46(m,1H), 8,32(breit,1H), 7,65(breit,1H), 7,36-7,33(m,1H), 7,07-7,05(m,1H), 6,93-6,90(m,1H), 3,92-3,83(m,1H), 3,68-3,61(m,1H), 3,13(breit,3H), 2,81(breit,3H), 2,16(s,3H) |
| Ib-48 | 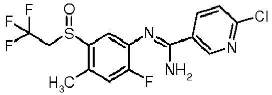 | 1H-NMR(D6-DMSO): 8,96-8,95(m,1H), 8,38-8,35(m,1H), 7,65-7,63(m,1H), 7,43-7,41(m,1H), 7,27-7,24(m,1H), 7,00(breit,2H), 4,10-4,02(q,2H), 2,35(s,3H) |
| Ib-49 | 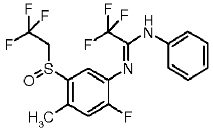 | logP(HCOOH)=3,22, logP(neutral)=3,12 |
| Ib-50 | 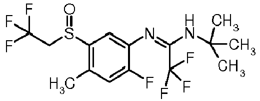 | 1H-NMR(D6-DMSO): 7,40(s,1H), 7,25-7,19(m,2H), 4,11-4,08(m,1H), 4,00-3,97(m,1H), 2,30(s,3H), 1,42(s,9H) |
| Ib-51 | 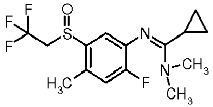 | 1H-NMR(D6-DMSO): 7,24-7,22(m,1H), 7,11-7,08(m,1H), 4,11-3,92(m,2H), 3,08(s,6H), 2,30(s,3H), 1,66(m,1H), 0,68-0,63(m,2H), 0,32-0,30(m,1H), 0,23-0,21(m,1H) |
| Ib-52 | 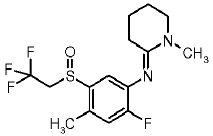 | 1H-NMR(D6-DMSO): 7,20(d,1H), 7,13(d,1H), 4,09-3,97(m,2H), 3,32(s,3H), 3,28(t,2H), 2,22(s,3H), 2,20-2,05(m,2H), 1,78-1,72(m,2H), 1,62-1,56(m,2H) |
| Ib-53 | 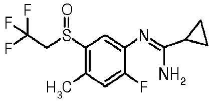 | 1H-NMR(D6-DMSO): 7,15-7,12(m,1H), 6,94-6,92(m,1H), 4,06-3,88(m,2H), 2,32(s,3H), 1,55(m,1H), 0,88-0,84(m,2H), 0,74(m,2H) |
| Ib-54 | 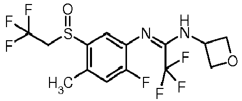 | 1H-NMR(D6-DMSO): 8,97(breit,1H), 7,30-7,22(m,2H), 4,77(breit,2H), 4,59(breit,3H), 4,19-4,07(m,1H), 3,97(breit,1H), 2,32(s,3H) |
| Ib-55 | 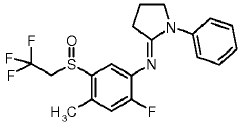 | 1H-NMR(D6-DMSO): 7,86(d,2H), 7,37(m,3H), 7,22(d,1H), 7,09(dd,1H), 4,14-4,02(m,2H), 3,73(dd,2H), 2,67-2,57(m,2H), 2,33(s,3H), 2,07-1,99(m,2H) |
| Ib-56 | 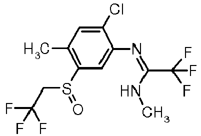 | 1H-NMR(D6-DMSO): 2,30(s,3H), 2,62(breit,3H), 4,17-3,95(m,2H), 7,28(beit,1H), 7,44(s,1H), 8,22(breit,1H) |
| Ib-57 | 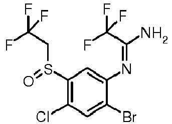 | 1H-NMR(D6-DMSO): 8,32(s,1H), 7,40-7,36(breit,1H), 7,29(s,1H), 4,19-4,14(m,2H) |
| Ib-58 | 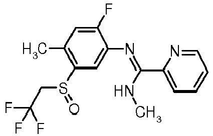 | 1H-NMR(D6-DMSO): 8,56-8,55(m,1H), 7,77-7,73(m,1H), 7,69(breit,1H), 7,41-7,38(m,2H), 7,10-7,08(m,1H), 7,02-6,99(m,1H), 3,97-3,91(m,1H), 3,73-3,67(m,1H), 2,84-2,83(d,3H), 2,23(s,3H) |
| Ib-59 | 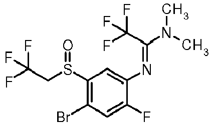 | 1H-NMR(D6-DMSO): 7,78(d,1H), 7,32(d,1H), 4,20-4,06(m,2H), 3,05(s,3H) |
| Ib-60 | 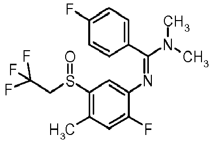 | 1H-NMR(D6-DMSO): 7,29-7,12(m,4H), 6,97(d,1H), 6,93(d,1H), 3,88-3,79(m,1H), 3,62-3,50(m,1H), 3,11(breit,3H), 2,79-2,77(breit,3H), 2,17(s,3H) |
| Ib-61 | 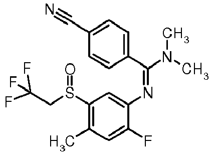 | 1H-NMR(D6-DMSO): 7,81-7,79(m,2H), 7,46(breit,1H), 7,29(breit,1H), 6,98(d,1H), 6,94(d,1H), 3,88-3,79(m,1H), 3,63-3,52(m,1H), 3,14(breit,3H), 2,76(breit,3H), 2,16(s,3H) |
| Ib-62 | 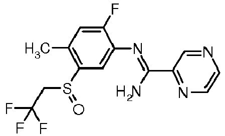 | 1H-NMR(D6-DMSO): 9,46-9,45(m,1H), 8,84(m,1H), 8,74-8,73(m,1H), 7,48-7,46(m,1H), 7,29-7,26(m,1H), 7,12(breit,2H), 4,12-4,04(m,2H), 2,36(s,3H) |
| Ib-63 | 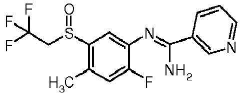 | 1H-NMR(D6-DMSO): 9,13(s,1H), 8,70-8,69(m,1H), 8,32-8,30(m,1H), 7,52-7,49(m,1H), 7,43-7,41(m,1H), 7,26-7,23(m,1H), 6,94(breit,2H), 4,11-4,03(m,2H), 2,35(s,3H) |
| Ib-64 | 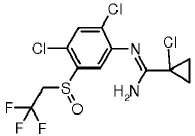 | 1H-NMR(D6-DMSO): 7,84(m,1H), 7,35(m,1H), 6,85(breit,2H), 4,16-4,08(m,2H), 1,65(m,2H), 1,37(m,2H) |
| Ib-65 | 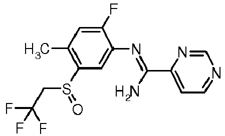 | 1H-NMR(D6-DMSO): 9,34(m,1H), 9,01-9,00(m,1H), 8,28-8,27(m,1H), 7,47-7,45(m,1H), 7,29-7,25(m,1H), 7,16(breit,2H), 4,12-4,01(m,2H), 2,36(s,3H) |
| Ib-66 | 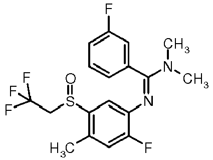 | 1H-NMR(D6-DMSO): 7,38-7,33(m,1H), 7,15-7,10(m,1H), 7,03(breit,2H), 6,98-6,92(m,2H), 3,89-3,82(m,1H), 3,61-3,55(m,1H), 3,12(breit,3H), 2,83(breit,3H), 2,17(s,3H) |
| Ib-67 | 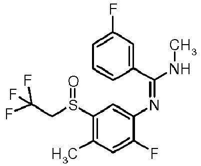 | 1H-NMR(D6-DMSO): 7,56-7,54(m,1H), 7,36-7,30(m,1H), 7,19-6,95(m,5H), 3,94-3,85(m,1H), 3,70-3,64(m,1H), 2,89-2,88(m,3H), 2,21(s,3H) |
| Ib-68 | 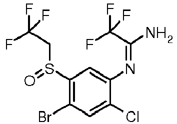 | 1H-NMR(D6-DMSO): 7,99(s,1H), 7,63(breit,2H), 7,32(s,1H), 4,18-4,06(m,2H) |
| Ib-69 | 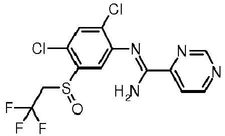 | 1H-NMR(D6-DMSO): 9,35(m,1H), 9,03-9,02(m,1H), 8,32-8,28(m,1H), 7,87(s,1H), 7,41(s,1H), 7,08(breit,2H), 4,19-4,11(m,2H) |
| Ib-71 | 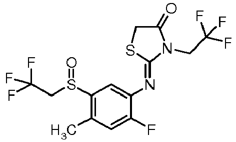 | 1H-NMR(D6-DMSO): 7,51(d,1H), 7,38(d,1H), 4,62-4,57(m,2H), 4,26-4,14(m,3H), 4,04-3,94(m,1H), 2,36(s,3H) |
| Ib-72 | 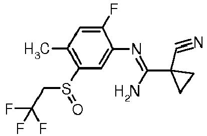 | 1H-NMR(D6-DMSO): 7,29-7,27(m,1H), 7,21-7,18(m,1H), 6,42(breit,2H), 4,07-3,99(m,2H), 2,32(s,3H), 1,68-1,59(m,4H) |
| Ib-73 | 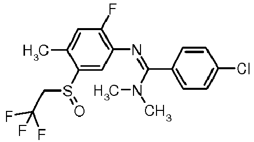 | 1H-NMR(D6-DMSO): 7,3-6,93(m,6H), 3,88-3,79(m,1H), 3,58-3,47(m,1H), 3,10(breit,3H), 2,78(breit,3H), 2,17(s,3H) |
| Ib-74 | 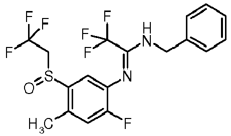 | 1H-NMR(D6-DMSO): 8,87(breit,1H), 7,33-7,16(m,7H), 4,47(breit,2H), 4,12-4,06(m,1H), 3,90(breit,1H), 2,30(s,3H) |
| Ib-75 | 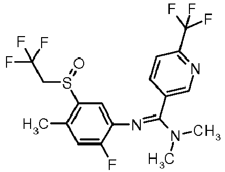 | 1H-NMR(D6-DMSO): 8,49(m,1H), 8,09(m,1H), 7,90-7,88(m,1H), 7,03-6,96(m,2H), 3,90-3,84(m,1H), 3,56-3,50(m,1H), 3,17(breit,3H), 2,80(breit,3H), 2,17(s,3H) |
| Ib-76 | 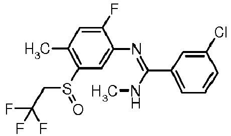 | 1H-NMR(D6-DMSO): 7,57-7,56(q,1H) 7,40-7,38(m,1H), 7,31-7,27(m, 2H), 7,13-7,08(m,2H), 6,98-6,95(m,1H), 3,92-3,83(m,1H), 3,70-3,63(m,1H), 2,89-2,87(d,3H), 2,21(s,3H) |
| Ib-77 | 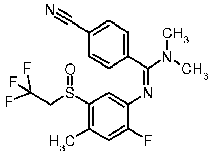 | 1H-NMR(D6-DMSO): 7,81-7,79(m,2H), 7,46(breit,1H), 7,29(breit,1H), 6,98(d,1H), 6,94(d,1H), 3,88-3,79(m,1H), 3,63-3,52(m,1H), 3,14(breit,3H), 2,75(breit,3H), 2,16(2,3H) |
| Ib-78 | 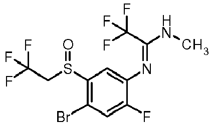 | 1H-NMR(D6-DMSO): 8,49(breit,1H), 7,78(d,1H), 7,33(d,1H), 4,24-4,02(m,2H), 2,73(breit,3H) |
| Ib-79 | 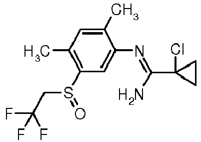 | 1H-NMR(D6-DMSO): 7,11(s,1H), 7,08(s,1H), 6,19(breit,2H), 4,00-3,92(m,2H), 2,27(s,3H), 2,00(s,3H), 1,58(m,2H), 1,30-1,29(m,2H) |
| Ib-80 | 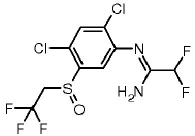 | 1H-NMR(D6-DMSO): 7,8(s,1H), 7,30(s,1H), 7,15(breit,2H), 6,40(t,1H), 4,18-4,10(m,2H) |
| Ib-81 | 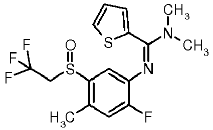 | 1H-NMR(D6-DMSO): 7,61-7,60(m,1H), 7,04-6,97(m,4H), 3,92-3,86(m,1H), 3,65-3,52(m,1H), 3,02(s,6H), 2,20(s,3H) |
| Ib-82 | 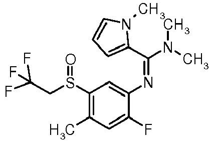 | 1H-NMR(D6-DMSO): 6,96-6,91(m,2H), 6,70(breit,1H), 5,90(breit,2H), 3,83-3,74(m,2H), 3,18(s,6H), 2,21(breit,6H) |
| Ib-83 | 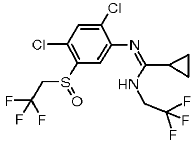 | 1H-NMR(D6-DMSO): 7,78(s,1H), 7,26(m,1H), 7,01(breit,1H), 4,22-4,01(m,4H), 1,40-1,38(m,1H), 0,96-0,86(m,2H), 0,78-0,71(m,2H) |
| Ib-84 | 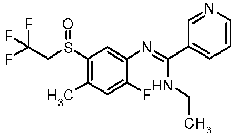 | 1H-NMR(D6-DMSO): 8,50-8,48(m,1H), 8,38(m,1H), 7,65-7,59(m,2H), 7,33-7,30(m,1H), 7,15-7,13(m,1H), 6,95-6,92(m,1H), 3,97-3,88(m,1H), 3,79-3,70(m,1H), 3,44-3,37(m,2H), 2,20(s,3H), 1,22(t,3H) |
| Ib-85 | 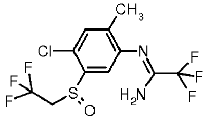 | 1H-NMR(D6-DMSO): 7,52-7,51(m,1H), 7,3(breit,2H), 7,18(s,1H), 4,16-4,02(m,2H), 2,10(s,3H) |
| Ib-86 | 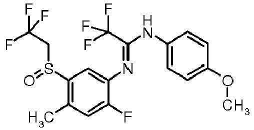 | logP(HCOOH)=3,04, logP(neutral)=3,05 |
| Ib-87 | 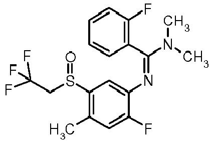 | 1H-NMR(D6-DMSO): 7,35-6,92(m,6H), 3,85-3,79(m,1H), 3,58-3,51(m,1H), 3,16(breit,3H), 2,79(breit,3H), 2,16(s,3H) |
| Ib-88 | 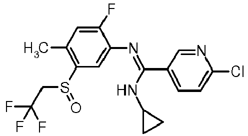 | 1H-NMR(D6-DMSO): 8,22(m,1H), 7,80(m,1H), 7,66(m,1H), 7,48-7,46(m,1H), 7,20-7,21(m,1H), 7,01-6,98(m,1H), 4,05-3,93(m,1H), 3,80(m,1H), 2,93(m,1H), 2,22(s,3H), 0,73(m,2H), 0,59(m,2H) |
| Ib-89 | 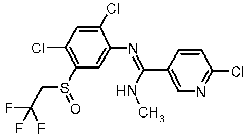 | 1H-NMR(D6-DMSO): 8,28-8,27(m,1H), 7,90(breit,1H), 7,67-7,64(m,2H), 7,50-7,48(m,1H), 7,03(s,1H), 4,17-4,05(m,1H), 3,89-3,80(m,1H), 2,92-2,91(d,3H) |
| Ib-90 | 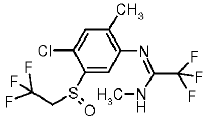 | 1H-NMR(D6-DMSO): 8,09(breit,1H), 7,44(s,1H), 7,12(breit,1H), 4,18-4,05(m,2H), 2,68-2,63(breit,3H), 2,11(s,3H) |
| Ib-91 | 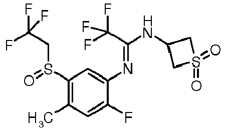 | 1H-NMR(D6-DMSO): 9,01(breit,1H), 7,38(d,1H), 7,24(d,1H), 4,58-4,57(m,2H), 4,35(breit,3H), 4,16-4,09(m,1H), 3,93(breit,1H), 2,32(s,3H) |
| Ib-92 | 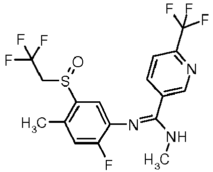 | 1H-NMR(D6-DMSO): 8,60(s,1H), 7,84-7,81(m,3H), 7,13-7,11(m,1H), 7,02-6,99(m,1H), 3,97-3,88(m,1H), 3,67-3,61(m,1H), 2,94-2,93(d,3H), 2,21(s,3H) |
| Ib-93 | 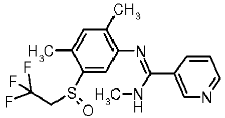 | 1H-NMR(D6-DMSO): 8,46-8,45(m,1H) 8,32(breit,1H), 7,58-7,56(m,1H), 7,37-7,29(m,2H), 6,99(s,1H), 6,66(s,1H), 3,74-3,68(m,2H), 2,91-2,90(d,3H), 2,17(s,3H), 2,15(s,3H) |
| Ib-94 | 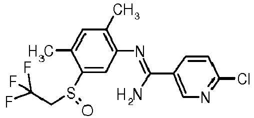 | 1H-NMR(D6-DMSO): 8,98-8,97(m,1H), 8,40-8,37(m,1H), 7,71-7,68(m,1H), 7,40-7,38(m,1H), 7,26-7,24(m,1H), 4,06-3,96(m,2H), 2,33(s,3H), 2,16(s,3H) |
| Ib-95 | 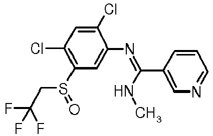 | 1H-NMR(D6-DMSO): 8,53-8,51(m,1H), 8,41(m,1H), 7,85(q,1H), 7,63(s,1H), 7,62-7,59(m,1H), 7,36-7,33(m,1H), 7,00(s,1H), 4,12-4,00(m,1H), 3,86-3,75(m,1H), 2,93-2,92(d,3H) |
| Ib-96 | 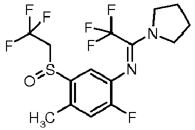 | 1H-NMR(D6-DMSO): 7,29(d,1H), 7,21(d,1H), 4,15-3,99(m,2H), 3,41(breit,4H), 2,31(s,3H), 1,86(breit,4H) |
| Ib-97 | 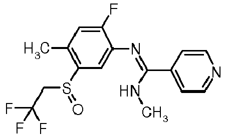 | 1H-NMR(D6-DMSO): 8,52-8,50(m,1H), 7,65-7,64(q,1H), 7,19-7,12(m,3H), 6,97-6,95(m,1H), 3,96-3,87(m,1H), 3,74-3,68(m,1H), 2,90-2,89(d,3H), 2,20(s,3H) |
| Ib-98 | 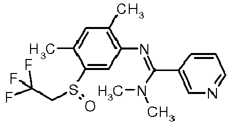 | 1H-NMR(D6-DMSO): 8,44-8,43(m,1H), 8,32-8,29(m,1H), 7,60(m,1H), 7,35-7,31(m,1H), 6,94(s,1H), 6,66(s,1H), 3,70-3,64(m,1H), 3,30-3,24(m,1H), 2,96(breit,6H), 2,14(s,3H), 2,13(s,3H) |
| Ib-99 | 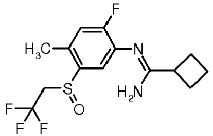 | 1H-NMR(D6-DMSO): 7,30-7,25(m,1H), 7,09(m,1H), 6,65(breit,2H), 4,07-4,02(m,2H), 2,97-2,93(m,1H), 2,33(s,3H), 2,22-1,71(m,6H) |
| Ib-100 | 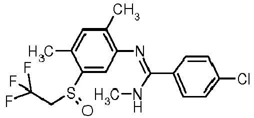 | 1H-NMR(D6-DMSO): 7,35-7,15(m,4H), 6,98-6,96(m,2H), 6,61(s,1H), 3,69-3,63(m,1H), 3,22-3,17(m,1H), 2,88(breit,3H), 2,17(s,3H), 2,16(s,3H) |
| Ib-101 | 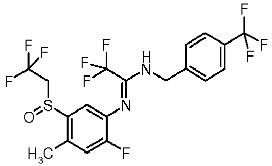 | 1H-NMR(D6-DMSO): 8,98(breit,1H), 7,72-7,70(m,2H), 7,52-7,51(breit,1H), 7,21-7,19(m,3H), 4,56(breit,2H), 4,13-4,06(m,1H), 3,93-3,90(m,1H), 2,28(s,3H) |
| Ib-102 | 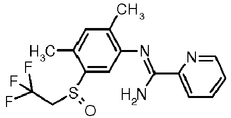 | 1H-NMR(D6-DMSO): 8,66-8,64(m,1H), 8,37-8,32(m,1H), 7,99-7,95(m,1H), 7,59-7,56(m,1H), 7,27(s,1H), 7,19(s,1H), 6,60(breit,2H), 4,05-3,97(m,2H), 2,31(s,3H), 2,13(s,3H) |
| Ib-103 | 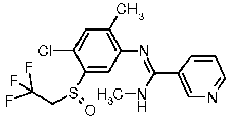 | 1H-NMR(D6-DMSO): 8,50-8,49(m,1H), 8,36(s,1H), 7,58-7,57(m,2H), 7,34-7,30(m,2H), 6,68(breit,1H), 3,91-3,82(m,1H), 3,59-3,53(m,1H), 2,92-2,91(d,3H), 2,20(s,3H) |
| Ib-104 | 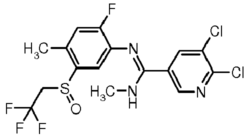 | 1H-NMR(D6-DMSO): 8,16-8,14(m,2H), 7,78-7,77(breit,1H), 7,23-7,21(m,1H), 7,03-7,00(m,1H), 4,02-3,93(m,1H), 3,84-3,77(m,1H), 2,91-2,90(d,3H), 2,23(s,3H) |
| Ib-105 | 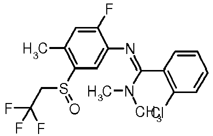 | 1H-NMR(D6-DMSO): 7,46-6,94(m,6H), 3,85-3,74(m,1H), 3,54-3,48(m,1H), 3,17(breit,3H), 2,74(breit,3H), 2,15(s,3H) |
| Ib-106 | 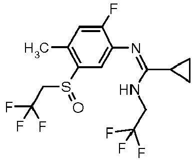 | 1H-NMR(D6-DMSO): 7,29-7,27(m,1H), 7,20-7,17(m,1H), 6,84(breit,1H), 4,11-3,64(m,4H), 2,30(s,3H), 1,44-1,41(m,1H), 0,91-0,84(m,2H), 0,72-0,69(m,2H) |
| Ib-107 | 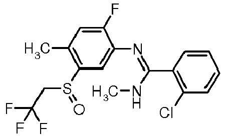 | 1H-NMR(D6-DMSO): 7,52-6,95(m,7H), 3,82-3,72(m,2H), 2,91-2,90(d,3H), 2,18(s,3H) |
| Ib-108 | 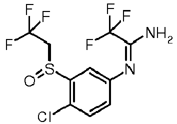 | 1H-NMR(D6-DMSO): 7,57(d,1H), 7,42(breit,2H), 7,30(d,1H), 7,10-7,07(m,1H), 4,22-4,04(m,2H) |
| Ib-109 | 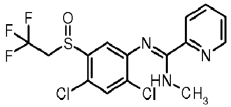 | 1H-NMR(D6-DMSO): 8,57-8,55(m,1H), 7,91(breit,1H), 7,82-7,77(m,1H), 7,66(s,1H), 7,44-7,41(m,2H), 6,96(s,1H), 4,10-4,01(m,1H), 3,87-3,78(m,1H), 2,84-2,83(d,3H) |
| Ib-110 | 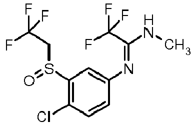 | 1H-NMR(D6-DMSO): 8,14(breit,1H), 7,48(d,1H), 7,22(d,1H), 7,03-7,00(m,1H), 4,25-4,01(m,2H), 3,67(breit,3H) |
| Ib-111 | 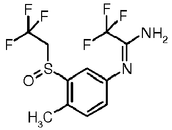 | 1H-NMR(D6-DMSO): 7,31-7,29(m,2H), 7,25(breit,2H), 6,98-6,95(m,1H), 4,09-4,00(m,2H), 2,33(s,3H) |
| Ib-112 | 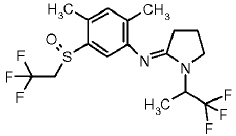 | 1H-NMR(D6-DMSO): 7,09(m,2H), 5,17-5,13(m,1H), 4,03-3,93(m,2H), 3,51-3,45(m,1H), 3,32-3,30(m,1H), 2,43-2,07(m,2H), 2,27(s,3H), 2,06(s,3H), 1,95-1,88(m,2H), 1,40-1,38(d,3H) |
| Ib-113 | 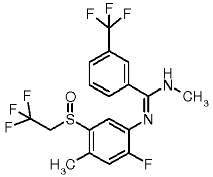 | 1H-NMR(D6-DMSO): 7,70-7,61(m,2H), 7,57(s,1H), 7,53-7,44(m,2H), 7,12(d,1H), 7,65(d,1H), 3,87-3,77(m,1H), 3,66-3,54(m,1H), 2,90(d,3H), 2,19(s,3H) |
| Ib-114 | 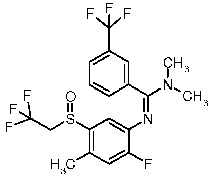 | 1H-NMR(D6-DMSO): 7,65(d,2H), 7,54(t,1H), 7,39(breit,1H), 7,04(breit,1H), 6,92(d,1H), 3,83-3,70(m,2H), 3,14(breit,3H), 3,28(breit,3H), 2,16(s,3H) |
| Ib-115 | 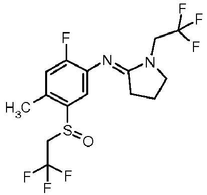 | 1H-NMR(D6-DMSO): 7,31-7,29(m,1H), 7,20-7,17(m,1H), 4,27(q,2H), 4,12-4,00(m,2H), 3,52(t,2H), 2,47-2,25(m,2H), 2,31(s,3H), 2,00-1,92(m,2H) |
| Ib-116 | 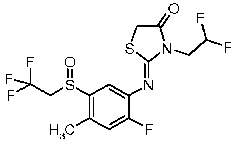 | 1H-NMR(D6-DMSO): 7,57(d,1H), 7,37(d,1H), 6,33(tt,1H), 4,25-4,13(m,5H), 4,01-3,89(m,2H), 2,36(s,3H) |
| Ib-117 | 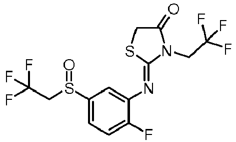 | 1H-NMR(D6-DMSO): 7,62-7,56(m,2H), 7,50-7,48(m,1H), 4,60(q,2H), 4,27-4,00(m,4H) |
| Ib-118 | 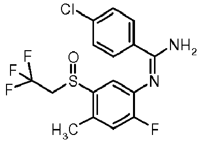 | 1H-NMR(D6-DMSO): 8,00(d,2H), 7,53(d,2H), 7,39(d,1H), 7,23(d,1H), 6,80(breit,2H), 4,06(q,2H), 2,34(s,3H) |
| Ib-119 | 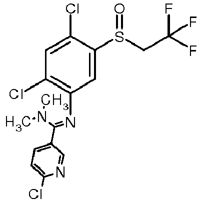 | 1H-NMR(D6-DMSO): 8,23(breit,1H), 7,73-7,72(breit,1H), 7,62(s,1H), 7,52-7,50(m,1H), 6,97(s,1H), 4,14-4,02(m,1H), 3,82-3,70(m,1H), 3,00(breit,6H) |
| Ib-120 | 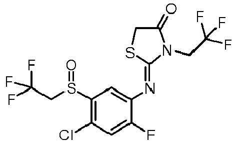 | 1H-NMR(D6-DMSO): 7,86(d,1H), 7,53(d,1H), 4,60(q,2H), 4,36-4,24(m,3H), 4,17-4,06(m,1H) |
| Ib-121 | 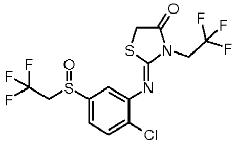 | 1H-NMR(D6-DMSO): 7,81(d,1H), 7,56(dd,1H), 7,47(d,1H), 4,61(q,2H), 4,31-4,07(m,4H) |
| Ib-122 | 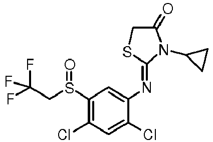 | 1H-NMR(D6-DMSO): 7,96(s,1H), 7,51(s,1H), 4,36-4,24(m,1H), 4,14-4,00(m,3H), 2,75-2,70(m,1H), 1,02-0,92(m,4H) |
| Ib-123 | 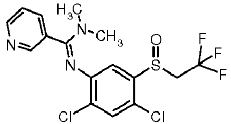 | 1H-NMR(D6-DMSO): 8,50-8,49(m,1H), 8,36(m,1H), 7,67-7,64(m,1H), 7,60(s,1H), 7,39-7,36(m,1H), 6,96(s,1H), 4,05-3,96(m,1H), 3,78-3,66(m,1H), 3,05(breit,6H) |
| Ib-124 | 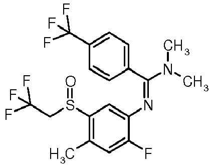 | 1H-NMR(D6-DMSO): 7,69(breit,2H), 7,54(breit,1H), 7,27(breit,1H), 6,97-6,14(m,2H), 3,84-3,70(m,1H), 3,43-3,30(m,1H), 3,15(breit,3H), 2,77(breit,3H), 2,16(s,3H) |
| Ib-125 | 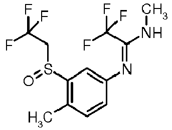 | 1H-NMR(D6-DMSO): 8,02(breit,1H), 7,22-7,17(m,2H), 6,89(breit,1H), 4,12-4,06(m,1H), 3,92(breit,1H), 2,76(breit,3H), 2,30(s,3H) |
| Ib-126 | 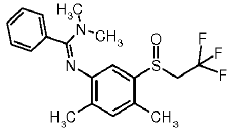 | 1H-NMR(D6-DMSO): 7,27(m,3H), 7,12(breit,2H), 6,92(s,1H), 6,62(s,1H), 3,50(m,2H), 2,93(breit,6H), 2,16(s,3H), 2,13(s,3H) |
| Ib-127 | 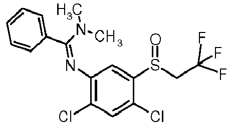 | 1H-NMR(D6-DMSO): 7,57(s,1H), 7,34-7,30(m,3H), 7,16(m,2H), 6,86(s,1H), 3,97-3,85(m,1H), 3,61-3,49(m,1H), 3,05(breit,6H) |
| Ib-128 | 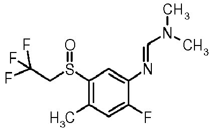 | 1H-NMR(D6-DMSO): 7,86(s,1H), 7,44(d,1H), 7,13(d,1H), 4,10-3,98(m,2H), 3,05(bs,3H), 2,95(bs,3H), 2,30 (s,3H) |
| Ib-129 | 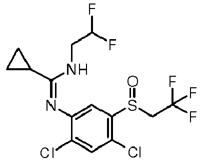 | 1H-NMR(D6-DMSO): 7,78(s,1H), 7,34(s,1H), 6,95(breit,1H), 6,21(tt,1H), 4,22-4,07(m,2H), 3,59-3,52(m,2H), 1,37(m,1H), 0,90-0,87(m,2H), 0,73-0,71(m,2H) |
| Ib-130 | 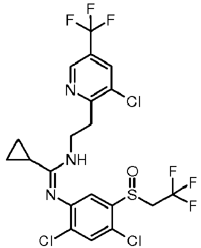 | 1H-NMR(D6-DMSO): 8,88(s,1H), 8,39(m,1H), 7,73(s,1H), 7,27(s,1H), 6,66(m,1H), 4,21-4,05(m,2H), 3,68-3,59(m,2H), 2,50(m,2H), 1,34(m,1H), 0,82-0,81(m,2H), 0,68-0,66(m,2H) |
| Ib-131 | 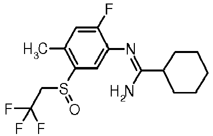 | 1H-NMR(D6-DMSO): 7,73-7,72(m,1H), 7,45(m,1H), 4,19-3,97(m,2H), 2,40(s,3H), 1,93-1,19(m,10H) |
| Ib-132 | 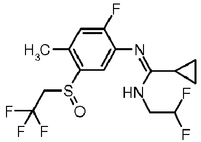 | 1H-NMR(D6-DMSO): 7,35-7,33(m,1H), 7,19-7,16(m,1H), 6,75(breit,1H), 6,15(tt,1H), 4,11-3,96(m,2H), 3,60-3,50(m,2H), 2,31(s,3H), 1,40(breit,1H), 0,90-0,82(m,2H), 0,73-0,67(m,2H) |
| Ib-133 | 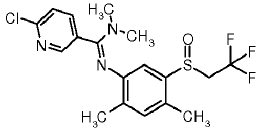 | 1H-NMR(D6-DMSO): 8,15(breit,1H), 7,70(breit,1H), 7,48-7,46(m,1H), 6,96(s,1H), 6,63(s,1H), 3,77-3,71(m,1H), 3,35-3,26(m,1H), 2,96(breit,6H), 2,15(s,3H), 2,13(s,3H) |
| Ib-134 | 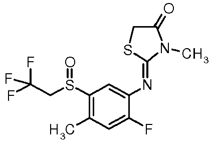 | 1H-NMR(D6-DMSO): 7,53(d,1H), 7,36(d,1H), 4,22-4,10(m,3H), 4,04-3,92(m,1H), 3,19(s,3H), 2,35(s,3H) |
| Ib-135 | 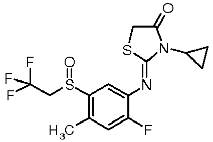 | 1H-NMR(D6-DMSO): 7,50(d,1H), 7,35(d,1H), 4,28-4,12(m,1H), 4,02-3,91(m,3H), 2,75-2,55(m,1H), 2,35(s,3H), 1,01-0,88(m,4H) -Peaks vom Hauptisomer- |
| Ib-136 | 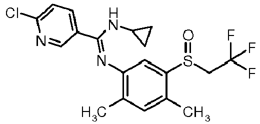 | 1H-NMR(D6-DMSO): 8,15(breit,1H), 7,60-7,44(m,3H), 7,01(s,1H), 6,70(s,1H), 3,79(m,2H), 2,90(m,1H), 2,19(s,3H), 2,16(s,3H), 0,72(m,2H), 0,58(m,2H) |
| Ib-137 | 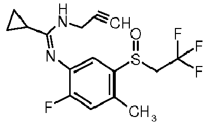 | 1H-NMR(D6-DMSO): 7,90-7,86(m,1H), 7,65-7,63(m,1H), 6,66(s,1H), 4,30-4,16(m,2H), 1,95(d,2H), 1,41(m,1H), 0,81-0,70(m,4H) |
| Ib-138 | 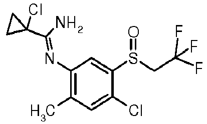 | 1H-NMR(D6-DMSO): 7,90(s,1H), 7,41(s,1H), 6,41(breit,2H), 4,10-3,97(m,2H), 2,04(s,3H), 1,64-1,58(m,2H), 1,32-1,29(m,2H) |
| Ib-139 | 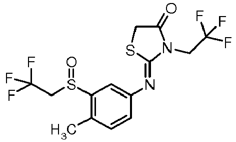 | 1H-NMR(D6-DMSO): 7,44(d,1H), 7,37(d,1H), 7,08(dd,1H), 4,58(q,2H), 4,23-4,11(m,3H), 4,00-3,88(m,1H), 2,35(s,3H) |
| Ib-140 | 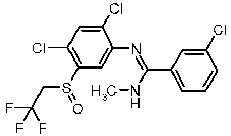 | 1H-NMR(D6-DMSO): 7,77(breit,1H), 7,64(s,1H), 7,43-7,41(m,1H), 7,34-7,30(m,2H), 7,09-7,07(m,1H), 6,97(s,1H), 4,08-3,96(m,1H), 3,81-3,70(m,1H), 2,90(d,3H) |
| Ib-141 | 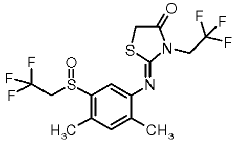 | 1H-NMR(D6-DMSO): 7,34(s,1H), 7,26(s,1H), 4,61(q,2H), 4,20(s,2H), 4,19-4,07(m,1H),3,91-3,79(m,1H), 2,31(s,3H), 2,14(s,3H) |
| Ib-142 | 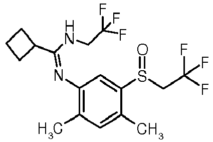 | 1H-NMR(D6-DMSO): 7,13(t,1H), 7,07(s,1H), 6,92(s,1H), 4,15-3,88(m,4H), 3,16-3,12(m,1H), 2,26(s,3H), 2,17-2,05(m,2H), 2,00(s,3H), 1,80-1,60(m,4H) |
| Ib-143 | 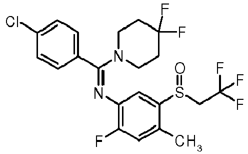 | 1H-NMR(D6-DMSO): 7,40-7,38(m,2H), 7,29(breit,2H), 7,10-7,08(m,1H), 6,98-6,95(m,1H), 3,94-3,82(m,1H), 3,61-3,48(m,1H), 3,31(breit,4H), 2,18(s,3H), 2,06(breit,4H) |
| Ib-144 | 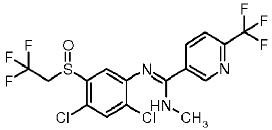 | 1H-NMR(D6-DMSO): 8,62(breit,1H), 7,99(breit,1H), 7,91-7,87(m,2H), 7,67(s,1H), 6,98(s,1H), 4,15-4,03(m,1H), 3,79-3,68(m,1H), 2,96-2,95(d,3H) |
| Ib-145 | 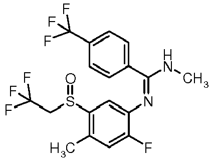 | 1H-NMR(D6-DMSO): 7,68(d,2H), 7,63(d,1H), 7,42(d,2H), 7,02-7,96(m,2H), 3,90-3,79(m,1H), 3,52-3,40(m,1H), 2,91(d,3H), 2,20(s,3H) |
| Ib-146 | 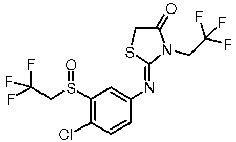 | 1H-NMR(D6-DMSO): 7,66(d,1H), 7,45(d,1H), 7,21(dd,1H), 4,59(q,2H), 4,30-4,21(m,3H), 4,14-4,04(m,1H) |
| Ib-147 | 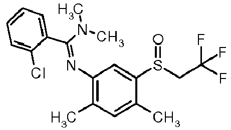 | 1H-NMR(D6-DMSO): 7,47-7,20(m,4H), 6,94(s,1H), 6,80-6,76(m,1H), 3,64-3,51(m,1H), 3,19(breit,3H), 3,06-2,97(m,1H), 2,74(breit,3H), 2,21(d,3H), 2,13(s,3H) |
| Ib-148 | 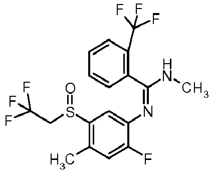 | 1H-NMR(D6-DMSO): 7,70-7,43(m,5H), 7,00-6,95(m,2H), 3,90-3,70(m,1H), 3,47-3,24(m,1H), 2,89(d,3H), 2,16(s,3H) |
| Ib-149 | 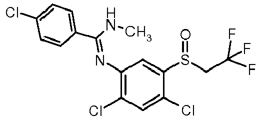 | 1H-NMR(D6-DMSO): 7,73-7,72(breit,1H), 7,63(s,1H), 7,40-7,38(m,2H), 7,22-7,20(m,2H), 6,89(s,1H), 4,05-3,96(m,1H), 3,73-3,66(m,1H), 2,91-2,90(d,3H) |
| Ib-150 | 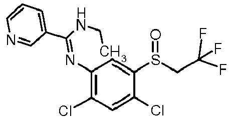 | 1H-NMR(D6-DMSO): 8,52-8,51(m,1H), 8,40(m,1H), 7,85(t,1H), 7,63(s,1H), 7,61-7,58(m,1H), 7,35-7,32(m,1H), 6,97(s,1H), 4,11-4,00(m,1H), 3,83-3,76(m,1H), 3,43(m,2H), 1,25(t,3H) |
| Ib-151 | 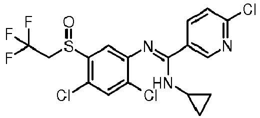 | 1H-NMR(D6-DMSO): 8,33(breit,1H), 7,98(breit,1H), 7,72(breit,1H), 7,66(s,1H), 7,50-7,48(m,1H), 7,13(breit,1H), 4,16-4,07(m,1H), 3,96-3,88(m,1H), 2,77(breit,1H),0,66-0,62(m,4H) |
| Ib-152 | 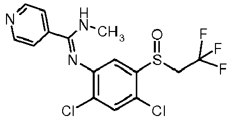 | 1H-NMR(D6-DMSO): 8,55-8,53(m,2H), 7,85-7,84(m,1H), 7,64(s,1H), 7,19-7,18(m,2H), 6,97(s,1H), 4,09-4,00(m,1H), 3,83-3,74(m,1H), 2,92-2,90(d,3H) |
| Ib-153 | 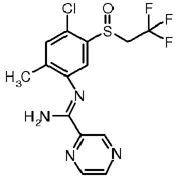 | 1H-NMR(D6-DMSO): 9,50(s,1H), 8,84-8,83(m,1H), 8,74-8,73(m,1H), 7,51(s,1H), 7,30(s,1H), 6,95(breit,2H), 4,16-4,02(m,2H), 2,18(s,3H) |
| Ib-154 | 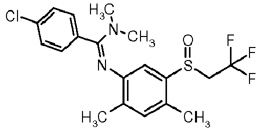 | 1H-NMR(D6-DMSO): 7,36-7,34(m,2H), 7,15(breit,2H), 6,93(s,1H), 6,60(s,1H), 3,67-3,60(m,1H), 3,21-3,14(m,1H), 2,94(breit,6H), 2,14(s,6H) |
| Ib-155 | 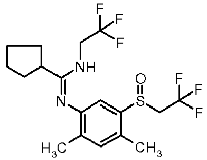 | 1H-NMR(D6-DMSO): 7,09(s,1H), 6,99(t,1H), 6,93(s,1H), 4,13-3,84(m,4H), 2,49(m,1H), 2,26(s,3H), 2,00(s,3H), 1,68-1,67(breit,6H), 1,38-1,37(breit,2H) |
| Ib-156 | 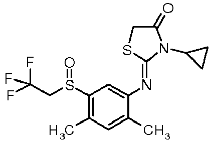 | 1H-NMR(D6-DMSO): 7,32(s,1H), 7,24(s,1H), 4,14-4,05(m,1H), 3,94(s,2H), 3,91-3,82(m,1H), 2,73(septet,1H), 2,31(s,3H), 2,15(s,3H), 1,02-0,90(m,4H) |
| Ib-157 | 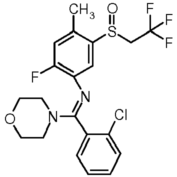 | 1H-NMR(D6-DMSO): 7,45-6,96(m,6H), 3,86-3,47(m,8H), 3,03(m,2H), 2,16(s,3H) |
| Ib-158 | 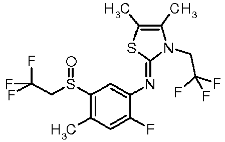 | 1H-NMR(D6-DMSO): 7,54(d,1H), 7,27(d,1H), 4,82(q,2H), 4,17-4,07(m,1H), 4,02-3,93(m,1H), 2,32(s,3H), 2,12(s, 3H), 2,05(s,3H) |
| Ib-159 | 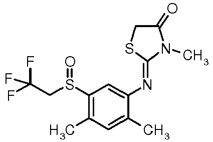 | 1H-NMR(D6-DMSO): 7,34(s,1H), 7,24(s,1H), 4,14-4,05(m,3H), 3,87-3,81(m,1H), 3,20(s,3H), 2,31(s,3H), 2,15(s,3H) |
| Ib-160 | 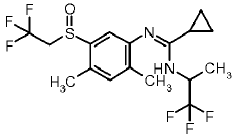 | 1H-NMR(D6-DMSO): 7,10-7,09(m,2H), 6,25-6,23(m,1H), 5,10-5,00(m,1H), 4,03-3,87(m,2H), 2,26(s,3H), 2,05(s,3H), 1,36-1,33(m,1H), 1,29-1,28(d,3H), 1,01-0,96(m,1H),0,82-0,76(m,1H), 0,68-0,61(m,2H) |
| Ib-161 | 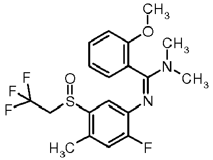 | logP(HCOOH)=1,28, logP(neutral)=2,95 |
| Ib-162 | 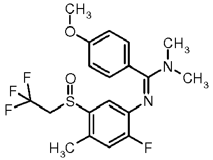 | 1H-NMR(D6-DMSO): 7,91-7,89(m,2H), 7,72-7,70(m,1H), 7,57-7,53(m,1H), 6,94-6,84(m,2H), 3,84-3,72(m,1H), 3,69(s,3H), 3,44-3,32(m,1H), 2,95(breit,6H), 2,17(s,3H) |
| Ib-163 | 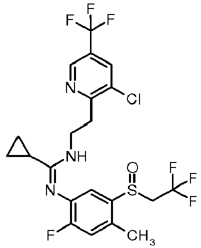 | 1H-NMR(D6-DMSO): 8,87(m,1H), 8,38(m,1H), 7,28-7,26(m,1H), 7,14-7,12(m,1H), 6,45(m,1H), 4,10-3,93(m,2H), 3,63-3,60(m,2H), 3,25(t,2H), 2,29(s,3H), 1,36(m,1H), 0,78-0,75(m,2H), 0,63-0,61(m,2H) |
| Ib-165 | 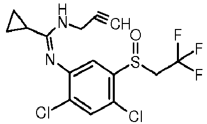 | 1H-NMR(D6-DMSO): 8,30(s,1H), 7,89-7,88(m,1H), 6,69(s,1H), 4,43-4,29(m,2H), 1,94-1,91(d,2H), 1,39(m,1H), 0,81-0,74(m,4H) |
| Ib-166 | 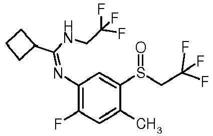 | 1H-NMR(D6-DMSO): 7,35(breit,1H), 7,17-7,13(m,2H), 4,12-3,98(m,4H), 3,21-3,16(m,1H), 2,30(s,3H), 2,16-2,09(m,2H), 1,84-1,61(m,4H) |
| Ib-167 | 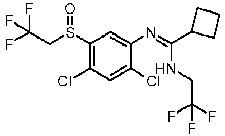 | 1H-NMR(D6-DMSO): 7,74(s,1H), 7,50(breit,1H), 7,12(s,1H), 4,24-4,07(m,4H), 3,23-3,14(m,1H), 2,18-1,59(m,6H) |
| Ib-168 | 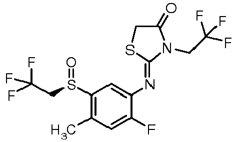 | 1H-NMR(D6-DMSO): 7,51(d,1H), 7,38(d,1H), 4,62-4,57(m,2H), 4,26-4,14(m,3H), 4,04-3,94(m,1H), 2,36(s,3H) |
| Ib-169 | 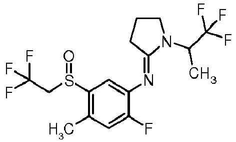 | 1H-NMR(D6-DMSO): 7,31-7,29(m,1H), 7,20-7,17(m,1H), 5,14-5,10(m,1H), 4,12-4,01(m,2H), 3,55-3,49(m,2H), 2,47-2,28(m,2H), 2,30(s,3H), 1,96-1,92(m,2H), 1,39-1,38(d,3H) |
| Ib-170 | 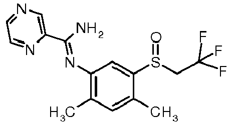 | 1H-NMR(D6-DMSO): 9,51(d,1H), 8,83-8,82(d,1H), 8,73(s,1H), 7,30(s,1H), 7,21(s,1H), 6,75(breit,2H), 4,06-3,97(m,2H), 2,32(s,3H), 2,15(s,3H) |
| Ib-171 | 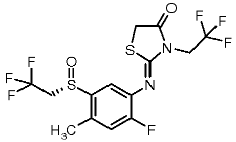 | 1H-NMR(D6-DMSO): 7,51(d,1H), 7,38(d,1H), 4,62-4,57(m,2H), 4,26-4,14(m,3H), 4,04-3,94(m,1H), 2,36(s,3H) |
| Ib-172 | 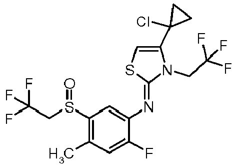 | 1H-NMR(D6-DMSO): 7,55(d,1H), 7,31(d,1H), 6,76(s,1H), 4,96(q,2H), 4,18-3,95(m,2H), 2,33(s,3H), 1,52-1,45(m,4H) |
| Ib-173 | 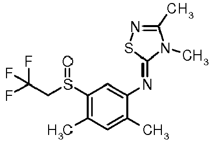 | 1H-NMR(D6-DMSO): 7,37(s,1H), 7,22(s,1H), 4,12-4,06(m,1H), 3,89-3,82(m,2H), 3,47(s,3H), 2,37(s,3H), 2,29(s,3H), 2,20(s,3H) |
| Ib-174 | 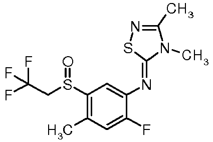 | 1H-NMR(D6-DMSO): 7,55(d,1H), 7,53(d,1H), 4,19-4,13(m,1H), 4,00-3,93(m,1H), 3,45(s,3H), 2,38(s,3H), 2,33(s,3H) |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-d₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Wenn auf Grund der Rundung des δ-Wertes auf zwei Nachkommastellen fallweise Signale mit gleichem δ-Wert auftreten, ergeben deren Intensitäten nach Addition dasselbe Bild, das auch in einem Ausdruck eines klassischen NMR's im Bereich dieses δ-Wertes beobachtet werden kann.

Gemäß den oben beschriebenen Herstellverfahren lassen sich die Verbindungen der Formel (II) erhalten, beispielsweise die folgenden Verbindungen der Formel (II):

### N-{2-Chlor-4-fluor-5-[(2,2,2-trifluorethyl)sulfanyl]phenyl}-2,2,2-trifluorethanimidoylchlorid (II-4)

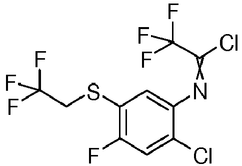

GC-MS: EI-Masse (m/z): 373 (2Cl) [M]⁺

Gemäß den oben beschriebenen Herstellverfahren lassen sich die Verbindungen der Formel (III) erhalten, beispielsweise die folgenden Verbindungen der Formel (III):

### 2,2,2-Trifluor-N-{4-fluor-2-methyl-5-[(2,2,2-trifluorethyl)sulfanyl]phenyl}acetamid (III-4)

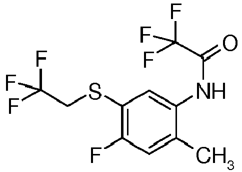

logP(HCOOH): 3,16; logP(neutral): 3,1; 1H-NMR (D6-DMSO, 400MHz) δ ppm 11,05(s,1H), 7,59(d,1H), 7,31(d,1H), 3,96(q,2H), 2,17(s,3H)

### N-{4-Brom-2-chlor-5-[(2,2,2-trifluorethyl)sulfanyl]phenyl}-2,2,2-trifluoracetamid (III-5)

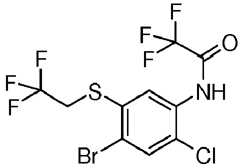

logP(HCOOH): 3,78; 1H-NMR (D6-DMSO, 400MHz) δ ppm 4,17-4,24(m,2H),7,79(s,1H),8,01(s,1H)11,45(s,1H)

### N-{2,4-Dibrom-5-[(2,2,2-trifluorethyl)sulfanyl]phenyl}-2,2,2-trifluoracetamid (III-6)

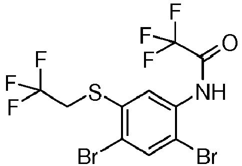

logP(HCOOH): 3,81; 1H-NMR (D6-DMSO, 400MHz) δ ppm 11,21(s,1H), 7,90(s,1H), 7,56(s,1H), 3,99(q,2H)

### N-{2-Chlor-4-fluor-5-[(2,2,2-trifluorethyl)sulfanyl]phenyl}-2,2,2-trifluoracetamid (III-7)

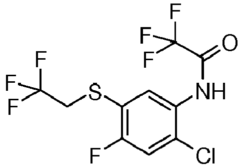

logP(HCOOH): 3,33; logP(neutral): 3,11; 1H-NMR (D6-DMSO, 400MHz) δ ppm 11,39(s,1H), 7,85(d,1H), 7,76(d,1H), 4,08(q,2H)

### N-{4-Chlor-2-fluor-5-[(2,2,2-trifluorethyl)sulfanyl]phenyl}-2,2,2-trifluoracetamid (III-8)

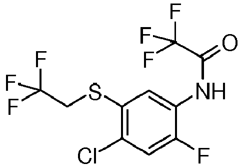

logP(HCOOH): 3,34; logP(neutral): 3,14; 1H-NMR (D6-DMSO, 400MHz) δ ppm 11,47(bs,1H), 7,85(d,1H), 7,76(d,1H), 4,09(q,2H)

### N-{4-Brom-2-chlor-5-[(2,2,2-trifluorethyl)sulfanyl]phenyl}-2,2,2-trifluoracetamid (III-9)

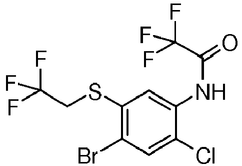

logP(HCOOH): 3,46; logP(neutral): 3,11; 1H-NMR (D6-DMSO, 400MHz) δ ppm 11,46(s,1H), 7,87(d,1H), 7,82(d,1H), 4,11(q,2H)

Gemäß den oben beschriebenen Herstellverfahren lassen sich die Verbindungen der Formel (IV) erhalten, beispielsweise die folgenden Verbindungen der Formel (IV):

### N,N'-[Disulfanediylbis(4-fluor-6-methylbenzol-3,1-diyl)]bis(2,2,2-trifluoracetamid) (IV-2)

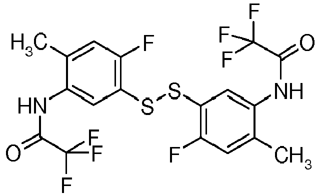

logP(HCOOH): 4,0; logP(neutral): 3,92; 1H-NMR (D6-DMSO, 400MHz) δ ppm 11,04(s,2H), 7,62(d,2H), 7,32(d,2H), 2,19(s,6H)

### N,N'-[Disulfanediylbis(4-brom-6-chlorbenzol-3,1-diyl)]bis(2,2,2-trifluoracetamid) (IV-3)

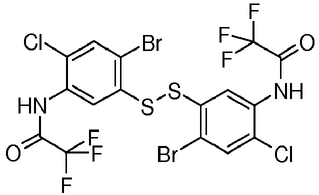

logP(HCOOH): 5,42

### N,N'-[Disulfanediylbis(4,6-dibrombenzol-3,1-diyl)]bis(2,2,2-trifluoracetamid) (IV-4)

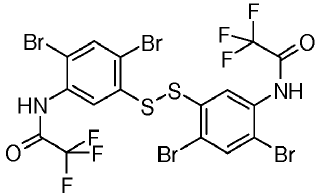

logP(HCOOH): 5,62; logP(neutral): 4,49; 1H-NMR (D6-DMSO, 400MHz) δ ppm 11,38(s,2H), 8,18(s,2H), 7,77(d,2H)

### N,N'-[Disulfanediylbis(6-chlor-4-fluorbenzol-3,1-diyl)]bis(2,2,2-trifluoracetamid) (IV-5)

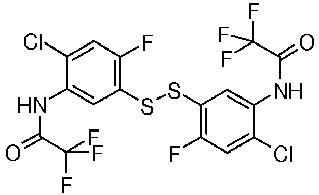

logP(HCOOH): 4,45; logP(neutral): 3,81; 1H-NMR (D6-DMSO, 400MHz) δ ppm 11,37(s,2H), 7,88(d,2H), 7,80(d,2H)

### N,N'-[Disulfanediylbis(4-chlor-6-fluorbenzol-3,1-diyl)]bis(2,2,2-trifluoracetamid) (IV-6)

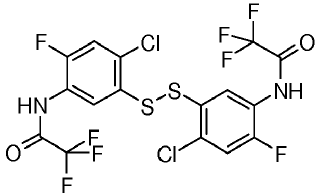

logP(HCOOH): 4,60; logP(neutral): 3,82; 1H-NMR (D6-DMSO, 400MHz) δ ppm 11,44(s,2H), 7,95(d,2H), 7,83(d,2H)

### N,N'-[Disulfanediylbis(4-brom-6-fluorbenzol-3,1-diyl)]bis(2,2,2-trifluoracetamid) (IV-7)

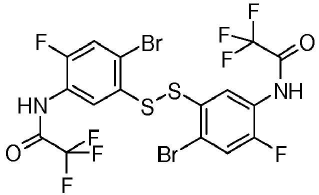

logP(HCOOH): 4,76; logP(neutral): 4,02; 1H-NMR (D6-DMSO, 400MHz) δ ppm 11,44(s,2H), 7,95-7,89 (m,4H)

Gemäß den oben beschriebenen Herstellverfahren lassen sich die Verbindungen der Formel (V) erhalten, beispielsweise die folgenden Verbindungen der Formel (V):

### 2-Fluor-4-methyl-5-[(trifluoracetyl)amino]benzolsulfonylchlorid (V-2)

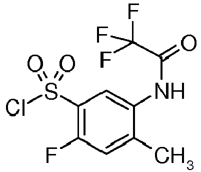

1H-NMR (D6-DMSO, 400MHz) δ ppm 10,98 (s,1H), 7,48 (d,1H), 7,12 (d,1H), 2,15 (s,3H)

### 2,4-Dibrom-5-[(trifluoracetyl)amino]benzolsulfonylchlorid (V-3)

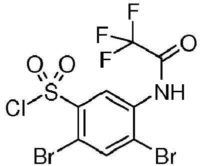

1H-NMR (D6-DMSO, 400MHz) δ ppm 9,20(bs,1H), 8,56(s,1H), 8,36(s,1H)

### 4-Chlor-2-fluor-5-[(trifluoracetyl)amino]benzolsulfonylchlorid (V-4)

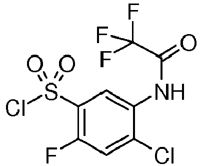

1H-NMR (D6-DMSO, 400MHz) δ ppm 11,30(s,1H), 7,72-7,67(m,1H), 7,59-7,55(d,1H)

### 2-Brom-4-fluor-5-[(trifluoracetyl)amino]benzolsulfonylchlorid (V-5)

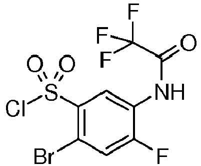

1H-NMR (D6-DMSO, 400MHz) δ ppm 9,38(bs,1H), 8,00-7,97(m,1H), 7,68(d,1H)

Gemäß den oben beschriebenen Herstellverfahren lassen sich die Verbindungen der Formel (VI) erhalten, beispielsweise die folgenden Verbindungen der Formel (VI):

### 2,2,2-Trifluor-N-(4-fluor-2-methylphenyl)acetamid (VI-2)

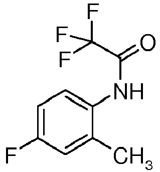

logP(HCOOH): 2,2; logP(neutral): 2,19; 1H-NMR (D6-DMSO, 400MHz) δ ppm 10,97(s,1H), 7,28-7,30(m,1H), 7,19-7,21(m,1H), 7,08-7,11(m,1H), 2,18 (s,3H)

### N-(4-Brom-2-chlorphenyl)-2,2,2-trifluoracetamid (VI-3)

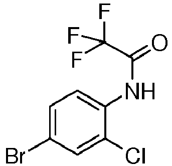

logP(HCOOH): 3,01; logP(neutral): 2,81; 1H-NMR (D6-DMSO, 400MHz) δ ppm 11,35 (s,1H), 7,92 (d, 1H), 7,65 (dd,1H), 7,45 (d,1H)

### N-(2,4-Dibromphenyl)-2,2,2-trifluoracetamid (VI-4)

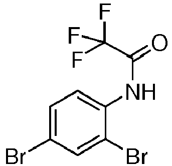

logP(HCOOH): 3,10; logP(neutral): 2,89; 1H-NMR (D6-DMSO, 400MHz) δ ppm 11,36(s,1H), 8,04(d,1H), 7,69(dd,1H), 7,43(d,1H)

### N-(2-Chlor-4-fluorphenyl)-2,2,2-trifluoracetamid (VI-5)

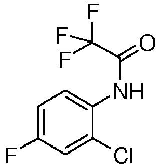

logP(HCOOH): 2,46; logP(neutral): 2,31; 1H-NMR (D6-DMSO, 400MHz) δ ppm 11.29(s,1H), 7,64(dd,1H), 7,53(dd,1H), 7,35-7,30(m,1H)

### N-(4-Chlor-2-fluorphenyl)-2,2,2-trifluoracetamid (VI-6)

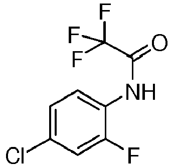

logP(HCOOH): 2,53; logP(neutral): 2,40; 1H-NMR (D6-DMSO, 400MHz) δ ppm 11.29(s,1H), 7,62(dd,1H), 7,55(dd,1H), 7,37(dd,1H)

### N-(4-Brom-2-fluorphenyl)-2,2,2-trifluoracetamid (VI-7)

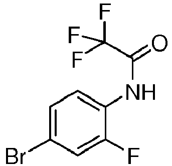

logP(HCOOH): 2,73; logP(neutral): 2,51; 1H-NMR (D6-DMSO, 400MHz) δ ppm 11,36(s,1H), 7,74(d,1H), 7,52-7,46(m,2H)

Gemäß den oben beschriebenen Herstellverfahren lassen sich die Verbindungen der Formel (X) erhalten, beispielsweise die folgenden Verbindungen der Formel (X):

### 4-Chlor-2-fluor-5-[(2,2,2-trifluorethyl)sulfanyl]anilin (X-6)

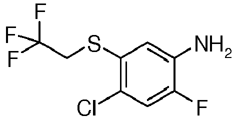

11,0 g (30,9 mmol) *N*-{4-Chlor-2-fluor-5-[(2,2,2-trifluorethyl)sulfanyl]phenyl}-2,2,2-trifluoracetamid in 150 ml Dioxan werden vorsichtig zu einer Lösung aus 10,3 ml (186 mmol) Schwefelsäure (96%ig) in 100 ml Wasser zu gegeben. Anschließend wird das Reaktionsgemisch über Nacht refluxiert. Nach Abkühlen wird die Lösung mit einer gesättigten Natriumhydrogencarbonatlösung und etwas Natriumcarbonat auf pH 7 gebracht und dreimal mit Essigester extrahiert. Die vereinten organischen Phasen werden mit einer gesättigten Natriumchloridlösung gewaschen, über Natriumsulfat getrocknet, filtriert und eingeengt. Der Rückstand enthält 8,27 g (96% Reinheit, 99% d.Th.) der Titelverbindung als schwarzes Öl/Feststoff-Gemisch.

logP(HCOOH): 3,02; logP(neutral): 3,00; 1H-NMR (D6-DMSO, 400MHz) δ ppm 7,27(d,1H), 7,04(d,1H), 5,46(bs,2H), 3,85(q,2H)

### 2-Chlor-5-[(2,2,2-trifluorethyl)sulfanyl]anilin (X-7)

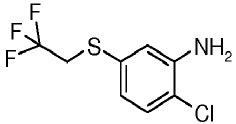

logP(HCOOH): 3,00; logP(neutral): 2,95; 1H-NMR (D6-DMSO, 400MHz) δ ppm 7,16(d,1H), 6,89(d,1H), 6,68-6,65(m,1H), 5,48(breit,2H), 3,89(q,2H) ; GC-MS: EI-Masse (m/z): 241 (1Cl) [M]+

### 2-Fluor-5-[(2,2,2-trifluorethyl)sulfanyl]anilin (X-8)

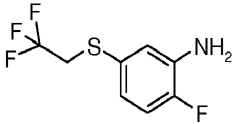

logP(HCOOH): 2,57; logP(neutral): 2,53; 1H-NMR (D6-DMSO, 400MHz) δ ppm 7,01-6.97(m,1H), 6,92-6,89(m,1H), 6,69-6,64(m,1H), 5,31(breit,2H), 3,82(q,2H) ; GC-MS: EI-Masse (m/z): 225 [M]+

Gemäß den oben beschriebenen Herstellverfahren lassen sich die Verbindungen der Formel (XXI) erhalten, beispielsweise die folgenden Verbindungen der Formel (XXI):

### 1,1'-Disulfanediylbis(4-chlor-3-nitrobenzol) (XXI-2)

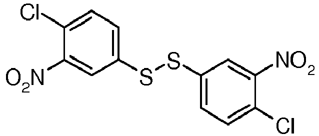

logP(HCOOH): 4,58; logP(neutral): 4,58; 1H-NMR (D6-DMSO, 400MHz) δ ppm 8,29(d,2H), 7,89-7,86(m,2H), 7,81(d,2H); GC-MS: EI-Masse (m/z): 376 (2Cl) [M]+

### 1,1'-Disulfanediylbis(4-fluor-3-nitrobenzol) (XXI-3)

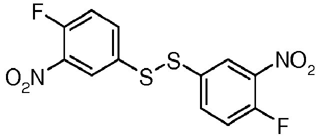

logP(HCOOH): 3,83; logP(neutral): 3,79; 1H-NMR (D6-DMSO, 400MHz) δ ppm 8,32-8,29(m,2H), 8,03-7,97(m,2H), 7,69-7,63(m,2H); GC-MS: EI-Masse (m/z): 344 [M]+

### 1,1'-Disulfanediylbis(2,4-dichlor-5-nitrobenzol) (XXI-4)

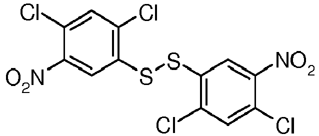

logP(HCOOH): 5,69; logP(neutral): 5,64; 1H-NMR (D6-DMSO, 400MHz) δ ppm 8,33(s,2H), 8,21(s,2H);

Gemäß den oben beschriebenen Herstellverfahren lassen sich die Verbindungen der Formel (XXII) erhalten, beispielsweise die folgenden Verbindungen der Formel (XXII):

### 3,3'-Disulfanediylbis(6-chloranilin) (XXII-2)

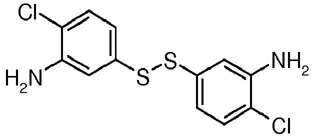

logP(HCOOH): 3,84; logP(neutral): 3,83; 1H-NMR (D6-DMSO, 400MHz) δ ppm 7,18(d,2H), 6,94(d,2H), 6,65-6,62(m,2H), 5,59(breit,4H); GC-MS: EI-Masse (m/z): 316 (2Cl) [M]+

### 3,3'-Disulfanediylbis(6-fluoranilin) (XXII-3)

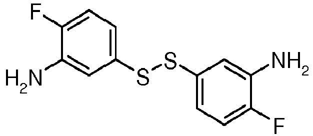

logP(HCOOH): 2,98; logP(neutral): 2,97; 1H-NMR (D6-DMSO, 400MHz) δ ppm 7,02-6,95(m,2H), 6,95-6,82(m,2H), 6,62-6,57(m,2H), 5,40(breit,4H); GC-MS: EI-Masse (m/z): 284 [M]+

### 3,3'-Disulfanediylbis(4,6-dichloranilin) (XXII-4)

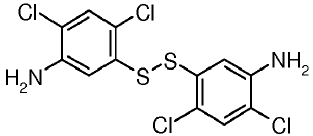

logP(HCOOH): 5,14; logP(neutral): 4,95; 1H-NMR (D6-DMSO, 400MHz) δ ppm 7,41(s,2H), 6,95(s,2H), 5,78(breit,4H); GC-MS: EI-Masse (m/z): 386 (4Cl) [M]+

Gemäß den oben beschriebenen Herstellverfahren lassen sich die Verbindungen der Formel (XXIV) erhalten, beispielsweise die folgenden Verbindungen der Formel (XXIV):

### 1-Chlor-2-fluorcyclopropancarboxamid (XXIV-1)

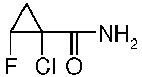

1H-NMR(D6-DMSO) δ ppm : 7,84(breit,1H), 7,75(breit,1H), 5,05-4,86(m,1H), 1,89-1,81(m,1H), 1,69-1,60(m,1H)

### N-(2,2,2-Trifluorethyl)cyclopentancarboxamid (XXIV-2)

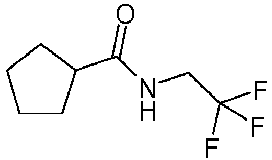

1H-NMR(D6-DMSO) δ ppm: 8,43(breit,1H), 3,92-3,83(m,2H), 3,08-3,07(m,1H), 2,67-2,59(m,1H), 1,79-1,49(m,7H)

### N-(2,2-Difluorethyl)cyclopentancarboxamid (XXIV-3)

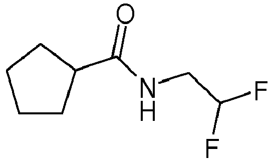

1H-NMR(D6-DMSO) δ ppm: 8,18(t,1H), 5,97(tt,1H), 3,50-3,40(m,2H), 3,08-3,06(m,1H), 2,64-2,57(m,1H), 1,78-1,45(m,7H)

### N-(1,1,1-Trifluorpropan-2-yl)cyclopropancarboxamid (XXIV-4)

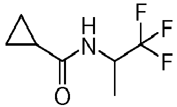

1H-NMR(D6-DMSO) δ ppm: 8,62-8,60(d,1H), 4,62-4,55(m,1H), 1,63-1,59(m,1H), 1,24-1,23(d,3H), 0,72-0,70(m,4H)

### (3-Pyridyl)(4,4-difluorpiperidin-1-yl)methanon (XXIV-5)

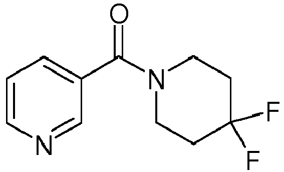

1H-NMR(D6-DMSO) δ ppm: 8,67-8,66(m,2H), 7,90-7,87(m,1H), 7,51-7,48(m,1H), 3,73(m,2H), 3,42 (m,2H), 2,06(m,4H)

### (2-Chlorphenyl)(4,4-difluorpiperidin-1-yl)methanon (XXIV-6)

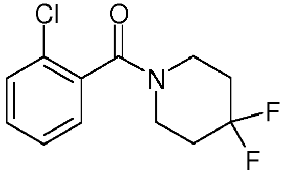

1H-NMR(D6-DMSO) δ ppm: 7,56-7,53(m,1H), 7,49-7,41(m,3H), 3,90-3,84(m,1H), 3,70-3,64(m,1H), 3,34-3,22(m,2H), 2,15-1,90(m,4H)

### Anwendungsbeispiele

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100 % bei einer Aufwandmenge von 500 g/ha: Ia-02, Ia-04, Ia-05, Ia-13, Ia-14, Ia-15, Ia-18, Ia-19, Ib-02, Ib-10, Ib-11, Ib-14, Ib-16

### Tetranychus - test, OP-resistent (TETRUR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 80 % bei einer Aufwandmenge von 500g/ha: Ia-09, Ia-17, Ib-04, Ib-05

Bei diesem Test zeigen z. B. die folgenden Verbindungen der Herstellungsbeispiele eine Wirkung von 100 % bei einer Aufwandmenge von 500g/ha: Ia-02, Ia-03, Ia-05, Ia-07, Ia-12, Ia-13, Ia-15, Ia-18, Ia-23, Ia-26, Ib-02, Ib-03, Ib-06, Ib-07, Ib-08, Ib-09, Ib-12, Ib-13, Ib-15

### Meloidogyne incognita- Test (MELGIN)

| | | |
|---|---|---|
| Lösungsmittel: | 80,0 | Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit Sand, Wirkstofflösung, Meloidogyne incognita Ei- Larven- Suspension und Salatsamen gefüllt. Die Salatsamen keimen und die Pflänzchen entwickeln sich. An den Wurzeln entwickeln sich die Gallen.

Nach 14 Tagen wird die nematizide Wirkung an hand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigt z. B. die folgende Verbindung der Herstellungsbeispiele eine Wirkung von 90 % bei einer Aufwandmenge von 20 ppm : Ia-07

Bei diesem Test zeigt z. B. die folgende Verbindung der Herstellungsbeispiele eine Wirkung von 100 % bei einer Aufwandmenge von 20 ppm : Ib-02

### Lucilia cuprina (48h)

| | |
|---|---|
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration. Etwa 20 Lucilia cuprina-Larven werden in ein Teströhrchen gebracht, welches ca. 1 cm³ Pferdefleisch und 0.5 ml der zu testende Wirkstoffzubereitung enthält. Nach 48 Stunden wird die Wirksamkeit der Wirkstoffzubereitung als % Larvenmortalität ermittelt.

Bei diesem Test zeigen z.B. die folgenden Verbindungen der Herstellungsbeispiele eine Wirkung von 100% bei einer Aufwandmenge von 100 ppm: Ia-21, Ib-11

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigt z.B. die folgende Verbindung der Herstellungsbeispiele eine Wirkung von 80 % bei einer Aufwandmenge von 20µg / Tier : Ia-04

Bei diesem Test zeigen z.B. die folgenden Verbindungen der Herstellungsbeispiele eine Wirkung von 100 % bei einer Aufwandmenge von 20µg / Tier : Ia-01, Ia-21, Ia-22, Ib-01, Ib-10, Ib-11, Ib-14, Ib-16

Wenn nicht anders erwähnt, wurde die Testlösung in den folgenden Beispielen wie folgt vorbereitet:
Man mischt 1 Gewichtsteil Wirkstoff mit den angegebenen Mengen eines Lösungsmittel und Emulgator - Gemisches (3 Gewichtsteile Dimethylformamide als Lösungsmittel und 1 Gewichtsteil Polyoxyethylene alkyl phenyl ether als Emulgator) und verdünnt die Lösung mit Wasser auf die gewünschte Konzentation

### Test gegen Spinnmilbe (Tetranychus urticae)

50 bis 100 erwachsene Spinnmilben werden auf die Blätter einer Kidney-Bohnenpflanze im Zweiblattstadium gesetzt, die in einem Topfen mit 6cm Durchmesser wächst.

Nach einem Tag wird die Pflanze mit Hilfe einer Farbspritzpistole mit der Wirkstoffzubereitung der gewünschten Konzentration in ausreichender Menge gespritzt und in einem Gewächshaus aufgestellt. Nach 7 Tagen wird die akarizide Wirkung ermittelt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100 % bei einer Wirkstoffkonzentation von 100ppm:
Ia-21, Ia-01, Ib-11, Ib-01, Ia-06, Ia-14, Ia-15, Ib-14, Ia-20, Ia-25, Ia-19, Ib-16 und Ib-10

### Test gegen den Kürbis-Blattkäfer (Aulacophora femoralis)

Gurkenblätter werden in die Testlösung mit der entsprechenden Konzentration getaucht und dann an der Luft getrocknet. Danach werden die Blätter in eine Plastikschale mit steriler Erde und fünf, im zweiten Larvenstadium befindlichen, Aulacophora femoralis Larven gesteckt Die Schalen werden in einem klimatisierten Raum bei 25°C aufgestellt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von 100 % bei einer Wirkstoffkonzentation von 500ppm:
Ia-21, Ib-11, Ib-01, Ib-14, Ib-16 und Ib-10

### Boophilus microplus - Diptest (BOOPMI Dip)

| | |
|---|---|
| Testtiere: | Rinderzecken (*Boophilus microplus)* Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken *(Boophilus microplus)* werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: Ia-189, Ib-115, Ib-33

### Boophilus microplus - Injektionstest (BOOPMI Inj)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: Ia-01, Ia-115, Ia-116, Ia-118, Ia-120, Ia-13, Ia-189, Ia-206, Ia-21, Ia-22, Ia-27, Ia-34, Ia-39, Ia-44, Ia-50, Ia-66, Ia-80, Ib-01, Ib-02, Ib-03, Ib-07, Ib-10, Ib-108, Ib-11, Ib-115, Ib-12, Ib-14, Ib-14, Ib-16, Ib-17, Ib-18, Ib-19, Ib-20, Ib-33, Ib-35, Ib-38, Ib-40, Ib-47, Ib-48, Ib-58, Ib-59, Ib-68, Ib-85

Bei diesem Test zeigen z.B. die folgenden Verbindungen der Herstellungsbeispiele eine Wirkung von 90% bei einer Aufwandmenge von 20µg/Tier: Ib-44

Bei diesem Test zeigen z.B. die folgenden Verbindungen der Herstellungsbeispiele eine Wirkung von 80% bei einer Aufwandmenge von 20µg/Tier: Ia-04, Ib-24, Ib-89

### Cooperia curticei - Test (COOPCU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit "Ringerlösung" auf die gewünschte Konzentration.

Gefäße mit der Wirkstoffzubereitung der gewünschten Konzentration werden mit ca. 40 Nematodenlarven *(Cooperia curticei)* besetzt.

Nach 5 Tagen wird die Abtötung in % bestimmt. Dabei bedeutet 100 %, dass alle Larven abgetötet wurden; 0 % bedeutet, dass keine der Larven abgetötet wurde.

Bei diesem Test zeigen z.B. die folgenden Verbindungen der Herstellungsbeispiele eine Wirkung von 100% bei einer Aufwandmenge von 100ppm: Ia-220

### Lucilia cuprina - Test (LUCICU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: Ia-21, Ib-11

Bei diesem Test zeigen z.B. die folgenden Verbindungen der Herstellungsbeispiele eine Wirkung von 90% bei einer Aufwandmenge von 100ppm: Ia-13

Bei diesem Test zeigen z.B. die folgenden Verbindungen der Herstellungsbeispiele eine Wirkung von 80% bei einer Aufwandmenge von 100ppm: Ib-02

### Myzus persicae - Sprühtest (MYZUPE)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha: Ia-206, Ia-228, Ib-44

Bei diesem Test zeigen z. B. die folgenden Verbindungen der Herstellungsbeispiele Wirkung von 90% bei einer Aufwandmenge von 500g/ha: Ia-34, Ia-116, Ib-123

Bei diesem Test zeigen z. B. die folgenden Verbindungen der Herstellungsbeispiele Wirkung von 80% bei einer Aufwandmenge von 100g/ha: Ib-42

### Phaedon cochleariae - Sprühtest (PHAECO)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: Ia-02, Ia-04, Ia-05, Ia-13, Ia-14, Ia-15, Ia-18, Ia-19, Ia-27, Ia-28, Ia-29, Ia-31, Ia-35, Ia-36, Ia-38, Ia-45, Ia-78, Ia-79, Ia-115, Ia-118, Ia-121, Ia-135, Ia-139, Ia-149, Ia-162, Ia-191, Ia-202, Ia-209, Ia-229, Ib-02, Ib-10, Ib-11, Ib-14, Ib-16, Ib-17, Ib-20, Ib-20, Ib-27, Ib-39, Ib-43, Ib-44, Ib-56, Ib-57, Ib-68, Ib-78, Ib-83, Ib-101, Ib-110, Ib-111, Ib-115

Bei diesem Test zeigen z. B. die folgenden Verbindungen der Herstellungsbeispiele Wirkung von 83% bei einer Aufwandmenge von 500g/ha: Ia-33, Ia-92, Ia-130, Ia-134, Ia-171, Ia-204, Ia-207, Ia-210, Ib-18, Ib-30, Ib-108, Ib-137, Ib-143

### Tetranychus urticae - Sprühtest, OP-resistent (TETRUR)

| | | |
|---|---|---|
| Lösungsmittel: | 78,0 | GewichtsteileAceton |
| | 1,5 | Gewichtsteile Dimethylformamid |
| | | |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: Ia-01, Ia-02, Ia-03, Ia-04, Ia-05, Ia-06, Ia-07, Ia-08, Ia-11, Ia-12, Ia-13, Ia-14, Ia-15, Ia-16, Ia-18, Ia-19, Ia-20, Ia-21, Ia-22, Ia-23, Ia-25, Ia-26, Ia-27, Ia-28, Ia-29, Ia-30, Ia-31, Ia-32, Ia-33, Ia-34, Ia-35, Ia-36, Ia-37, Ia-38, Ia-39, Ia-40, Ia-41, Ia-42, Ia-43, Ia-44, Ia-46, Ia-47, Ia-48, Ia-49, Ia-50, Ia-51, Ia-52, Ia-53, Ia-54, Ia-55, Ia-56, Ia-57, Ia-60, Ia-61, Ia-64, Ia-65, Ia-66, Ia-67, Ia-69, Ia-70, Ia-71, Ia-72, Ia-73, Ia-74, Ia-75, Ia-76, Ia-77, Ia-78, Ia-79, Ia-80, Ia-81, Ia-82, Ia-83, Ia-84, Ia-85, Ia-86, Ia-87, Ia-89, Ia-93, Ia-94, Ia-95, Ia-96, Ia-97, Ia-98, Ia-99, Ia-100, Ia-115, Ia-116, Ia-119, Ia-120, Ia-121, Ia-122, Ia-123, Ia-124, Ia-125, Ia-126, Ia-127, Ia-129, Ia-130, Ia-131, Ia-132, Ia-133, Ia-134, Ia-135, Ia-136, Ia-138, Ia-139, Ia-140, Ia-141, Ia-141, Ia-143, Ia-144, Ia-145, Ia-146, Ia-147, Ia-148, Ia-150, Ia-151, Ia-152, Ia-153, Ia-156, Ia-158, Ia-161, Ia-162, Ia-163, Ia-165, Ia-166, Ia-187, Ia-188, Ia-189, Ia-191, Ia-193, Ia-194, Ia-195, Ia-197, Ia-201, Ia-202, Ia-203, Ia-205, Ia-206, Ia-207, Ia-208, Ia-209, Ia-210, Ia-211, Ia-213, Ia-214, Ia-217, Ia-221, Ia-222, Ia-224, Ia-225, Ia-226, Ia-227, Ia-230, Ia-233, Ia-234, Ia-236, Ia-238, Ia-239, Ia-242, Ia-247, Ia-249, Ia-272, Ia-275, Ia-276, Ib-02, Ib-03, Ib-06, Ib-07, Ib-08, Ib-09, Ib-10, Ib-11, Ib-12, Ib-13, Ib-14, Ib-15, Ib-16, Ib-17, Ib-19, Ib-20, Ib-21, Ib-22, Ib-24, Ib-25, Ib-26, Ib-27, Ib-28, Ib-29, Ib-30, Ib-31, Ib-32, Ib-33, Ib-34, Ib-35, Ib-36, Ib-37, Ib-38, Ib-39, Ib-40, Ib-42, Ib-43, Ib-45, Ib-46, Ib-47, Ib-48, Ib-49, Ib-50, Ib-51, Ib-52, Ib-53, Ib-56, Ib-57, Ib-58, Ib-59, Ib-61, Ib-62, Ib-63, Ib-64, Ib-65, Ib-68, Ib-69, Ib-71, Ib-72, Ib-75, Ib-76, Ib-78, Ib-79, Ib-80, Ib-81, Ib-82, Ib-83, Ib-84, Ib-85, Ib-86, Ib-87, Ib-88, Ib-89, Ib-90, Ib-92, Ib-93, Ib-94, Ib-95, Ib-97, Ib-98, Ib-99, Ib-100, Ib-108, Ib-109, Ib-110, Ib-112, Ib-113, Ib-114, Ib-115, Ib-117, Ib-118, Ib-119, Ib-120, Ib-121, Ib-123, Ib-125, Ib-126, Ib-127, Ib-129, Ib-130, Ib-131, Ib-132, Ib-135, Ib-137, Ib-138, Ib-139, Ib-140, Ib-141, Ib-148, Ib-151, Ib-160, Ib-169, Ib-174

Bei diesem Test zeigen z. B. die folgenden Verbindungen der Herstellungsbeispiele eine Wirkung von 90% bei einer Aufwandmenge von 500g/ha: Ia-59, Ia-62, Ia-63, Ia-68, Ia-83, Ia-88, Ia-91, Ia-92, Ia-101, Ia-102, Ia-103, Ia-104, Ia-105, Ia-106, Ia-107, Ia-117, Ia-118, Ia-128, Ia-149, Ia-155, Ia-159, Ia-160, Ia-167, Ia-168, Ia-169, Ia-171, Ia-172, Ia-173, Ia-175, Ia-176, Ia-177, Ia-178, Ia-180, Ia-190, Ia-192, Ia-196, Ia-204, Ia-205, Ia-212, Ia-215, Ia-216, Ia-218, Ia-219, Ia-220, Ia-223, Ia-228, Ia-229, Ia-231, Ia-232, Ia-235, Ia-237, Ia-240, Ia-241, Ia-243, Ia-244, Ia-245, Ia-248, Ia-250, Ia-251, Ia-252, Ia-253, Ia-254, Ia-255, Ia-256, Ia-257, Ia-258, Ia-259, Ia-260, Ia-261, Ia-262, Ia-263, Ia-264, Ia-265, Ia-266, Ia-267, Ia-268, Ia-269, Ia-270, Ia-278, Ib-01, Ib-23, Ib-41, Ib-54, Ib-60, Ib-66, Ib-67, Ib-73, Ib-74, Ib-77, Ib-91, Ib-96, Ib-101, Ib-102, Ib-103, Ib-104, Ib-111, Ib-116, Ib-122, Ib-124, Ib-128, Ib-133, Ib-134, Ib-136, Ib-142, Ib-143, Ib-144, Ib-145, Ib-146, Ib-147, Ib-149, Ib-150, Ib-152, Ib-153, Ib-154, Ib-155, Ib-156, Ib-157, Ib-158, Ib-166, Ib-167, Ib-172

Bei diesem Test zeigen z. B. die folgenden Verbindungen der Herstellungsbeispiele eine Wirkung von 80% bei einer Aufwandmenge von 500g/ha: Ia-09, Ia-17, Ia-45, Ia-90, Ia-108, Ia-109, Ib-04, Ib-05, Ib-18

Bei diesem Test zeigen z. B. die folgenden Verbindungen der Herstellungsbeispiele eine Wirkung von 100% bei einer Aufwandmenge von 100g/ha: Ia-58

Bei diesem Test zeigen z. B. die folgenden Verbindungen der Herstellungsbeispiele eine Wirkung von 90% bei einer Aufwandmenge von 100g/ha: Ia-181

### Tetranychus urticae - Sprühtest; OP-resistent (TETRUR)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen oder/und Penetrationsförderern werden diese jeweils in einer Konzentration von 1000 ppm der Präparatelösung zugefügt.

Bohnenpflanzen (*Phaseolus vulgaris*), die stark von allen Stadien der Gemeinen Spinnmilbe (*Tetranychus urticae*) befallen sind, werden durch Spritzen mit der Wirkstoffzubereitung in der gewünschten Konzentration behandelt.

Nach 7 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele Wirkung von 90%bei einer Aufwandmenge von 100ppm: Ib-44

Bei diesem Test zeigen z. B. die folgenden Verbindungen der Herstellungsbeispiele Wirkung von 100%bei einer Aufwandmenge von 20ppm: Ib-168

Bei diesem Test zeigen z. B. die folgenden Verbindungen der Herstellungsbeispiele Wirkung von 80%bei einer Aufwandmenge von 20ppm: Ib-159

## Patentansprüche

1. N-Arylamidine-substituierte Trifluoroethylsulfid-Derivate der Formel (I) 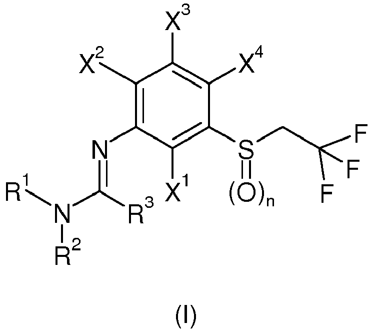 in welcher
n für die Zahl 0, 1 oder 2 steht,
X¹, X², X³, X⁴ unabhängig von einander für Wasserstoff, Halogen, Hydroxy, Amino, OCN, SCN, SF₅, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₄)-alkoxy, (C₁-C₆)Alkoxycarbonyl-(C₁-C₄)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₇)Alkoxycarbonyl-(C₁-C₆)alkoxy, (C₁-C₆)Alkoxy-(C₁-C₆)alkoxy, (C₁-C₇)Alkylhydroxyimino, (C₁-C₇)Alkoxyimino, (C₁-C₆)Alkyl-(C₁-C₇)alkoxyimino, (C₁-C₆)Halogenalkyl-(C₁-C₇)alkoxyimino, (C₁-C₆)Alkylthio, (C₁-C₆)Halogenalkylthio, (C₁-C₆)Alkoxy-(C₁-C₆)alkylthio, (C₁-C₆)Alkylthio-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₇)Alkylcarbonyl, (C₁-C₇)Halogenalkylcarbonyl, Carboxyl, (C₁-C₇)Alkylcarbonyloxy, (C₁-C₇)Alkoxycarbonyl, (C₁-C₇)Halogenalkoxycarbonyl, (C₁-C₇)Alkoxycarbonyl-(C₁-C₆)alkyl, Aminocarbonyl, (C₁-C₇)Alkylaminocarbonyl, Di-(C₁-C₇)alkyl-aminocarbonyl, (C₁-C₇)Alkenylaminocarbonyl, Di-(C₁-C₇)-alkenylaminocarbonyl, (C₃-C₈)Cycloalkylaminocarbonyl, (C₁-C₆)Alkylsulfonylamino, Aminosulfonyl, (C₁-C₆)Alkylaminosulfonyl, Di-(C₁-C₆)alkyl-aminosulfonyl, (C₁-C₆)Alkylsulfoximino, Aminothiocarbonyl, (C₁-C₆)Alkylaminothiocarbonyl, Di-(C₁-C₆)alkyl-aminothiocarbonyl,
oder für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkyl-aminosulfonyl substituiertes gegebenenfalls durch ein oder zwei Heteroatome aus der Reihe O, S oder N unterbrochenes, gegebenenfalls gesättigtes oder ungesättigtes (C₃-C₈)Cycloalkyl, (C₃-C₈)Cycloalkoxy, (C₃-C₈)Cycloalkyl(C₁-C₆)alkyl oder (C₃-C₈)Cycloalkyl-(C₁-C₆)alkoxy substituiertes Phenyl-(C₁-C₆)alkyl, Phenoxy, Phenyl-(C₁-C₄)alkyloxy, Phenoxy-(Ci-C₄)alkyl, Phenylthio, Phenylthio-(C₁-C₄)alkyl, Phenylsulfinyl, Phenylsulfonyl, Hetaryl-(Ci-C₆)alkyl, Hetaryloxy, Hetaryl-(C₁-C₄)alkyloxy, Hetarylthio, Hetarylsulfinyl, Hetarylsulfonyl, gegebenenfalls gesättigtes oder ungesättigtes (C₃-C₈)Cycloalkyl-(C₁-C₆)alkyl, (C₃-C₈)Cycloalkyloxy, (C₃-C₈)Cycloalkyl-(C₁-C₆)alkoxy, (C₃-C₈)Cycloalkylthio, (C₃-C₈)Cycloalkyl-(C₁-C₆)alkylthio, (C₃-C₈)Cycloalkylsulfinyl, (C₃-C₈)Cycloalkyl-(C₁-C₆)alkylsulfinyl, (C₃-C₈)Cycloalkylsulfonyl, (C₃-C₈)Cycloalkyl-(C₁-C₆)alkylsulfonyl,
oder für NR⁴R⁵, wobei R⁴ und R⁵ unabhängig voneinander für Wasserstoff, Cyano, (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Alkyl-(C₁-C₆)thioalkyl, (C₂-C₈)Alkenyl, (C₂-C₈)Halogenalkenyl, (C₂-C₈)Cyanoalkenyl, (C₂-C₈)Alkinyl, (C₂-C₈)Halogenalkinyl, (C₂-C₈)Cyanoalkinyl, Acyl, (C₁-C₇)Alkoxycarbonyl stehen oder R⁴ und R⁵ gemeinsam mit dem N-Atom, an dem sie gebunden sind, einen gegebenenfalls gleich oder verschieden durch Halogen, Cyano, Hydroxy, Amino, (C₁-C₆)Alkyl, (C₁-C₆)Halogenalkyl, (C₁-C₆)Alkoxy, (C₁-C₆)Halogenalkoxy, oder durch gegebenenfalls gleich oder verschieden durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl oder (C₃-C₈)Cycloalkyl(C₁-C₆)alkyl, substituierten und gegebenenfalls durch Heteroatome aus der Reihe O, S oder N unterbrochenen gesättigten oder ungesättigten fünf- bis siebengliedrigen Ring bilden können, stehen,
oder für (C₃-C₈)Cycloalkyl, (C₃-C₈)Cycloalkoxy, (C₃-C₈)Cycloalkylthio, (C₃-C₈)Cycloalkenyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl oder Triazolyl, welches gegebenenfalls einfach oder mehrfach, gleich oder verschieden substituiert ist mit Halogen, Cyano, Nitro, (C₁-C₆)Alkyl, (C₁-C₆)Halogenalkyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, oder durch gegebenenfalls durch Halogen, Cyano, (C₁-C₆)Alkyl, (C₁-C₆)Alkoxy, (C₁-C₆)Halogenalkyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl(-C₁-C₆)alkyl oder (C₃-C₈)Cycloalkyl-(C₁-C₆)alkoxy, steht,
oder, X² und X³, oder X³ und X⁴, können die folgenden 5 oder 6-gliedrigen Ring ausbilden, die gegebenenfalls gleich oder verschieden durch Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, subsitutiert sind, 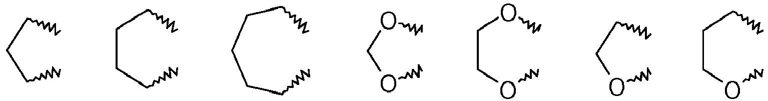
oder X² und X³, oder X³ und X⁴, können die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Halogen, Cyano, (C₁-C₆)Alkyl, (C₃-C₈)Cycloalkyl, (C₁-C₆)Halogenalkyl, (C₃-C₈)Halogencycloalkyl, (C₁-C₆)Alkoxy, (C₁-C₆)Halogenalkoxy, (C₁-C₆)Alkoxy(C₁-C₆)alkyl, (C₁-C₆)Alkylthio, (C₁-C₆)Alkylsulfonyl, Amino, (C₁-C₆)Alkylamino, Di-(C₁-C₆)-alkylamino oder (C₃-C₈)Cycloalkylamino, 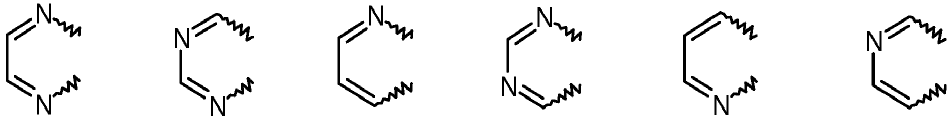 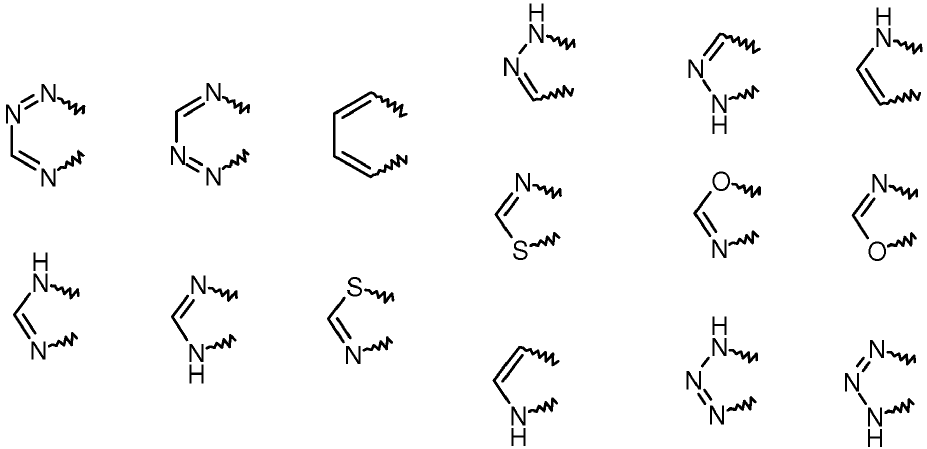 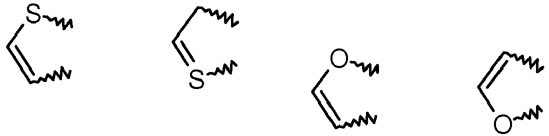
R³ für Wasserstoff, (C₂-C₆)Alkyl, Cyano, (C₁-C₆)Halogenalkyl, (C₁-C₆)Alkoxy-(C₁-C₆)alkyl, (C₁-C₆)Cyanoalkyl, (C₁-C₆)Alkylthio-(C₁-C₆)alkyl, (C₂-C₆)Alkenyl, (C₂-C₆)Halogenalkenyl, (C₂-C₆)Alkinyl, (C₂-C₆)Halogenalkinyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₆)Cycloalkylamino, (C₁-C₆)Alkylsulfonylamino, Aminosulfonyl, (C₁-C₆)Alkylaminosulfonyl oder Di-(C₁-C₆)alkyl-aminosulfonyl, oder substituiert mit gegebenenfalls gleich oder verschieden durch Halogen, Cyano, Nitro, Hydroxy, Amino, Carboxy, (C₁-C₆)Alkyl, (C₁-C₆)Halogenalkyl, (C₁-C₆)Alkoxy, (C₁-C₆)Halogenalkoxy substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy steht,
R¹ für Wasserstoff, Cyano, (C₁-C₆)Alkyl, (C₁-C₆)Halogenalkyl, (C₁-C₆)Alkoxy, (C₁-C₆)Cyanoalkyl, (C₁-C₆)Hydroxyalkyl, (C₁-C₆)Alkoxy-(C₁-C₆)alkyl, (C₁-C₆)Alkylthio-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, oder
für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylamino, Di-(C₁-C₆)alkyl-amino substituierte (C₁-C₆)Alkylcarbonyl, (C₁-C₇)Alkoxycarbonyl, Arylcarbonyl, Hetarylcarbonyl, Aryloxycarbonyl, Hetaryloxycarbonyl, (C₁-C₆)Alkylsulfinyl, (C₁-C₆)Halogenalkylsulfinyl, Arylsulfinyl, Aryl(C₁-C₆)alkylsulfinyl, Hetarylsulfinyl, Hetaryl(C₁-C₆)alkylsulfinyl, (C₁-C₆)Alkylsulfonyl, (C₁-C₆)Halogenalkylsulfonyl, Arylsulfonyl, Aryl(C₁-C₆)alkylsulfonyl, Hetarylsulfonyl, Hetaryl(C₁-C₆)alkylsulfonyl steht,
oder für einen 3- bis 6-gliedrigen gesättigten, teilgesättigten oder aromatischen Ring, der gegebenenfalls ein bis drei Heteroatome aus der Reihe O, S oder N enthalten kann, gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)-alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)-alkylaminosulfonyl, oder mit gegebenenfalls substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy steht,
oder für -(CH₂)ₘ-R⁶, -O-(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkylamino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl, oder gleich oder verschieden mit gegebenenfalls durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkyl-aminosulfonyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy, steht, wobei m für die Zahl 1, 2 oder 3 steht,
R² für Cyano, (C₁-C₆)Alkoxy, (C₁-C₆)Cyanoalkyl, (C₁-C₆)Hydroxyalkyl, (C₁-C₆)Alkoxy-(C₁-C₆)alkyl, (C₁-C₆)Alkylthio-(C₁-C₆)alkyl, (C₂-C₆)Halogenalkenyl, (C₂-C₆)Cyanoalkenyl, (C₂-C₆)Halogenalkinyl, (C₂-C₆)Cyanoalkinyl, oder
für gegebenenfalls gleich oder verschieden durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylamino, Di-(C₁-C₆)alkyl-amino substituierte (C₁-C₆)Alkylcarbonyl, (C₁-C₇)Alkoxycarbonyl, Arylcarbonyl, Hetarylcarbonyl, Aryloxycarbonyl, Hetaryloxycarbonyl, (C₁-C₆)Alkylsulfinyl, (C₁-C₆)Halogenalkylsulfinyl, Arylsulfinyl, Aryl(C₁-C₆)alkylsulfinyl, Hetarylsulfinyl, Hetaryl(C₁-C₆)alkylsulfinyl, (C₁-C₆)Alkylsulfonyl, (C₁-C₆)Halogenalkylsulfonyl, Arylsulfonyl, Aryl(C₁-C₆)alkylsulfonyl, Hetarylsulfonyl, Hetaryl(C₁-C₆)alkylsulfonyl steht,
oder für einen 3- bis 6-gliedrigen gesättigten, teilgesättigten oder aromatischen Ring, der gegebenenfalls ein bis drei Heteroatome aus der Reihe O, S oder N enthalten kann, gegebenenfalls einfach oder zweifach durch C=O, SO oder SO₂ unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)-alkylamino, (C₁-C₇)Alkylcarbonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)-alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)-alkylaminosulfonyl, oder mit gegebenenfalls substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy steht,
oder für -(CH₂)ₘ-R⁶, -O-(CH₂)ₘR⁶, -(CH₂)ₘ-O-R⁶, wobei R⁶ für einen 3- bis 6-gliedrigen gesättigten, teilgesättigten oder aromatischen Ring, der gegebenenfalls ein bis drei Heteroatome aus der Reihe O, S oder N enthalten kann, der gegebenenfalls einfach oder zweifach durch C=O unterbrochen sein kann und der gegebenenfalls einfach oder mehrfach, gleich oder verschieden substituiert ist mit Halogen, Cyano, Nitro, Hydroxy, Amino, Carboxy, (C₁-C₆)Alkyl, (C₁-C₆)Halogenalkyl, (C₂-C₆)Alkenyl, (C₂-C₆)Halogenalkenyl, (C₂-C₆)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkylamino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₇)Alkylaminocarbonyl, Di-(C₁-C₇)-alkylaminocarbonyl, Aminothiocarbonyl, (C₁-C₇)Alkylaminothiocarbonyl, Di-(C₁-C₇)alkylaminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkylaminosulfonyl, oder gleich oder verschieden mit gegebenenfalls durch Halogen, Cyano, Nitro, Hydroxy, Amino, Carboxy, (C₁-C₆)Alkyl, (C₁-C₆)Halogenalkyl, (C₂-C₈)Alkenyl, (C₂-C₈)Halogenalkenyl, (C₂-C₈)Alkinyl, (C₁-C₆)Alkoxy, (C₁-C₆)Halogenalkoxy, (C₁-C₆)Alkylthio, (C₁-C₆)Alkylsulfinyl, (C₁-C₆)Alkylsulfonyl, (C₁-C₆)Alkylsulfonyloxy, (C₁-C₆)Halogenalkylthio, (C₁-C₆)Halogenalkylsulfinyl, (C₁-C₆)Halogenalkylsulfonyl, (C₁-C₆)Alkylamino, Di-(C₁-C₆)alkyl-amino, (C₁-C₇)Alkylcabonylamino, (C₁-C₇)Alkoxycarbonyl, (C₁-C₇)Alkylcarbonyl, (C₁-C₇)Alkylcarbonyloxy, Aminocarbonyl, (C₁-C₆)Alkylaminocarbonyl, Di-(C₁-C₆)alkyl-aminocarbonyl, Aminothiocarbonyl, (C₁-C₆)Alkylaminothiocarbonyl, Di-(C₁-C₆)alkyl-aminothiocarbonyl, (C₃-C₈)Cycloalkylamino, (C₁-C₆)Alkylsulfonylamino, Aminosulfonyl, (C₁-C₆)Alkylaminosulfonyl oder Di-(C₁-C₆)alkyl-aminosulfonyl substituiertes gegebenenfalls durch Heteroatome aus der Reihe O, S oder N unterbrochenes (C₃-C₈)Cycloalkyl, (C₃-C₈)Cycloalkoxy, (C₃-C₈)Cycloalkyl-(C₁-C₆)alkyl oder (C₃-C₈)Cycloalkyl-(C₁-C₆)alkoxy, steht, wobei m für die Zahl 1, 2 oder 3 steht, oder
R¹ und R² zusammen mit den Atomen, an das sie gebunden sind, einen gesättigten oder ungesättigten und gegebenenfalls einfach oder mehrfach durch (C₁-C₄)Alkyl, (C₁-C₄)Alkoxy, Halogen, (C₁-C₄)Halogenalkyl substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe stehen, oder
R¹ und R³ zusammen mit den Atomen, an die sie gebunden sind, einen gesättigten oder ungesättigten und gegebenenfalls einfach oder mehrfach gleich oder verschieden durch (C₁-C₄)Alkyl, (C₁-C₄)Alkoxy, Halogen, (C₁-C₄)Halogenalkyl, Cyano, (C₃-C₆)Cycloalkyl, (C₃-C₆)Halogencycloalkyl, (C₃-C₆)Cyanocycloalkyl, (C₁-C₂)Alkyl(C₃-C₆)Cycloalkyl (C₂-C₄)Alkandiyl, (C₂-C₄)Alkendiyl, Butandienyl (wobei Alkandiyl, Alkendiyl und Butandienyl gegebenenfalls durch (C₁-C₄)Alkyl, (C₁-C₄)Alkoxy, Halogen, (C₁-C₄)Halogenalkyl substituiert sein können und/oder gegebenenfalls durch mindestens ein Sauerstoff- oder/und Stickstoffatom unterbrochen sein können) substituierten 4- bis 8-gliedrigen Ring bilden können, der gegebenenfalls durch ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine Carbonylgruppe enthalten kann.

2. Verbindungen der allgemeinen Formel (I) gemäß Anspruch 1, wobei
n für die Zahl 0 oder 1 steht,
X¹, X², X³, X⁴ unabhängig von einander für Wasserstoff, Fluor, Chlor, Brom, Jod, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, Aminothiocarbonyl,
oder für gegebenenfalls einfach oder zweifach gleich oder verschieden durch Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, oder durch gegebenenfalls durch ein Heteroatom aus der Reihe O, S oder N unterbrochenes, gegebenenfalls gesättigtes oder ungesättigtes (C₃-C₆)Cycloalkyl substituiertes Phenyl-(Ci-C₄)alkyl, Phenoxy, Hetaryl-(C₁-C₄)alkyl, Hetaryloxy, gegebenenfalls gesättigtes oder ungesättigtes (C₃-C₆)Cycloalkyl-(C₁-C₄)alkyl, (C₃-C₆)Cycloalkyloxy, (C₃-C₆)Cycloalkyl-(C₁-C₄)alkoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl, der gegebenenfalls einfach oder zweifach, gleich oder verschieden substituiert ist mit Fluor, Chlor, Brom, Cyano, Nitro, (C₁-C₄)Alkyl, (C₁-C₄)Halogenalkyl, (C₁-C₄)Alkoxy, (C₁-C₄)Halogenalkoxy, (C₃-C₆)Cycloalkyl steht,
oder, X² und X³, oder X³ und X⁴, können die folgenden 5 oder 6-gliedrigen Ring ausbilden, die gegebenenfalls einfach bis vierfach durch Fluor oder (C₁-C₄)Alkyl subsitutiert sind, 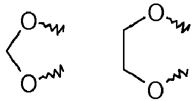
oder X² und X³, oder X³ und X⁴, können die folgenden anellierten Ringe ausbilden, die gegebenenfalls einfach oder zwei, gleich oder verschieden substituiert sind, wobei die Substituenten unabhängig voneinander ausgewählt sein können aus Wasserstoff, Fluor, Chlor, Brom, Cyano, (C₁-C₄)Alkyl, (C₃-C₆)Cycloalkyl, (C₁-C₄)Halogenalkyl, (C₁-C₄)Alkoxy, (C₁-C₄)Halogenalkoxy, 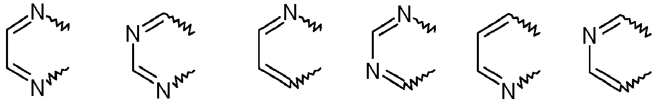 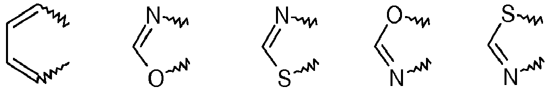
R³ für Wasserstoff, (C₂-C₄)Alkyl, Cyano, (C₁-C₄)Halogenalkyl, (C₁-C₄)Alkoxy-(C₁-C₄)alkyl, (C₁-C₄)Cyanoalkyl,
oder für einen 3- bis 6-gliedrigen gesättigten, teilgesättigten oder aromatischen Ring, der gegebenenfalls ein bis zwei Heteroatome aus der Reihe O, S oder N enthalten kann und der gegebenenfalls einfach oder dreifach, gleich oder verschieden substituiert ist mit Fluor, Chlor, Brom, Jod, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy, oder substituiert mit (C₃-C₆)Cycloalkyl steht,
R¹ für Wasserstoff, Cyano, (C₁-C₄)Alkyl, (C₁-C₄)Halogenalkyl, (C₁-C₄)Alkoxy, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Alkinyl, oder
für gegebenenfalls einfach bis dreifach unabhängig voneinander durch Fluor, Chlor, Brom, Cyano, Nitro, (C₁-C₄)Alkyl, (C₁-C₄)Halogenalkyl, (C₂-C₄)Alkenyl, (C₂-C₄)Halogenalkenyl, (C₂-C₄)Alkinyl, (C₁-C₄)Alkoxy, (C₁-C₄)Halogenalkoxy, (C₁-C₄)Alkylthio, (C₁-C₄)Alkylsulfinyl, (C₁-C₄)Alkylsulfonyl, (C₁-C₄)Alkylamino, Di-(C₁-C₄)alkyl-amino substituiertes (C₁-C₄)Alkylcarbonyl, (C₁-C₅)Alkoxycarbonyl, Arylcarbonyl, Thiophenylcarbonyl, Pyridylcarbonyl, Pyrimidylcarbonyl, Thiazolylcarbonyl, Pyrazolylcarbonyl, (C₁-C₄)Alkylsulfinyl, (C₁-C₄)Halogenalkylsulfinyl, Arylsulfinyl, Aryl(C₁-C₄)alkylsulfinyl, Hetarylsulfinyl, Hetaryl(C₁-C₄)alkylsulfinyl, (C₁-C₄)Alkylsulfonyl, (C₁-C₄)Halogenalkylsulfonyl, Arylsulfonyl, Aryl(C₁-C₄)alkylsulfonyl, Hetarylsulfonyl, Hetaryl(C₁-C₄)alkylsulfonyl steht,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl steht,
oder für -(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl, steht, wobei m für die Zahl 1 oder 2 steht,
R² für Cyano, (C₁-C₄)Alkoxy, (C₁-C₄)Cyanoalkyl, (C₁-C₄)Hydroxyalkyl, (C₁-C₄)Alkoxy-(C₁-C₄)alkyl, oder
für gegebenenfalls einfach bis dreifach unabhängig voneinander durch Fluor, Chlor, Brom, Cyano, Nitro, (C₁-C₄)Alkyl, (C₁-C₄)Halogenalkyl, (C₂-C₄)Alkenyl, (C₂-C₄)Halogenalkenyl, (C₂-C₄)Alkinyl, (C₁-C₄)Alkoxy, (C₁-C₄)Halogenalkoxy, (C₁-C₄)Alkylthio, (C₁-C₄)Alkylsulfinyl, (C₁-C₄)Alkylsulfonyl, (C₁-C₄)Alkylamino, Di-(C₁-C₄)alkyl-amino substituiertes (C₁-C₄)Alkylcarbonyl, (C₁-C₅)Alkoxycarbonyl, Arylcarbonyl, Thiophenylcarbonyl, Pyridylcarbonyl, Pyrimidylcarbonyl, Thiazolylcarbonyl, Pyrazolylcarbonyl, (C₁-C₄)Alkylsulfinyl, (C₁-C₄)Halogenalkylsulfinyl, Arylsulfinyl, Aryl(C₁-C₄)alkylsulfinyl, Hetarylsulfinyl, Hetaryl(C₁-C₄)alkylsulfinyl, (C₁-C₄)Alkylsulfonyl, (C₁-C₄)Halogenalkylsulfonyl, Arylsulfonyl, Aryl(C₁-C₄)alkylsulfonyl, Hetarylsulfonyl, Hetaryl(C₁-C₄)alkylsulfonyl steht,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl steht,
oder für -(CH₂)ₘ-R⁶, -(CH₂)ₘ-O-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach oder dreifach, gleich oder verschieden substituiert ist mit Fluor, Chlor, Brom, Cyano, Nitro, (C₁-C₄)Alkyl, (C₁-C₄)Halogenalkyl, (C₂-C₄)Alkenyl, (C₂-C₄)Alkinyl, (C₁-C₄)Alkoxy, (C₁-C₄)Halogenalkoxy oder (C₃-C₆)Cycloalkyl, steht, wobei m für die Zahl 1 oder 2 steht, oder
R¹ und R² zusammen mit dem N-Atom, an das sie gebunden sind, einen gesättigten oder ungesättigten und gegebenenfalls einfach oder vierfach durch Fluor, Chlor, (C₁-C₄)Alkyl, (C₁-C₄)Alkoxy, (C₁-C₄)Halogenalkyl substituierten 3- bis 6-gliedrigen Ring bilden können, der gegebenenfalls durch ein weiteres Heteroatom aus der Reihe Schwefel, Sauerstoff und Stickstoff enthalten kann und/oder mindestens eine Carbonylgruppe stehen, oder
R¹ und R³ zusammen mit den Atomen, an die sie gebunden sind, einen gesättigten oder ungesättigten und gegebenenfalls einfach oder mehrfach durch Methyl, Ethyl, Methoxy, Ethoxy, Fluor, Chlor, Trifluoromethyl, Cyclopropyl, Cyano, Chlorcyclopropyl, Fluorcyclopropyl, Cyanocyclopropyl, Methylcyclopropyl, (C₂-C₄)Alkandiyl, (C₂-C₄)Alkendiyl, Butandienyl (wobei Butandienyl gegebenenfalls einfach oder zweifach durch Methyl, Fluor, Chlor, Brom, Methoxy oder Trifluoromethyl substituiert sein können und/oder gegebenenfalls durch mindestens ein Sauerstoff- oder/und Stickstoffatom unterbrochen sein können) substituierten 5- bis 6-gliedrigen Ring bilden können, der gegebenenfalls ein weiteres Schwefel- oder Sauerstoff- oder Stickstoffatom und/oder eine Carbonylgruppe enthalten kann.

3. Verbindungen der allgemeinen Formel (I) gemäß einem der Ansprüche 1 bis 2, wobei
n für die Zahl 0 oder 1 steht,
X¹ und X³ für Wasserstoff stehen,
X² und X⁴ unabhängig voneinander für Wasserstoff, Fluor, Chlor, Brom, Methyl, Ethyl, Trifluoromethyl, Difluoromethyl, Difluoromethoxy, Trifluoromethoxy, OCH₂CF₃,
insbesonders stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl,
R³ für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl,
oder für Aryl steht, insbesondere für Phenyl steht,
R¹ und R² zusammen mit dem N-Atom, an das sie gebunden sind, einen gesättigten oder ungesättigten 5- bis 6-gliedrigen Ring bilden können, der gegebenenfalls ein Sauerstoffatom enthalten kann, insbesondere werden folgende Ringe explizit genannt, 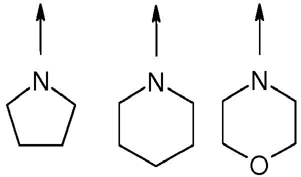
in denen das N-Atom, an das R¹ und R² gebunden sind, den Cyclus darstellen und der Pfeil zum Rest des Moleküls zeigt,

4. Verbindungen der allgemeinen Formel (1) gemäß einem der Ansprüche 1 bis 2, wobei
n für die Zahl 0 oder 1 steht,
X¹ und X³ für Wasserstoff stehen,
X² und X⁴ unabhängig voneinander für Wasserstoff, Fluor, Chlor, Brom, Methyl, Ethyl, Trifluoromethyl, Difluoromethyl, Vinyl, Ethinyl,Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl, Methoxy/Methyl, Methyl/Methoxy, Methyl/H, Ethyl/H, Chlor/H, Brom/H, Fluor/H, Methoxy/H, H/Chlor, H/Fluor, H/Brom, H/Methyl, H/Methoxy, H/Ethyl
R³ für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der ein oder zwei weitere Heteroatome aus der Reihe Schwefel oder Stickstoff enthält,
oder zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 4-gliedrigen Ring bilden können, der gegebenenfalls ein weiteres Heteroatom aus der Reihe Sauerstoff, Schwefel oder Stickstoff enthält
insbesondere werden folgende Ringe explizit genannt, 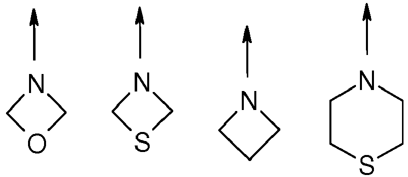 in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

5. Verbindungen der allgemeinen Formel (I) gemäß einem der Ansprüche 1 bis 2,
wobei
n für die Zahl 0 oder 1 steht,
X¹ und X³ für Wasserstoff stehen,
X² und X⁴ unabhängig voneinander für Wasserstoff, Fluor, Chlor, Brom, Methyl, Ethyl, Trifluoromethyl, Difluoromethyl, Vinyl, Ethinyl,Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl, Methoxy/Methyl, Methyl/Methoxy, Methyl/H, Ethyl/H, Chlor/H, Brom/H, Fluor/H, Methoxy/H, H/Chlor, H/Fluor, H/Brom, H/Methyl, H/Methoxy, H/Ethyl
R³ für Methoxymethyl, Methoxyethyl, Cyanomethyl, Allyl, Butenyl, Oxolanyl, Pentandienyl, Butadienyl,
oder für Cyclopropyl, Cyclobutyl, Cyclopentyl, Cyclohexyl, Pyridyl, Pyrimidyl, Pyrazinyl, Pyridazinyl, Pyrrolyl, Furanyl, Thiazolyl, Pyrazolyl oder Thienyl steht, die gegebenenfalls einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert sein können,
oder für Aryl, insbesondere für Phenyl steht, welches einfach oder mehrfach, gleich oder verschieden mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl substituiert ist,
R¹ für Wasserstoff, Cyano, Methyl, Ethyl, Propyl, Butyl, *sec*-Butyl, *iso*-Propyl, *tert*-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl steht,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl, steht, wobei m für die Zahl 1 steht, insbesondere werden folgende R⁶ explizit genannt: Cyclopropyl, Phenyl und Pyridyl, die gegebenenfalls mit den unter ganz besonderen bevorzugten genannten Subtituenten substituiert sein können,
R² für Cyano, Methoxy, Ethoxy, Cyanomethyl, Methoxymethyl, Methoxyethyl, Methylcarbonyl, Ethylcarbonyl, Propylcarbonyl, Isopropylcarbonyl, Methoxycarbonyl, Ethoxycarbonyl,
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl steht,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl, steht, wobei m für die Zahl 1 steht, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder
R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 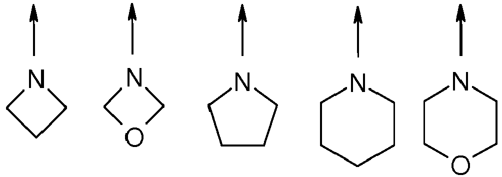
in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können.

6. Verbindungen der allgemeinen Formel (I) gemäß einem der Ansprüche 1 bis 2, wobei
n für die Zahl 0 oder 1 steht,
X¹ und X³ für Wasserstoff stehen,
X² und X⁴ für folgende Kombinationen X²/X⁴ stehen: Vinyl/H, H/Vinyl, Ethinyl/H, H/Ethinyl, Methoxy/H, H/Methoxy, Ethoxy/H, H/Ethoxy, Aminothiocarbonyl/H, H/Aminothiocarbonyl, Vinyl/Methyl, Methyl/Vinyl, Ethinyl/Methyl, Methyl/Ethinyl, Methoxy/Methyl, Methyl/Methoxy, Ethoxy/Methyl, Methyl/Ethoxy, Aminothiocarbonyl/Methyl, Methyl/Aminothiocarbonyl, Vinyl/F, F/Vinyl, Ethinyl/F, F/Ethinyl, Methoxy/F, F/Methoxy, Ethoxy/F, F/Ethoxy, Aminothiocarbonyl/F, F/Aminothiocarbonyl, Vinyl/Cl, Cl/Vinyl, Ethinyl/Cl, Cl/Ethinyl, Methoxy/Cl, Cl/Methoxy, Ethoxy/Cl, Cl/Ethoxy, Aminothiocarbonyl/Cl, Cl/Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
R³ für Wasserstoff, Ethyl, Propyl, Cyano, Trifluoromethyl, Difluoromethyl, Dichlormethyl, Trichlormethyl, Difluorchlormethyl, Dichlorfluormethyl, (2,2,2)Trifluoroethyl, 2-Chloro-(2,2)difluoroethyl, (2,2)Dichloro-2-fluoroethyl, (2,2,2)Trichloroethyl, Pentafluoroethyl, Methoxymethyl, Methoxyethyl, Cyanomethyl, Allyl, Butenyl,
oder für einen 3- bis 6-gliedrigen gesättigten, teilgesättigten oder aromatischen Ring, der gegebenenfalls ein bis drei Heteroatome aus der Reihe O, S oder N enthalten kann und der gegebenenfalls einfach oder zweifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Phenyl, Pyridyl, Pyrimidyl, Thiazolyl, Pyrazolyl und Thienyl, die gegebenenfalls mit den unter ganz besonderen bevorzugten genannten Subtituenten substituiert sein können,
R¹ für Wasserstoff, Cyano, Methyl, Ethyl, Propyl, Butyl, *sec*-Butyl, *iso*-Propyl, *tert*-Butyl, (2,2,2)Trifluoroethyl, (2,2)Difluoromethyl, Methoxy, Ethoxy, Cyanomethyl, Methoxymethyl, Methoxyethyl, Allyl, Butenyl, Propinyl, Methylcarbonyl, Ethylcarbonyl, Propylcarbonyl, Isopropylcarbonyl, Methoxycarbonyl, Ethoxycarbonyl, oder
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl steht,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl, steht, wobei m für die Zahl 1 steht, insbesondere werden folgende R⁶ explizit genannt: Cyclopropyl, Phenyl und Pyridyl, die gegebenenfalls mit den unter ganz besonderen bevorzugten genannten Subtituenten substituiert sein können,
R² für Cyano, Methoxy, Ethoxy, Cyanomethyl, Methoxymethyl, Methoxyethyl, Methylcarbonyl, Ethylcarbonyl, Propylcarbonyl, Isopropylcarbonyl, Methoxycarbonyl, Ethoxycarbonyl, oder
für gegebenenfalls einfach oder zweifach unabhängig voneinander durch Fluor, Chlor, Brom, Cyano, Nitro, Methyl, Trifluoromethyl, Allyl, Butenyl, Methoxy, Trifluoromethoxy, Methylthio, Methylsulfinyl, Methylsulfonyl, Methylamino, Dimethylamino substituiertes Arylcarbonyl, Pyridylcarbonyl, Pyrimidylcarbonyl, Thiazolylcarbonyl, Pyrazolylcarbonyl, Methylsulfinyl, Trifluoromethylsulfinyl, Methylsulfonyl, Trifluoromethylsulfonyl steht,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl, steht, wobei m für die Zahl 1 steht, insbesondere werden folgende R⁶ explizit genannt: Cyclopropyl, Phenyl und Pyridyl, die gegebenenfalls mit den unter ganz besonderen bevorzugten genannten Subtituenten substituiert sein können, oder R¹ und R² zusammen mit dem N-Atom, an das sie gebunden sind, einen gesättigten oder ungesättigten und gegebenenfalls einfach, zweifach, dreifach oder vierfach durch Methyl, Ethyl, Methoxy, Ethoxy, Fluor, Chlor, Brom, Trifluoromethyl, Difluoromethyl substituierten 3- bis 6-gliedrigen Ring bilden können, der gegebenenfalls ein oder zwei weitere Heteroatome aus der Reihe Schwefel, Sauerstoff und Stickstoff enthalten kann (wobei Sauerstoffatome nicht unmittelbar benachbart sein dürfen) und/oder mindestens eine CH2-Gruppe durch eine Carbonylgruppe ersetzt ist, insbesondere werden folgende Ringe explizit genannt, 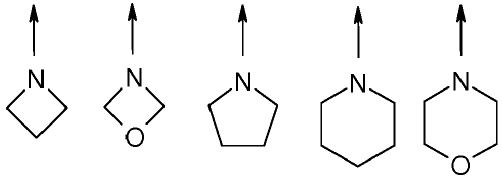
in denen das N-Atom, an das R¹ und R² gebunden sind, den Cyclus darstellen und der Pfeil zum Rest des Moleküls zeigt, welche gegebenenfalls einfach oder zweifach durch Methyl, Ethyl, Methoxy, Ethoxy, Fluor, Chlor, Brom, Trifluoromethyl, Difluoromethyl substituiert sein können, oder
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, Difluoromethyl, Cyano, Cyclopropyl, Chlorcyclopropyl, Fluorcyclopropyl, Cyanocyclopropyl, Methylcyclopropyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5-bis 6-gliedrigen Ring bilden können, der gegebenenfalls ein weiteres Schwefel- oder Sauerstoff- oder Stickstoffatom und/oder eine Carbonylgruppe enthalten kann, insbesondere stehen R¹ und R³ zusammen mit den Atomen, an die sie gebunden sind für folgende Gruppe, 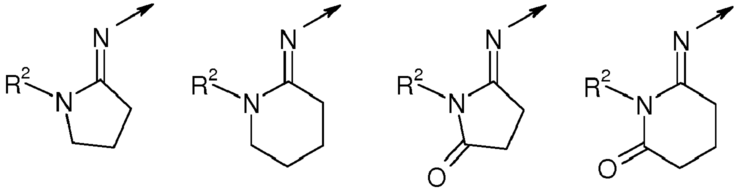 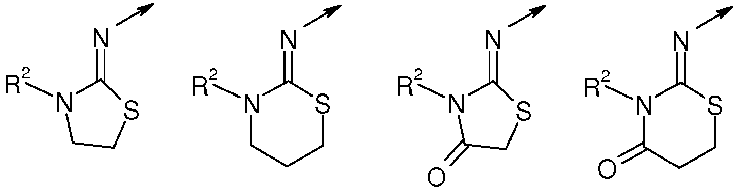 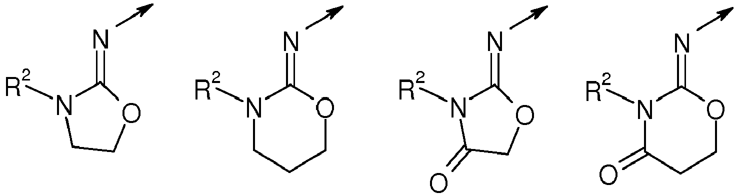 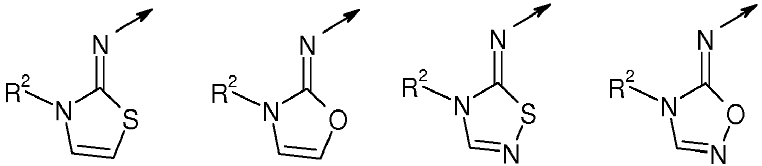 die jeweils gegebenenfalls einfach oder zweifach durch Methyl, Ethyl, Methoxy, Ethoxy, Fluor, Chlor, Trifluoromethyl, Difluoromethyl, Cyclopropyl, Chlorcyclopropyl, Fluorcyclopropyl, Cyanocyclopropyl, Methylcyclopropyl substituiert sein können,
wobei der Pfeil zum Rest des Moleküls zeigt.

7. Verbindungen der allgemeinen Formel (I) gemäß einem der Ansprüche 1 bis 2, wobei
n für die Zahl 0 oder 1 steht,
X¹ und X³ für Wasserstoff stehen,
X² und X⁴ unabhängig voneinander für Wasserstoff, Fluor, Chlor, Brom, Methyl, Ethyl, Trifluoromethyl, Difluoromethyl, Vinyl, Ethinyl, Methoxy, Ethoxy, Difluoromethoxy, Trifluoromethoxy, OCH₂CF₃, Aminothiocarbonyl,
oder gegebenenfalls einfach oder zweifach gleich oder verschieden durch Fluor, Chlor, Brom, Cyano, Nitro, Methyl, Ethyl, Trifluoromethyl, Difluoromethyl, Methoxy, Trifloromethoxy, Difluoromethoxy, oder Cyclopropyl substituiertes Phenylmethyl, Phenoxy, Pyridylmethyl, Pyrimidylmethyl, Thiazolylmethyl, Pyrazolylmethyl, Pyridyloxy, Pyrimidyloxy, Thiazolyloxy, Pyrazolyloxy, Cyclopropylmethyl, Cyclopropylmethoxy,
oder für einen (C₃-C₆)Cycloalkyl, Phenyl, Pyridyl, Pyrimidyl, Pyridazinyl, Pyrazinyl, Triazinyl, Thiazolyl, Thiadiazolyl, Oxazolyl, Oxadiazolyl, Pyrazolyl, Triazolyl oder Tetrazolyl der gegebenenfalls einfach oder zweifach, gleich oder verschieden substituiert ist mit Fluor, Chlor, Brom, Cyano, Nitro, Methyl, Ethyl, Trifluoromethyl, Difluoromethyl, Methoxy, Trifluoromethoxy oder mit Cyclopropyl steht,
insbesonders stehen X² und X⁴ für folgende Kombinationen X²/X⁴: F/F, Cl/Cl, F/Cl, Cl/F, Br/Br, Br/Cl, Cl/Br, Br/F, F/Br, Methyl/Methyl, Methyl/F, F/Methyl, Methyl/Cl, Cl/Methyl, Methyl/Br, Br/Methyl, Ethyl/Ethyl, Ethyl/F, F/Ethyl, Ethyl/Cl, Cl/Ethyl, Ethyl/Br, Br/Ethyl, Methoxy/Methyl, Methyl/Methoxy, Methyl/H, Ethyl/H, Chlor/H, Brom/H, Fluor/H, Methoxy/H, H/Chlor, H/Fluor, H/Brom, H/Methyl, H/Methoxy, H/Ethyl
R¹ und R³ zusammen mit den Atomen, an die sie gebunden sind, einen gesättigten oder ungesättigten und/oder gegebenenfalls einfach oder zweifach durch Methyl, Ethyl, Methoxy, Ethoxy, Fluor, Chlor, Trifluoromethyl, Difluoromethyl, Cyano, Cyclopropyl, Chlorcyclopropyl, Fluorcyclopropyl, Cyanocyclopropyl, Methylcyclopropyl, (C₃-C₄)Alkandiyl, (C₃-C₄)Alkendiyl, Butandienyl (wobei Butandienyl gegebenenfalls einfach oder zweifach durch Methyl, Fluor, Chlor, Brom, Methoxy oder Trifluoromethyl substituiert und/oder gegebenenfalls durch mindestens ein Sauerstoff- oder/und Stickstoffatom unterbrochen sein kann) substituierten 5- bis 6-gliedrigen Ring bilden können, der gegebenenfalls ein weiteres Schwefel- oder Sauerstoff- oder Stickstoffatom und/oder eine Carbonylgruppe enthalten kann, insbesondere stehen R¹ und R³ zusammen mit den Atomen, an die sie gebunden sind für folgende Gruppe, 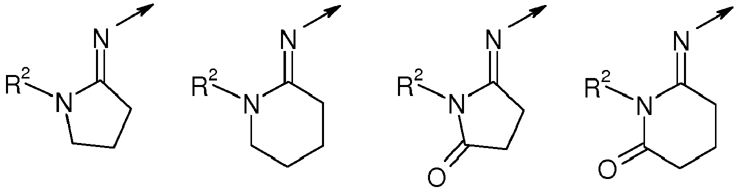 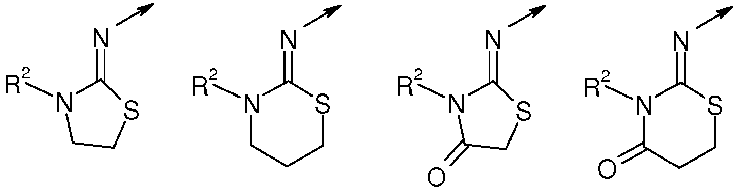 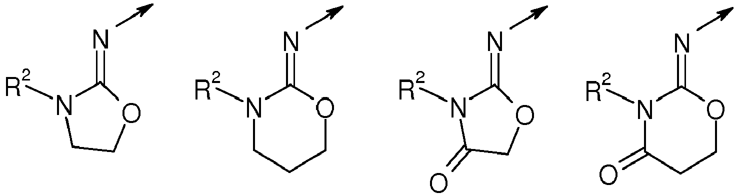 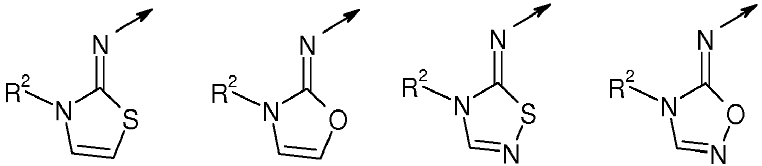 die jeweils gegebenenfalls einfach oder zweifach durch Methyl, Ethyl, Methoxy, Ethoxy, Fluor, Chlor, Trifluoromethyl, Difluoromethyl, Cyclopropyl, Chlorcyclopropyl, Fluorcyclopropyl, Cyanocyclopropyl, Methylcyclopropyl substituiert sein können,
wobei der Pfeil zum Rest des Moleküls zeigt,
R² für Cyano, Methylsulfinyl, Trifluoromethylsulfinyl, Methylsulfonyl oder Trifluoromethylsulfonyl steht,
oder für einen 3- bis 6-gliedrigen gesättigten oder aromatischen Ring, der gegebenenfalls ein bis zwei Heteroatome aus der Reihe O, S oder N enthalten kann, der gegebenenfalls einfach, zweifach oder dreifach durch C=O unterbrochen sein kann und der gegebenenfalls einfach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, insbesondere werden folgende Ringe explizit genannt: Cyclopropyl, Cyclobutyl, Cyclopentyl, Cyclohexyl, Oxetan, Phenyl, Thienyl und Pyridyl, die gegebenenfalls mit den unter ganz besonderen bevorzugten genannten Subtituenten substituiert sein können,
oder für -(CH₂)ₘ-R⁶, wobei R⁶ für einen 3- bis 6-gliedrigen gesättigten, teilgesättigten oder aromatischen Ring, der gegebenenfalls ein bis zwei Heteroatome aus der Reihe O, S oder N enthalten kann, gegebenenfalls einfach oder zweifach durch C=O unterbrochen sein kann und der gegebenenfalls einfach, zweifach oder dreifach, gleich oder verschieden substituiert ist mit Fluor, Chlor, Brom, Cyano, Nitro, Methyl, Ethyl, Trifluoromethyl, Vinyl, Ethinyl, Methoxy, Ethoxy, Difluoromethoxy, Trifluoromethoxy, Trifluoroethoxy oder Cyclopropyl steht, wobei m für die Zahl 1 steht, insbesondere werden folgende R⁶ explizit genannt: Cyclopropyl, Cyclobutyl, Cyclopentyl, Cyclohexyl, Phenyl und Pyridyl, die gegebenenfalls mit den unter ganz besonderen bevorzugten genannten Subtituenten substituiert sein können.

8. Verfahren zur Herstellung von Verbindungen der Formel (I) gemäß einem der Ansprüche 1 bis 7, **dadurch gekennzeichnet, dass** man
(A) Aniline der Formel (VII) 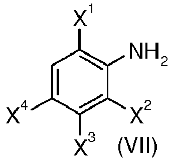 in Gegenwart von Säuren, Säureanhydriden oder Säurechloriden der Formel (VIII) 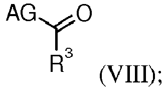 wobei AG für eine Abgangsgruppe steht,
umsetzt zu Aniliden der allgemeinen Formel (VI) 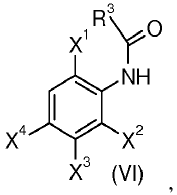
(B) Anilide der Formel (VI) durch Chlorsulfonierung mit Chlorsulfonsäure in Sulfonylchloride der allgemeinen Formel (V) umsetzt, 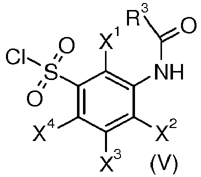
(C) Sulfonylchloride der Formel (V) durch Reduktion mit Eisen in Salzsäure oder Iodid in Disulfide der allgemeinen Formel (IV) 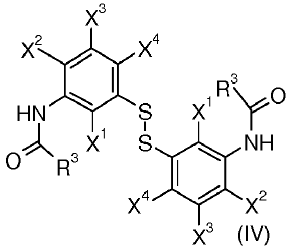 umsetzt,
(D) Disulfide der Formel (IV) mit Trifluorethylelektrophilen der Formel (IX), 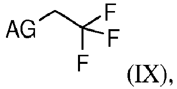 wobei AG für eine Abgangsgruppe wie zum Beispiel Chlor, Brom, Tosylat, Mesylat oder Triflat steht, in Sulfide der allgemeinen Formel (III) 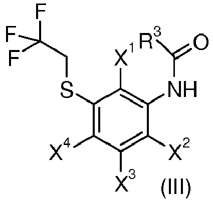 umsetzt,
(E) die Verbindungen der Formel (III) mit Diphenylchlorphosphat oder Phosphorpentachlorid zu Imidoylchloriden der allgemeinen Formel (II) umsetzt, 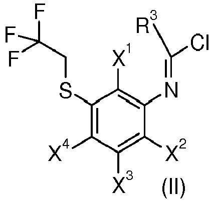
(F) die Verbindungen der allgemeinen Formel (II) mit Aminen zu Amidinen der allgemeinen Formel (Ia) 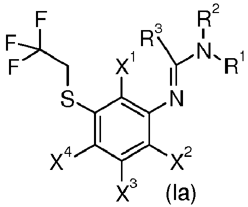 umsetzt,
(G) die Verbindungen der Formel (IA) mit Oxidationsmitteln in Sulfoxide der allgemeinen Formel (Ib) 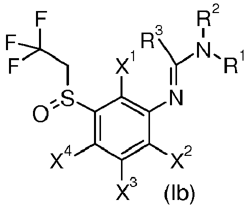 umsetzt,
wobei X¹, X², X³, X⁴, R¹, R² und R³ die oben angegebenen Bedeutungen haben.

9. Verfahren zur Herstellung von Verbindungen der Formel (I) gemäß einem der Ansprüche 1 bis 7, **dadurch gekennzeichnet, dass** man
(A) Aniline der Formel (X) 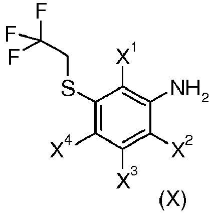 mit Isocyanaten oder Isothiocyanaten gegebenenfalls in Anwesenheit einer Base und gegebenenfalls in Anwesenheit eines organischen Lösungsmittels zu Verbindungen der Formel (XXXVIII) umsetzt 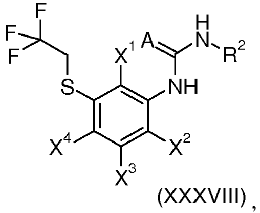
(B) Verbindungen der Formel (XXXVIII) gegebenenfalls durch Cycloacylierung zu Verbindungen der allgemeinen Formel (Ia-g) umsetzt 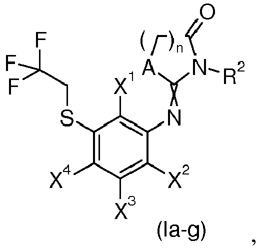
(C) diese Verbindungen der Formel (Ia-g) oxidiert zu Verbindungen der Formel (Ib-g), 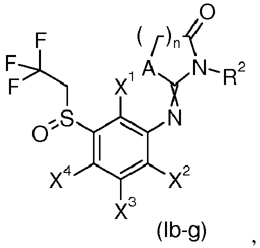 wobei X¹, X², X³, X⁴ und R² die oben angegebenen Bedeutungen haben und A für O oder S steht.

10. Verbindungen der Formel (XXXVIII) 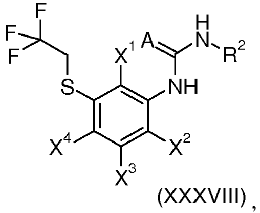 wobei X¹, X², X³, X⁴ und R² die oben angegebenen Bedeutungen haben.

11. Verbindungen der Formel X 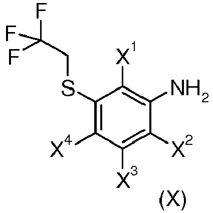 wobei X¹, X², X³, X⁴ die oben angegebenen Bedeutungen haben.

12. Wirkstoffzusammensetzung enthaltend mindestens eine Verbindung der allgemeinen Formel (I) gemäß einem der Ansprüche 1 bis 7 und mindestens einen weiteren insektiziden, akariziden oder nematiziden Wirkstoff ausgewählt aus der Gruppe bestehend aus
(1) Acetylcholinesterase (AChE) Inhibitoren, wie beispielsweise
Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
Cyclodien-organochlorine, z.B. Chlordane und Endosulfan; oder
Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
DDT; oder Methoxychlor.
(4) Nikotinerge Acetylcholin-Rezeptor (nAChR) Agonisten, wie beispielsweise
Neonikotinoide, z.B. Acetamiprid, Clothianidin, Dinotefuran, Imidacloprid, Nitenpyram, Thiacloprid und Thiamethoxam; oder
Nikotin.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
Avermectine/Milbemycine, z.B. Abamectin, Emamectin-benzoat, Lepimectin und Milbemectin.
(7) Juvenilhormon-Imitatoren, wie beispielsweise
Juvenilhormon-Analoge, z.B. Hydroprene, Kinoprene und Methoprene; oder
Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, CrylFa, Cry2Ab, mCry3A, Cry3Ab, Cry3Bb, Cry34/35Abl.
(12) Inhibitoren der oxidativen Phosphorylierung, ATP-Disruptoren, wie beispielsweise Diafenthiuron; oder
Organozinnverbindungen, z.B. Azocyclotin, Cyhexatin und Fenbutatin-oxid; oder
Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
METI-Akarizide, z.B. Fenazaquin, Fenpyroximate, Pyrimidifen, Pyridaben, Tebufenpyrad und Tolfenpyrad; oder
Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
Tetron- und Tetramsäurederivate, z.B. Spirodiclofen, Spiromesifen und Spirotetramat.
(24) Komplex-IV-Elektronentransportinhibitoren, wie beispielsweise
Phosphine, z.B. Aluminiumphosphid, Calciumphosphid, Phosphin und Zinkphosphid; oder
Cyanid.
(25) Komplex-II-Elektronentransportinhibitoren, wie beispielsweise Cyenopyrafen.
(28) Ryanodinrezeptor-Effektoren, wie beispielsweise
Diamide, z.B. Chlorantraniliprole und Flubendiamide;
weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid 4-{[(6-Brompyrid-3-yl)methyl](2-fluorethyl)amino}furan-2(5H)-on (bekannt aus WO2007/115644), 4-{ [(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl] (cyclopropyl)amino } -1,3 -oxazol-2(5H)-on, 4- { [(6-Chlorpyridin-3 -yl)methyl] (2,2-difluorethyl)amino} -1,3 -oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925) und Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233),
und/oder mindestens einen weiteren fungiziden Wirkstoff ausgewählt aus der Gruppe bestehend aus
(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazol-p (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).
(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-empimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{ [4-(trifluormethyl)pyridin-2-yl]oxy}phenyl)ethyl]quinazolin-4-amin (1210070-84-0) (bekannt aus WO2010025451), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2) (bekannt aus WO 2004/058723), (3.9) Famoxadon (131807-57-3) (bekannt aus WO 2004/058723), (3.10) Fenamidon (161326-34-7) (bekannt aus WO 2004/058723), (3.11) Fenoxystrobin (918162-02-4), (3.12) Fluoxastrobin (361377-29-9) (bekannt aus WO 2004/058723), (3.13) Kresoxim-Methyl (143390-89-0) (bekannt aus WO 2004/058723), (3.14) Metominostrobin (133408-50-1) (bekannt aus WO 2004/058723), (3.15) Orysastrobin (189892-69-1) (bekannt aus WO 2004/058723), (3.16) Picoxystrobin (117428-22-5) (bekannt aus WO 2004/058723), (3.17) Pyraclostrobin (175013-18-0) (bekannt aus WO 2004/058723), (3.18) Pyrametostrobin (915410-70-7) (bekannt aus WO 2004/058723), (3.19) Pyraoxystrobin (862588-11-2) (bekannt aus WO 2004/058723), (3.20) Pyribencarb (799247-52-2) (bekannt aus WO 2004/058723), (3.21) Triclopyricarb (902760-40-1), (3.22) Trifloxystrobin (141517-21-7) (bekannt aus WO 2004/058723), (3.23) (2E)-2-(2-{[6-(3-Chlor-2-methylphenoxy)-5-fluorpyrimidin-4-yl]oxy}phenyl)-2-(methoxyimino)-N-methylethanamid (bekannt aus WO 2004/058723), (3.24) (2E)-2-(Methoxyimino)-N-methyl-2-(2-{[({(1E)-1-[3-(trifluormethyl)phenyl]ethyliden}amino)oxy]methyl}phenyl)ethanamid (bekannt aus WO 2004/058723), (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).
(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).
(5) Verbindungen mit Multisite-Aktivität, wie beispielsweise (5.1) Bordeauxmischung (8011-63-0), (5.2) Captafol (2425-06-1), (5.3) Captan (133-06-2) (bekannt aus WO 02/12172), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).
(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).
(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7) (bekannt aus WO2005070917).
(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).
(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).
(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).
(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl{3-methyl-1-[(4-methylbenzoyl)amino]butan-2-yl}carbamat (851524-22-6) (bekannt aus WO2005042474).
(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).
(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).
(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).
(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9) (bekannt aus EP-A 1 559 320), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2) (bekannt aus WO2003035617), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0) (bekannt aus WO2005070917), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4) (bekannt aus WO2009094442), (15.75) 5-Fluor-2-[(4-methylbenzyl)oxy]pyrimidin-4-amin (1174376-25-0) (bekannt aus WO2009094442), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl}-2-phenylacetamid (221201-92-9), (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1 -yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid (bekannt aus EP-A 1 559 320), (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N- [4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl{6-[({[(Z)-(1-methyl-1H-tetrazol-5-yl)(phenyl)methylen]amino}oxy)methyl]pyridin-2-yl}carbamat.

13. Agrochemische Zusammensetzungen, **dadurch gekennzeichnet, dass** sie mindestens eine Verbindung der Formel (I) gemäß den Ansprüchen 1 bis 7 oder eine Zusamensetzung gemäß Anspruch 12, sowie Streckmittel und/oder oberflächenaktive Stoffe enthalten.

14. Verfahren zur Herstellung agrochemischer Zusammensetzungen, **dadurch gekennzeichnet, dass** man Verbindungen der Formel (I) gemäß den Ansprüchen 1 bis 7 oder eine Zusammensetzung gemäß Anspruch 12 mit Streckmitteln und/oder oberflächenaktiven Stoffen vermischt.

15. Verfahren zur Bekämpfung tierischer Schädlinge, **dadurch gekennzeichnet, dass** man Verbindungen der Formel (I) gemäß den Ansprüchen 1 bis 7 oder eine Zusammensetzung gemäß Anspruch 12 auf tierische Schädlinge und/oder deren Lebensraum einwirken lässt.

16. Verwendung von Verbindungen der Formel (I) gemäß einem der Ansprüche 1 bis 7 oder einer Zusammensetzung gemäß Anspruch 12 zur Bekämpfung tierischer Schädlinge im Pflanzenschutz, im Materialschutz und/oder im veterinärmedizinischen Sektor
